# EUROPEAN PATENT APPLICATION

(11) **EP 4 703 362 A1**
(43) Date of publication of application: **04.03.2026**
(21) Application number: 24796261.6
(22) Date of filing: 26.04.2024
(51) Int. Cl.: C07D 491/048, A61K 31/496, A61P 29/00

(54) **COMPOUND CONTAINING OXO-PYRIDO-ALIPHATIC RING OR ALIPHATIC HETEROCYCLIC STRUCTURE, AND MEDICAL USE THEREOF**

(30) Priority: 28.04.2023 CN 202310479996; 27.11.2023 CN 202311608661
(71) Applicant: Chengdu Sibeibo Pharmaceutical Technology Co., Ltd, Chengdu, Sichuan 610095 (CN)
(72) Inventor: YANG, Anle, Chengdu, Sichuan 610095 (CN); ZHU, Jun, Chengdu, Sichuan 610095 (CN); ZHANG, Dewei, Chengdu, Sichuan 610095 (CN); LI, Zerui, Chengdu, Sichuan 610095 (CN); YU, Pan, Chengdu, Sichuan 610095 (CN); ZOU, Lianxin, Chengdu, Sichuan 610095 (CN); ZHOU, Libo, Chengdu, Sichuan 610095 (CN); YANG, You, Chengdu, Sichuan 610095 (CN); HE, Quanhong, Chengdu, Sichuan 610095 (CN); YANG, Hao, Chengdu, Sichuan 610095 (CN); YUE, Jingmei, Chengdu, Sichuan 610095 (CN); DU, Shuwen, Chengdu, Sichuan 610095 (CN); WANG, Zhenyu, Chengdu, Sichuan 610095 (CN); SU, Zhonghai, Chengdu, Sichuan 610095 (CN)
(74) Representative: Studio Torta S.p.A.
(86) International application number: PCT/CN2024/090110
(87) International publication number: WO 2024/222889

(57) **Abstract**

The present invention belongs to the technical field of medicine. Provided is a compound containing an oxo-pyrido-aliphatic ring or an aliphatic heterocyclic structure as represented by formula I, wherein R², R³, X¹, X², X³, X⁴, X⁵, Y¹, Y², A, o and n are as defined in the description. The compound with the new structure provided in the present invention has excellent CGRP antagonistic activity, and has the potential to treat various CGRP-mediated diseases, especially migraines or neuropathic pain.

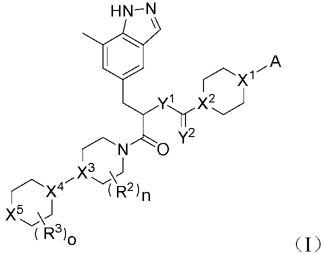

## Description

### TECHNICAL FIELD

The present invention belongs to the technical field of medicine, and provides a compound containing an oxo-pyrido-aliphatic ring or aliphatic heterocyclic ring structure, and an application thereof as a CGRP antagonist for treating related diseases.

### BACKGROUND

Migraine, as a common neurovascular disease, is mainly manifested as a recurrent and generally unilateral moderate and severe pulsatile headache, which usually lasts for 4 to 72 hours, accompanied by symptoms such as nausea, vomiting, and sensitivity to sound and light (intolerance of sound and light) [1]. Patients often go through a period of sensory disturbance before the migraine attack, this kind of migraine is called migraine with aura, and 1/7 patients with the migraine in China have aura symptoms [2]. Main aura types comprise visual disorders such as dark spots, bright spots and flashes, and symptoms such as abnormal sense of smell, dizziness, tinnitus and difficulty in language expression. At present, more than 1 billion people in the world are affected by the migraine [3]. A consultation rate of patients with the migraine in China is only 52.9%, a correct diagnosis rate of doctors is only 13.8%, and there are widespread situations such as insufficient preventive treatment and excessive use of analgesic drugs [2]. The migraine attack may last for hours or even days, and may seriously affect normal daily activities, so that there is a significant demand for an acute therapy capable of quickly relieving the migraine. In recent years, with the research progress of the migraine at home and abroad, new therapeutic targets such as a calcitonin gene-related peptide (CGRP) have emerged, thereby enriching therapeutic methods for the migraine.

The calcitonin gene-related peptide (CGRP) is a polypeptide composed of 37 amino acids, in which an N-terminal disulfide bond and an aminated C-terminal play an important role in activating a receptor [4]. The CGRP is mainly located in a c fiber and an Aδ fiber in a trigeminal ganglion and a dorsal root ganglion, and in a central nervous system [5]. There are two forms of CGRPs in human body, which are α-CGRP and β-CGRP, with a difference of only 1-3 amino acids in different species, wherein, the α-CGRP is highly expressed in a sensory neuron, while the β-CGRP mainly plays a role in an enteric nervous system [6]. The CGRP exerts its biological function by binding to a membrane receptor. A CGRP receptor is mainly composed of a calcitonin receptor-like receptor (CLR) and a receptor activity modifying protein 1 (RAMP1), and in addition, a receptor component protein (RCP) is also needed to play a role of signal transmission [7]. The CGRP mainly plays a role of vasodilatation in vivo, and studies suggest that the CGRP plays an important role in the occurrence and development of the migraine. Clinical evidences show that a CGRP content in the jugular vein of the patients with the migraine is increased during the attack, and the intravenous injection with the CGRP may cause moderate to severe migraine attacks, and the use of a CGRP antagonist can relieve the pain and related symptoms of the migraine [4].

At present, specific methods for acute-stage treatment in guidelines for migraine diagnosis and treatment comprise triptans, ergotamines, ditans and gepants [2]. Triptan drugs are 5-HT1B/1D receptor agonists, which have been widely used in the acute treatment of the migraine in the past twenty years. The tripotan drugs have high evidence and recommendation levels in the guidelines, but there is also an obvious cardiovascular risk, and there are also many cases such as a low response rate of the patients. Ditan drugs are 5-HT1F receptor agonists, which cause no adverse reaction of vasoconstriction in the triptan drugs, but the ditan drugs have an inhibitory effect on the central nervous system [8]. Gepant drugs - antagonists developed for the CGRP receptor are a new treatment method for the migraine, without the cardiovascular risk and the central inhibition effect [7], which provide a new selection for patients who do not respond to the triptan drugs and the ditan drugs. At present, marketed gepant drugs developed for the CGRP receptor comprise ubrogepant and atogepant from AbbVie, and rimegepant from Pfizer. These three drugs are all oral tablets, and the patients with the migraine often have symptoms such as nausea and vomiting, so that the feasibility of oral administration is low at this time. Moreover, the migraine attack lasts for a long time, which severely affects the daily life. A novel drug zavegepant (vazegepant) from Pfizer was marketed in the United States in March 2023 in a marketed dosage form of nasal spray for approved indication of acute treatment of adult migraine with or without aura. However, the drug cannot be used for prevention of the migraine, and causes the most common adverse reactions (an effect of at least 2% ZAVZPRET is greater than that of placebo in treatment of the patients) such as taste disorder, nausea, nasal discomfort and vomiting.

BMS disclosed and protected a compound structure of Zavegepant in CN102834388B applied in 2011. Before CN102834388B, BMS also applied for CN100558728C, CN100558428C, CN1914193A, CN1972929A and other related patents in 2003-2005, which also disclosed a series of heterocyclic CGRP antagonists related to the Zavegepant structure for treatment of the migraine.

Boehringer Ingelheim also applied for CN101146799A and WO2005084672A in 2005-2006, which also disclosed a CGRP antagonist respectively, and the antagonist was expected to be applied in treatment of pain and other related diseases.

Previously marketed drugs related to the effect of the CGRP above or compounds disclosed in literatures are all different from those in the present invention. Considering a current treatment status of migraine and neuropathic pain, in order to meet and enrich clinical needs, there is still a demand for a compound with a novel structure for specific treatment of migraine and neuropathic headache.

### SUMMARY

In order to solve the above problems in the prior art, and meet and enrich clinical needs, the present invention is intended to provide a novel compound containing an oxo-pyrido-aliphatic ring or aliphatic heterocyclic ring structure as a CGRP antagonist, which is different from compounds disclosed in previously marketed drugs or literatures in structure, so as to be used for preventing and treating CGRP-mediated diseases, especially migraine and neuropathic headache.

Firstly, the present invention provides a compound as represented by formula I, or an isomer, pharmaceutically acceptable salt or solvate thereof: 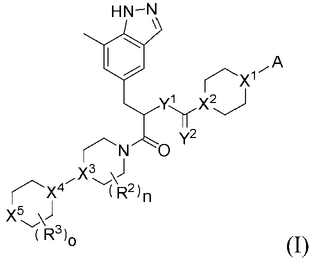 wherein,
X¹ and X² are respectively independently CH or N, and X² is preferably N;
X³ and X⁴ are respectively independently C, CH or N; and as a selection, X³ and X⁴ are optionally not N at the same time;
X⁵ is C, CH, CH₂, NH or N;
Y¹ is CH₂, O or NH, and Y¹ is preferably O or NH;
Y² is O or S, and Y² is preferably O;
R² is independently -H, -(C₁-C₆)alkyl or -(C₃-C₈)cycloalkyl when occurring each time, and n is 0, 1, 2, 3, 4, 5 or 6,
or, two R² form a 3-6 membered aliphatic ring or aliphatic heterocyclic ring together with atoms to which R² attach, the aliphatic ring or aliphatic heterocyclic ring optionally forms a spiro structure, a fused ring structure or a bridged ring structure with the ring to which R² attaches, and heteroatoms in the aliphatic heterocyclic ring are O, N or S,
or, -(R²)ₙ forms a 3-6 membered aliphatic ring or aliphatic heterocyclic ring together with atoms to which -(R²)ₙ attach, the aliphatic ring or aliphatic heterocyclic ring optionally forms a spiro structure, a fused ring structure or a bridged ring structure with the ring to which -(R²)ₙ attach, and heteroatoms in the aliphatic heterocyclic ring are O, N or S;
R³ is independently -H, -(C₁-C₆)alkyl or -(C₃-C₈)cycloalkyl when occurring each time, and o is 0, 1, 2, 3, 4, 5 or 6,
or, two R³ form a 3-6 membered aliphatic ring or aliphatic heterocyclic ring together with atoms to which R³ attach, the aliphatic ring or aliphatic heterocyclic ring optionally forms a spiro structure, a fused ring structure or a bridged ring structure with the ring to which R³ attach, and heteroatoms in the aliphatic heterocyclic ring are O, N or S,
or, -(R³)ₒ forms a 3-6 membered aliphatic ring or aliphatic heterocyclic ring together with atoms to which -(R³)ₒ attach, the aliphatic ring or aliphatic heterocyclic ring optionally forms a spiro structure, a fused ring structure or a bridged ring structure with the ring to which -(R³)ₒ attach, and heteroatoms in the aliphatic heterocyclic ring are O, N or S;
A has a structure as represented by the following formula II: 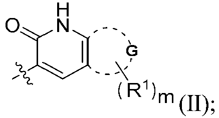
a G-chain group forms a 5-7 membered aliphatic ring or aliphatic heterocyclic ring together with two carbon atoms to which the G-chain group attaches, and ring-forming heteroatoms in the aliphatic heterocyclic ring are -O-, -S- or 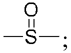
R¹ is independently -H, -(C₁-C₆)alkyl or -(C₃-C₈)cycloalkyl when occurring each time, and m is 0, 1, 2, 3, 4, 5 or 6,
or, two R¹ form a 3-8 membered aliphatic ring or aliphatic heterocyclic ring together with atoms to which R¹ attach, the aliphatic ring or aliphatic heterocyclic ring optionally forms a spiro structure, a fused ring structure or a bridged ring structure with the ring to which R¹ attach, and heteroatoms in the aliphatic heterocyclic ring are O, N or S,
or, -(R¹)ₘ forms a 3-6 membered aliphatic ring or aliphatic heterocyclic ring together with atoms to which -(R¹)ₘ attach, the aliphatic ring or aliphatic heterocyclic ring optionally forms a spiro structure, a fused ring structure or a bridged ring structure with the ring to which -(R¹)ₘ attach, and heteroatoms in the aliphatic heterocyclic ring are O, N or S.

In some embodiments, the compound as represented by the formula I of the present invention has a chiral structure as represented by formula I-2 or formula I-3: 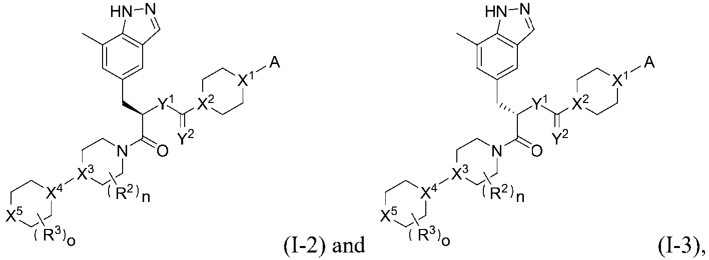 wherein, X¹, X², X³, X⁴, X⁵, Y¹, Y², A, R², R³, n and o are as defined above. In some embodiments, the present invention may make a further selection for an A group as follows:
selection of the G-chain group in A: for example, the G-chain group forms a 5 or 6 membered aliphatic ring together with the two carbon atoms to which the G-chain group attaches, and the G-chain group optionally has 0 or 1 double bond, and the remaining bonds are all single bonds; for example, the G-chain group forms a 7 membered aliphatic ring together with the two carbon atoms to which the G-chain group attaches, and the G chain group optionally has 0, 1 or 2 double bonds, and the remaining bonds are all single bonds; for example, the G-chain group forms a 5 membered aliphatic heterocyclic ring together with the two carbon atoms to which the G-chain group attaches, and bonds on the G-chain group are all single bonds; and for example, the G-chain group forms a 6 or 7 membered aliphatic heterocyclic ring together with the two carbon atoms to which the G-chain group attaches, and the G-chain group optionally has 0 or 1 double bond, and the remaining bonds are all single bonds. In A, the G-chain group may be further specifically selected from: 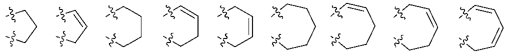 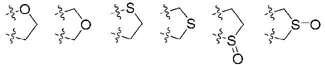 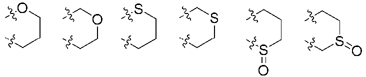 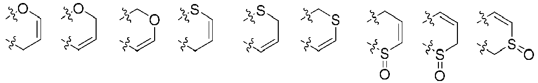 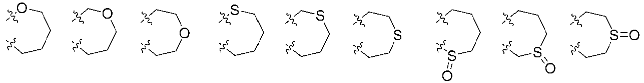 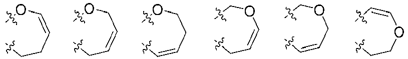 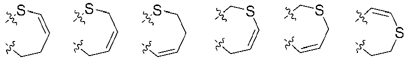 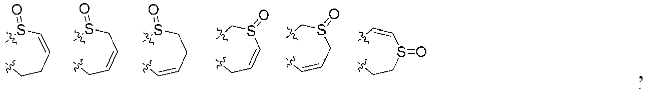 in futher specific selection of the G-chain group, there are some symmetric groups such as 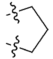 and asymmetric groups such as 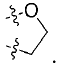 Considering an asymmetry of a group attached at two linking sites of the G-chain group, when the attached asymmetric group is attached, a specific selective structure of the asymmetric group of each G-chain group represents that there are two different specific attaching modes, for example, the asymmetric group 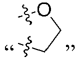 represents two cases 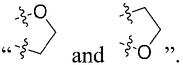 As an example, when the attached asymmetric group has an oxo-pyridine structure 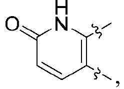 still taking the asymmetric group 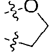 of the G-chain group as an example, the two cases represented by the asymmetric group are attached to the oxo-pyridine structure in a mode of:

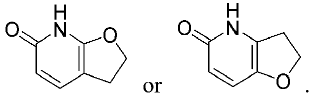

Selection of R¹ group in A: R¹ is independently -H, -(C₁-C₃)alkyl or -(C₃-C₆)cycloalkyl when occurring each time, and m is 0, 1, 2 or 3, or, two R¹ form a 3-6 membered aliphatic ring or aliphatic heterocyclic ring together with atoms to which R¹ attach, the aliphatic ring or aliphatic heterocyclic ring optionally forms a spiro structure, a fused ring structure or a bridged ring structure with the ring to which R¹ attach, and heteroatoms in the aliphatic heterocyclic ring are O, N or S, or, -(R¹)ₘ forms a 3-6 membered aliphatic ring or aliphatic heterocyclic ring together with atoms to which -(R¹)ₘ attach, the aliphatic ring or aliphatic heterocyclic ring optionally forms a spiro structure, a fused ring structure or a bridged ring structure with the ring to which -(R¹)ₘ, attach and heteroatoms in the aliphatic heterocyclic ring are O, N or S. Further selection of R¹ group: R¹ is independently -H, methyl, ethyl or cyclopropyl when occurring each time, and m is 0, 1 or 2, or, two R¹ form cyclopropane, cyclobutane or cyclopentane together with atoms to which R¹ attach, and the cyclopropane, the cyclobutane or the cyclopentane forms a spiro structure, a fused ring structure or a bridged ring structure with the ring to which R¹ attach, or, -(R¹)ₘ forms cyclopropane, cyclobutane or cyclopentane together with atoms to which -(R¹)ₘ attach, and the cyclopropane, the cyclobutane or the cyclopentane forms a spiro structure, a fused ring structure or a bridged ring structure with the ring to which -(R¹)ₘ attach.

According to the embodiments of the present invention, the A group preferably has a structure as represented by the following formula II-1, II-2 or II-3: 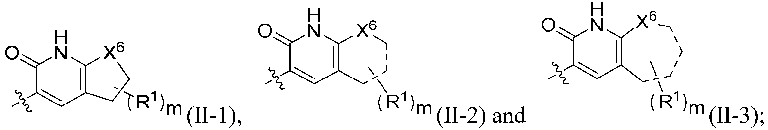
in the formulas II-1, II-2 and II-3, X⁶ is -O-, -S- or 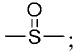
in the formula II-2, two "- - - " both refer to single bonds, or any one of the two "- - - " is a double bond and the other one is a single bond; and
in the formula II-3, three "- - - " all refer to single bonds, or any one of the three "- - - " is a double bond and the other two are both single bonds.

When the A group preferably has the structure as represented by the formula II-1, II-2 or II-3, the compound containing the oxo-pyrido-aliphatic ring or aliphatic heterocyclic ring structure provided by the present invention has a better CGRP antagonistic activity, a lower side reaction and/or a better PK metabolic effect.

As is verified by embodiments of the present invention that, compared with a case that Y² is O, when Y² is S, a compound in which X⁶ is -S- usually has a better CGRP antagonistic activity. Therefore, in one embodiment, Y² is S, and X⁶ is -S-.

According to the Examples of the present invention, the A group may further preferably have a structure as represented by the following formula II-4 or II-5: 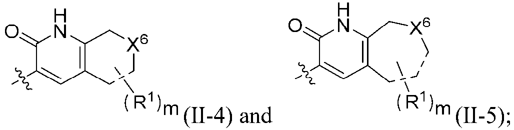
in the formula II-4 or II-5, X⁶ is -O-, -S- or 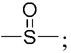
in the formula II-4, "- - - " refers to a single bond or a double bond; and
in the formula II-5, two " -- - " both refer to single bonds, or any one of the two " - - - " is a double bond and the other one is a single bond.

As is verified by embodiments of the present invention , when the position of the heteroatom X⁶ in A is gradually far away from the -NH- group in A, the CGRP antagonistic activity of the compound tends to decrease. However, when the heteroatom X⁶ is on a larger ring, such as a 6 membered ring or a 7 membered ring, even if the heteroatom X⁶ is in a position as shown in the II-4 or II-5, the CGRP antagonistic activity of the compound is still in an acceptable range at this time.

According to the examples of the present invention, when the G-chain group in A forms the aliphatic ring together with the two carbon atoms to which the G-chain group attaches, the aliphatic ring is preferably unsubstituted.

In one embodiment, the G-chain group in A forms a 5-7 membered aliphatic ring together with two carbon atoms to which the G-chain group attaches, and m is 0.

According to the embodiments of the present invention, the A group may be specifically selected as follows: 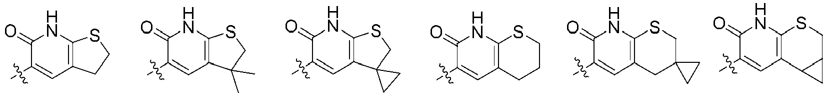 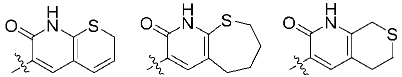 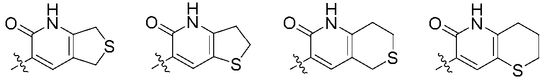 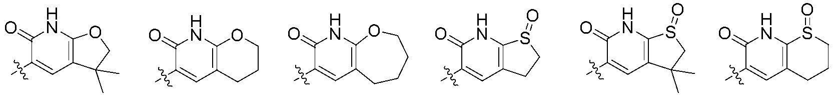 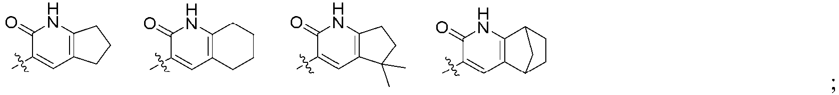 and the A group is particularly preferably selected from 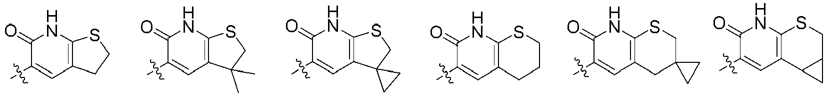 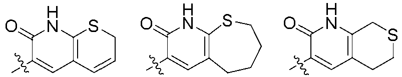 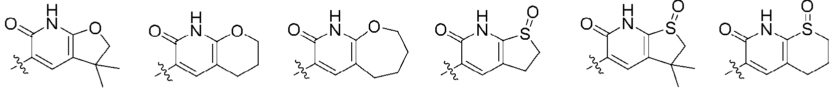 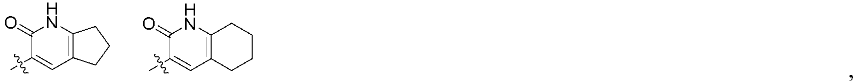 and based on the particularly preferred selection of the A group at this time, the compound containing the oxo-pyrido-aliphatic ring or aliphatic heterocyclic ring structure provided by the present invention has a better CGRP antagonistic activity, a lower side reaction and/or a better PK metabolic effect.

According to the embodiments of the present invention, the A group may be specifically selected as follows: 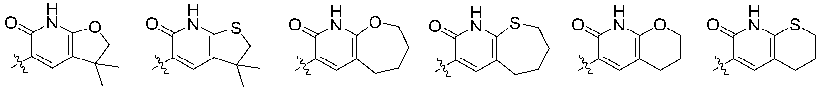 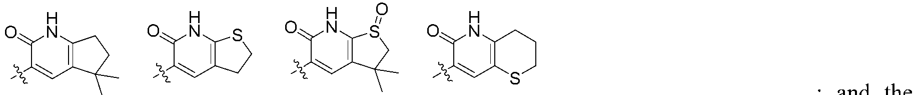 A group is particularly preferably selected from 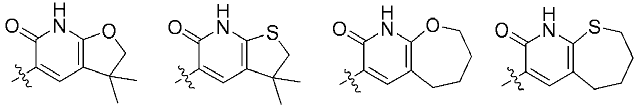 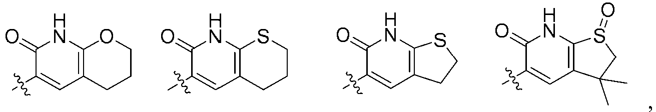 and based on the particularly preferred selection of the A group at this time, the compound containing the oxo-pyrido-aliphatic ring or aliphatic heterocyclic ring structure provided by the present invention has a better CGRP antagonistic activity, a lower side reaction and/or a better PK metabolic effect.

In other embodiments, further selection of the R² group made according to the present invention is as follow: R² is independently -H, -(C₁-C₃)alkyl or -(C₃-C₆)cycloalkyl when occurring each time, and n is 0, 1, 2 or 3; or, two R² form a 3-6 membered aliphatic ring or aliphatic heterocyclic ring together with atoms to which R² attach, the aliphatic ring or aliphatic heterocyclic ring optionally forms a spiro structure, a fused ring structure or a bridged ring structure with the ring to which R² attach, and the atoms on the ring provided by the two R² in the aliphatic ring or aliphatic heterocyclic ring are both C atoms; or, -(R²)ₙ forms a 3-6 membered aliphatic ring or aliphatic heterocyclic ring together with atoms to which -(R²)ₙ attach, the aliphatic ring or aliphatic heterocyclic ring optionally forms a spiro structure, a fused ring structure or a bridged ring structure with the ring to which -(R²)ₙ attach, and the atoms on the ring provided by -(R²)ₙ in the aliphatic ring or aliphatic heterocyclic ring are all C atoms. Further selection of R² group: R² is independently -H, methyl, ethyl, cyclopropyl or cyclobutyl when occurring each time, and n is 0, 1 or 2; or, two R² form a 3-5 membered aliphatic ring or aliphatic heterocyclic ring together with atoms to which R² attach, the aliphatic ring or aliphatic heterocyclic ring optionally forms a spiro structure with the ring to which R² attach, and the atoms on the ring provided by the two R² in the aliphatic ring or aliphatic heterocyclic ring are both C atoms; or, -(R²)ₙ forms a 3-5 membered aliphatic ring or aliphatic heterocyclic ring together with atoms to which -(R²)ₙ attach, the aliphatic ring or aliphatic heterocyclic ring optionally forms a spiro structure with the ring to which -(R²)ₙ attach, and the atoms on the ring provided by -(R²)ₙ in the aliphatic ring or aliphatic heterocyclic ring are all C atoms.

In other embodiments, further selection of R³ group made according to the present invention is as follow: R³ is independently -H, -(C₁-C₃)alkyl or -(C₃-C₆)cycloalkyl when occurring each time, and o is 0, 1, 2 or 3; or, two R³ form a 3-6 membered aliphatic ring or aliphatic heterocyclic ring together with atoms to which R³ attach, the aliphatic ring or aliphatic heterocyclic ring optionally forms a spiro structure, a fused ring structure or a bridged ring structure with the ring to which R³ attach, and the atoms on the ring provided by the two R³ in the aliphatic ring or aliphatic heterocyclic ring are both C atoms; or, -(R³)ₒ forms a 3-6 membered aliphatic ring or aliphatic heterocyclic ring together with atoms to which -(R³)ₒ attach, the aliphatic ring or aliphatic heterocyclic ring optionally forms a spiro structure, a fused ring structure or a bridged ring structure with the ring to which -(R³)ₒ attach, and the atoms on the ring provided by -(R³)ₒ in the aliphatic ring or aliphatic heterocyclic ring are all C atoms. Further selection of R³ group: R³ is independently -H, -(C₁-C₃)alkyl or -(C₃-C₆)cycloalkyl when occurring each time, and o is 0, 1, 2 or 3; or, two R³ form a 3-5 membered aliphatic ring or aliphatic heterocyclic ring together with atoms to which R³ attach, the aliphatic ring or aliphatic heterocyclic ring optionally forms a spiro structure with the ring to which R³ attach, and the atoms on the ring provided by the two R³ in the aliphatic ring or aliphatic heterocyclic ring are both C atoms; or, -(R³)ₒ forms a 3-5 membered aliphatic ring or aliphatic heterocyclic ring together with atoms to which -(R³)ₒ attach, the aliphatic ring or aliphatic heterocyclic ring optionally forms a spiro structure with the ring to which -(R³)ₒ attach, and the atoms on the ring provided by -(R³)ₒ in the aliphatic ring or aliphatic heterocyclic ring are all C atoms.

In other embodiments, the compound containing the oxo-pyrido-aliphatic ring or aliphatic heterocyclic ring structure as represented by the formula I of the present invention more preferably has a structure as represented by a formula I-1: 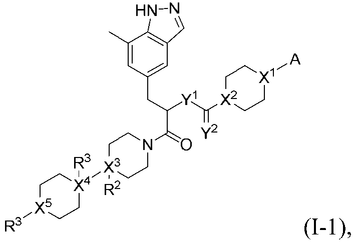 in the formula I-1, X¹, X², X³, X⁴, X⁵, Y¹, Y² and A are optionally as defined in any one of the foregoing items of the present invention when occurring. In the formula I-1, R² and R³ are independently -H, -CH₃ or absent when occurring each time.

In other embodiments, more than one H in the compound containing the oxo-pyrido-aliphatic ring or aliphatic heterocyclic ring structure as represented by the formula I of by the present invention, or the more preferred compound as represented by the formula I-1, I-2 or I-3 are substituted by D, especially more than one H in the G-chain group or R¹ are substituted by D.

According to some embodiments provided by the present invention, more than one H in R³ in the compound containing the oxo-pyrido-aliphatic ring or aliphatic heterocyclic ring structure as represented by the formula I of the present invention, or the more preferred compound as represented by the formula I-1, I-2 or I-3 are substituted by D, and in further selection, R³ attached to X⁵ is -CD₃.

According to some embodiments provided by the present invention, H on the ring of pyrazole group in indazolyl in the compound containing the oxo-pyrido-aliphatic ring or aliphatic heterocyclic ring structure as represented by the formula I of the present invention, or the more preferred compound as represented by the formula I-1, I-2 or I-3 are substituted by D.

In other embodiments, the compound containing the oxo-pyrido-aliphatic ring or aliphatic heterocyclic ring structure of the present invention may be specifically selected from:

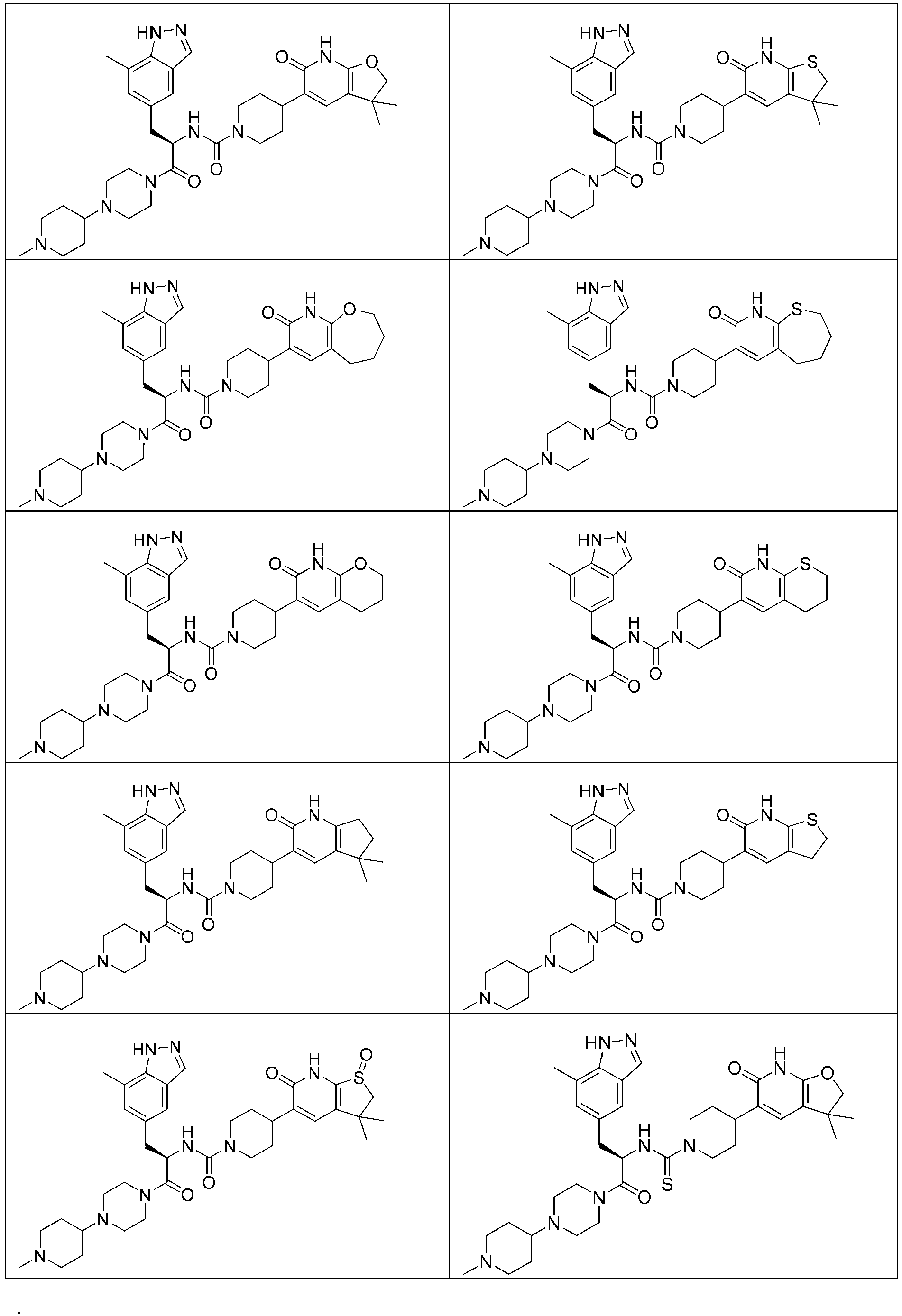

In other embodiments, the compound containing the oxo-pyrido-aliphatic ring or aliphatic heterocyclic ring structure of the present invention may also be specifically selected from:

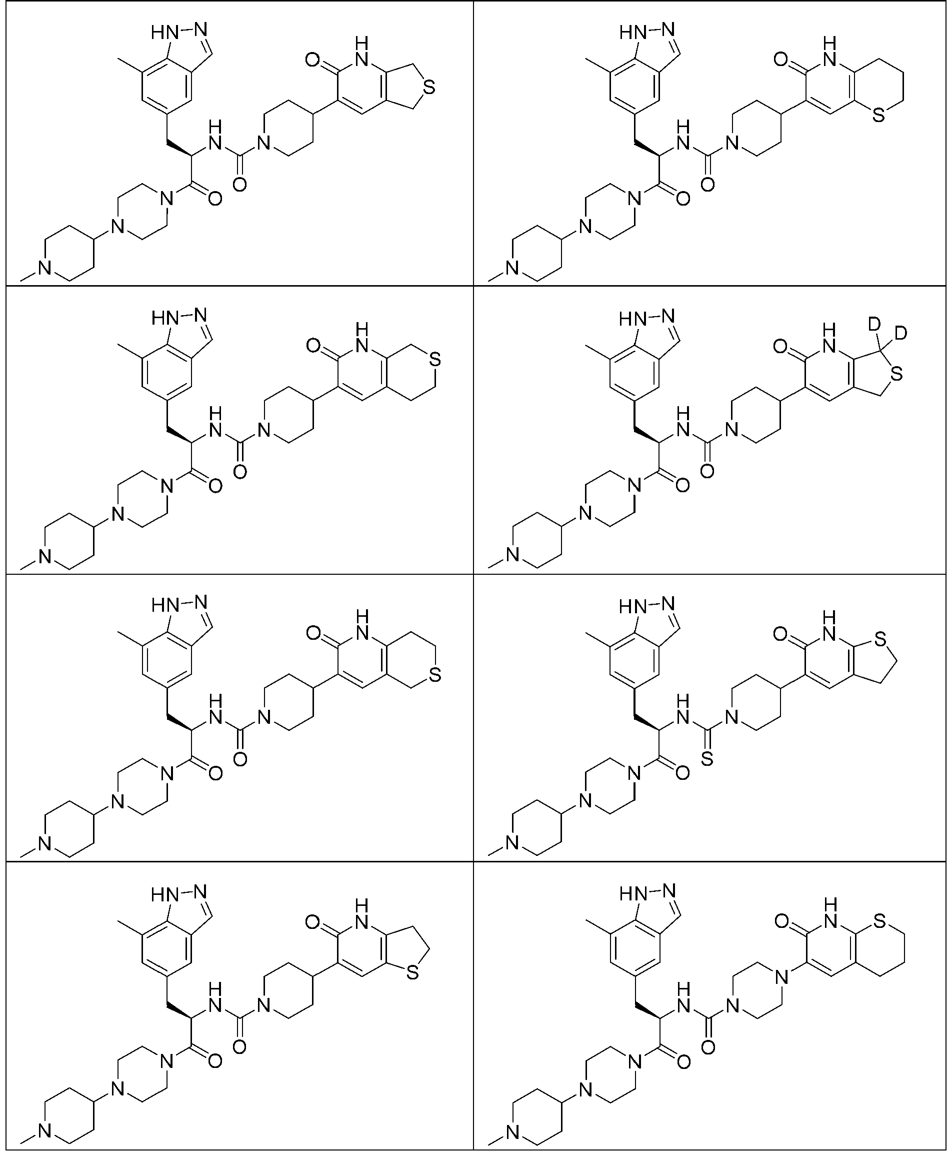

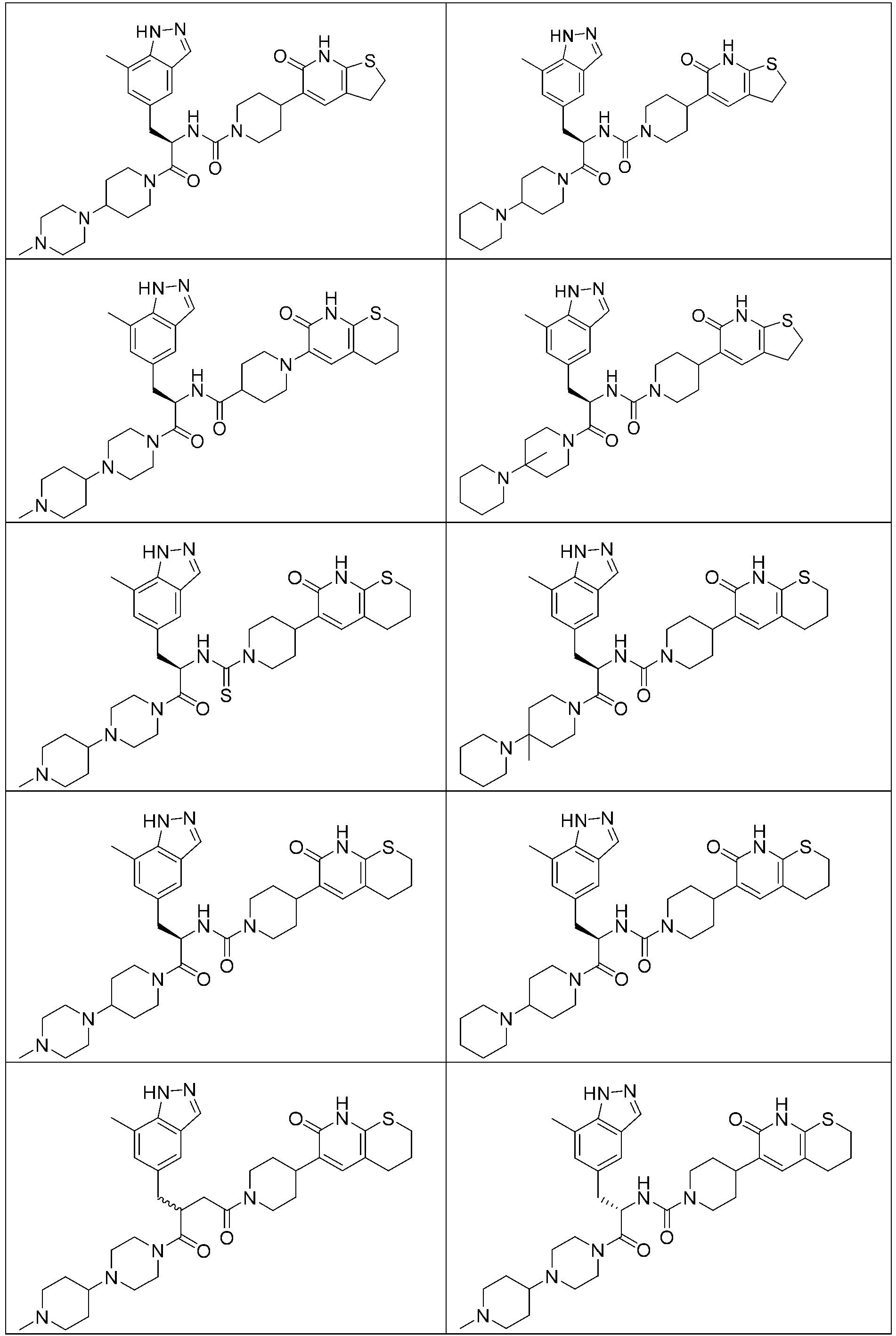

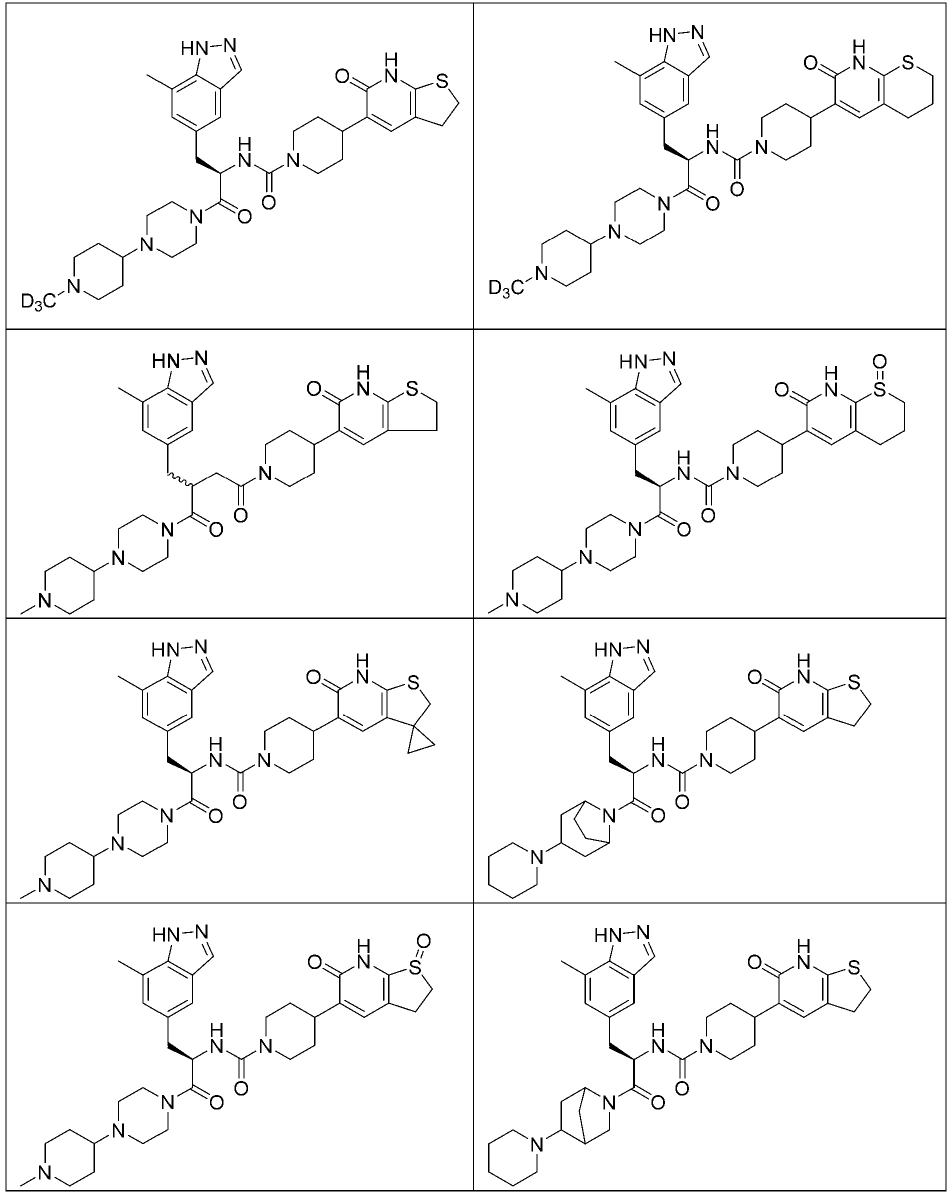

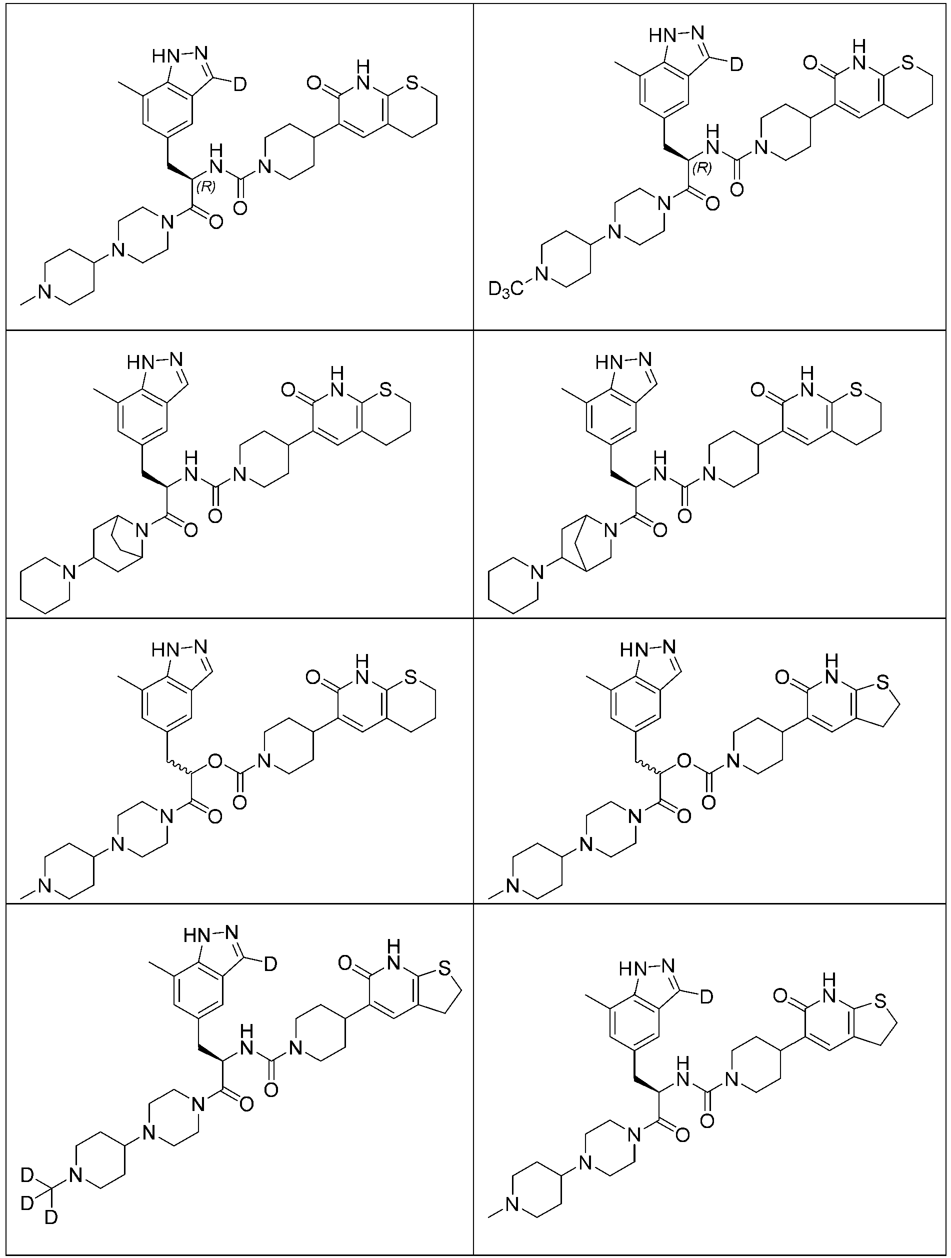

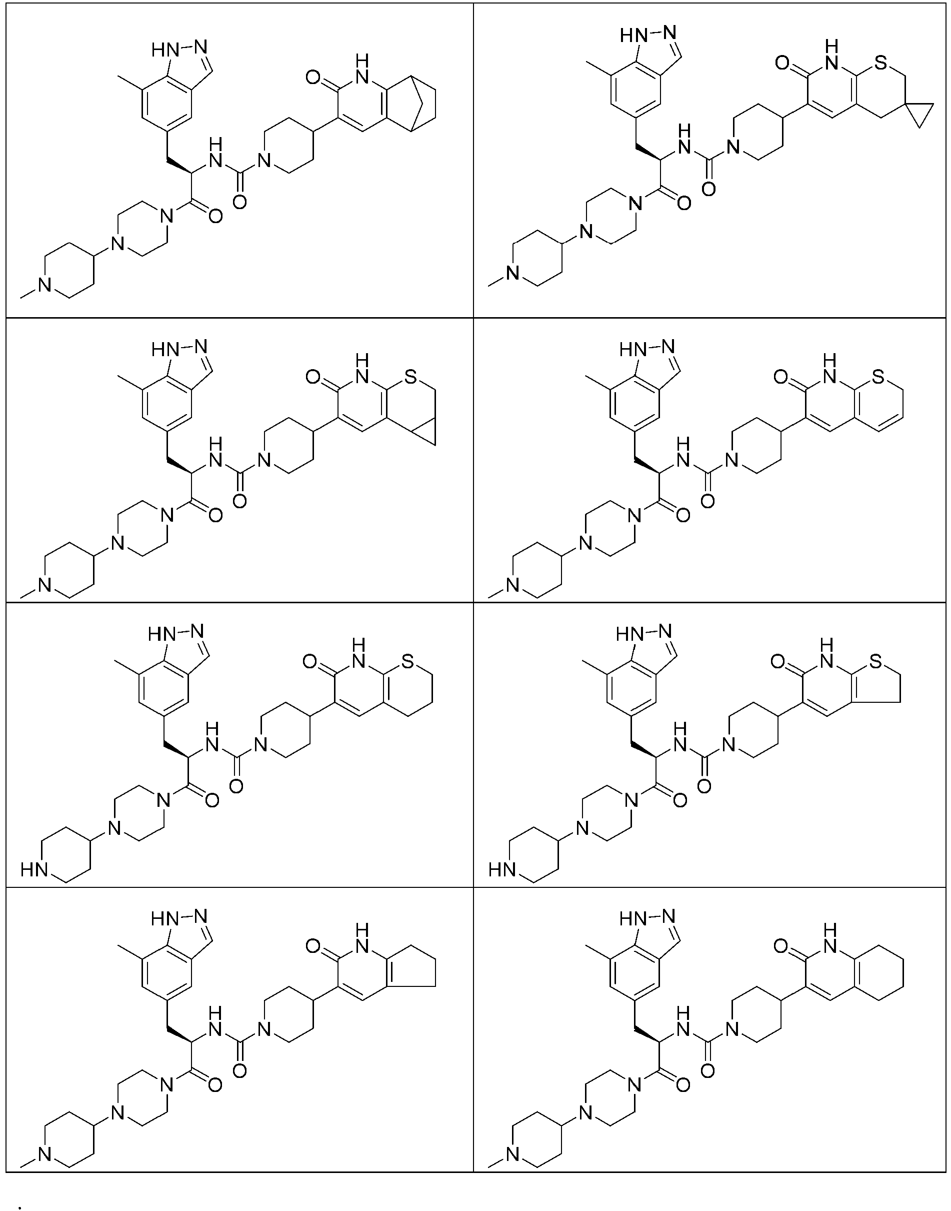

On the basis of the compounds provided above, the present invention further provides a series of important intermediate compounds, the compound with CGRP antagonism of the present invention may be acquired conveniently and smoothly through these important intermediate compounds.

In one embodiment, a typical important intermediate compound provided is an intermediate compound as represented by formula III or an isomer, pharmaceutically acceptable salt or solvate thereof: 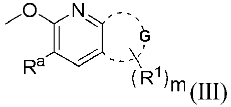 in the formula III, R^{a} is Cl, Br, 1, -OTf or -B(OH)₂, the G-chain group, R¹ and m are as defined in any part of the compound as represented by the formula I of the present invention or any part of the compound of the present invention above, under conditions that: when R^{a} is Br, the ring formed by the G-chain group together with the two carbon atoms attached to the G-chain group is not cyclopentane, cyclohexane and 1,4-dioxane, when R^{a} is Cl, the ring formed by the G-chain group together with the two carbon atoms attached to the G-chain group is not cyclohexane, when the ring formed by the G-chain group together with the two carbon atoms attached to the G-chain group is tetrahydrofuran, and m is 1, R¹ is not isopropyl.

In some embodiments, the intermediate compound as represented by the formula III is selected from formula III-1, III-2, III-3, III-4 or III-5, 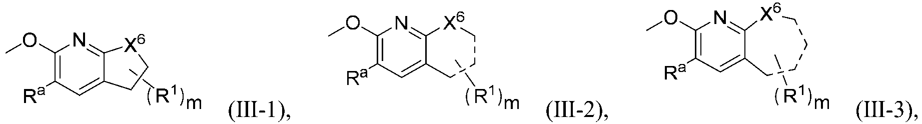 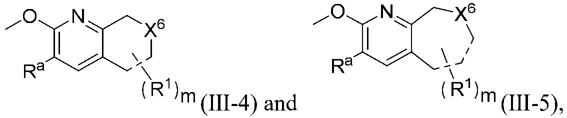
in the formulas III-1, III-2, III-3, III-4 and III-5, X⁶ is -O-, -S- or 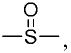
in the formula III-2, two "- - -" both refer to single bonds, or any one of the two " - - -" is a double bond and the other one is a single bond,
in the formula III-3, three "- - -" all refer to single bonds, or any one of the three "- - -" is a double bond and the other two are both single bonds,
in the formula III-4, "- - -" refers to a single bond or a double bond, and
in the formula III-5, two "- - -" both refer to single bonds, or any one of the two " - - -" is a double bond and the other one is a single bond.

In one embodiment, a typical important intermediate compound provided is an intermediate compound as represented by formula IV or an isomer, pharmaceutically acceptable salt or solvate thereof: 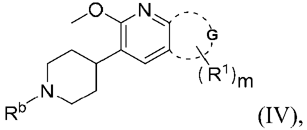 in the formula IV, R^{b} is -H or an amino protecting group, and the amino protecting group is optionally -Cbz, -Boc, -Fmoc, -PMB, -Bn, -Trt, -Tos or -Alloc, and the amino protecting group is preferably -Boc. The G-chain group, R¹ and m are as defined in any part of the compound as represented by the formula I of the present invention or any part of the compound of the present invention above.

In some embodiments, the intermediate compound as represented by the formula IV is selected from formula IV-1, IV-2, IV-3, IV-4 or IV-5, 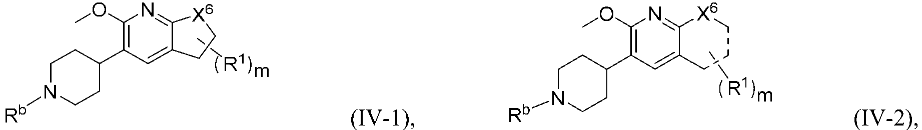 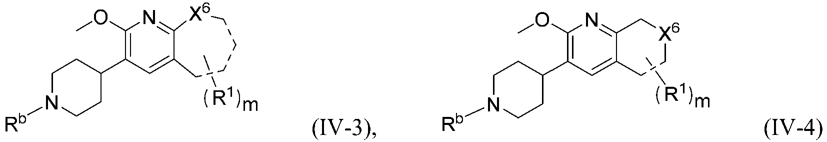 and 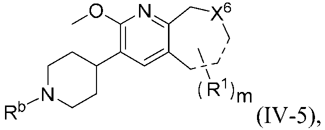
in the formulas IV-1, IV-2, IV-3, IV-4 and IV-5, X⁶ is -O-, -S- or 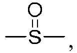
in the formula IV-2, two " - - -" both refer to single bonds, or any one of the two " - - -" is a double bond and the other one is a single bond,
in the formula IV-3, three "- - -" all refer to single bonds, or any one of the three "- - - " is a double bond and the other two are both single bonds,
in the formula IV-4, "- - - " refers to a single bond or a double bond, and
in the formula IV-5, two " - - -" both refer to single bonds, or any one of the two " - - -" is a double bond and the other one is a single bond.

In one embodiment, a typical important intermediate compound provided is an intermediate compound as represented by formula V or an isomer, pharmaceutically acceptable salt or solvate thereof: 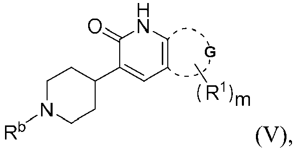
in the formula V, R^{b} is -H or an amino protecting group, and the amino protecting group is optionally -Cbz, -Boc, -Fmoc, -PMB, -Bn, -Trt, -Tos or -Alloc, and the amino protecting group is preferably -Boc,
when R^{b} is -Boc, the formula V is optionally not 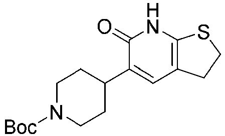 or 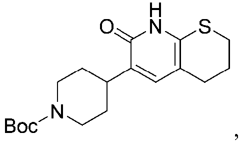
the G-chain group, R¹ and m are as defined in any part of the compound as represented by the formula I of the present invention or any part of the compound of the present invention above.

In some embodiments, the intermediate compound as represented by the formula V is selected from formula V-1, V-2, V-3, V-4 or V-5, 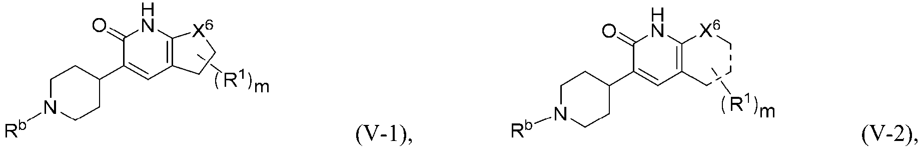 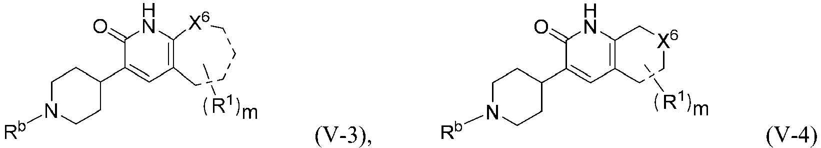 and 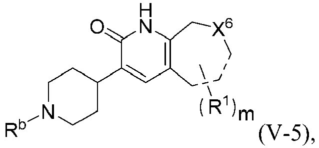
in the formulas V-1, V-2, V-3, V-4 and V-5, X⁶ is -O-, -S- or 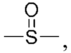
in the formula V-2, two "- - -" both refer to single bond, or any one of the two "- - -" is a double bond and the other one is a single bond,
in the formula V-3, three "- - -" all refer to single bonds, or any one of the three "- - -" is a double bond and the other two are both single bonds,
in the formula V-4, "- - - " refers to a single bond or a double bond, and
in the formula V-5, two "- - - " both refer to single bonds, or any one of the two "- - - " is a double bond and the other one is a single bond.

In some embodiments, the salt of the intermediate compound as represented by the formula III, IV or V above is hydrochloride or trifluoroacetate.

In some embodiments, more than one H in the intermediate compound as represented by the formula III, IV or V above are substituted by D, and optionally, more than one H in the G-chain group or R¹ are substituted by D.

In other embodiments, the intermediate compound of the present invention may be specifically selected from:

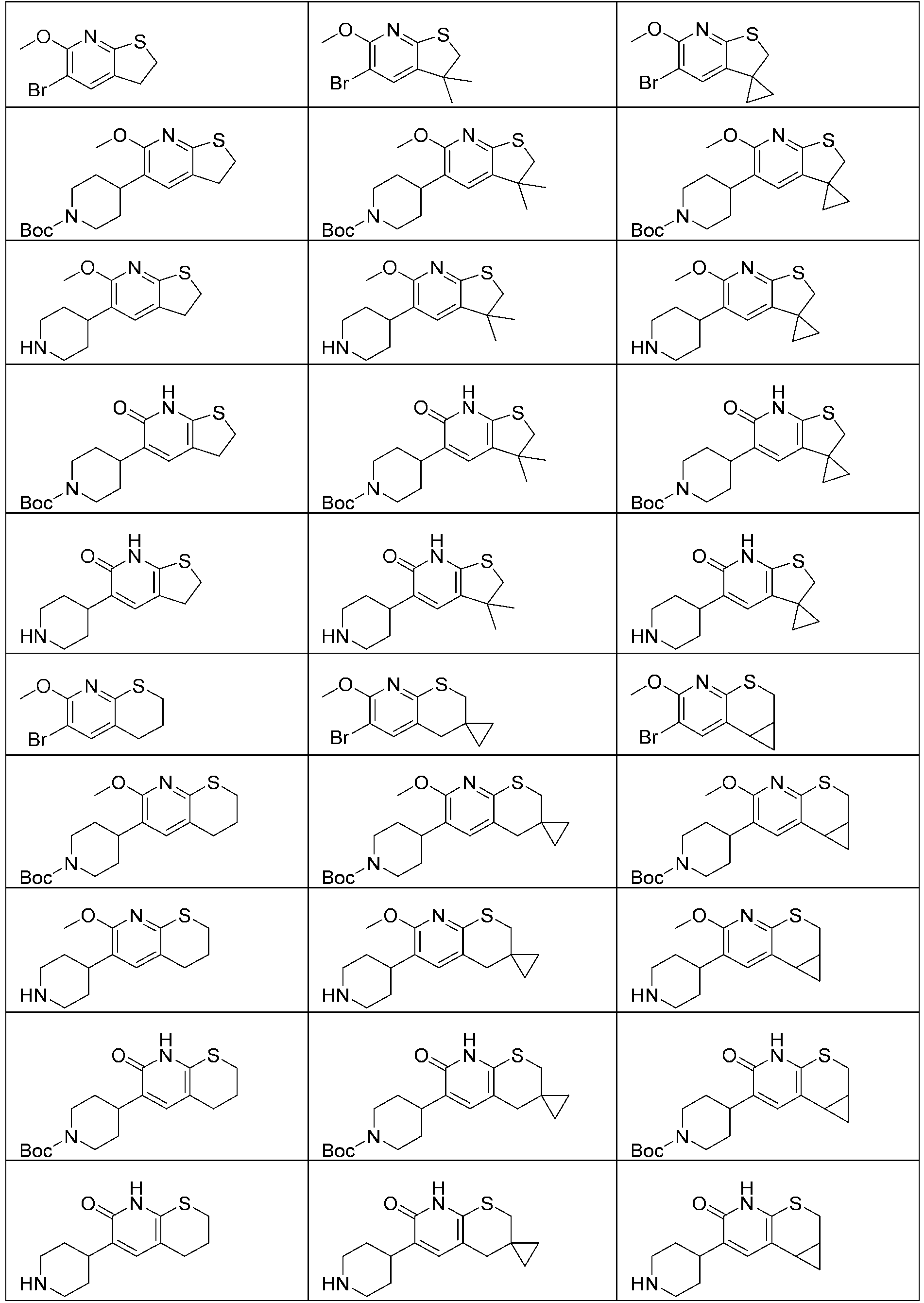

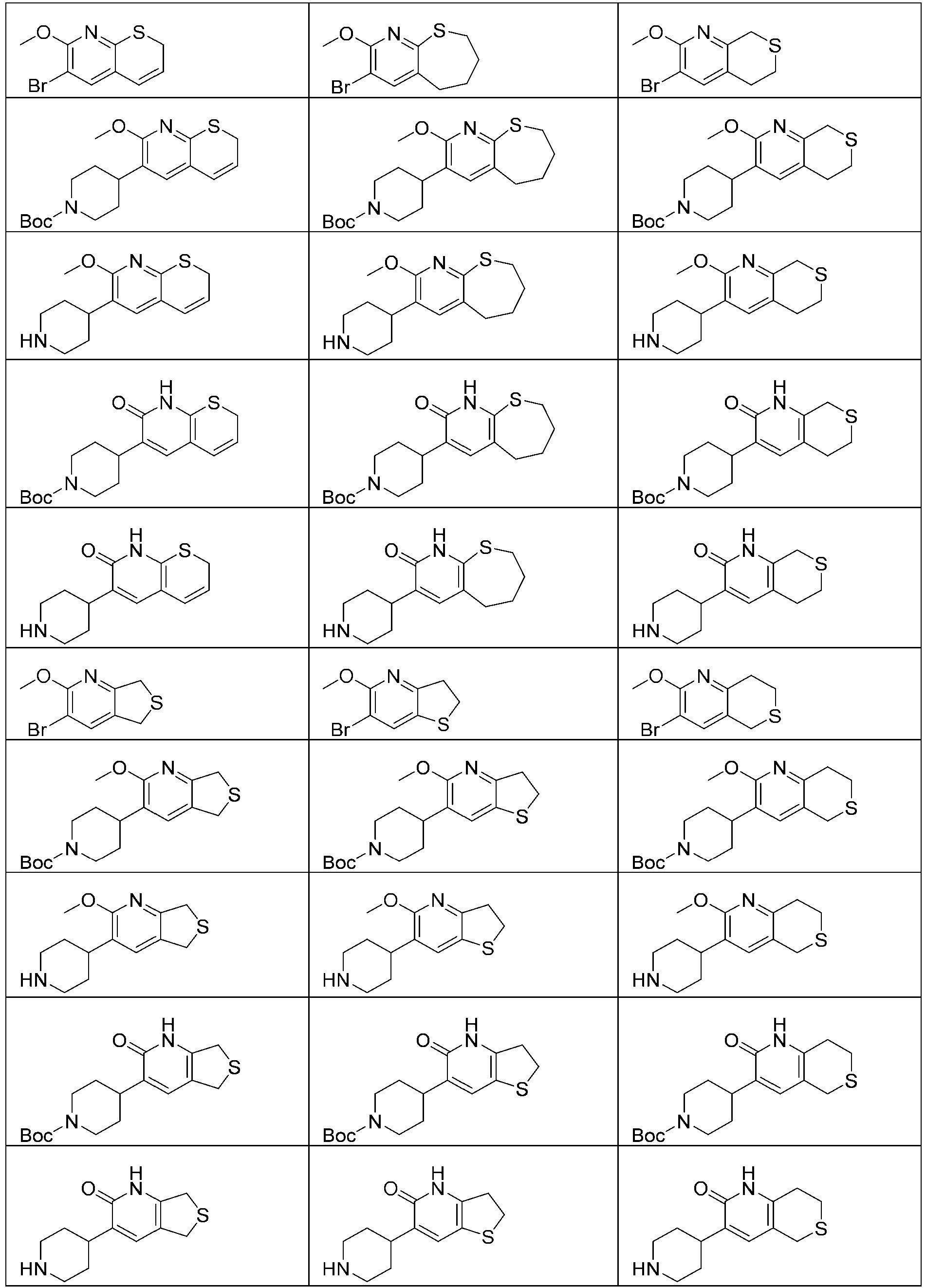

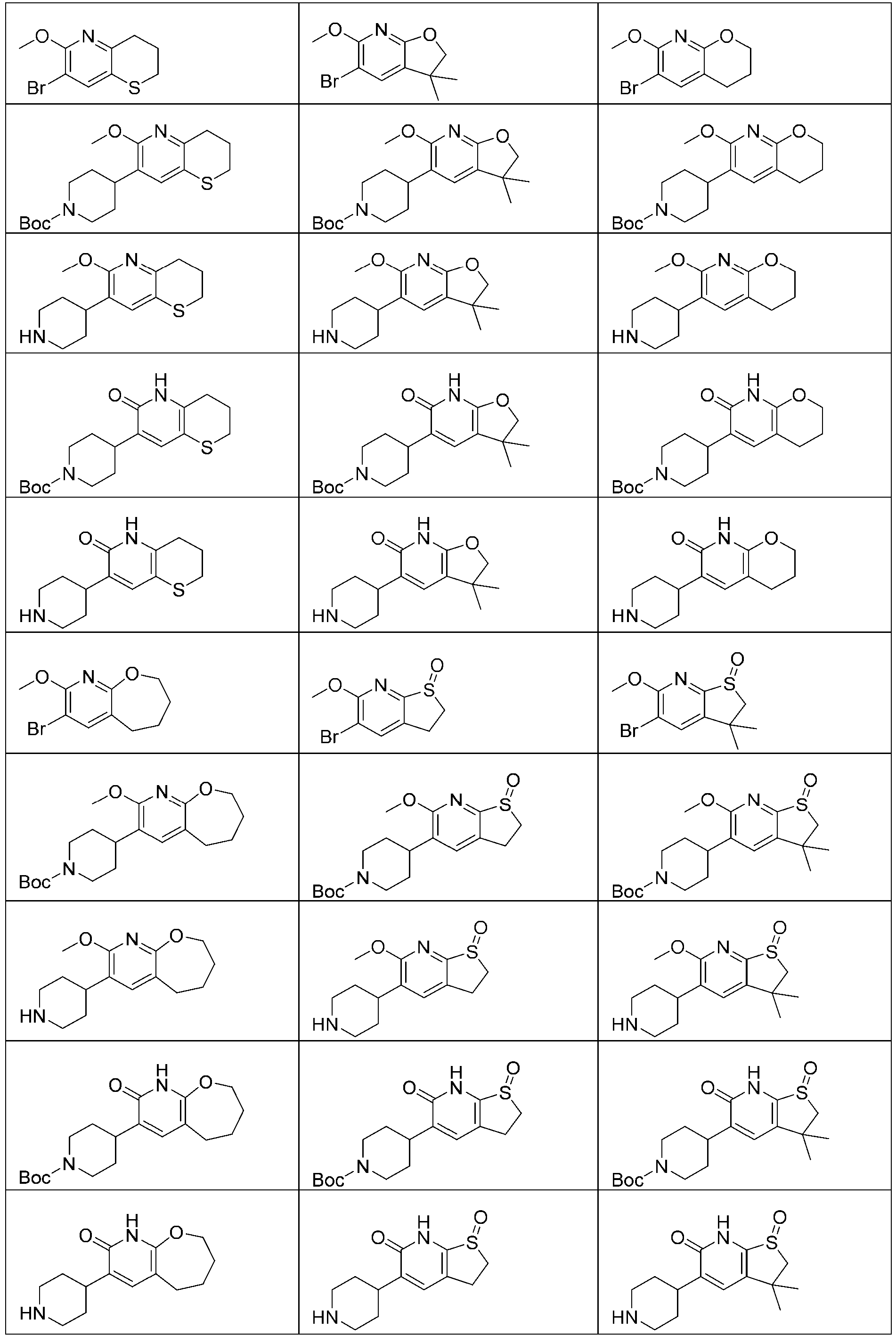

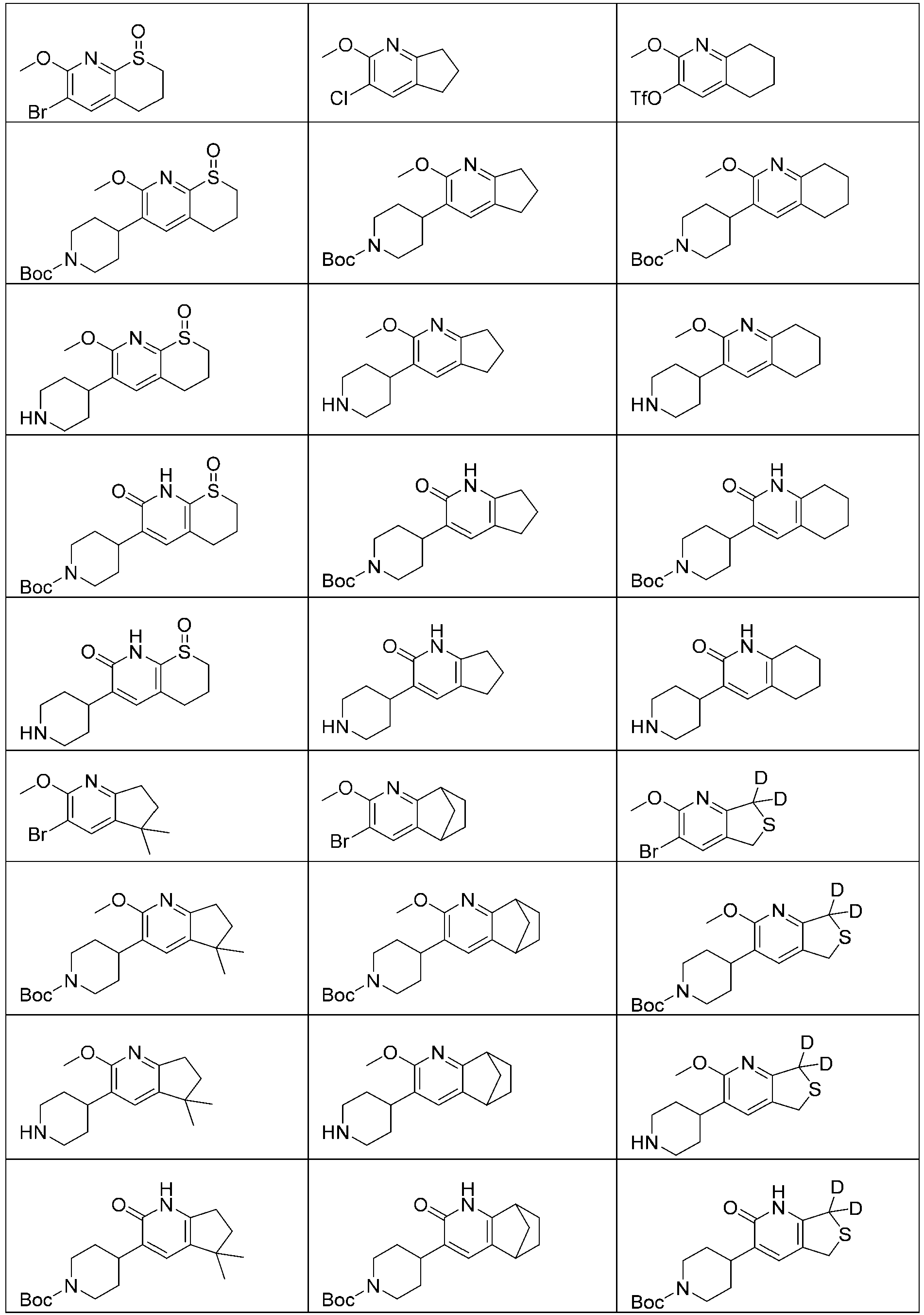

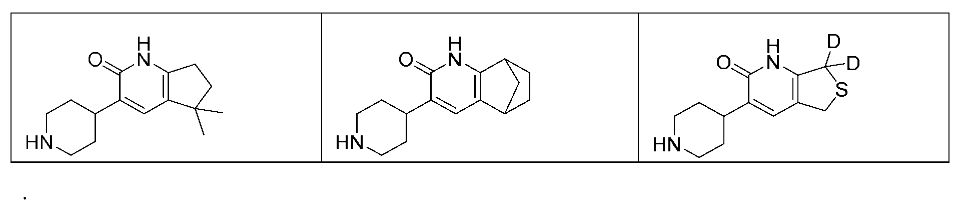

In another embodiment, the present invention provides an intermediate compound as represented by formula VI or an isomer, pharmaceutically acceptable salt or solvate thereof:

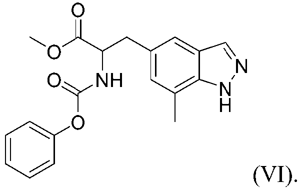

In some embodiments, the intermediate compound as represented by the formula VI is further selected from any one of the following structures: 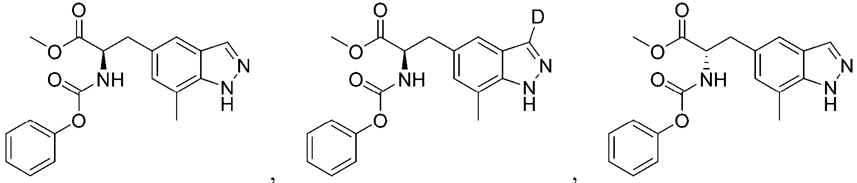 and

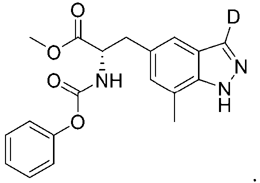

When specific preparation examples and embodiments of the present invention are implemented, the obtained intermediate compounds are characterized and confirmed by MS and ¹H NMR. In order to avoid repeated description, specific ¹H NMR characterization information of some intermediate compounds is only illustratively provided as follows:

| **Intermediate compound** | **¹H NMR** |
|---|---|
| 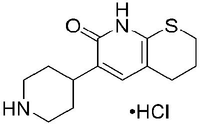 | ¹H NMR (400 MHz, DMSO-*d*₆) δ 9.19 - 9.05 (m, 1H), 8.97 (d, *J* = 12.0 Hz, 1H), 6.92 (s, 1H), 3.29 (d, *J =* 12.5 Hz, 2H), 3.03 (dd, *J =* 7.0, 3.9 Hz, 2H), 2.94 (q, *J =* 12.1 Hz, 2H), 2.83 (td, *J =* 10.2, 8.4, 6.0 Hz, 1H), 2.57 (t, *J* = 6.2 Hz, 2H), 1.95 (p, *J* = 5.8 Hz, 2H), 1.86 (d, *J =* 13.4 Hz, 2H), 1.74 (qd, *J =* 13.0, 3.9 Hz, 2H). |
| 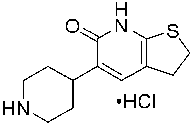 | ¹H NMR (400 MHz, DMSO-*d*₆) δ 9.32 - 8.89 (m, 2H), 7.18 (s, 1H), 3.41 (t, *J* = 7.9 Hz, 2H), 3.30 (d, *J =* 12.6 Hz, 2H), 3.08 (t, *J =* 7.9 Hz, 2H), 3.00 - 2.81 (m, 3H), 1.91 - 1.70 (m, 4H). |
| 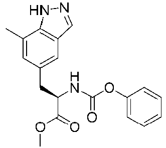 | ¹H NMR (400 MHz, DMSO-*d*₆) δ 13.08 (s, 1H), 8.32 (d, *J =* 8.0 Hz, 1H), 8.02 (d, *J =* 1.4 Hz, 1H), 7.46 (s, 1H), 7.36 - 7.29 (m, 2H), 7.17 (td, *J =* 7.2, 1.2 Hz, 1H), 7.07 (d, *J =* 1.3 Hz, 1H), 7.01 - 6.91 (m, 2H), 4.32 (ddd, *J =* 9.9, 8.0, 5.1 Hz, 1H), 3.66 (s, 3H), 3.16 (dd, *J =* 13.8, 5.1 Hz, 1H), 3.00 (dd, *J =* 13.8, 9.9 Hz, 1H), 2.50 (d, *J* = 1.2 Hz, 3H). |

In addition to directly characterizing and confirming corresponding intermediate compounds by ¹H NMR above, the intermediate compound of the present invention may also be confirmed by a combination of MS provided and ¹H NMR of a final active compound product correspondingly synthesized from the intermediate compound.

On the basis of the structure of the important intermediate compound provided above, the present invention further provides a preparation method for a compound as represented by formula I, which comprises any one or a combination of the following synthetic routes (1)-(3): 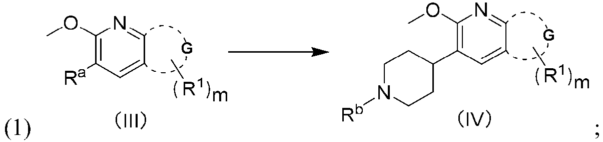 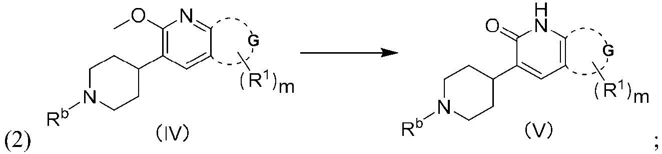 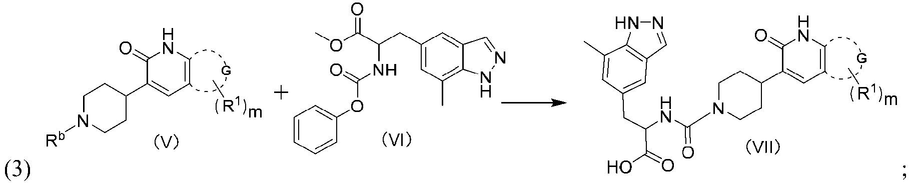 wherein, formulas III, IV, V and VI are respectively as defined in the formulas III, IV and V of the intermediate compound of the present invention above, and the G-chain group, R¹ and m in formula VII are as defined in the G-chain group, R¹ and m in any structure of the formula III, IV or V in the intermediate compound of the present invention above.

The present invention further provides a synthetic method for a compound as a CGRP antagonist, which comprises the following synthetic route: 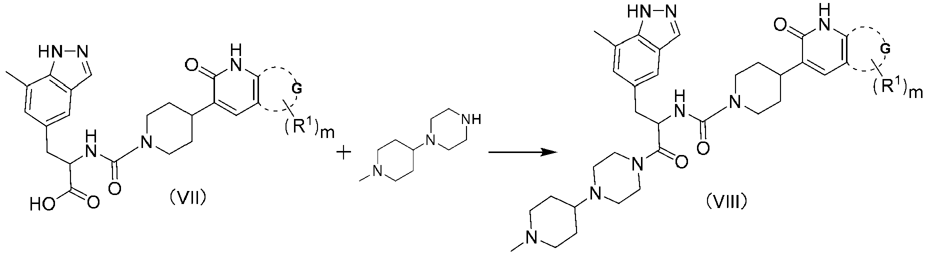 wherein, the G-chain group, R¹ and m in formulas VII and VIII are as defined in the G-chain group, R¹ and m in any structure of the compound of the present invention above.

The present invention further provides a synthetic method for a compound as a CGRP antagonist, which comprises the following synthetic route: 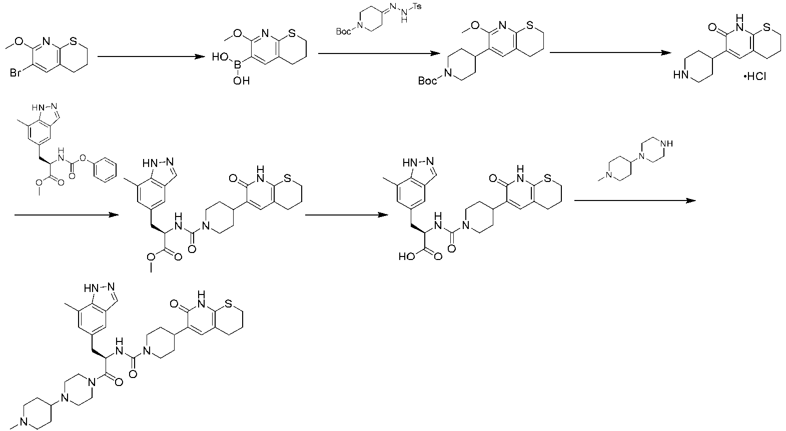 or,

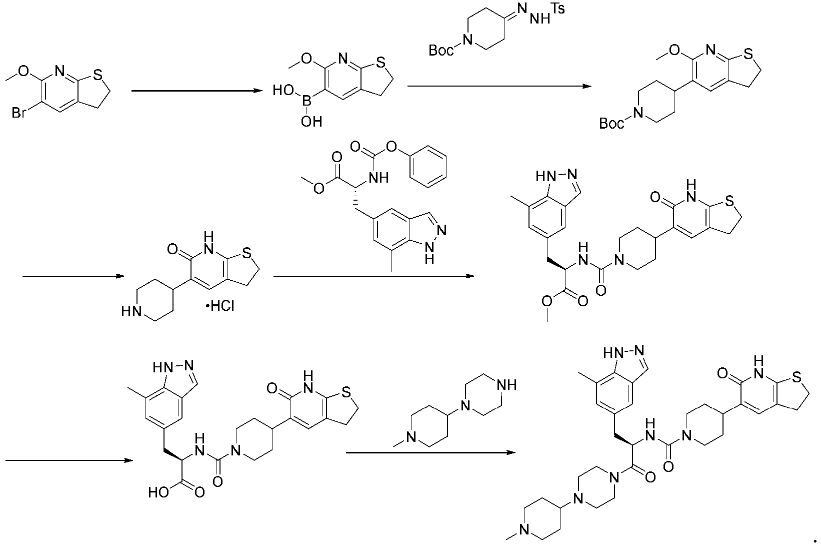

In the present invention, when the latter technical solution further defines the former technical solution, only some technical features may be further defined, and at this time, the undefined technical features may be optionally defined in any occurring part in the former technical solution or the present invention.

As mentioned above, the present invention provides a class of compounds with structural features of general formula I, and it is found according to studies that this class of compounds can effectively inhibit generation of cAMP stimulated by CGRP, so as to serve as a CGRP antagonists to prevent, and relieve or treat CGRP-related diseases, especially migraine or neuropathic pain. Specifically, the novel compounds provided by the present invention have a lower IC₅₀ value, especially some specific structural compounds with common characteristics have a significantly lower IC₅₀ value, which can reach a pM level. Other experimental data show that the compounds of the present invention also have a good liver microsome stability, a lower expected potential side effect and a better PK characteristic.

Therefore, the present invention provides the compound, or the isomer, pharmaceutically acceptable salt or solvate thereof in any embodiment of the present invention above as the CGRP antagonist.

Based on the above, the present invention further provides a pharmaceutical composition containing the compound or the isomer, pharmaceutically acceptable salt or solvate thereof in any embodiment of the present invention above, the pharmaceutical composition further comprises a pharmaceutically acceptable excipient, and the pharmaceutical composition may be presented in any pharmaceutically acceptable dosage form and administration route.

Secondly, the present invention further provides a use of the compound or the pharmaceutical composition of the present invention above as the CGRP antagonist, and further provides a use of the compound or th pharmaceutical composition of the present invention in preparation of a drug for preventing, treating or relieving CGRP-mediated diseases. In one embodiment, the diseases comprise migraine and neuropathic pain. Correspondingly, the present invention further provides a method for preventing, treating or relieving CGRP-mediated diseases, such as migraine or neuropathic pain, which comprises: administering the compound, the isomer, pharmaceutically acceptable salt or solvate thereof in any embodiment of the present invention above.

### DESCRIPTION OF THE DRAWINGS

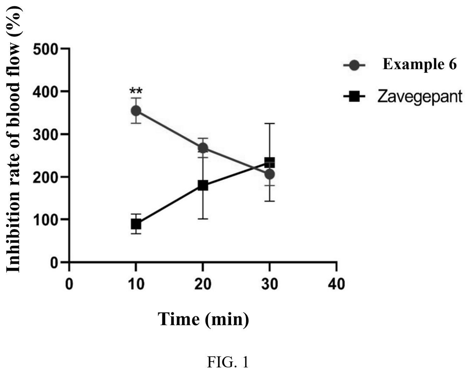

FIG. 1 shows comparison results of blood flow inhibition rates of a compound in Example 6 of the present invention and Zavegepant in an in-vivo pharmacodynamic study of a capsaicin-induced change of blood flow in forearm skin of cynomolgus monkeys.

### EMBODIMENT

Unless stated to the contrary or defined separately, the following terms used in the specification and the claims have the following meanings.

"Alkyl" refers to an aliphatic hydrocarbon group, and refers to saturated hydrocarbyl. The alkyl may be linear-chain alkyl or branched-chain alkyl, such as -(C₁-C₆)alkyl or -(C₁-C₃)alkyl. The -(C₁-C₆)alkyl refers to alkyl with 1 to 6 carbon atoms, such as alkyl with 1 carbon atom, 2 carbon atoms, 3 carbon atoms, 4 carbon atoms, 5 carbon atoms and 6 carbon atoms. Non-restrictive examples of alkyl comprise methyl, ethyl, n-propyl, isopropyl, n-butyl, isobutyl, sec-butyl, tert-butyl, n-pentyl, neopentyl, n-hexyl, and the like.

"Ring" refers to any covalently closed structure, comprising, for example, carbocyclic rings (such as aryl and cycloalkyl), heterocyclic groups (such as heteroaryl and heterocycloalkyl), aromatic groups (such as aryl and heteroaryl) and nonaromatic groups (such as cycloalkyl and heterocycloalkyl). The ring may be a monocyclic ring or a polycyclic ring. Typical polycyclic rings generally comprise a bicyclic ring and a tricyclic ring. The ring of the present invention generally has 3-20 ring atoms, such as 3 ring atoms, 4 ring atoms, 5 ring atoms, 6 ring atoms, 7 ring atoms, 8 ring atoms, 9 ring atoms, 10 ring atoms, 11 ring atoms, 12 ring atoms, 13 ring atoms, 14 ring atoms, 15 ring atoms, 16 ring atoms, 17 ring atoms, 18 ring atoms, 19 ring atoms or 20 ring atoms.

"Aliphatic ring" is saturated or unsaturated cyclic hydrocarbon with no aromatic property, and ring-forming skeleton atoms of the aliphatic ring are all carbon atoms. The aliphatic ring in the present invention may usually have 3-8, 3-6 or 5-7 ring carbon atoms, and may also be called a 3-8 membered aliphatic ring, a 3-6 membered aliphatic ring or a 5-7 membered aliphatic ring, and typical examples of the aliphatic ring are cyclopropane, cyclobutane, cyclopentane, cyclohexane, cycloheptane, cyclooctane, cyclopropene, cyclobutene, cyclopentene, cyclohexene, cycloheptene, and the like.

"Cycloalkyl" refers to a saturated cyclic hydrocarbon substituent, and is preferably cyclopropyl, cyclobutyl, cyclopentyl, cyclohexanyl, cycloheptyl or cyclooctyl. The cycloalkyl may be substituted or unsubstituted.

"Aliphatic heterocyclic ring", also called "aliphatic heterocyclic ring" in the present invention, is a heterocyclic ring with no aromatic property formed by substituting one or more ring-forming skeleton carbon atoms with heteroatoms on the basis of the "aliphatic ring", and the substituted heteroatoms may usually be O, S, N, and the like.

"Membered" refers to the number of the ring-forming skeleton carbon atoms. Typical 5 membered rings comprise, for example, cyclopentyl, pyrrole, imidazole, thiazole, furan and thiophene; and typical 6 membered rings comprise, for example, cyclohexanyl, pyridine, pyran, pyrazine, thiapyran, pyridazine, pyrimidine and benzene, wherein, a ring formed by skeleton atoms containing heteroatoms is a heterocyclic ring.

"Spiro" or "spiro structure" refers to a structure formed by two rings sharing one skeleton ring atom, such as

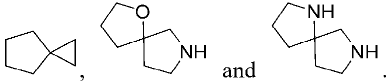

"Bridged ring" or "bridged ring structure" refers to a structure formed by two rings sharing two skeleton ring atoms, wherein the two shared skeleton ring atoms are not adjacent, such as

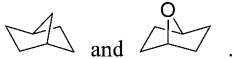

"Fused ring" or "fused ring structure" refers to a structure formed by two rings sharing two adjacent skeleton ring atoms, such as

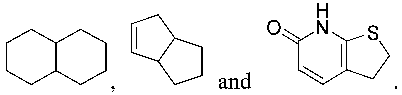

"Oxo" means that hydrogens on carbon are substituted by =O.

"Substituted" means that one or more hydrogen atoms, preferably at most 5 (such as 1, 2, 3, 4 and 5) hydrogen atoms, and more preferably 1-3 hydrogen atoms, in a group, are independently substituted by a corresponding number of substituents. It goes without saying that the substituents are only in their possible chemical positions, and those skilled in the art can determine (by experiment or theory) possible or impossible substitutions without excessive efforts. For example, amino or hydroxyl with free hydrogen may be unstable when combined with carbon atoms with unsaturated bonds.

"Antagonist" refers to a drug that has no intrinsic activity, but may block a receptor agonist-mediated action after binding with a receptor.

"Optionally" means that the subsequently described event or environment may, but does not have to, occur, and the description comprises an occasion in which the event or environment occurs or does not occur.

The term "substituted or unsubstituted" herein means that any group is monosubstituted or polysubstituted by a specified substituent to an extent that such monosubstitution or polysubstitution (comprising multi-substitution in the same part) is chemically permitted, and each substituent may be located in any available position on the group and may be linked by any available atom on the substituent. "Any available position" refers to any position on the group that may be chemically obtained by a method known in the art or a method taught herein, and does not produce excessively unstable molecules. When there are two or more substituents on any group, each substituent is defined independently of any other substituent, so that the substituents may be the same or different.

Those skilled in the art should understand that some compounds represented by formula (I) may contain one or more chiral centers, so that there are two or more stereoisomers. Therefore, the term "isomer" used herein comprises a single enantiomer, a racemic mixture of enantiomers and an enantiomer-enriched mixture, and a mixture in which diastereomers and specific diastereomers are partially enriched when there are two or more chiral centers. Furthermore, under appropriate circumstances, the term "isomer" used herein further comprises a tautomer.

The term "compound of the present invention" used herein is intended to cover a compound as represented by general formula (I) as defined herein or any preferred or specific embodiment thereof (comprising compounds as represented by formulas (I-1), (I-2), (I-3), and the like, and compounds in the embodiments), and their stereoisomers, pharmaceutically acceptable salts, tautomers or solvates.

The term "pharmaceutically acceptable" used herein refers to molecular entities and compositions that are approved by or may be approved by corresponding institutions in various countries, or are listed in the generally recognized pharmacopoeia for animals and more specific humans, or will not produce adverse, allergic or other adverse reactions when administered to animals such as humans in appropriate amounts.

The term "pharmaceutically acceptable salt" used herein refers to a salt of the compound of the present invention that is pharmaceutically acceptable and has a pharmacological activity required by a parent compound. Specifically, such salts are nontoxic, and may be inorganic acid addition salts, organic acid addition salts and alkali addition salts.

" 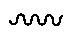 " in the chemical structural formula herein means that the structure here is chiral, but there is no chiral resolution in the current compound, that is, the meaning of the current compound contains a case of different chiral structures.

" 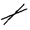 " in the chemical structural formula herein means that the double bond structure here contains cis and trans configurations, but there are no cis and trans resolutions in the current compound, that is, the meaning of the current compound contains a case of different cis and trans configurations.

It should be understood that, when each group in the structure of the compound of the present invention is selected, the groups connected, coordinated or influenced with each other should be selected correspondingly on the premise of conforming to chemical valence-bond rules.

According to the contents of the present invention, on the basis of common technical knowledge and means in the art, other modifications, substitutions or changes may be made in various forms without departing from the above basic technical idea of the present invention.

In order to further explain the present invention, an active compound provided by the present invention, a preparation method therefor and an application thereof are described in detail hereinafter with reference to the embodiments.

The following abbreviations or terms have the following meanings:
K₂CO₃ represents potassium carbonate; H₂O represents water;
DMF represents *N,N-*dimethylformamide; KF represents potassium fluoride;
DIPEA or DIEA represents *N,N-*diisopropylethylamine; n-BuLi represents n-butyllithium;
THF represents tetrahydrofuran; TFA represents trifluoroacetic acid;
HCl/Dioxane represents a hydrogen chloride dioxane solution; HCl represents a hydrogen chloride solution;
DMSO represents dimethyl sulfoxide; CH₃I or MeI represents methyl iodide;
H₂ represents hydrogen; Pd₂(dba)₃ represents tris(dibenzylideneacetone)dipalladium;
t-BuOK represents potassium tert-butoxide; NMP represents N-methylpyrrolidone;
MeOH represents methanol; EtOH represents ethanol;
Pd(PPh₃)₂Cl₂ represents bis(triphenylphosphine)palladium dichloride; DIBAL-H represents diisobutyl aluminium hydride;
TEA represents triethylamine; DCM represents dichloromethane;
DCE represents 1,2-dichloroethane; dioxane represents 1,4-dioxane;
NBS represents N-bromosuccinimide; NaH represents sodium hydride;
LiHMDS represents lithium bis(trimethylsilyl)amide; NaBH₄ represents sodium borohydride;
Pd(dppf)Cl₂ represents [1,1'-bis(diphenylphosphino)ferrocene]palladium dichloride; Zn represents zinc;
Ruphos represents 2-dicyclohexylphosphino-2',6'-diisopropoxy-1,1'-biphenyl; I₂ represents iodine;
NaSH represents sodium hydrosulfide; CuI represents cuprous iodide;
Pd/C represents palladium carbon; AcSH represents thioacetic acid;
DIAD represents diisopropyl azodiformate; PPh₃ represents triphenylphosphine;
CH₃CN or MeCN represents acetonitrile; PtO₂ represents platinum dioxide;
NaOMe represents sodium methoxide; KHMDS represents potassium bis(trimethylsilyl)amide;
DEAD represents diethyl azodiformate; MWI represents a microwave-induced reaction;
AcCl represents acetyl chloride; NaOH represents sodium hydroxide;
TBTU represents 2-(1H-benzotriazol-1-yl)-1,1,3,3-tetramethyluronium tetrafluoroborate;
DMAP represents 4-dimethylaminopyridine; Ms₂O represents methanesulfonic anhydride;
LiOH represents lithium hydroxide; B(OCH₃)₃ represents trimethyl borate;
Cs₂CO₃ represents cesium carbonate; TCDI represents N,N'-thiocarbonyl diimidazole;
TosCl represents p-toluenesulfonyl chloride; DSC represents N,N'-disuccinimyl carbonate;
LDA represents lithium diisopropylamide; XantPhos represents 4,5-bis(diphenylphosphino)-9,9-dimethylxanthene;
LAH represents lithium aluminum hydride; DPPA represents diphenylphosphoryl azide;
DMC represents dimethyl carbonate; TMSCN represents trimethylsilyl cyanide;
s-BuLi represents sec-butyllithium; PyBOP represents 1H-benzotriazol-1-yloxytripyrrolidinophosphonium hexafluorophosphate;
DBU represents 1,8-diazabicyclo[5.4.0]undec-7-ene; DMSu represents dimethyl succinate;
Pd(dppf)Cl₂·DCM represents a [1,1'-bis(diphenylphosphino)ferrocene]palladium dichloride dichloromethane complex.

In addition, in the following preparation examples and embodiments, when the structural formula of the compound is inconsistent with the chemical name, the structural formula of the compound shall prevail.

A synthesis method for an intermediate compound of the compound of the present invention is illustratively provided as follows first. Starting materials and other additive reagent materials used in the synthesis of the intermediate compound provided are all commercially available, or may be acquired through the methods reported in the existing technical literatures combined with the conventional chemical reaction synthesis means. Some intermediate compounds are also commercially available directly, or may be acquired through the methods reported in the existing technical literatures combined with the conventional chemical reaction synthesis means.

### EXAMPLES

### Preparation Example 1 of intermediate compound: preparation of 2-(2-chloro-6-methoxypyridine-3-yl)-2-methylpropane-1-ol (available for Preparation Example 2 of intermediate compound)

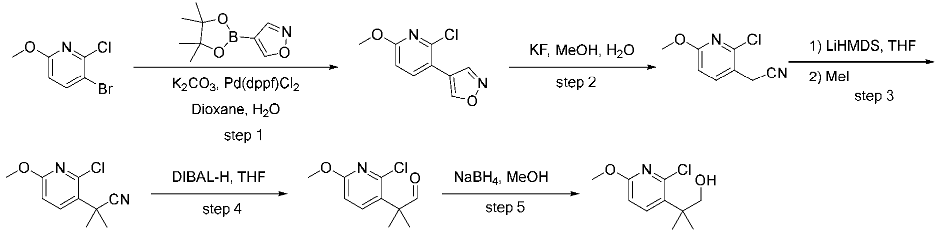

### Step 1: preparation of 4-(2-chloro-6-methoxypyridine-3-yl)isoxazole

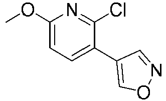

In a nitrogen atmosphere, 3-bromo-2-chloro-6-methoxypyridine (10.0 g) and potassium carbonate (12.4 g) were dissolved in 1,4-dioxane (100 mL) and water (10 mL), and then 4-isoxazole boric acid (9.7 g) was dissolved in 1,4-dioxane (100 mL), put into a 150 mL constant-pressure dropping funnel, and added with 1,1'-bis(diphenylphosphino)ferrocene palladium dichloride (1.63 g). The system was transferred to 90°C and slowly dropwise added with 4-isoxazole boric acid at this temperature for 3 hours, and then to react for 2 hours after the addition. After TLC showed that the reaction was completed, the reaction system was transferred to room temperature, added with 500 mL of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 9.0 g of the title compound.

MS (ESI) m/z (M+H)⁺ = 211.0.

### Step 2: preparation of 2-(2-chloro-6-methoxypyridine-3-yl)acetonitrile

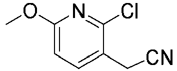

4-(2-chloro-6-methoxypyridine-3-yl)isoxazole (9.0 g) was dissolved in methanol (100 mL) and water (10 mL), and added with potassium fluoride (12.4 g). The system was put into a thick-walled reaction bottle to react in a closed mode at 100°C for 6 hours. After TLC showed that the reaction was completed, the reaction system was transferred to room temperature, concentrated to remove methanol, added with 500 mL of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 7.1 g of the title compound.

MS (ESI) m/z (M+H)⁺ = 183.0.

### Step 3: preparation of 2-(2-chloro-6-methoxypyridine-3-yl)-2-methylpropionitrile

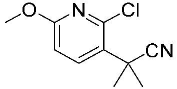

In a nitrogen atmosphere, 2-(2-chloro-6-methoxypyridine-3-yl)acetonitrile (7.1 g) was dissolved in anhydrous tetrahydrofuran (80 mL). The system was cooled to -78°C and slowly added with lithium bis(trimethylsilyl)amide (1 M, 117.0 mL) to react for 10 minutes. The system was added with methyl iodide (12.7 g) to continuously react for 20 minutes. After TLC showed that the reaction was completed, the reaction system was transferred to room temperature, added with 200 mL of saturated ammonium chloride aqueous solution, and extracted with ethyl acetate for three times, and the organic phases were combined, backwashed with a saturated sodium chloride solution once, dried with anhydrous sodium sulfate, filtered and concentrated to obtain 8.1 g of crude product.

MS (ESI) m/z (M+H)⁺ = 211.1.

### Step 4: preparation of 2-(2-chloro-6-methoxypyridine-3-yl)-2-methylpropanal

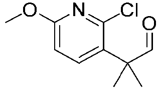

In a nitrogen atmosphere, 2-(2-chloro-6-methoxypyridine-3-yl)-2-methyl propionitrile (8.1 g) was dissolved in anhydrous tetrahydrofuran (80 mL). The system was cooled to -78°C and slowly added with diisobutyl aluminium hydride (1 M, 64.3 mL) to react for 2 hours. After TLC showed that the reaction was completed, the reaction system was heated to 0°C, diluted with tetrahydrofuran (200 mL), sequentially added with water (3.9 mL), 10% sodium hydroxide aqueous solution (3.9 mL) and water (9.64 mL), transferred to room temperature and stirred for 10 minutes, then added with an appropriate amount of anhydrous sodium sulfate, and continuously stirred for 20 minutes. The mixture was filtered, the filtrate was concentrated, and the obtained crude product was purified by column chromatography to obtain 5.0 g of the title compound.

MS (ESI) m/z (M+H)⁺ = 214.1.

### Step 5: preparation of 2-(2-chloro-6-methoxypyridine-3-yl)-2-methylpropane-1-ol

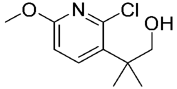

2-(2-chloro-6-methoxypyridine-3-yl)-2-methylpropanal (5.0 g) was dissolved in methanol (50 mL) and added with sodium borohydride (446.0 mg) to react at room temperature for 10 minutes. After TLC showed that the reaction was completed, the reaction system was concentrated, and the obtained crude product was purified by column chromatography to obtain 4.0 g of the title compound.

MS (ESI) m/z (M+H)⁺ = 216.1.

### Preparation Example 2 of intermediate compound: preparation of 5-bromo-6-methoxy-3,3-dimethyl-2,3-dihydrofuran[2,3-b]pyridine (available for Example 1)

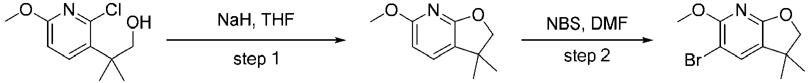

### Step 1: preparation of 6-methoxy-3,3-dimethyl-2,3-dihydrofuran[2,3-b]pyridine

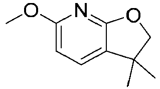

2-(2-chloro-6-methoxypyridine-3-yl)-2-methylpropane-1-ol (1.0 g) was dissolved in anhydrous tetrahydrofuran (10 mL), slowly added into an anhydrous tetrahydrofuran (10 mL) suspension of sodium hydride (220.8 mg) and heated to 60°C to react for 2 hours. After TLC showed that the reaction was completed, in ice water bath, the reaction system was dropwise added into 50 mL of saturated ammonium chloride aqueous solution, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 516 mg of the title compound.

MS (ESI) m/z (M+H)⁺ = 180.1.

### Step 2: preparation of 5-bromo-6-methoxy-3,3-dimethyl-2,3-dihydrofuran[2,3-b]pyridine

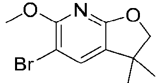

6-methoxy-3,3-dimethyl-2,3-dihydrofuran[2,3-b]pyridine (510.0 mg) was dissolved in anhydrous N,N-dimethylformamide (5 mL) and added with N-bromosuccinimide (558 mg) to react at room temperature for 20 minutes. After TLC showed that the reaction was completed, the reaction system was added with 30 mL of water, and extracted with ethyl acetate for three times, and the organic phases were combined, backwashed with pure water twice, backwashed with a saturated sodium chloride solution once, dried with anhydrous sodium sulfate, filtered and concentrated. The obtained crude product was purified by column chromatography to obtain 610.0 mg of the title compound.

MS (ESI) m/z (M+H)⁺ = 258.0, 260.0.

### Preparation Example 3 of intermediate compound: preparation of methyl (R)-2-amino-3-(7-methyl-1H-indazole-5-yl)propionate trifluoroacetate (available for Example 1)

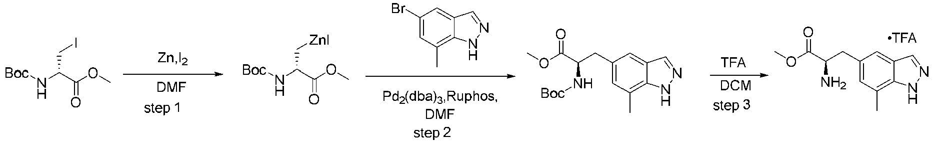

### Step 1: preparation of (S)-(2-((tert-butoxycarbonyl)amino) -3-methoxy-3-oxopropyl)zinc iodide (II)

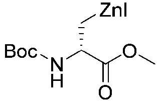

In a nitrogen atmosphere, activated zinc powder (2.37 g) was added into a 100 mL three-necked flask, and iodine (926.37 mg) was dissolved in anhydrous N,N-dimethylformamide, and dropwise added into the three-necked flask. Then, the system was placed at 60°C to react for 15 minutes. (S)-2-((tert-butoxycarbonyl)amino)-3-iodopropionic acid methyl ester (6.00 g) was dissolved in anhydrous N,N-dimethylformamide (35 mL) and slowly dropwise added into the above reaction system to react at 70°C for 2.5 hours. After the reaction was ended, the reaction system was transferred to room temperature, and the obtained reaction solution was directly used in the next step of reaction.

### Step 2: preparation of methyl (R)-2-((tert-butoxycarbonyl)amino)-3-(7-methyl-1H-indazole-5-yl)propionate

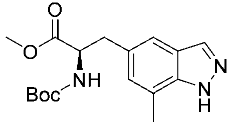

In a nitrogen atmosphere, 5-bromo-7-methyl-1H-indazole (2.26 g), tris(dibenzylideneacetone)dipalladium (489.52 mg) and 2-dicyclohexylphosphine-2',6'-diisopropoxy-1,1'-biphenyl (499.29 mg) were dissolved in anhydrous N,N-dimethylformamide (5 mL), and then the solution obtained in the step 1 was added into the reaction system and heated at 70°C to react for 3 hours. After the reaction was ended, the reaction system was filtered by diatomite, the filtrate was collected, added with 30 mL of water, and extracted with ethyl acetate, and the organic phases were combined, dried with anhydrous sodium sulfate, filtered and concentrated. The obtained crude product was separated and purified by silica gel column chromatography to obtain 2.1 g of the title compound.

MS (ESI) m/z (M+H)⁺ = 334.1.

### Step 3: preparation of methyl (R)-2-amino-3-(7-methyl-1H-indazole-5-yl)propionate trifluoroacetate

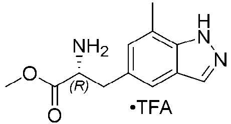

Methyl (R)-2-((tert-butoxycarbonyl)amino)-3-(7-methyl-1H-indazole-5-yl)propionate (1 g) was dissolved in dichloromethane (10 mL) and trifluoroacetic acid (10 mL) to react at room temperature for 1 hour. After the reaction was ended, the reaction system was concentrated to obtain a crude product, which could be directly used in the next step of reaction without purification.

MS (ESI) m/z (M+H)⁺ = 234.1.

### Preparation Example 4 of intermediate compound: preparation of 5-bromo-6-methoxy-3,3-dimethyl-2,3-dihydrothiophene[2,3-b]pyridine (available for Example 2)

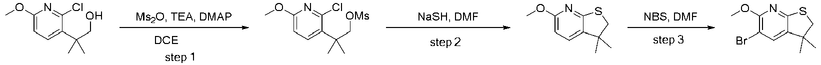

### Step 1: preparation of 2-(2-chloro-6-methoxypyridine-3-yl)-2-methylpropyl methanesulfonate

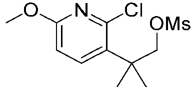

2-(2-chloro-6-methoxypyridine-3-yl)-2-methylpropane-1-ol (600.0 mg) was dissolved in 1,2-dichloroethane (10 mL), and sequentially added with triethylamine (848.4 mg), 4-dimethylaminopyridine (34.1 mg) and methanesulfonic anhydride (584.6 mg) to react at room temperature for 30 minutes. After TLC showed that the reaction was completed, the reaction system was added into 30 mL of water and extracted with dichloromethane for three times, and the organic phases were combined, backwashed with a saturated sodium chloride solution once, dried with anhydrous sodium sulfate, filtered and concentrated to obtain 820 mg of crude product.

MS (ESI) m/z (M+H)⁺ = 294.0.

### Step 2: preparation of 6-methoxy-3,3-dimethyl-2,3-dihydrothieno[2,3-b]pyridine

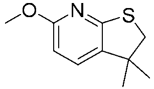

The 2-(2-chloro-6-methoxypyridine-3-yl)-2-methylpropyl methanesulfonate crude product (850.0 mg) was dissolved in N,N-dimethylformamide (10 mL), added with sodium hydrosulfide (487.4 mg), and heated at 130°C to react for 3 hours. After TLC showed that the reaction was completed, the reaction system was added with 30 mL of water, and extracted with ethyl acetate for three times, and the organic phases were combined, backwashed with pure water twice, backwashed with a saturated sodium chloride solution once, dried with anhydrous sodium sulfate, filtered and concentrated. The obtained crude product was purified by column chromatography to obtain 450.0 mg of the title compound.

MS (ESI) m/z (M+H)⁺ = 196.1.

### Step 3: preparation of 5-bromo-6-methoxy-3,3-dimethyl-2,3-dihydrothiophene[2,3-b]pyridine

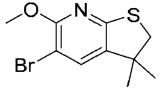

6-methoxy-3,3-dimethyl-2,3-dihydrothieno[2,3-b]pyridine (450.0 mg) was dissolved in N,N-dimethylformamide (5 mL) and added with N-bromosuccinimide (452.0 mg) to react at room temperature for 10 minutes. After TLC showed that the reaction was completed, the reaction system was added with 30 mL of water, and extracted with ethyl acetate for three times, and the organic phases were combined, backwashed with pure water twice, backwashed with a saturated sodium chloride solution once, dried with anhydrous sodium sulfate, filtered and concentrated. The obtained crude product was purified by column chromatography to obtain 300.0 mg of the title compound.

MS (ESI) m/z (M+H)⁺ = 274.0, 276.0.

### Preparation Example 5 of intermediate compound: preparation of methyl (R)-3-(7-methyl-1H-indazole-5-yl)-2-((phenoxycarbonyl)amino)propionate (available for Example 2)

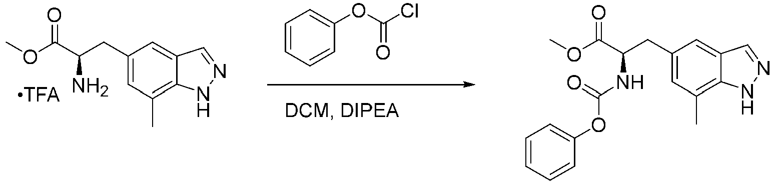

In ice water bath, methyl (R)-2-amino-3-(7-methyl-1H-indazole-5-yl)propionate trifluoroacetate (990 mg) was dissolved in dichloromethane (20 mL) and dropwise added with N,N-diisopropylethylamine (1.16 g). Then, phenyl chloroformate (469.71 mg) was dissolved in dichloromethane (10 mL) and dropwise added into the above reaction system to react for 1 hour. After the reaction was ended, the reaction solution was poured into water and extracted with ethyl acetate, and the organic phases were combined, washed with a saturated sodium chloride aqueous solution, dried with anhydrous sodium sulfate, filtered and concentrated. The obtained crude product was separated and purified by silica gel column chromatography to obtain 1 g of the title compound.

MS (ESI) m/z (M+H)⁺ = 354.1.

### Preparation Example 6 of intermediate compound: preparation of 4-(2-chloro-6-methoxypyridine-3-yl)butan-1-ol (available for Preparation Example 7 of intermediate compound)

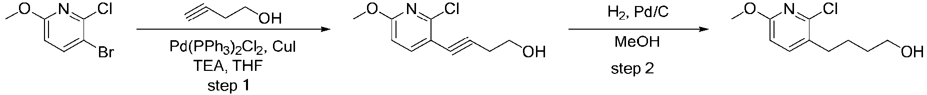

### Step 1: preparation of 4-(2-chloro-6-methoxypyridine-3-yl)butan-3-yn-1-ol

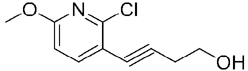

3-bromo-2-chloro-6-methoxypyridine (3.0 g) was dissolved in tetrahydrofuran (20 mL) and triethylamine (10 mL), and 3-butyne-1-ol (2.9 g) was diluted with tetrahydrofuran (5 mL) and then put into a constant-pressure dropping funnel. After nitrogen replacement, the system was added with bis(triphenylphosphine)palladium dichloride (476.0 mg) and cuprous iodide (64.0 mg). After nitrogen replacement again, the system was heated to 50°C and slowly dropwise added with 3-butyn-1-ol at this temperature for two hours, and then to continuously react at this temperature for 1 hour. After TLC showed that the reaction was completed, the reaction system was added with 30 mL of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 1.8 g of the title compound.

MS (ESI) m/z (M+H)⁺ = 212.0.

### Step 2: preparation of 4-(2-chloro-6-methoxypyridine-3-yl)butan-1-ol

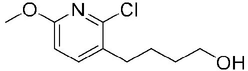

4-(2-chloro-6-methoxypyridine-3-yl)butan-3-yn-1-ol (1.8 g) was dissolved in methanol (100 mL), added with palladium carbon (10%, 180.0 mg), subjected to air replacement, and continuously introduced with hydrogen to react at room temperature for 1 hour. After TLC showed that the reaction was completed, the reaction system was filtered, and the filtrate was collected and concentrated. The obtained crude product was purified by column chromatography to obtain 1.5 g of the title compound.

MS (ESI) m/z (M+H)⁺ = 216.0.

### Preparation Example 7 of intermediate compound: preparation of 7-bromo-8-methoxy-2,3,4,5-tetrahydro-oxepino[2,3-b]pyridine (available for Example 3)

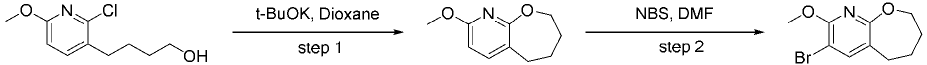

### Step 1: preparation of 8-methoxy-2,3,4,5-tetrahydro-oxepino[2,3-b]pyridine

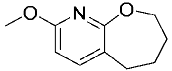

4-(2-chloro-6-methoxypyridine-3-yl)butan-1-ol (430.0 mg) was dissolved in 1,4-dioxane (6 mL), added with potassium tert-butoxide (448.0 mg) and heated to 85°C to react for 1 hour. After TLC showed that the reaction was completed, the system was added into dilute hydrochloric acid at 0°C, and a pH value of the system was adjusted to about 7. The reaction system was extracted with ethyl acetate for three times, and the organic phases were combined, backwashed with a saturated sodium chloride solution once, dried with anhydrous sodium sulfate, filtered and concentrated to obtain 0.3 g of crude product.

MS (ESI) m/z (M+H)⁺ =180.1.

### Step 2: preparation of 7-bromo-8-methoxy-2,3,4,5-tetrahydro-oxepino[2,3-b]pyridine

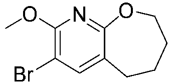

The 8-methoxy-2,3,4,5-tetrahydro-oxepino[2,3-*b*]pyridine crude product (300.0 mg) was dissolved in anhydrous *N,N*-dimethylformamide (4 mL) and added with *N*-bromosuccinimide (298.0 mg) to react at room temperature for 30 minutes. After TLC showed that the reaction was completed, the reaction system was added with 30 mL of water, and extracted with ethyl acetate for three times, and the organic phases were combined, backwashed with pure water twice, backwashed with a saturated sodium chloride solution once, dried with anhydrous sodium sulfate, filtered and concentrated. The obtained crude product was purified by column chromatography to obtain 195.0 mg of the title compound.

MS (ESI) m/z (M+H)⁺ = 258.0, 260.0.

### Preparation Example 8 of intermediate compound: preparation of 7-bromo-8-methoxy-2,3,4,5-tetrahydro-thiazepino[2,3-b]pyridine (available for Example 4)

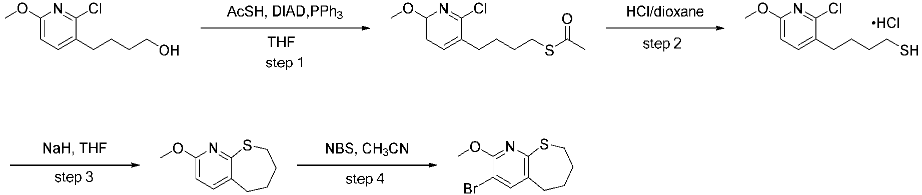

### Step 1: preparation of S-4-(2-chloro-6-methoxypyridine-3-yl)butyl)thioethyl ester

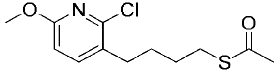

In ice water bath, 4-(2-chloro-6-methoxypyridine-3-yl)butan-1-ol (1.4 g) and triphenylphosphine (2.6 g) were dissolved in dry tetrahydrofuran (20 mL), added with diisopropyl azodiformate (2.0 g) and transferred to room temperature to react for 10 minutes. In ice water bath, the system was added with thioacetic acid (741.7 mg) and transferred to room temperature to react for 30 minutes. After TLC showed that the reaction was completed, the reaction system was added with 30 mL of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 1.0 g of the title compound.

MS (ESI) m/z (M+H)⁺ = 274.1.

### Step 2: preparation of 4-(2-chloro-6-methoxypyridine-3-yl)butane-1-thiol hydrochloride

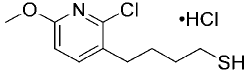

S-4-(2-chloro-6-methoxypyridine-3-yl)butyl)thioethyl ester (1.0 g) was dissolved in a 1,4-dioxane-hydrochloric acid solution (4 M, 10 mL) and heated to 40°C to react for 1 hour. After TLC showed that the reaction was completed, the system was directly concentrated to obtain 930.0 mg of crude product.

MS (ESI) m/z (M+H)⁺ = 232.0.

### Step 3: preparation of 8-methoxy-2,3,4,5-tetrahydro-thiepino[2,3-b]pyridine

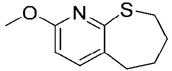

The 4-(2-chloro-6-methoxypyridine-3-yl)butane-1-thiol hydrochloride crude product (930.0 mg) was dissolved in tetrahydrofuran (20 mL), and the above system was slowly dropwise added into a tetrahydrofuran (5 mL) suspension of sodium hydride (288.0 mg) and heated to 80°C to react for 1hour. After TLC showed that the reaction was completed, in ice water bath, the system was dropwise added into 30 mL of water and extracted with ethyl acetate for three times, and the organic phases were combined, backwashed with a saturated sodium chloride solution once, dried with anhydrous sodium sulfate, filtered and concentrated to obtain 830.0 mg of crude product.

MS (ESI) m/z (M+H)⁺ = 196.1.

### Step 4: preparation of 7-bromo-8-methoxy-2,3,4,5-tetrahydro-thiepino[2,3-b]pyridine

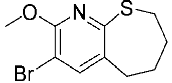

The 8-methoxy-2,3,4,5-tetrahydro-thiepino[2,3-b]pyridine crude product (800.0 mg) was dissolved in acetonitrile (9 mL) and added with N-bromosuccinimide (721.6 mg) to react at room temperature for 10 minutes. After TLC showed that the reaction was completed, the reaction system was added with 30 mL of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 336.0 mg of the title compound.

MS (ESI) m/z (M+H)⁺ = 274.0, 276.0.

### Preparation Example 9 of intermediate compound: preparation of tert-butyl 4-(2-tosylhydrazone)piperidine-1-formate (available for Example 4)

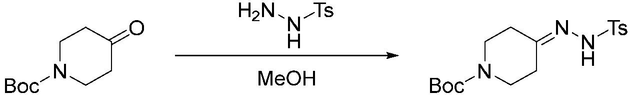

Tert-butyl 4-oxopiperidine-1-formate (4.5 g) was dissolved in methanol (50 mL), added with 4-methylbenzenesulfonyl hydrazine (4.2 g) and heated to 70°C to react for 4 hours. After TLC monitoring showed that the reaction was completed, the reaction solution was concentrated, and the crude product was purified by column chromatography to obtain 7.0 g of the title compound.

MS (ESI) m/z (M+H)⁺ = 368.1.

### Preparation Example 10 of intermediate compound: preparation of 6-bromo-7-methoxy-3,4-dihydro-2H-pyrano[2,3-b]pyridine (available for Example 5)

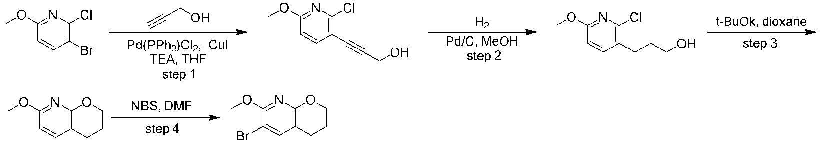

Corresponding common commercially available reagents were used to prepare the title compound by a preparation method similar to those in Preparation Example 6 of intermediate compound and Preparation Example 7 of intermediate compound.

MS (ESI) m/z (M+H)⁺ = 244.0, 246.0.

### Preparation Example 11 of intermediate compound: preparation of 6-bromo-7-methoxy-3,4-dihydro-2H-thiopyrano[2,3-b]pyridine (available for Example 6)

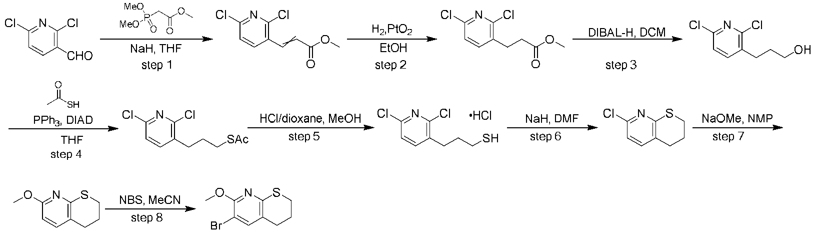

### Step 1: preparation of methyl 3-(2,6-dichloropyridine-3-yl)acrylate

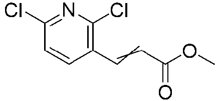

In ice water bath, in a nitrogen atmosphere, methyl 2-(dimethoxyphosphonyl)acetate (4.66 g) was slowly added into anhydrous tetrahydrofuran (10 mL) containing sodium hydride (1.02 g) to react for half an hour. An anhydrous tetrahydrofuran (20 mL) solution of 2,6-dichloronicotinaldehyde (3.00 g) was slowly dropwise added into the above system and transferred to room temperature to react for 1 hour. After LCMS showed that the reaction was completed, the system was added with an appropriate amount of water and extracted with ethyl acetate for 3 times, and the organic phases were combined, dried with anhydrous sodium sulfate, filtered and concentrated. The obtained crude product was purified by column chromatography to obtain 3.7 g of the title compound.

MS (ESI) m/z (M+H)⁺ = 231.9.

### Step 2: preparation of methyl 3-(2,6-dichloropyridine-3-yl)propionate

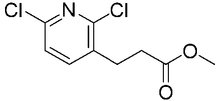

Methyl 3-(2,6-dichloropyridine-3-yl)acrylate (3.7 g) was dissolved in ethanol (40 mL), added with platinum dioxide (400 mg) and subjected to hydrogen replacement for 3 times to react at room temperature for 1 hour. After LCMS showed that the reaction of raw materials was completed, the reaction solution was filtered, and the filtrate was collected and concentrated to obtain 3.7 g of crude product.

MS (ESI) m/z (M+H)⁺ = 233.9.

### Step 3: preparation of 3-(2,6-dichloropyridine-3-yl)propan-1-ol

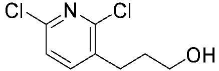

In a nitrogen atmosphere, methyl 3-(2,6-dichloropyridine-3-yl)propionate (3.7 g) was dissolved in dichloromethane (20 mL) at -20°C, slowly added with diisobutyl aluminum hydride (1.5 M, 21.09 mL) and transferred to room temperature to react for 1 hour. After LCMS showed that the reaction of raw materials was completed, the reaction solution was quenched with a saturated ammonium chloride solution (10 mL) and extracted with dichloromethane for 3 times, the organic phases were combined, dried with anhydrous sodium sulfate, filtered and concentrated. The obtained crude product was purified by column chromatography to obtain 2.2 g of the title compound.

MS (ESI) m/z (M+H)⁺ = 205.9.

### Step 4: preparation of S-(3-(2,6-dichloropyridine-3-yl)propyl)thioacetate

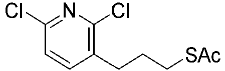

In a nitrogen atmosphere, 3-(2,6-dichloropyridine-3-yl)propan-1-ol (2.2 g) and triphenylphosphine (4.2 g) were dissolved in anhydrous tetrahydrofuran (20 mL), and sequentially added with diisopropyl azodiformate (3.24 g) and thioacetic acid (1.22 g) to react at room temperature for 1 hour. After LCMS showed that the reaction was completed, the reaction solution was concentrated. The obtained crude product was purified by column chromatography to obtain 2.0 g of the title compound.

MS (ESI) m/z (M+H)⁺ = 263.9.

### Step 5: preparation of 3-(2,6-dichloropyridine-3-yl)propane-1-thiol hydrochloride

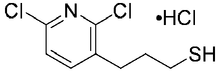

S-(3-(2,6-dichloropyridine-3-yl)propyl)thioacetate (2.0 g) was dissolved in a hydrochloric acid-1,4-dioxane solution (4 M, 20 mL) to react at room temperature overnight. After LCMS showed that the reaction of raw materials was completed, the reaction solution was concentrated to obtain 1.6 g of crude product.

MS (ESI) m/z (M+H)⁺ = 221.9.

### Step 6: preparation of 7-chloro-3,4-dihydro-2H-thiopyrano[2,3-b]pyridine

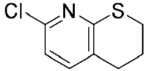

In ice water bath, in a nitrogen atmosphere, an anhydrous N,N-dimethylformamide (10 mL) solution containing sodium hydride (864.3 mg) was slowly added with an anhydrous N,N-dimethylformamide (10 mL) solution of 3-(2,6-dichloropyridine-3-yl)propane-1-thiol hydrochloride (1.6 g) to react for 1 hour. After LCMS showed that the reaction of raw materials was completed, the reaction solution was quenched with a saturated ammonium chloride solution and extracted with ethyl acetate for 3 times, and the organic phases were combined, dried with anhydrous sodium sulfate, filtered and concentrated. The obtained crude product was purified by column chromatography to obtain 1.2 g of the title compound.

MS (ESI) m/z (M+H)⁺ = 186.1.

### Step 7: preparation of 7-methoxy-3,4-dihydro-2H-thiopyrano[2,3-b]pyridine

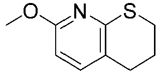

7-chloro-3,4-dihydro-2H-thiopyrano[2,3-*b*]pyridine (700 mg) and sodium methoxide (814.7 mg) were dissolved in N-methylpyrrolidone (10 mL) and heated at 110°C to react for 15 hours. After LCMS showed that the reaction of raw materials was completed, the reaction solution was added with an appropriate amount of water and extracted with ethyl acetate for 3 times, and the organic phases were combined, dried with anhydrous sodium sulfate, filtered and concentrated. The obtained crude product was purified by column chromatography to obtain 580 mg of the title compound.

MS (ESI) m/z (M+H)⁺ = 181.9.

### Step 8: preparation of 6-bromo-7-methoxy-3,4-dihydro-2H-thiopyrano[2,3-b]pyridine

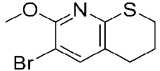

7-methoxy-3,4-dihydro-2H-thiopyrano[2,3-b]pyridine (420 mg) was dissolved in acetonitrile (4 mL), and added with N-bromosuccinimide (412.4 mg) to react at room temperature for 0.5 hour. After LCMS showed that the reaction of raw materials was completed, the reaction system was added with 30 mL of water and extracted with ethyl acetate for 3 times, and the organic phases were combined, dried with anhydrous sodium sulfate, filtered and concentrated. The obtained crude product was purified by column chromatography to obtain 580 mg of the title compound.

MS (ESI) m/z (M+H)⁺ = 260.1, 262.1.

### Preparation Example 12 of intermediate compound: preparation of 3-bromo-2-methoxy-5,5-dimethyl-6,7-dihydro-5H-cyclopentadieno[b]pyridine (available for Example 7)

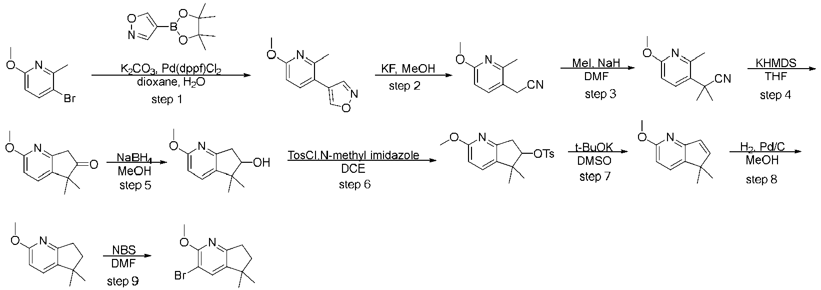

### Step 1: preparation of 4-(6-methoxy-2-methylpyridine-3-yl)isoxazole

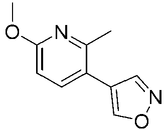

In a nitrogen atmosphere, 3-bromo-6-methoxy-2-methylpyridine (10.0 g), potassium carbonate (10.25 g) and [1,1'-bis(diphenylphosphino)ferrocene]palladium dichloride (2.54 g) were dissolved in 1,4-dioxane (70 mL) and water (20 mL). The system was heated to 90°C and dropwise added with a 4-isoxazoleboronic acid pinacol ester (9.65 g, dissolved in 80 ml of 1,4-dioxane) solution at a constant speed within 3 hours to continuously react for 1.5 hours. The system was transferred to room temperature, added with water for quenching, and extracted with ethyl acetate for three times, and the organic phases were combined, washed with a saturated sodium chloride solution, dried with anhydrous sodium sulfate, filtered and concentrated. The crude product was purified by column chromatography to obtain 7.0 g of the title compound.

MS (ESI) m/z (M+H)⁺ = 191.1.

### Step 2: preparation of 2-(6-methoxy-2-methylpyridine-3-yl)acetonitrile

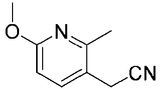

4-(6-methoxy-2-methylpyridine-3-yl)isoxazole (7.0 g) was dissolved in methanol (80 mL), added with potassium fluoride (10.7 g) and heated in an autoclave at 120°C in a closed mode to react for 4 hours. The reaction system was cooled to room temperature and concentrated to remove the solvent. The reaction system was added with water for quenching, and extracted with ethyl acetate for three times, and the organic phases were combined, washed with a saturated sodium chloride solution, dried with anhydrous sodium sulfate, filtered and concentrated. The crude product was purified by column chromatography to obtain 5.1 g of the title compound.

MS (ESI) m/z (M+H)⁺ = 163.1.

### Step 3: preparation of 2-(6-methoxy-2-methylpyridine-3-yl)-2-methylpropionitrile

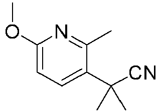

In a nitrogen atmosphere, 2-(6-methoxy-2-methylpyridine-3-yl)acetonitrile (5.1 g) was dissolved in N,N-dimethylformamide (50 mL) and added with sodium hydride (3.78 g) at -20°C to react for 10 minutes. The reaction system was added with methyl iodide (11.18 g) to continuously react for 1.5 hours. The reaction system was added with a saturated ammonium chloride solution for quenching, and extracted with ethyl acetate for three times, and the organic phases were combined, sequentially washed with water and a saturated sodium chloride solution, dried with anhydrous sodium sulfate, filtered and concentrated. The crude product was purified by column chromatography to obtain 5.8 g of the title compound.

MS (ESI) m/z (M+H)⁺ = 191.1.

### Step 4: preparation of 2-methoxy-5,5-dimethyl-5,7-dihydro-6H-cyclopentadieno[b]pyridine-6-one

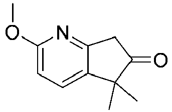

In a nitrogen atmosphere, 2-(6-methoxy-2-methylpyridine-3-yl)-2-methylpropionitrile (5.8 g) was dissolved in anhydrous tetrahydrofuran (60 mL), dropwise added with potassium bis(trimethylsilyl)amide (1 M, 45 mL) at -40°C and transferred to room temperature to react for 0.5 hour. The reaction system was added with a saturated ammonium chloride solution for quenching, and extracted with ethyl acetate for three times, and the organic phases were combined, sequentially washed with water and a saturated sodium chloride solution, dried with anhydrous sodium sulfate, filtered and concentrated. The crude product was purified by column chromatography to obtain 5.02 g of the title compound.

MS (ESI) m/z (M+H)⁺ = 192.1.

### Step 5: preparation of 2-methoxy-5,5-dimethyl-6,7-dihydro-5H-cyclopentadieno[b]pyridine-6-ol

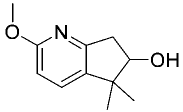

2-methoxy-5,5-dimethyl-5,7-dihydro-6*H*-cyclopentadieno[*b*]pyridine-6-one (4.3 g) was dissolved in methanol (40 mL) and added with sodium borohydride (1.08 g) in batches to react at room temperature for 1 hour. After LCMS monitoring showed that the reaction was ended, the reaction system was concentrated to remove the solvent. The reaction system was added with a saturated ammonium chloride solution for quenching, and extracted with ethyl acetate for three times, and the organic phases were combined, sequentially washed with water and a saturated sodium chloride solution, dried with anhydrous sodium sulfate, filtered and concentrated. The crude product was purified by column chromatography to obtain 4.1 g of the title compound.

MS (ESI) m/z (M+H)⁺ = 194.1.

### Step 6: preparation of 2-methoxy-5,5-dimethyl-6,7-dihydro-5H-cyclopentadieno[b]pyridine-6-yl-4-methyl benzenesulfonate

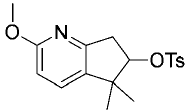

2-methoxy-5,5-dimethyl-6,7-dihydro-5*H*-cyclopentadieno[*b*]pyridine-6-ol (4.1 g) was dissolved in 1,2-dichloroethane (30 mL), sequentially added with N-methylimidazole (3.48 g) and p-toluenesulfonyl chloride (5.26 g), and heated at 70°C to react for 3 hours. The reaction system was added with water (80 mL) to stop the reaction, and extracted with dichloromethane for three times, and the organic phases were combined, sequentially washed with water and a saturated sodium chloride solution, dried with anhydrous sodium sulfate, filtered and concentrated. The crude product was purified by column chromatography to obtain 6.3 g of the title compound.

MS (ESI) m/z (M+H)⁺ = 348.1.

### Step 7: preparation of 2-methoxy-5,5-dimethyl-5H-cyclopentadieno[b]pyridine

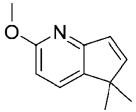

2-methoxy-5,5-dimethyl-6,7-dihydro-5*H*-cyclopentadieno[*b*]pyridine-6-yl-4-methyl benzenesulfonate (6.3 g) was dissolved in dimethyl sulfoxide (50 mL), added with potassium tert-butoxide (4.07 g) and heated at 50°C to react for 2 hours. After the reaction was ended, the system was concentrated, added with a saturated ammonium chloride solution for quenching, and extracted with ethyl acetate for three times, and the organic phases were combined, sequentially washed with water and a saturated sodium chloride solution, dried with anhydrous sodium sulfate, filtered and concentrated. The crude product was purified by column chromatography to obtain 2.85 g of the title compound.

MS (ESI) m/z (M+H)⁺ = 176.1.

### Step 8: preparation of 2-methoxy-5,5-dimethyl-6,7-dihydro-5H-cyclopentadieno[b]pyridine

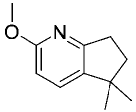

2-methoxy-5,5-dimethyl-5*H*-cyclopentadieno[*b*]pyridine (0.4 g) was dissolved in methanol (10 mL), and added with palladium carbon (10%, 100 mg). The system was subjected to air replacement and heated at 50°C in a hydrogen atmosphere to react for 2 hours. After the reaction was ended, the system was filtered, and the filtrate was collected and concentrated to obtain 400 mg of crude product.

MS (ESI) m/z (M+H)⁺ = 178.1.

### Step 9: preparation of 3-bromo-2-methoxy-5,5-dimethyl-6,7-dihydro-5H-cyclopentadieno[b]pyridine

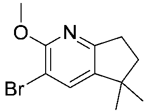

2-methoxy-5,5-dimethyl-6,7-dihydro-5*H*-cyclopentadieno[*b*]pyridine (0.4 g) was dissolved in N,N-dimethylformamide (8 mL), added with *N*-bromosuccinimide (407 mg) and heated 50°C to react for 1 hour. After the reaction was ended, the system was added with an appropriate amount of water and extracted with ethyl acetate for three times, and the organic phases were combined, sequentially washed with water and a saturated sodium chloride solution, dried with anhydrous sodium sulfate, filtered and concentrated. The crude product was purified by column chromatography to obtain 520 mg of the title compound.

MS (ESI) m/z (M+H)⁺ = 256.1, 258.1.

### Preparation Example 13 of intermediate compound: preparation of 5-bromo-6-methoxy-2,3-dihydrothieno[2,3-b]pyridine (available for Example 8)

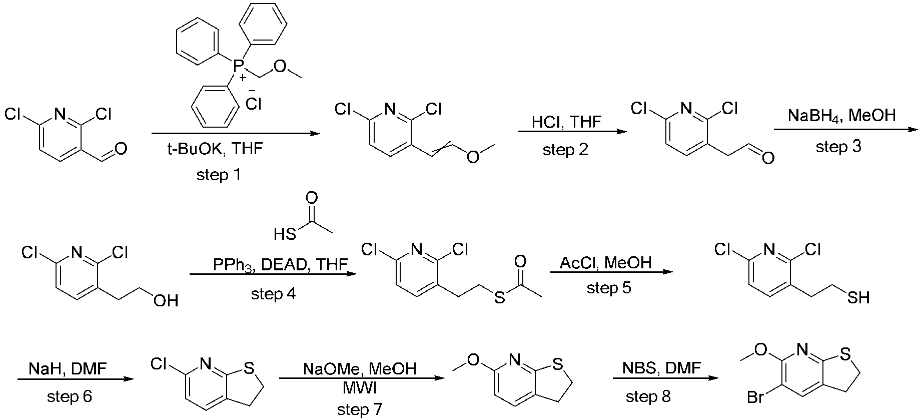

### Step 1: preparation of 2,6-dichloro-3-(2-methoxyvinyl)pyridine

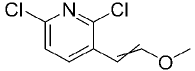

In ice water bath, in a nitrogen atmosphere, (methoxymethyl)triphenylphosphonium chloride (20.0 g) was dissolved in tetrahydrofuran (160 mL) and added with a potassium tert-butoxide (6.53 g dissolved in 58.3 mL of tetrahydrofuran) solution to react for 30 minutes. The reaction system was dropwise added with a 2,6-dichloronicotinaldehyde (6.0 g dissolved in 50 mL of tetrahydrofuran) solution to react for 20 minutes. The reaction system was transferred to room temperature to continuously react for 1 hour. The reaction system was added with water for quenching, and extracted with ethyl acetate for three times, and the organic phases were combined, washed with a saturated sodium chloride solution, dried with anhydrous sodium sulfate, filtered and concentrated. The crude product was purified by column chromatography to obtain 6.1 g of the title compound.

MS (ESI) m/z (M+H)⁺ = 204.1.

### Step 2: preparation of 2-(2,6-dichloropyridine-3-yl)acetaldehyde

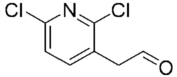

2,6-dichloro-3-(2-methoxyvinyl)pyridine (6.1 g) was dissolved in tetrahydrofuran (41 mL) and added with hydrogen chloride aqueous solution (4.0 M, 24 mL). The mixture was heated and refluxed for 3 hours. The reaction system was cooled to room temperature and concentrated to remove the solvent. The residue was dissolved in ethyl acetate, sequentially washed with a saturated sodium bicarbonate solution and a saturated sodium chloride solution, dried with anhydrous sodium sulfate, filtered and concentrated to obtain 6.1 g of crude product.

MS (ESI) m/z (M+H)⁺ = 190.1.

### Step 3: preparation of 2-(2,6-dichloropyridine-3-yl)ethanol

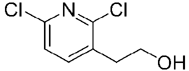

In ice water bath, the 2-(2,6-dichloropyridine-3-yl)acetaldehyde crude product (6.1 g) was dissolved in methanol (51 mL) and added with sodium borohydride (1.0 g) to react for 30 minutes. The reaction system was quenched with a saturated ammonium chloride solution and extracted with ethyl acetate for three times, and the organic phases were combined, dried with anhydrous sodium sulfate, filtered and concentrated. The crude product was purified by column chromatography to obtain 2.3 g of the title compound.

MS (ESI) m/z (M+H)⁺ = 192.1.

### Step 4: preparation of S-(2-(2,6-dichloropyridine-3-yl)ethyl)thioacetate

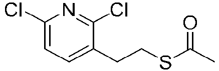

In ice water bath, 2-(2,6-dichloropyridine-3-yl)ethanol (2.3 g) and triphenylphosphine (6.3 g) were dissolved in anhydrous tetrahydrofuran (60 mL), and added with diethyl azodiformate (3.8 mL). 10 minutes later, the system was added with thioacetic acid (1.72 mL). The reaction system was transferred to room temperature to react for 2 hours. The reaction solution was diluted with n-hexane and filtered to remove precipitates, the filtrate was collected and concentrated, and the crude product was purified by column chromatography to obtain 2.5 g of the title compound.

MS (ESI) m/z (M+H)⁺ = 250.1.

### Step 5: preparation of 2-(2,6-dichloropyridine-3-yl)ethane-1-thiol

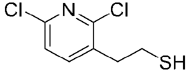

S-(2-(2,6-dichloropyridine-3-yl)ethyl)thioacetate (1.5 g) was dissolved in methanol (40 mL) and added with acetyl chloride (2.36 g) to react at room temperature for 24 hours. After LCMS monitoring showed that the reaction was ended, the reaction system was concentrated to remove the solvent, so as to obtain 1.6 g of crude product.

MS (ESI) m/z (M+H)⁺ = 208.1.

### Step 6: preparation of 6-chloro-2,3-dihydrothieno[2,3-b]pyridine

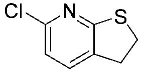

In a nitrogen atmosphere, in ice water bath, the 2-(2,6-dichloropyridine-3-yl)ethane-1-thiol crude product (1.6 g) was dissolved in N,N-dimethylformamide (30 mL) and added with sodium hydride (720 mg) to react for 1.5 hours. The reaction system was added with water (80 mL) to stop the reaction, and extracted with ethyl acetate for three times, and the organic phases were combined, sequentially washed with water and a saturated sodium chloride solution, dried with anhydrous sodium sulfate, filtered and concentrated. The crude product was purified by column chromatography to obtain 1.0 g of the title compound.

MS (ESI) m/z (M+H)⁺ = 172.1.

### Step 7: preparation of 6-methoxy-2,3-dihydrothieno[2,3-b]pyridine

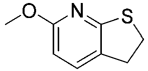

6-chloro-2,3-dihydrothieno[2,3-*b*]pyridine (1.0 g) and sodium methoxide (1.08 g) were dissolved in methanol (15 mL) and heated at 115°C to react under induction of microwave for 8 hours. After the reaction was ended, the system was concentrated, and the crude product was purified by column chromatography to obtain 730 mg of the title compound.

MS (ESI) m/z (M+H)⁺ = 168.1.

### Step 8: preparation of 5-bromo-6-methoxy-2,3-dihydrothieno[2,3-b]pyridine

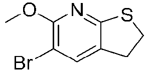

6-methoxy-2,3-dihydrothieno[2,3-*b*]pyridine (0.7 g) was dissolved in *N,N*-dimethylformamide (10 mL) and added with *N*-bromosuccinimide (0.8 g) to react for 1 hour. After the reaction was ended, the system was added into water, extracted with ethyl acetate for three times, sequentially washed with water and a saturated sodium chloride solution, dried with anhydrous sodium sulfate, filtered and concentrated. The crude product was purified by column chromatography to obtain 910 mg of the title compound.

MS (ESI) m/z (M+H)⁺ = 246.1, 248.1.

### Preparation Example 14 of intermediate compound: preparation of (R)-2-amino-3-(7-methyl-1H-indazole-5-yl)-1-(4-(1-methylpiperidine-4-yl)piperazine-1-yl)p ropan-1-one trifluoroacetate (available for Example 10)

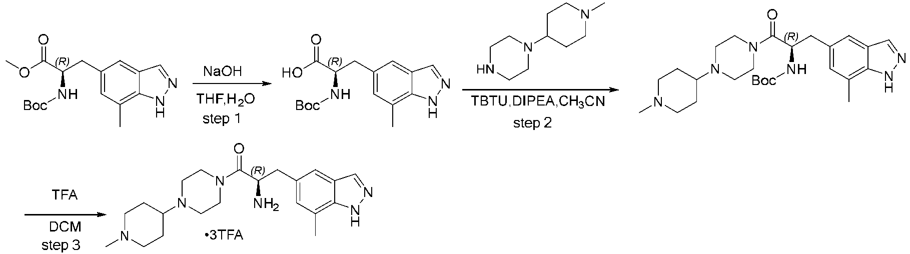

### Step 1: preparation of (R)-2-((tert-butoxycarbonyl)amino)-3-(7-methyl-1H-indazole-5-yl)propionic acid

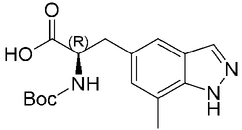

Methyl (R)-2-((tert-butoxycarbonyl)amino)-3-(7-methyl-1H-indazole-5-yl)propionate (100 mg) was dissolved in tetrahydrofuran (3 mL) and water (1 mL), and added with sodium hydroxide (120 mg) to react at room temperature overnight. After the reaction was ended, the system was added with an appropriate amount of hydrochloric acid (0.5 N) to adjust a pH value of the system to be 5-6, and extracted with ethyl acetate for three times, and the organic phases were combined, dried with anhydrous sodium sulfate, filtered and concentrated to obtain 90.0 mg of crude product.

MS (ESI) m/z (M+H)⁺ = 320.1.

### Step 2: preparation of tert-butyl (R)-(3-(7-methyl-1H-indazole-5-yl)-1-(4-(1-methylpiperidine-4-yl)piperazine-1-yl)-1-oxopropan -2-yl)carbamate

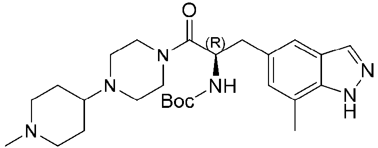

(R)-2-((tert-butoxycarbonyl)amino)-3-(7-methyl-1H-indazole-5-yl)propionic acid (50 mg), 1-(1-methylpiperidine-4-yl)piperazine (57.18 mg), 2-(1H-benzotriazol-1-yl)-1,1,3,3-tetramethylurea tetrafluoroborate (60.02 mg) and N,N-diisopropylethylamine (40.32 mg) were dissolved in acetonitrile (10 mL) to react at room temperature for 4 hours. After the reaction was ended, the reaction system was concentrated to obtain a crude product, and the crude product was purified by column chromatography to obtain 30 mg of the title compound.

MS (ESI) m/z (M+H)⁺ = 485.2.

### Step 3: preparation of (R)-2-amino-3-(7-methyl-1H-indazole-5-yl)-1-(4-(1-methylpiperidine-4-yl)piperazine-1-yl)prop an-1-one trifluoroacetate

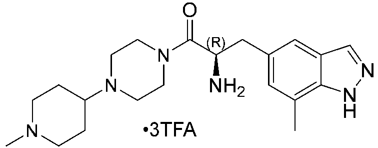

Tert-butyl (R)-(3-(7-methyl-1H-indazole-5-yl)-1-(4-(1-methylpiperidine-4-yl)piperazine-1-yl)-1-oxopropan -2-yl)carbamate (30 mg) was dissolved in dichloromethane (1 mL) and trifluoroacetic acid (0.5 mL) to react at room temperature for 1 hour. After the reaction was ended, the reaction system was concentrated to obtain a crude product, which could be used in the next step of reaction without purification.

MS (ESI) m/z (M+H)⁺ = 385.2.

### Preparation Example 15: preparation of 3-(piperidine-4-yl)-5,7-dihydrothieno[3,4-b]pyridine-2(1H)-one hydrochloride (available for Example 11)

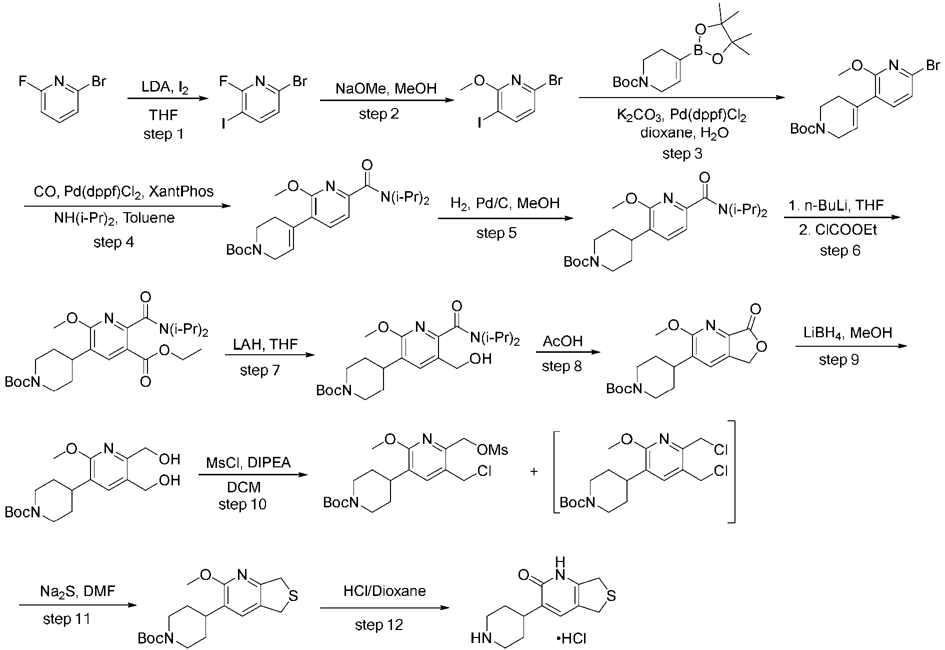

### Step 1: preparation of 6-bromo-2-fluoro-3-iodopyridine

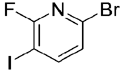

6-bromo-2-fluoropyridine (10.0 g) was dissolved in tetrahydrofuran (70 mL), subjected to nitrogen replacement, then cooled to -78°C, and dropwise added with lithium diisopropylamide (34.3 mL, 68.6 mmol) at this temperature to react at -78°C for 1 hour after the addition. The system was added with a tetrahydrofuran (50 mL) solution of iodine (14.5 g) to react at -78°C for 1.5 hours after the addition. The system was added with 10 mL of water at -60°C for quenching and transferred to room temperature, the system was added with 100 mL of water and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 8.2 g of the title compound.

MS (ESI) m/z (M+H)⁺ = 301.8.

### Step 2: preparation of 6-bromo-3-iodo-2-methoxypyridine

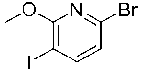

6-bromo-2-fluoro-3-iodopyridine (8.2 g) was dissolved in methanol (100 mL), added with sodium methoxide (4.4 g) and heated to 45°C to react for 1 hour. After TLC showed that the reaction was completed, the reaction system was transferred to room temperature, added with 500 mL of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 8.4 g of the title compound.

MS (ESI) m/z (M+H)⁺ =313.9.

### Step 3: preparation of tert-butyl 6-bromo-2-methoxy-3',6'-dihydro-[3,4'-bipyridine]-1'(2'H)-formate

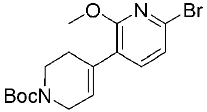

In a nitrogen atmosphere, 6-bromo-3-iodo-2-methoxypyridine (8.3 g), N-tert-butoxycarbonyl-1,2,5,6-tetrahydropyridine-4-boronic acid pinacol ester (7.8 g) and potassium carbonate (7.3 g) were dissolved in 1,4-dioxane (100 mL) and water (10 mL), and added with 1,1'-bis(diphenylphosphino)ferrocene palladium dichloride (1.9 g) to react at 70°C overnight. After TLC showed that the reaction was completed, the reaction system was added with 100 mL of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 7.3 g of the title compound.

MS (ESI) m/z (M+H)⁺ = 369.1.

### Step 4: preparation of tert-butyl 6-(diisopropyl carbamoyl)-2-methoxy-3',6'-dihydro-[3,4'-bipyridine]-1'(2'H)-formate

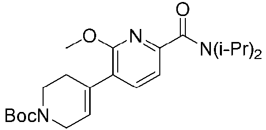

Tert-butyl 6-bromo-2-methoxy-3',6'-dihydro-[3,4'- bipyridine]-1'(2'H)-formate (1.45 g) was put into a 100 mL autoclave, and added with toluene (15 mL), diisopropylamine (1.2 g), triethylamine (2.0 g), 4,5-bis(diphenylphosphino)-9,9-dimethylxanthene (445.0 mg) and 1,1-bis(diphenylphosphine)ferrocene palladium dichloride (322.0 mg). The autoclave was sealed after the addition, carbon monoxide gas was introduced after nitrogen replacement, the pressure was kept at 13 atmospheres, and the reaction system was heated to 90°C to react for 10 hours. After TLC showed that the reaction was completed, the reaction system was added with 50 mL of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 1.5 g of the title compound.

MS (ESI) m/z (M+H)⁺ = 418.3.

### Step 5: preparation of tert-butyl 4-(6-(diisopropyl carbamoyl)-2-methoxypyridine-3-yl)piperidine-1-formate

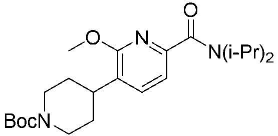

Tert-butyl 6-(diisopropyl carbamoyl)-2-methoxy-3',6'-dihydro-[3,4'-bipyridine]-1'(2'H)-formate (6.0 g) was dissolved in methanol (100 mL), added with palladium carbon (0.6 g), subjected to nitrogen replacement, and then continuously introduced with hydrogen to react at room temperature for 1 hour. After TLC showed that the reaction was completed, the system was directly filtered and concentrated to obtain 6.0 g of crude product.

MS (ESI) m/z (M+H)⁺ = 420.3.

### Step 6: preparation of ethyl 5-(1-(tert-butoxycarbonyl)piperidine-4-yl)-2-(diisopropyl carbamoyl)-6-methoxynicotinate.

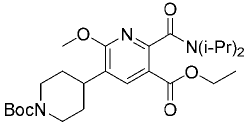

Tert-butyl 4-(6-(diisopropyl carbamoyl)-2-methoxypyridine-3-yl)piperidine-1-formate (5.8 g) was dissolved in anhydrous tetrahydrofuran (60 mL), subjected to nitrogen replacement, then cooled to -78°C, and dropwise added with a hexane solution of n-butyl lithium (2.5 M, 6.6 mL) to react for 0.5 hour. The above system was slowly dropwise added into a tetrahydrofuran solution (40 mL) of ethyl chloroformate (7.5 g) at -78°C to react for 0.5 hour. After TLC showed that the reaction was completed, the reaction system was transferred to room temperature, added into 50 mL of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 3.2 g of the title compound.

MS (ESI) m/z (M+H)⁺ = 492.3.

### Step 7: preparation of tert-butyl 4-(6-(diisopropyl carbamoyl)-5-(hydroxymethyl)-2-methoxypyridine-3-yl)piperidine-1-formate

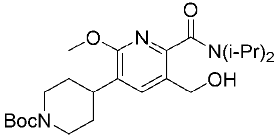

In ice water bath, ethyl 5-(1-(tert-butoxycarbonyl)piperidine-4-yl)-2-(diisopropyl carbamoyl)-6-methoxynicotinate (1.0 g) was dissolved in anhydrous tetrahydrofuran (5 mL), and slowly dropwise added into an anhydrous tetrahydrofuran (5 mL) suspension of lithium aluminum tetrahydroxide (77 mg) to react for 20 minutes. After TLC showed that the reaction was completed, the system was sequentially added with 77 µL of water, 77 µL of 15% NaOH aqueous solution and 231 µL of water in ice water bath, transferred to room temperature and stirred for 10 minutes, then added with an appropriate amount of anhydrous sodium sulfate, and stirred at room temperature for 10 minutes. The reaction system was filtered and concentrated to obtain 0.9 g of crude product.

MS (ESI) m/z (M+H)⁺ = 450.3.

### Step 8: preparation of tert-butyl 4-(2-methoxy-7-oxo-5,7-dihydrofuro[3,4-b]pyridine-3-yl)piperidine-1-formate

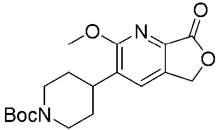

The tert-butyl 4-(6-(diisopropyl carbamoyl)-5-(hydroxymethyl)-2-methoxypyridine-3-yl)piperidine-1-formate crude product (0.9 g) was dissolved in acetic acid (9 mL), and heated to 90 °C to react for 2 hours. After TLC showed that the reaction was completed, the system was concentrated, and the obtained crude product was purified by column chromatography to obtain 502.0 mg of the title compound.

MS (ESI) m/z (M+H)⁺ = 349.2.

### Step 9: preparation of tert-butyl 4-(5,6-bis(hydroxymethyl)-2-methoxypyridine-3-yl)piperidine-1-formate

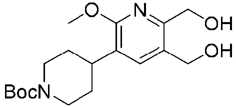

Tert-butyl 4-(2-methoxy-7-oxo-5,7-dihydrofuro[3,4-*b*]pyridine-3-yl)piperidine-1-formate (300.0 mg) was dissolved in methanol (3 mL), added with lithium borohydride (56.0 mg), and heated to 50°C to react for 2 hours. After TLC showed that the reaction was completed, the reaction solution was added with 1 M dilute hydrochloric acid to adjust pH to be neutral, the system was concentrated, and the obtained crude product was purified by column chromatography to obtain 210.0 mg of the title compound.

MS (ESI) m/z (M+H)⁺ = 353.2.

### Step 10: preparation of tert-butyl 4-(5-(chloromethyl)-2-methoxy-6-((methylsulfonyl)oxymethyl)pyridine-3-yl)piperidine-1-forma te mixture

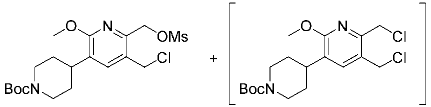

Methanesulfonyl chloride (195.0 mg) was dissolved in dichloromethane (2 mL), added with a dichloromethane (2 mL) mixed solution of tert-butyl 4-(5,6-bis(hydroxymethyl)-2-methoxypyridine-3-yl)piperidine-1-formate (150.0 mg) and *N,N*-diisopropylethylamine (219.0 mg) in ice water bath, stirred for 20 minutes, and transferred to room temperature to react for 1 hour. After TLC showed that the reaction was completed, the system was concentrated, and the obtained crude product was purified by column chromatography to obtain 180.0 mg of mixture.

MS (ESI) m/z (M+H)⁺ = 449.1 and 389.1.

### Step 11: preparation of tert-butyl 4-(2-methoxy-5,7-dihydrothieno[3,4-b]pyridine-3-yl)piperidine-1-formate

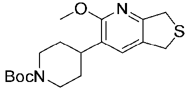

In a nitrogen atmosphere, the tert-butyl 4-(5-(chloromethyl)-2-methoxy-6-((methylsulfonyl)oxymethyl)pyridine-3-yl)piperidine-1-forma te mixture (200.0 mg) was dissolved in *N,N*-dimethylformamide (5 mL), and slowly dropwise added with an *N,N*-dimethylformamide (2 mL) suspension of sodium sulfide (37.0 mg) to react at room temperature for 1 hour. After TLC showed that the reaction was completed, the reaction system was added with 30 mL of water, and extracted with ethyl acetate for three times, and the organic phases were combined, backwashed with pure water twice, backwashed with a saturated sodium chloride solution once, dried with anhydrous sodium sulfate, filtered and concentrated. The obtained crude product was purified by column chromatography to obtain 67.0 mg of the title compound.

MS (ESI) m/z (M+H)⁺ =351.2.

### Step 12: preparation of 3-(piperidine-4-yl)-5,7-dihydrothieno[3,4-b]pyridine-2(1H)-one hydrochloride

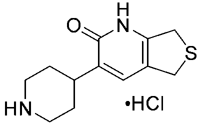

Tert-butyl 4-(2-methoxy-5,7-dihydrothieno[3,4-*b*]pyridine-3-yl)piperidine-1-formate (67.0 mg) was dissolved in a 1,4-dioxane solution (4 M, 5 mL) of hydrochloric acid, and heated to 90°C to react for 3 hours. After TLC showed that the reaction was completed, the system was concentrated to obtain 51.0 mg of crude product.

MS (ESI) m/z (M+H)⁺ = 237.1.

### Preparation Example 16: preparation of 7-(piperidine-4-yl)-3,4-dihydro-2H-thiopyrano[3,2-b]pyridine-6(5H)-one hydrochloride (available for Example 12)

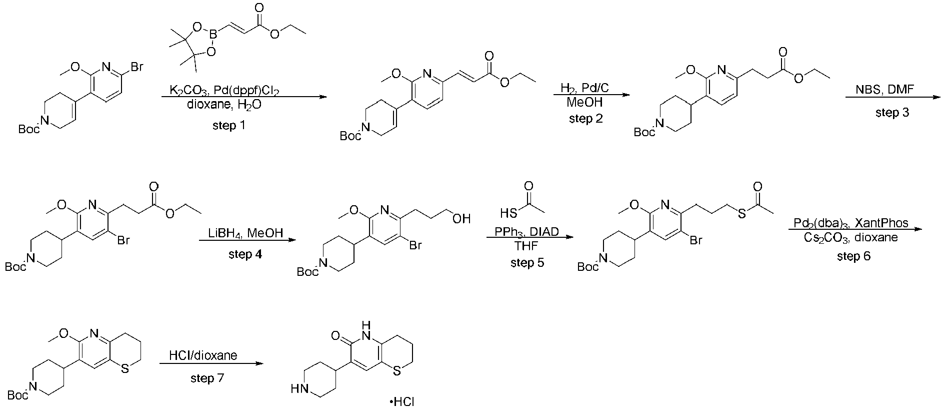

### Step 1: preparation of tert-butyl (E)-6-(3-ethoxy-3-oxopropan-1-ene-1-yl)-2-methoxy-3',6'-dihydro-[3,4'-bipyridine]-1'(2'H)-form ate

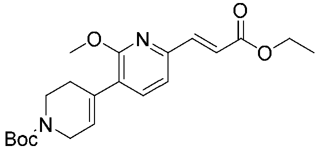

In a nitrogen atmosphere, tert-butyl 6-bromo-2-methoxy-3',6'-dihydro-[3,4'-bipyridine]-1'(2'*H*)-formate (2.0 g), ethyl (*E*)-3-(4,4,5,5-tetramethyl-1,3,2-dioxaborane-2-yl)acrylate (1.23 g), potassium carbonate (1.11 g) and [1,1'-bis(diphenylphosphine)ferrocene]palladium dichloride (II) (382 mg) were dissolved in 1,4-dioxane (15 mL) and water (1.5 mL) to react at 90°C for 3 hours. After the reaction was ended, the system was added into an appropriate amount of water and extracted with ethyl acetate (200 mL), and the organic phases were combined, washed with water and a saturated sodium chloride solution, dried with anhydrous sodium sulfate, filtered and concentrated. The obtained crude product was purified by column chromatography to obtain 2.0 g of the title compound.

MS (ESI) m/z (M+H)⁺ = 389.1.

### Step 2: preparation of tert-butyl 4-(6-(3-ethoxy-3-oxopropyl)-2-methoxypyridine-3-yl)piperidine-1-formate

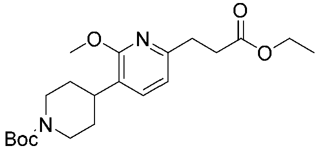

Tert-butyl (*E*)-6-(3-ethoxy-3-oxopropan-1-ene-1-yl)-2-methoxy-3',6'-dihydro-[3,4'-bipyridine]-1'(2'*H*)-form ate (2.0 g) was dissolved in methanol (30 mL), added with 10% palladium carbon (0.15 g), and heated at 55°C in a hydrogen atmosphere for 1 hour. After the reaction was ended, the palladium carbon was removed by suction filtration, the solid was washed with methanol (100 mL), and the filtrates were combined and concentrated under a reduced pressure to obtain 2.0 g of crude product. MS (ESI) m/z (M+H)⁺ = 393.1.

### Step 3: preparation of tert-butyl 4-(5-bromo-6-(3-ethoxy-3-oxopropyl)-2-methoxypyridine-3-yl)piperidine-1-formate

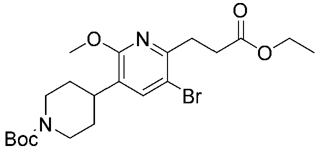

Tert-butyl 4-(6-(3-ethoxy-3-oxopropyl)-2-methoxypyridine-3-yl)piperidine-1-formate (850 mg) was dissolved in *N,N-*dimethylformamide (8 mL) and added with *N*-bromosuccinimide (386 mg) to react at room temperature for 2 hours. After the reaction was ended, the system was added into an appropriate amount of water and extracted with ethyl acetate (100 mL), and the organic phases were combined, washed with water and a saturated sodium chloride solution, dried with anhydrous sodium sulfate, filtered and concentrated to obtain 1.1 g of crude product, which was directly used in the next step of reaction.

MS (ESI) m/z (M+H)⁺ = 471.1.

### Step 4: preparation of tert-butyl 4-(5-bromo-6-(3-hydroxypropyl)-2-methoxypyridine-3-yl)piperidine-1-formate

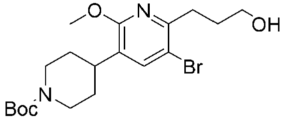

The tert-butyl 4-(5-bromo-6-(3-ethoxy-3-oxopropyl)-2-methoxypyridine-3-yl)piperidine-1-formate crude product (1.1 g) was dissolved in methanol (20 mL), and added with lithium borohydride (1.08 g) in batches to react at room temperature for 3 hours. After LCMS monitoring showed that the reaction was ended, the system was concentrated to remove the solvent, added with an appropriate amount of saturated ammonium chloride solution for quenching, and extracted with ethyl acetate for three times, and the organic phases were combined, backwashed with water and a saturated sodium chloride solution, dried with anhydrous sodium sulfate, filtered and concentrated. The obtain crude product was purified by column chromatography to obtain 770 mg of the title compound.

MS (ESI) m/z (M+H)⁺ = 429.1.

### Step 5: preparation of tert-butyl 4-(6-(3-(acetylthio)propyl)-5-bromo-2-methoxypyridine-3-yl)piperidine-1-formate

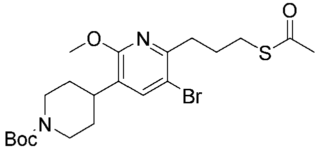

In a nitrogen atmosphere, in ice water bath, tert-butyl 4-(5-bromo-6-(3-hydroxypropyl)-2-methoxypyridine-3-yl)piperidine-1-formate (300 mg) and triphenylphosphine (368 mg) were dissolved in tetrahydrofuran (10 mL), and added with diethyl azodicarboxylate (284 mg). 10 minutes later, the system was added with thioacetic acid (106 mg) and transferred to room temperature to react for 1.5 hours. The reaction system was diluted with an appropriate amount of hexane, the white precipitate was filtered out, and the filtrate was collected and concentrated. The obtained crude product was purified by column chromatography to obtain 330 mg of the title compound.

MS (ESI) m/z (M+H)⁺ = 487.1.

### Step 6: preparation of tert-butyl 4-(6-methoxy-3,4-dihydro-2H-thiopyrano[3,2-b]pyridine-7-yl)piperidine-1-formate

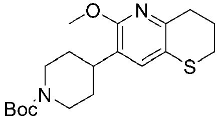

In a nitrogen atmosphere, tert-butyl 4-(6-(3-(acetylthio)propyl)-5-bromo-2-methoxypyridine-3-yl)piperidine-1-formate (330 mg), cesium carbonate (443 mg), 4,5-bis(diphenylphosphino)-9,9-dimethylxanthene (23 mg) and tris(dibenzylideneacetone)dipalladium (27 mg) were dissolved in 1,4-dioxane (3 mL), and heated at 100°C for 12 hours. After the reaction was ended, the system was added into an appropriate amount of water and extracted with ethyl acetate (20 mL), and the organic phases were combined, backwashed with water and a saturated sodium chloride solution, dried with anhydrous sodium sulfate, filtered and concentrated. The obtained crude product was purified by column chromatography to obtain 210 mg of the title compound.

MS (ESI) m/z (M+H)⁺ = 365.1.

### Step 7: preparation of 7-(piperidine-4-yl)-3,4-dihydro-2H-thiopyrano[3,2-b]pyridine-6(5H)-one hydrochloride

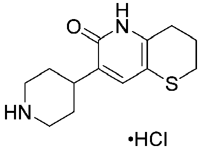

Tert-butyl 4-(6-methoxy-3,4-dihydro-2H-thiopyrano[3,2-b]pyridine-7-yl)piperidine-1-formate (210 mg) was dissolved in hydrogen chloride/1,4-dioxane solution (4.0 M, 5 mL) to react at 90°C for 5 hours. The reaction system was cooled to room temperature, and treated in vacuum to remove the solvent to obtain 230 mg of crude product.

MS (ESI) m/z (M+H)⁺ = 251.1.

### Preparation Example 17: preparation of 3-(piperidine-4-yl)-5,8-dihydro-1H-thiopyrano[3,4-b]pyridine-2(6H)-one hydrochloride (available for Example 13)

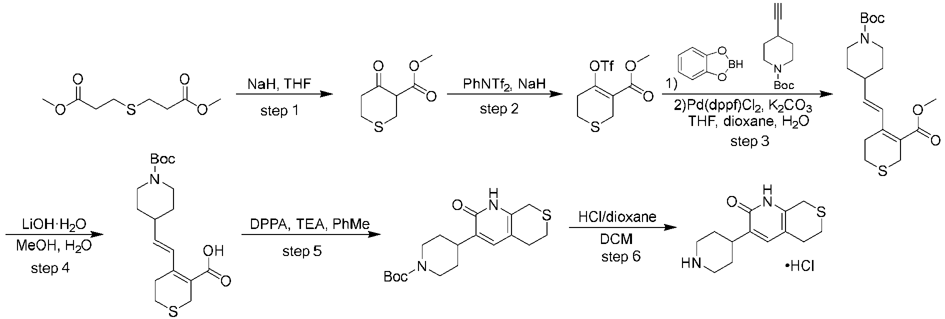

### Step 1: preparation of methyl 4-oxotetrahydro-2H-thiopyran-3-formate

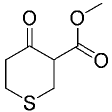

In a nitrogen atmosphere, in ice water bath, an anhydrous tetrahydrofuran (15 mL) suspension of sodium hydride (1.45 g) was slowly dropwise added with an anhydrous tetrahydrofuran (30 mL) solution of dimethyl 3,3'-thiodipropionate (5.0 g), and transferred to room temperature to react overnight. After LCMS showed that the reaction of raw materials was completed, the system was added with an appropriate amount of saturated ammonium chloride solution for quenching and extracted with ethyl acetate for 3 times, and the organic phases were combined, dried with anhydrous sodium sulfate, filtered and concentrated. The obtained crude product was purified by column chromatography to obtain 3.65 g of the title compound.

MS (ESI) m/z (M+H)⁺ = 175.0.

### Step 2: preparation of methyl 4-(((trifluoromethyl)sulfonyl)oxy)-5,6-dihydro-2H-thiopyran-3-formate

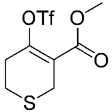

In a nitrogen atmosphere, in ice water bath, an anhydrous tetrahydrofuran (10 mL) suspension of sodium hydride (1.09 g) was slowly dropwise added with an anhydrous tetrahydrofuran (30 mL) solution of methyl 4-oxotetrahydro-2*H*-thiopyran-3-formate (3.65 g), and transferred to room temperature to react for 1 hour. The system was added with an anhydrous tetrahydrofuran (20 mL) solution of 1,1,1-trifluoro-*N*-phenyl-*N*-(trifluoromethyl)sulfonyl)methanesulfonamide (8.23 g) to react at room temperature overnight. After LCMS showed that the reaction of raw materials was completed, the system was added with an appropriate amount of saturated ammonium chloride solution for quenching and extracted with ethyl acetate for 3 times, and the organic phases were combined, dried with anhydrous sodium sulfate, filtered and concentrated. The obtained crude product was purified by column chromatography to obtain 5.2 g of the title compound.

MS (ESI) m/z (M+H)⁺ = 306.9.

### Step 3: preparation of tert-butyl (E)-4-(2-(5-(methoxycarbonyl)-3,6-dihydro-2H-thiopyran-4-yl)vinyl)piperidine-1-formate

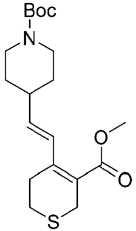

In a nitrogen atmosphere, tert-butyl 4-ethynyl piperidine-1-formate (1.0 g) was dissolved in anhydrous tetrahydrofuran (4 mL), slowly added with an anhydrous tetrahydrofuran (4 mL) solution of catecholborane (0.69 g) to react at 70°C for 4 hours, and then cooled to room temperature. Methyl 4-((trifluoromethyl)sulfonyl)oxy)-5,6-dihydro-2H-thiopyran-3-formate (1.5 g), 1,1'-bis(diphenylphosphino)ferrocene palladium dichloride (II) (183 mg) and potassium carbonate (2.03 g) were dissolved in dioxane (10 mL) and water (1 mL), slowly dropwise added into the above reaction solution, and heated to 70°C to continuously react for 1 hour. After LCMS showed that the reaction of raw materials was completed, the reaction solution was filtered, and the filtrate was collected and concentrated. The obtained crude product was purified by column chromatography to obtain 1.3 g of the title compound.

MS (ESI) m/z (M+H-100)⁺ = 267.9.

### Step 4: preparation of (E)-4-(2-(1-(tert-butoxycarbonyl)piperidine-4-yl)vinyl)-5,6-dihydro-2H-thiopyran-3-formic acid

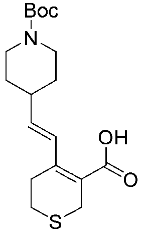

Tert-butyl (E)-4-(2-(5-(methoxycarbonyl)-3,6-dihydro-2H-thiopyran-4-yl)vinyl)piperidine-1-formate (230 mg) was dissolved in methanol (2 mL) and water (0.5 mL), and added with lithium hydroxide monohydrate (131.4 mg) to react overnight. After LCMS showed that the reaction was completed, the reaction solution was neutralized with 2 M hydrochloric acid and extracted with ethyl acetate for 3 times, and the organic phases were combined, dried with anhydrous sodium sulfate, filtered and concentrated. The obtained crude product was purified by column chromatography to obtain 200 mg of the title compound.

MS (ESI) m/z (M+H-100)⁺ = 253.9.

### Step 5: preparation of tert-butyl 4-(2-oxo-2,5,6,8-tetrahydro-1H-thiopyrano[3,4-b]pyridine-3-yl)piperidine-1-formate

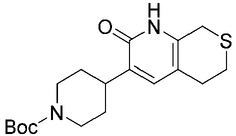

In a nitrogen atmosphere, (*E*)-4-(2-(1-(tert-butoxycarbonyl)piperidine-4-yl)vinyl)-5,6-dihydro-2*H*-thiopyran-3-formic acid (200 mg) and diphenylphosphoryl azide (155.7 mg) were dissolved in anhydrous toluene (3 mL), and added with triethylamine (85.9 mg) to react at room temperature for 2 hours. The reaction system was heated to 80°C to continuously react for 1 hour. After LCMS showed that the reaction of raw materials was completed, the reaction solution was concentrated, and the obtained crude product was purified by column chromatography to obtain 100 mg of the title compound.

MS (ESI) m/z (M+H-100)⁺ = 250.9.

### Step 6: preparation of 3-(piperidine-4-yl)-5,8-dihydro-1H-thiopyrano[3,4-b]pyridine-2(6H)-one hydrochloride

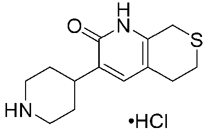

Tert-butyl 4-(2-oxo-2,5,6,8-tetrahydro-1H-thiopyrano[3,4-*b*]pyridine-3-yl)piperidine-1-formate (100 mg) was dissolved in dichloromethane (2 mL), and added with hydrochloric acid/1,4-dioxane solution (4 M, 1 mL) to react at room temperature for 1 hour. After LCMS showed that the reaction of raw materials was completed, the reaction solution was concentrated to obtain 80 mg of crude product, which was directly used in the next step of reaction.

MS (ESI) m/z (M+H)⁺ = 250.9.

### Preparation Example 18: preparation of 3-(piperidine-4-yl)-5,7-dihydrothieno[3,4-b]pyridine-2(1H)-one-7,7-d2 hydrochloride (available for Example 14)

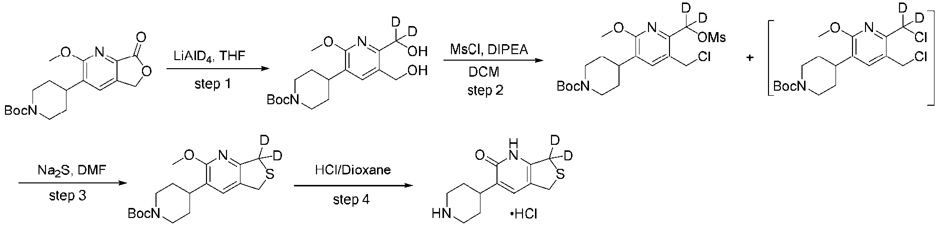

### Step 1: preparation of tert-butyl 4-(5-(hydroxymethyl)-6-(hydroxymethyl-d2)-2-methoxypyridine-3-yl)piperidine-1-formate

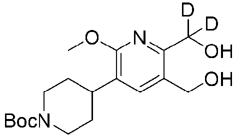

In ice water bath, tert-butyl 4-(2-methoxy-7-oxo-5,7-dihydrofuro[3,4-b]pyridine-3-yl)piperidine-1-formate (200.0 mg) was dissolved in anhydrous tetrahydrofuran (1 mL), and slowly dropwise added into an anhydrous tetrahydrofuran (1 mL) suspension of lithium aluminum deuteride (24.0 mg) to react for 20 minutes. After TLC showed that the reaction was completed, the system was sequentially added with 24 µL of water, 24 µL of 15% NaOH aqueous solution and 72 µL of water, transferred to room temperature and stirred for 10 minutes, then added with an appropriate amount of anhydrous sodium sulfate, and stirred at room temperature for 10 minutes. The reaction system was filtered, and the filtrate was collected and concentrated to obtain 166.0 mg of crude product.

MS (ESI) m/z (M+H)⁺ = 355.2.

Corresponding common commercially available reagents were used to subsequently prepare the title compound by a preparation method similar to that in Preparation Example 15 of intermediate compound above.

MS (ESI) m/z (M+H)⁺ = 239.1.

### Preparation Example 19: preparation of 3-piperidine-4-yl)-1,5,7,8-tetrahydro-2H-thiopyrano[4,3-b]pyridine-2-one hydrochloride (available for Example 15)

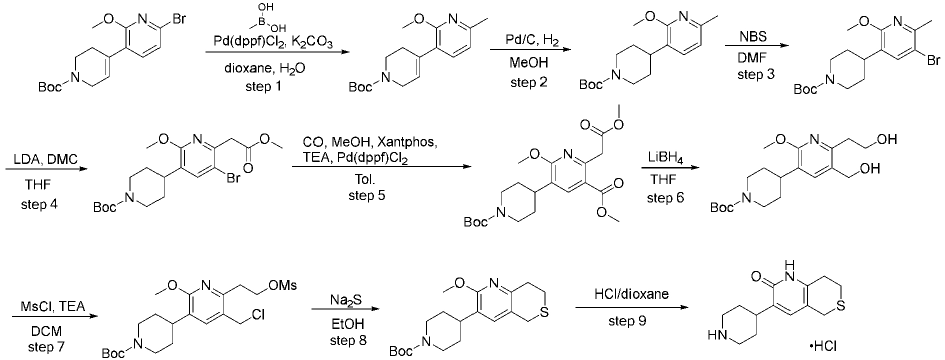

### Step 1: preparation of tert-butyl 2-methoxy-6-methyl-3',6'-dihydro-[3,4'-bipyridine]-1'(2'H)-formate

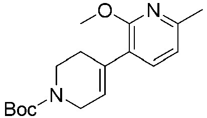

In a nitrogen atmosphere, tert-butyl 6-bromo-2-methoxy-3',6'-dihydro-[3,4'-bipyridine]-1'(2'*H*)-formate (3 g) was dissolved in 1,4-dioxane (30 mL) and water (3 mL), and added with methyl boric acid (1.5 g), potassium carbonate (2.3 g) and [1,1'-bis(di-tert-butylphosphine)ferrocene]palladium dichloride (0.53 g) to react at 80°C for 2 hours. After LCMS showed that the reaction was completed, the reaction system was transferred to room temperature, added with a small amount of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 2.4 g of the title compound.

MS (ESI) m/z (M+H)⁺ = 305.1.

### Step 2: preparation of tert-butyl 4-(2-methoxy-6-methylpyridine-3-yl)piperidine-1-formate

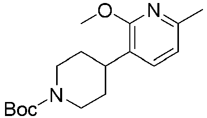

Tert-butyl 2-methoxy-6-methyl-3',6'-dihydro-[3,4'-bipyridine]-1'(2'*H*)-formate (2.4 g) was dissolved in methanol (30 mL) and added with palladium carbon (100 mg), and the reaction system was introduced with hydrogen to react at room temperature for 2 hours. After LCMS showed that the reaction was completed, the reaction solution was filtered, and the filtrate was collected and rotationally evaporated to remove excess solvent. The obtained crude product was purified by column chromatography to obtain 2.3 g of the title compound.

MS (ESI) m/z (M+H)⁺ = 307.1.

### Step 3: preparation of tert-butyl 4-(5-bromo-2-methoxy-6-methylpyridine-3-yl)piperidine-1-formate

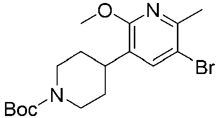

Tert-butyl 4-(2-methoxy-6-methylpyridine-3-yl)piperidine-1-formate (2.3 g) was dissolved in *N,N*-dimethylformamide (25 mL) and added with *N*-bromosuccinimide (1.6 g) to react at room temperature for 1 hour. After LCMS showed that the reaction was completed, the reaction system was added with a small amount of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 2.6 g of the title compound.

MS (ESI) m/z (M+H)⁺ = 385.0.

### Step 4: preparation of tert-butyl 4-(5-bromo-2-methoxy-6-(2-methoxy-2-oxoethyl)pyridine-3-yl)piperidine-1-formate

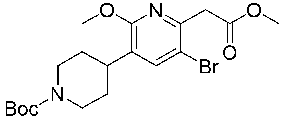

In a nitrogen atmosphere, tert-butyl 4-(5-bromo-2-methoxy-6-methylpyridine-3-yl)piperidine-1-formate (2.6 g) was dissolved in tetrahydrofuran (30 mL) at -78°C, slowly dropwise added with lithium diisopropylamine (1 M, 10.2 mL), and stirred for 20 minutes. Dimethyl carbonate (1.3 g) was dissolved in tetrahydrofuran (10 mL), slowly dropwise added into the above reaction solution, and stirred for 20 minutes. After TLC showed that the reaction was completed, the reaction solution was transferred to room temperature, added with an appropriate amount of saturated ammonium chloride aqueous solution, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 90 mg of the title compound.

MS (ESI) m/z (M+H)⁺ = 443.1.

### Step 5: preparation of methyl 5-(1-(tert-butoxycarbonyl)piperidine-4-yl)-6-methoxy-2-(2-methoxy 2-oxoethyl)nicotinate

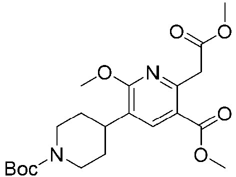

Tert-butyl 4-(5-bromo-2-methoxy-6-(2-methoxy-2-oxoethyl)pyridine-3-yl)piperidine-1-formate (1 g) was dissolved in toluene (10 mL) and methanol (1 mL), and added with triethylamine (0.7 g), 4,5-bis(diphenylphosphino)-9,9-dimethylxanthene (0.14 g) and [1,1'-bis(di-tert-butylphosphine)ferrocene]palladium dichloride (0.16 g). The reaction system was fully filled with carbon monoxide to react at 100°C for 10 hours. After LCMS showed that the reaction was completed, the reaction solution was transferred to room temperature, added with a small amount of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 750 mg of the title compound.

MS (ESI) m/z (M+H)⁺ = 423.1.

### Step 6: preparation of tert-butyl 4-(6-(2-hydroxyethyl)-5-(hydroxymethyl)-2-methoxypyridine-3-yl)piperidine-1-formate

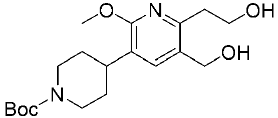

In ice water bath, methyl 5-(1-(tert-butoxycarbonyl)piperidine-4-yl)-6-methoxy-2-(2-methoxy 2-oxoethyl)nicotinate (750 mg) was dissolved in tetrahydrofuran (10 mL), added with lithium borohydride (112 mg), and transferred to room temperature to react for 1 hour. After LCMS showed that the reaction was completed, the reaction solution was added with an appropriate amount of saturated ammonium chloride aqueous solution, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 560 mg of the title compound.

MS (ESI) m/z (M+H)⁺ = 367.1.

### Step 7: preparation of tert-butyl 4-(5-(chloromethyl)-2-methoxy-6-(2-((methylsulfonyl)oxy)ethyl)pyridine-3-yl)piperidine-1-for mate

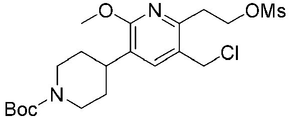

In ice water bath, tert-butyl 4-(6-(2-hydroxyethyl)-5-(hydroxymethyl)-2-methoxypyridine-3-yl)piperidine-1-formate (560 mg) was dissolved in dichloromethane (8 mL), added with triethylamine (455 mg) and methanesulfonyl chloride (431 mg) , and transferred to room temperature to react for 1 hour. After LCMS showed that the reaction was completed, the reaction solution was rotationally evaporated to remove excess solvent. The obtained crude product was purified by column chromatography to obtain 620 mg of the title compound.

MS (ESI) m/z (M+H)⁺ = 463.0.

### Step 8: preparation of tert-butyl 4-(2-methoxy-7,8-dihydro-5H-thiopyrano[4,3-b]pyridine-3-yl)piperidine-1-formate

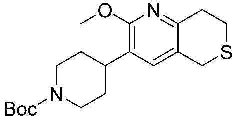

4-(5-(Chloromethyl)-2-methoxy-6-(2-((methylsulfonyl)oxy)ethyl)pyridine-3-yl)piperidine-1-for mate (180 mg) was dissolved in ethanol (20 mL) at room temperature, and added with sodium sulfide (70 mg) to react at 80°C for 0.5 hour. After LCMS showed that the reaction was completed, the reaction solution was rotationally evaporated to remove excess solvent, added with water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 110 mg of the title compound.

MS (ESI) m/z (M+H)⁺ = 365.1.

### Step 9: preparation of 3-(piperidine-4-yl)-1,5,7,8-tetrahydro-2H-thiopyrano[4,3-b]pyridine-2-one hydrochloride

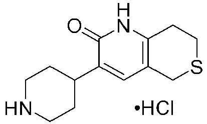

Tert-butyl 4-(2-methoxy-7,8-dihydro-5*H*-thiopyrano[4,3-*b*]pyridine-3-yl)piperidine-1-formate (100.0 mg, 0.3 mmol) was dissolved in a dioxane solution of 4 N hydrochloric acid (1 ml) at room temperature to react at 90°C for 1 hour. After LCMS monitoring showed that the reaction was completed, the reaction solution was directly spin-dried to obtain a pale yellow solid (110 mg, crude).

MS (ESI) m/z (M+H)⁺ = 251.0.

### Preparation Example 20: preparation of 6-(piperidine-4-yl)-2,3-dihydrothieno[3,2-b]pyridine-5(4H)-one hydrochloride (available for Example 17)

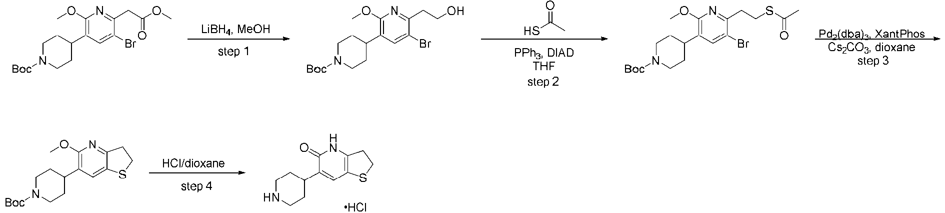

### Step 1: preparation of tert-butyl 4-(5-bromo-6-(2-hydroxyethyl)-2-methoxypyridine-3-yl)piperidine-1-formate

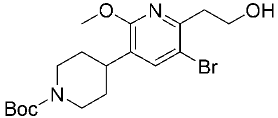

Tert-butyl 4-(5-bromo-2-methoxy-6-(2-methoxy-2-oxoethyl)pyridine-3-yl)piperidine-1-formate (120 mg) was dissolved in methanol (5 mL), and added with lithium borohydride (36 mg) in batches to react at room temperature for 3 hours. After LCMS monitoring showed that the reaction was ended, the system was treated in vacuum to remove the solvent, added with an appropriate amount of saturated ammonium chloride solution for quenching, and extracted with ethyl acetate for three times, and the organic phases were combined, backwashed with water and a saturated sodium chloride solution, dried with anhydrous sodium sulfate, filtered and concentrated. The obtain crude product was purified by column chromatography to obtain 100 mg of the title compound.

MS (ESI) m/z (M+H)⁺= 415.1.

### Step 2: preparation of tert-butyl 4-(6-(2-(acetylthio)ethyl)-5-bromo-2-methoxypyridine-3-yl)piperidine-1-formate

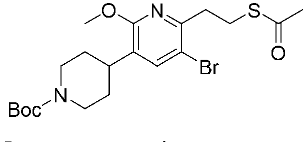

In a nitrogen atmosphere, in ice water bath, tert-butyl 4-(5-bromo-6-(2-hydroxyethyl)-2-methoxypyridine-3-yl)piperidine-1-formate (100 mg) and triphenylphosphine (131 mg) were dissolved in anhydrous tetrahydrofuran (5 mL), and added with diethyl azodiformate (101 mg). 10 minutes later, the system was added with thioacetic acid (38 mg) to react at room temperature for 1.5 hours. The reaction system was diluted with an appropriate amount of hexane, the white precipitate was filtered out, and the filtrate was collected and concentrated. The obtained crude product was purified by column chromatography to obtain 80 mg of the title compound.

MS (ESI) m/z (M+H)⁺= 473.1.

### Step 3: preparation of tert-butyl 4-(5-methoxy-2,3-dihydrothieno[3,2-b]pyridine-6-yl)piperidine-1-formate

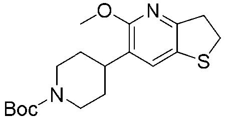

In a nitrogen atmosphere, tert-butyl 4-(6-(2-(acetylthio)ethyl)-5-bromo-2-methoxypyridine-3-yl)piperidine-1-formate (80 mg), cesium carbonate (110 mg), 4,5-bis(diphenylphosphino)-9,9-dimethylxanthene (23 mg) and tris(dibenzylideneacetone)dipalladium (27 mg) were dissolved in 1,4-dioxane (3 mL) to react at 100°C for 12 hours. After the reaction was ended, the system was added into an appropriate amount of water and extracted with ethyl acetate (10 mL), and the organic phases were combined, washed with water and a saturated sodium chloride solution, dried with anhydrous sodium sulfate, filtered and concentrated. The obtained crude product was purified by column chromatography to obtain 50 mg of the title compound.

MS (ESI) m/z (M+H)⁺= 351.1.

### Step 4: preparation of 6-(piperidine-4-yl)-2,3-dihydrothieno[3,2-b]pyridine-5(4H)-one hydrochloride

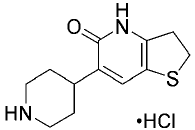

Tert-butyl 4-(5-methoxy-2,3-dihydrothieno[3,2-*b*]pyridine-6-yl)piperidine-1-formate (50 mg) was dissolved in hydrogen chloride/1,4-dioxane solution (4.0 M, 5 mL) to react at 90°C for 5 hours. The reaction system was cooled to room temperature, and treated in vacuum to remove the solvent to obtain 55 mg of crude product, which was directly used in the next step of reaction.

MS (ESI) m/z (M+H)⁺ = 237.1.

### Preparation Example 21: preparation of 6-piperazine-1-yl)-2,3,4,8-tetrahydro-7H-thiopyrano[2,3-b]pyridine-7-one hydrochloride (available for Example 18)

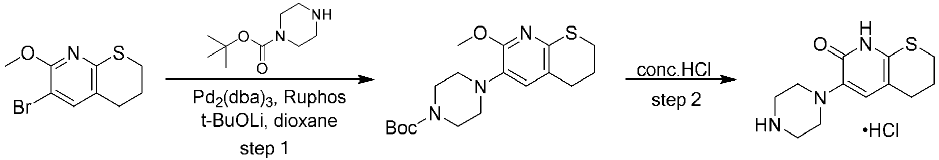

### Step 1: preparation of tert-butyl 4-(7-methoxy-3,4-dihydro-2H-thiopyrano[2,3-b]pyridine-6-yl)piperazine-1-formate

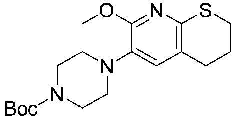

In a nitrogen atmosphere, 6-bromo-7-methoxy-3,4-dihydro-2*H*-thiopyrano[2,3-*b*]pyridine (50.0 mg), tert-butyl piperazine-1-formate (43.0 mg), tris(dibenzylideneacetone)dipalladium (17.4 mg), 2-dicyclohexylphosphine-2',6'-diisopropylbiphenyl (17.7 mg) and lithium tert-butoxide (30.4 mg) were dissolved in dioxane (1 mL) to react at 100°C for 1 hour. After LCMS showed that the reaction of raw materials was completed, the reaction solution was filtered, and the filtrate was collected and concentrated. The obtained crude product was purified by column chromatography to obtain 60.0 mg of the title compound.

MS (ESI) m/z (M+H)⁺ =365.9.

### Step 2: preparation of 6-(piperazine-1-yl)-2,3,4,8-tetrahydro-7H-thiopyrano[2,3-b]pyridine-7-one hydrochloride

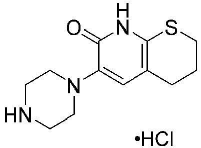

Tert-butyl 4-(7-methoxy-3,4-dihydro-2*H*-thiopyrano[2,3-*b*]pyridine-6-yl)piperazine-1-formate (60 mg) was dissolved in concentrated hydrochloric acid (2 mL) to react at 95°C for 2 hours. After LCMS showed that the reaction was completed, the reaction solution was concentrated to obtain 40 mg of crude product, which was directly used in the next step of reaction.

MS (ESI) m/z (M+H)⁺ =251.9.

### Preparation Example 22: preparation of 4'-methyl-1,4'-bipiperidine hydrochloride (available for Example 22)

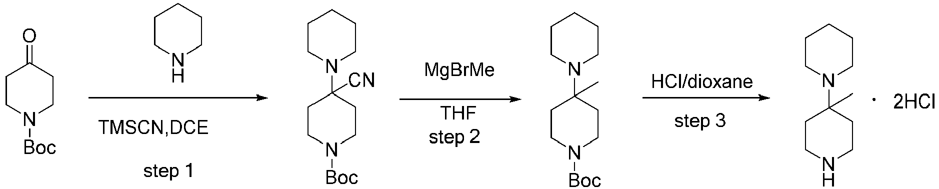

### Step 1: preparation of tert-butyl 4'-cyano-[1,4'-bipiperidine]-1'-formate

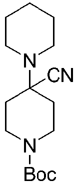

Tert-butyl 4-oxopiperidine-1-formate (2 g), piperidine (1.7 g) and trimethylsilyl cyanide (1.29 g) were dissolved in 1,2-dichloroethane (20 mL) to react at 70°C overnight. After the reaction was ended, the reaction solution was added with a small amount of water and extracted with ethyl acetate, and the organic phases were combined, washed with a saturated sodium chloride solution, dried with anhydrous sodium sulfate, filtered and concentrated. The obtained crude product was separated and purified by silica gel column chromatography to obtain 1.1 g of the title compound.

MS (ESI) m/z (M+H)⁺= 294.2.

### Step 2: preparation of tert-butyl 4'-methyl-[1,4'-bipiperidine]-1'-formate

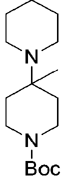

In a nitrogen atmosphere, in ice water bath, tert-butyl 4'-cyano-[1,4'-bipiperidine]-1'-formate (700 mg) was dissolved in anhydrous tetrahydrofuran (5 mL), and slowly dropwise added with a magnesium methyl bromide solution (3 N, 2.4 mL) to react for 1 hour. The reaction system was recovered to room temperature to continuously react for 1 hour. After the reaction was ended, the reaction system was added with a small amount of water for quenching and extracted with ethyl acetate, and the organic phases were combined, washed with a saturated sodium chloride solution, dried with anhydrous sodium sulfate, filtered and concentrated. The obtained crude product was separated and purified by silica gel column chromatography to obtain 480 mg of the title compound.

MS (ESI) m/z (M+H)⁺= 283.2.

### Step 3: preparation of 4'-methyl-1,4'-bipiperidine hydrochloride

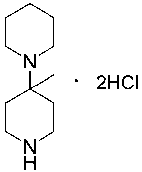

Tert-butyl 4'-methyl-[1,4'-bipiperidine]-1'-formate (100 mg) was dissolved in hydrochloric acid/1,4-dioxane solution (4 N, 4 mL) to react at room temperature for 2 hours. After the reaction was ended, the reaction system was concentrated to obtain 70 mg of crude product.

MS (ESI) m/z (M+H)⁺= 183.2.

### Preparation Example 23: preparation of 4-methoxy-3-((7-methyl-1H-indazole-5-yl)methyl)-4-oxobutyric acid (available for Example 27)

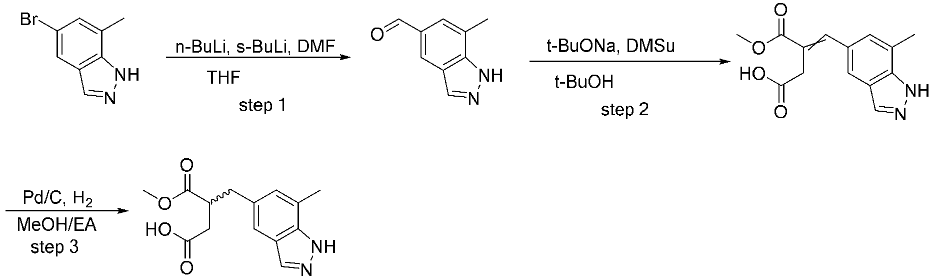

### Step 1: preparation of 7-methyl-1H-indazole-5-formaldehyde

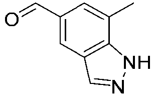

In a nitrogen atmosphere, 5-bromo-7-methyl-1H-indazole (500 mg) was dissolved in tetrahydrofuran (5 mL) at -78°C, added with n-butyl lithium (1.5 M, 2.4 mL) and sec-butyl lithium (1 M, 3.6 mL), and stirred for 1 hour. The system was dropwise added with N,N-dimethylformamide (533 mg) to continuously react for 1 hour. After LCMS showed that the reaction was completed, the reaction solution was transferred to room temperature, added with an appropriate amount of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 350 mg of the title compound.

MS (ESI) m/z (M+H)⁺ = 161.1.

### Step 2: preparation of 3-(methoxycarbonyl)-4-(7-methyl-1H-indazole-5-yl)but-3-enoic acid

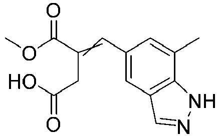

7-methyl-1*H*-indazole-5-formaldehyde (350 mg) was dissolved in tert-butyl alcohol (5 mL), and added with sodium tert-butoxide (422.4 mg) and dimethyl succinate (481.8 mg) to react at 50°C for 5 hours. After LCMS monitoring showed that the reaction was completed, the reaction solution was transferred to room temperature, added with an appropriate amount of water, and extracted with ethyl acetate for three times, and the organic phases were combined, backwashed with a saturated sodium chloride solution once, dried with anhydrous sodium sulfate, filtered and concentrated to obtain 800 mg of crude product.

MS (ESI) m/z (M+H)⁺ = 275.1

### Step 3: preparation of 4-methoxy-3-((7-methyl-1H-indazole-5-yl)methyl)-4-oxobutyric acid

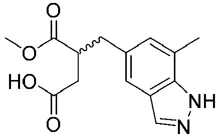

3-(methoxycarbonyl)-4-(7-methyl-1*H*-indazole-5-yl)but-3-enoic acid (800 mg) was dissolved in methanol (10 mL) and ethyl acetate (10 mL), and added with palladium carbon (100 mg). The reaction system was introduced with hydrogen to react at room temperature overnight. After LCMS monitoring showed that the reaction was completed, the reaction solution was filtered, and the filtrate was collected and rotationally evaporated to remove excess solvent to obtain 800 mg of crude product.

MS (ESI) m/z (M+H)⁺ = 277.1.

### Preparation Example 24: preparation of methyl (S)-3-(7-methyl-1H-indazole-5-yl)-2-((phenoxycarbonyl)amino)propionate (available for Example 28)

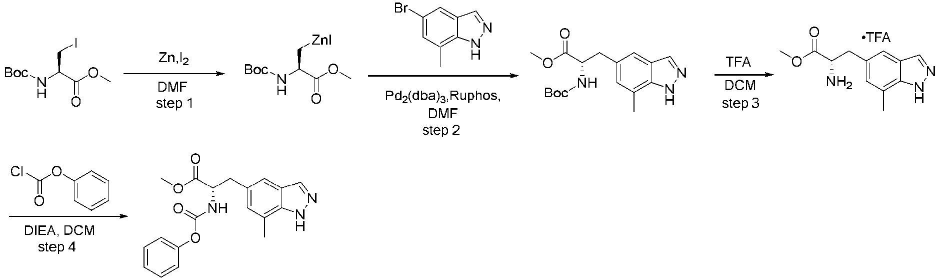

Corresponding common commercially available reagents were used to subsequently prepare the title compound by a preparation method similar to those in Preparation Example 3 of intermediate compound and Preparation Example 5 of intermediate compound above.

MS (ESI) m/z (M+H)⁺ = 354.1.

### Preparation Example 25: preparation of 1-(1-(methyl-d3)piperidine-4-yl)piperazine hydrochloride (available for Example 29)

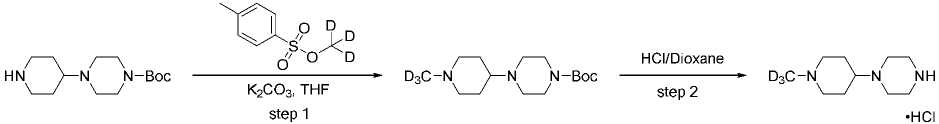

### Step 1: preparation of tert-butyl 4-(1-(methyl-d₃)piperidine-4-yl)piperazine-1-formate

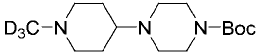

1-Boc-4-(piperidine-4-yl)-piperazine (355 mg) and potassium carbonate (365.0 mg) were dissolved in tetrahydrofuran (5 mL), and slowly dropwise added with methyl *d₃*-p-toluenesulfonate (250.0 mg) in batches to react at room temperature for 10 hours. After TLC showed that the reaction was completed, the system was filtered, and the mother solution was concentrated. The obtained crude product was purified by column chromatography to obtain 210.0 mg of the title compound.

MS (ESI) m/z (M+H)⁺ = 287.2.

### Step 2: preparation of 1-(1-(methyl-d₃)piperidine-4-yl)piperazine hydrochloride

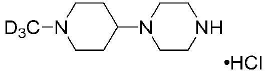

Tert-butyl 4-(1-(methyl-*d₃*) piperidine-4-yl)piperazine-1-formate (210.0 mg) was dissolved in 1,4-dioxane solution (4 M, 5 mL) of hydrochloric acid to react at room temperature for 1 hour. After TLC showed that the reaction was completed, the system was concentrated to obtain 180.0 mg of crude product.

MS (ESI) m/z (M+H)⁺ = 187.2.

### Preparation Example 26: preparation of 5'-(piperidine-4-yl)-2'H-spiro[cyclopropane-1,3'-thieno[2,3-b]pyridine]-6'(7'H)-one hydrochloride (available for Example 33)

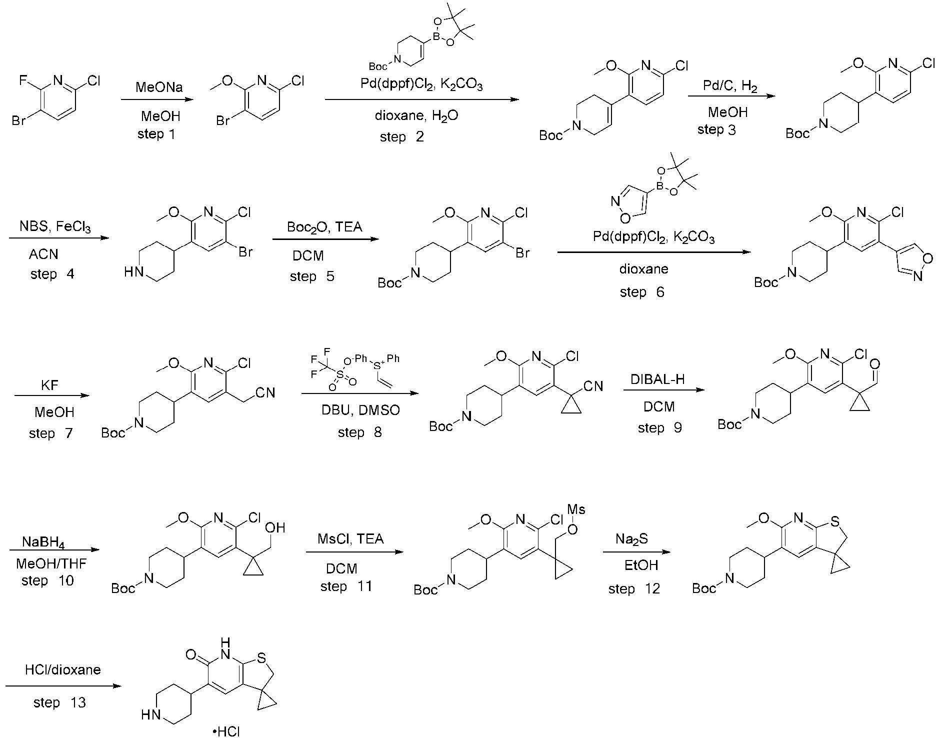

### Step 1: preparation of 3-bromo-6-chloro-2-methoxypyridine

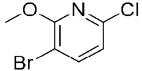

3-bromo-6-chloro-2-fluoropyridine (10 g) was dissolved in methanol (100 mL), and added with sodium methoxide (5.2 g) to react at 50°C for 2 hours. After TLC showed that the reaction was completed, the reaction solution was rotationally evaporated to remove excess solvent, added with an appropriate amount of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 10.1 g of the title compound.

### Step 2: preparation of tert-butyl 6-chloro-2-methoxy-3',6'-dihydro-[3,4'-bipyridine]-1'(2'H)-formate

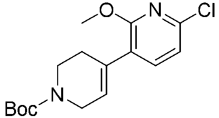

In a nitrogen atmosphere, 3-bromo-6-chloro-2-methoxypyridine (10.1 g) was dissolved in 1,4-dioxane (100 mL) and water (10 mL), and added with potassium carbonate (12.4 g), [1,1'-bis(di-tert-butylphosphine)ferrocene]palladium dichloride (3 g) and tert-butyl 4-(4,4,5,5-tetramethyl-1,3,2-dioxaborane-2-yl)-3,6-dihydropyridine-1(2*H*)-carboxylate (15.4 g) to react at 80°C for 3 hours. After LCMS showed that the reaction was completed, the reaction solution was transferred to room temperature, added with an appropriate amount of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 11.0 g of the title compound.

MS (ESI) m/z (M+H)⁺ = 325.1.

### Step 3: preparation of tert-butyl 4-(6-chloro-2-methoxypyridine-3-yl)piperidine-1-formate

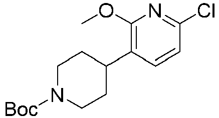

Tert-butyl 6-chloro-2-methoxy-3',6'-dihydro-[3,4'-bipyridine]-1'(2'*H*)-formate (9 g) was dissolved in methanol (50 mL) and added with palladium carbon (150 mg), and the reaction system was introduced with hydrogen to react at room temperature overnight. After LCMS showed that the reaction was completed, the reaction solution was filtered, and the filtrate was collected and rotationally evaporated to remove excess solvent. The obtained crude product was purified by column chromatography to obtain 9.0 g of the title compound.

MS (ESI) m/z (M+H)⁺ = 327.1.

### Step 4: preparation of 3-bromo-2-chloro-6-methoxy-5-(piperidine-4-yl)pyridine

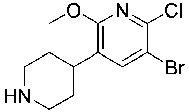

In ice water bath, tert-butyl 4-(6-chloro-2-methoxypyridine-3-yl)piperidine-1-formate (9 g) was dissolved in acetonitrile (60 mL), slowly dropwise added with a ferric trichloride (4.5 g dissolved in 10 mL of acetonitrile) solution, added with N-bromosuccinimide (4.8 g), and transferred to room temperature to react for 16 hours. After LCMS showed that the reaction was completed, the reaction solution was added with an appropriate amount of water and extracted with ethyl acetate for three times, and the organic phases were combined, backwashed with a saturated sodium thiosulfate solution and a sodium chloride solution for three times, dried with anhydrous sodium sulfate, filtered and concentrated to obtain 11 g of crude product.

MS (ESI) m/z (M+H)⁺ = 305.0.

### Step 5: preparation of tert-butyl 4-(5-bromo-6-chloro-2-methoxypyridine-3-yl)piperidine-1-formate

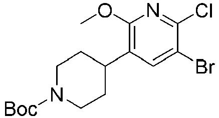

3-bromo-2-chloro-6-methoxy-5-(piperidine-4-yl)pyridine (11 g) was dissolved in dichloromethane (200 mL), and added with triethylamine (9.1 g) and di-tert-butyl dicarbonate (15.7 g) to react at room temperature for 1 hour. After LCMS showed that the reaction was completed, the reaction solution was rotationally evaporated to remove excess solvent and filtered, the solid residue was extracted with ethyl acetate for multiple times, and the filtrate was collected, washed with a saturated sodium chloride solution once, dried with anhydrous sodium sulfate, filtered and concentrated. The obtained crude product was purified by column chromatography to obtain 4.8 g of the title compound.

MS (ESI) m/z (M+H)⁺ = 405.0.

### Step 6: preparation of tert-butyl 4-(6-chloro-5-(isoxazole-4-yl)-2-methoxypyridine-3-yl)piperidine-1-formate

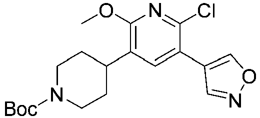

In a nitrogen atmosphere, tert-butyl 4-(5-bromo-6-chloro-2-methoxypyridine-3-yl)piperidine-1-formate(3.9 g) was dissolved in 1,4-dioxane (100 mL) and water (10 mL), and added with potassium carbonate (2.7 g), [1,1'-bis(di-tert-butylphosphine)ferrocene]palladium dichloride (650 mg) and 4-isoxazoleboronic acid pinacol ester (550 mg) to react at 80°C for 3 hours. After LCMS showed that the reaction was completed, the reaction solution was transferred to room temperature, added with a small amount of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 2.2 g of the title compound.

MS (ESI) m/z (M+H)⁺ = 394.1.

### Step 7: preparation of tert-butyl 4-(6-chloro-5-(cyanomethyl)-2-methoxypyridine-3-yl)piperidine-1-formate

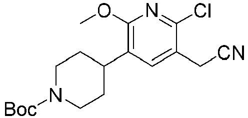

Tert-butyl 4-(6-chloro-5-(isoxazole-4-yl)-2-methoxypyridine-3-yl)piperidine-1-formate (2.2 g) was dissolved in methanol (20 mL), and added with potassium fluoride (0.67 g) to react at 90°C for 10 hours. After LCMS showed that the reaction was completed, the reaction solution was transferred to room temperature, rotationally evaporated to remove excess solvent, added with a small amount of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 2.0 g of the title compound.

MS (ESI) m/z (M+H)⁺ = 366.1.

### Step 8: preparation of tert-butyl 4-(6-chloro-5-(1-cyanocyclopropyl)-2-methoxypyridine-3-yl)piperidine-1-formate

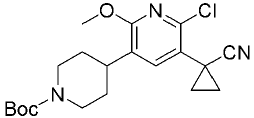

Tert-butyl 4-(6-chloro-5-(cyanomethyl)-2-methoxypyridine-3-yl)piperidine-1-formate (2 g) was dissolved in dimethyl sulfoxide (20 mL), and added with diphenyl(vinyl)sulfonium trifluoromethanesulfonate (2.4 g) and 1,8-diazabicyclo[5.4.0]undec-7-ene (2.5 g) to react at room temperature for 1 hour. After LCMS showed that the reaction was completed, the reaction solution was added with a small amount of water and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 1.8 g of the title compound.

MS (ESI) m/z (M+H)⁺ = 392.2.

### Step 9: preparation of tert-butyl 4-(6-chloro-5-(1-formylcyclopropyl)-2-methoxypyridine-3-yl)piperidine-1-formate

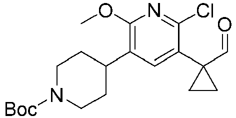

In a nitrogen atmosphere, tert-butyl 4-(6-chloro-5-(1-cyanocyclopropyl)-2-methoxypyridine-3-yl)piperidine-1-formate (1.8 g) was dissolved in dichloromethane (20 mL) at -78°C, and slowly added with diisobutyl aluminium hydride (1 M, 5 mL) to react for 1 hour. After LCMS showed that the reaction was completed, the reaction solution was transferred to room temperature, added with a small amount of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 1.4 g of the title compound.

MS (ESI) m/z (M+H)⁺ = 395.2.

### Step 10: preparation of tert-butyl 4-(6-chloro-5-(1-(hydroxymethyl)cyclopropyl)-2-methoxypyridine-3-yl)piperidine-1-formate

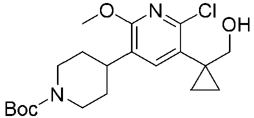

In ice water bath, tert-butyl 4-(6-chloro-5-(1-formylcyclopropyl)-2-methoxypyridine-3-yl)piperidine-1-formate (1.4 g) was dissolved in tetrahydrofuran (10 mL) and methanol (10 mL), and added with sodium borohydride (356.4 mg) to react at room temperature for 1 hour. After LCMS showed that the reaction was completed, the reaction solution was added with a small amount of water and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 1.3 g of the title compound.

MS (ESI) m/z (M+H)⁺ = 397.2.

Corresponding common commercially available reagents were used to prepare the title compound by a preparation method similar to that in Preparation Example 19 of intermediate compound above.

MS (ESI) m/z (M+H)⁺ = 263.1.

### Preparation Example 27: preparation of 3-(piperidine-1-yl)-8-azabicyclo[3.2.1]octane hydrochloride (available for Example 34)

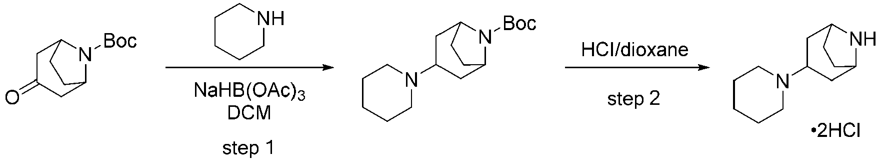

### Step 1: preparation of tert-butyl 3-(piperidine-1-yl)-8-azabicyclo[3.2.1]octane-8-formate

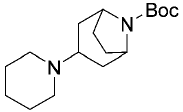

*N*-tert-butoxycarbonyl-nortropine (600 mg) and hexahydropyridine (226 mg) were dissolved in dichloromethane (10 mL), and added with sodium triacetoxyborohydride (848 mg) to react at room temperature for 3 hours. After the reaction was ended, the system was added into an appropriate amount of water and extracted with ethyl acetate (200 mL), and the organic phases were combined, washed with water and a saturated sodium chloride solution, dried with anhydrous sodium sulfate, filtered and concentrated. The obtained crude product was purified by column chromatography to obtain 430 mg of the title compound.

MS (ESI) m/z (M+H)⁺= 295.1.

### Step 2: preparation of 3-(piperidine-1-yl)-8-azabicyclo[3.2.1]octane hydrochloride

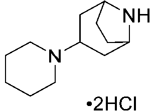

Tert-butyl 3-(piperidine-1-yl)-8-azabicyclo[3.2.1]octane-8-formate (200 mg) was dissolved in hydrogen chloride/1-4 dioxane solution (4.0 M, 5 mL) to react at room temperature for 1 hour. After monitoring showed that the reaction was completed, the reaction system was concentrated to remove the solvent to obtain 130 mg of crude product.

MS (ESI) m/z (M+H)⁺ = 195.1.

### Preparation Example 28: preparation of 5-(piperidine-1-yl)-2-azabicyclo[2.2.1]heptane hydrochloride (available for Example 36)

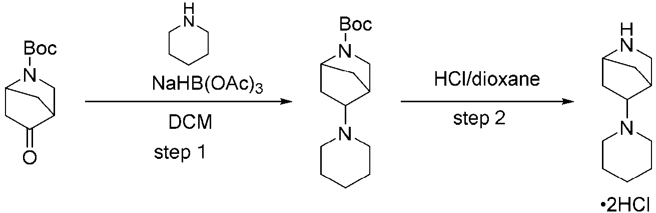

Corresponding common commercially available reagents were used to prepare the title compound by a preparation method similar to that in Preparation Example 27 of intermediate compound above.

MS (ESI) m/z (M+H)⁺ = 181.1.

### Preparation Example 29: preparation of methyl (R)-3-(7-methyl-1H-indazole-5-yl-3-d)-2-((phenoxycarbonyl)amino)propionate (available for Example 37)

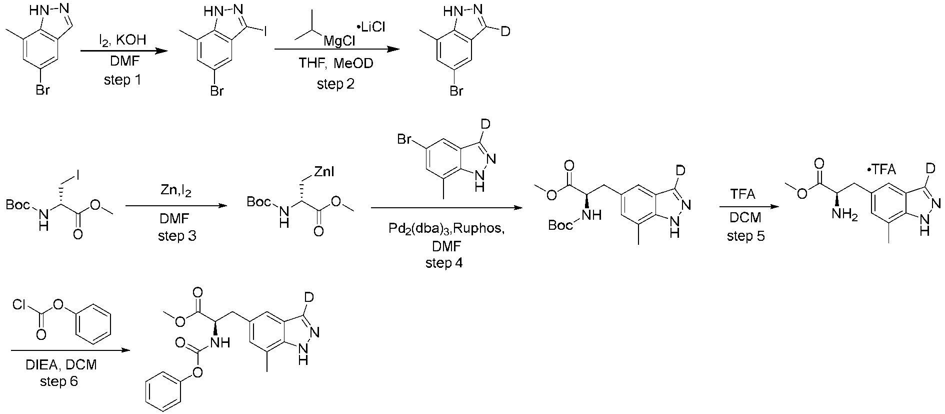

### Step 1: preparation of 5-bromo-3-iodo-7-methyl-1H-indazole

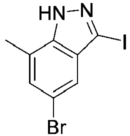

In ice water bath, 5-bromo-7-methyl-1*H*-indazole (250 mg) and potassium hydroxide (146.2 mg) were dissolved in anhydrous *N,N*-dimethylformamide (3 mL), and added with iodine (451 mg) to react for 1 hour. After LCMS showed that the reaction of raw materials was completed, the reaction solution was quenched with an appropriate amount of saturated sodium sulfite solution and extracted with ethyl acetate for 3 times, and the organic phases were combined, washed with a saturated saline solution once, dried with anhydrous sodium sulfate, filtered and concentrated. The obtained crude product was purified by column chromatography to obtain 390 mg of the title compound.

MS (ESI) m/z (M+H)⁺= 336.9.

### Step 2: preparation of 5-bromo-7-methyl-1H-indazole-3d

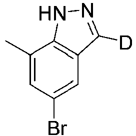

In ice water bath, in a nitrogen atmosphere, 5-bromo-3-iodo-7-methyl-1*H*-indazole (0.39 g) was dissolved in anhydrous tetrahydrofuran (4 mL), and slowly dropwise added with an isopropyl magnesium chloride-lithium chloride complex solution (1.96 mL, 1.3 M in THF) to react for 0.5 hour. The reaction system was added with deuterated methanol (1 mL) and transferred to room temperature to continuously react for 0.5 hour. After LCMS showed that the reaction of raw materials was completed, the reaction solution was concentrated, and the obtained crude product was purified by column chromatography to obtain 0.2 g of the title compound.

MS (ESI) m/z (M+H)⁺ = 211.9.

Corresponding common commercially available reagents were used to subsequently prepare the title compound by a preparation method similar to those in Preparation Example 3 of intermediate compound and Preparation Example 5 of intermediate compound above.

MS (ESI) m/z (M+H)⁺ = 354.9.

### Preparation Example 30: preparation of 2-hydroxy-3-(7-methyl-1H-indazole-5-yl)-1-(4-(1-methylpiperidine-4-yl)piperazine-1-yl)pro pan-1-one (available for Example 41)

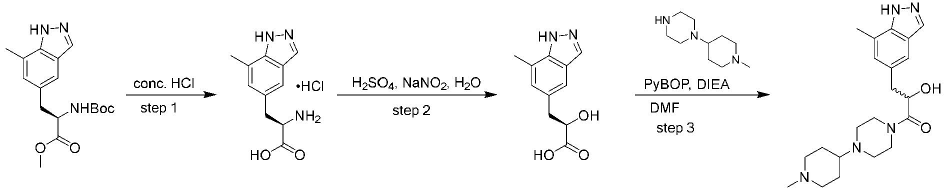

### Step 1: preparation of (R)-2-amino-3-(7-methyl-1H-indazole-5-yl)propionic acid hydrochloride

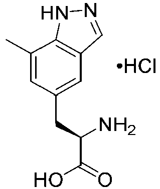

Methyl (*R*)-2-((tert-butoxycarbonyl)amino)-3-(7-methyl-1*H*-indazole-5-yl)propionate (500.0 mg) was dissolved in concentrated hydrochloric acid (3 mL) to react at 90°C for 2 hours. After LCMS showed that the reaction of raw materials was completed, the reaction solution was concentrated to obtain 300 mg of crude product, which was directly used in the next step of reaction.

MS (ESI) m/z (M+H)⁺ = 220.1.

### Step 2: preparation of (R)-2-hydroxy-3-(7-methyl-1H-indazole-5-yl)propionic acid

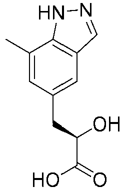

In ice water bath, (*R*)-2-amino-3-(7-methyl-1*H*-indazole-5-yl)propionic acid hydrochloride (300 mg) was dissolved in water (3 mL) and slowly dropwise added with concentrated sulfuric acid (0.45 mL). The system was added with sodium nitrite (567 mg) in batches to react at 40°C for 16 hours. After LCMS showed that the reaction was completed, The reaction solution was added with 2 N hydrochloric acid to adjust a pH value to be about 4 and extracted with ethyl acetate for 3 times, and the organic phases were combined, dried with anhydrous sodium sulfate, filtered and concentrated to obtain 200 mg of crude product, which was directly used in the next step of reaction.

MS (ESI) m/z (M+H)⁺ = 220.9.

### Step 3: preparation of 2-hydroxy-3-(7-methyl-1H-indazole-5-yl)-1-(4-(1-methylpiperidine-4-yl)piperazine-1-yl)propan -1-one

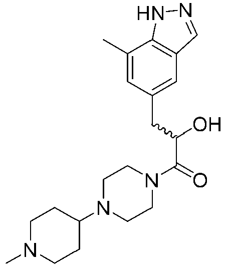

In ice water bath, (*R*)-2-hydroxy-3-(7-methyl-1*H*-indazole-5-yl)propionic acid (200 mg), 1-(1-methylpiperidine-4-yl)piperazine (332.9 mg) and *N,N*-diisopropylethylamine (234.8 mg) were dissolved in *N,N*-dimethylformamide (2.0 mL), and slowly added with 1H-benzotriazol-1-yloxytripyrrolidinophosphonium hexafluorophosphate (519.9 mg) to react for 0.5 hour. After LCMS showed that the reaction was completed, the reaction solution was concentrated, and the crude product was purified by a preparative liquid phase to obtain 100 mg of the title compound.

MS (ESI) m/z (M+H)⁺ = 385.9.

### Preparation Example 31: preparation of tert-butyl (E)-4-(2-(4,4,5,5-tetramethyl-1,3,2-dioxaborane-2-yl)vinyl)piperidine-1-formate

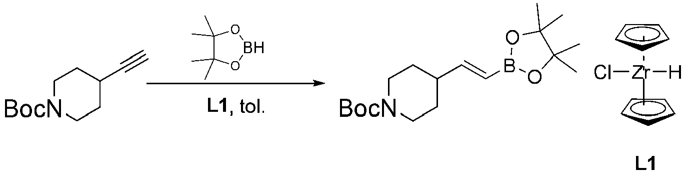

1-Boc-4- ethynyl piperidine (5.0 g) and bis(cyclopentadienyl)zirconium chloride hydride (307.32 mg) were dissolved in toluene (50 mL), dropwise added with 4,4,5,5-tetramethyl-1,3,2-dioxaborane (3.67 g), and heated at 60°C to react overnight. After the reaction was ended, the reaction solution was concentrated, the crude product was purified by column chromatography, and another configuration was removed to obtain 5.3 g of the title compound.

### Preparation Example 32: preparation of 3-piperidine-4-yl)-5,6,7,8-tetrahydro-5,8-methanoquinoline-2(1H)-one hydrochloride (available for Example 45)

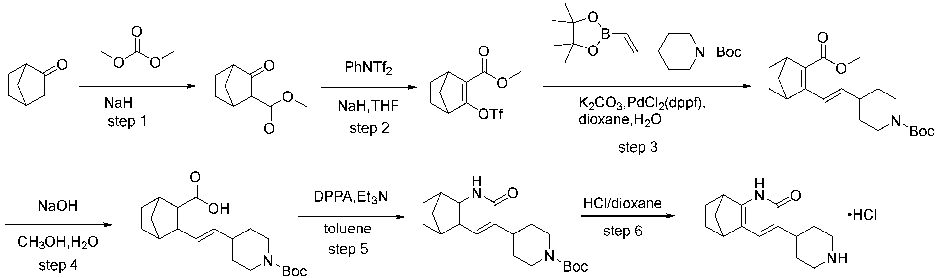

### Step 1: preparation of methyl 3-oxobicyclo[2.2.1]heptane-2-formate

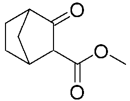

In a nitrogen atmosphere, sodium hydride (726 mg) was dissolved in dimethyl carbonate (9 mL), then bicyclo[2.2.1]heptane-2-one (1 g) was dissolved in dimethyl carbonate (6 mL), and the mixture was slowly dropwise added into a sodium hydride system to react at 90°C for 2 hours. After the reaction was ended, the reaction solution was added with water for quenching and extracted with ethyl acetate, and the organic phases were combined, dried with anhydrous sodium sulfate, filtered and concentrated to obtain a crude product. The obtained crude product was separated and purified by column chromatography to obtain 1.10 g of the title compound.

MS (ESI) m/z (M+H)⁺= 169.1 .

### Step 2: preparation of methyl 3-((trifluoromethyl)sulfonyl)oxy)bicyclo[2.2.1]hept-2-ene-2-formate

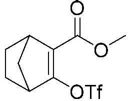

In ice water bath, sodium hydride (385 mg) was dissolved in anhydrous tetrahydrofuran (10 mL), then methyl 3-oxobicyclo[2.2.1]hept-2-formate (1.08 g) was dissolved in anhydrous tetrahydrofuran (5 mL), and the mixture was slowly dropwise added into a sodium hydride system and stirred for 30 hours. Then, N-phenyl-bis(trifluoromethanesulfonimide) (2.98 g) was dissolved in anhydrous tetrahydrofuran (10 mL) and slowly dropwise added into the above system to react at room temperature overnight. After the reaction was ended, the reaction system was added with water for quenching and extracted with ethyl acetate, and the organic phases were combined, dried with anhydrous sodium sulfate, filtered and concentrated to obtain a crude product. The obtained crude product was separated and purified by column chromatography to obtain 1.70 g of the title compound.

MS (ESI) m/z (M+H)⁺= 301.1 .

### Step 3: preparation of tert-butyl (E)-4-(2-(3-(methoxycarbonyl)bicyclo[2.2.1]hept-2-ene-2-yl)vinyl)piperidine-1-formate

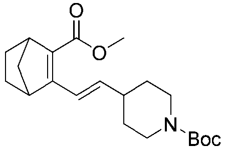

In a nitrogen atmosphere, methyl 3-((trifluoromethyl)sulfonyl)oxy)bicyclo[2.2.1]hept-2-ene-2-formate (600 mg), (E)-4-(2-(4,4,5,5-tetramethyl-1,3,2-dioxaborane-2-yl)vinyl)piperidine 1-formate (742 mg), [1,1'-bis(diphenylphosphino)ferrocene]palladium dichloride (163 mg) and potassium carbonate (690 mg) was dissolved in 1,4-dioxane (10 mL) and water (2 mL) to react at 80°C for 2 hours. After the reaction was ended, the reaction system was added with water for quenching, filtered by suction, and extracted with ethyl acetate, and the organic phases were combined, dried with anhydrous sodium sulfate, filtered and concentrated to obtain a crude product. The obtained crude product was separated and purified by column chromatography to obtain 660.00 mg of the title compound.

MS (ESI) m/z (M+H)⁺= 362.2 .

### Step 4: preparation of (E)-3-(2-(1-(tert-butoxycarbonyl)piperidine-4-yl)vinyl)bicyclo[2.2.1]hept-2-ene-2-formic acid

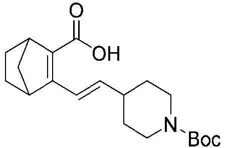

Tert-butyl (E)-4-(2-(3-(methoxycarbonyl)bicyclo[2.2.1]hept-2-ene-2-yl)vinyl)piperidine-1-formate (200 mg) was dissolved in methanol (10 mL) and water (1 mL), and added with sodium hydroxide (155 mg) to react at 50°C overnight. After the reaction was ended, the reaction system was added with 1 N hydrochloric acid to adjust to pH 5-6 and extracted with ethyl acetate, and the organic phases were combined, dried with anhydrous sodium sulfate, filtered and concentrated to obtain 180 mg of crude product.

MS (ESI) m/z (M+H)⁺ = 348.2.

### Step 5: preparation of tert-butyl 4-(2-oxo-1,2,5,6,7,8-hexahydro-5,8-methanoquinoline-3-yl)piperidine-1-formate

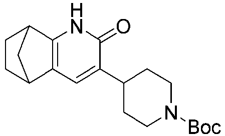

In a nitrogen atmosphere, (E)-3-(2-(1-(tert-butoxycarbonyl)piperidine-4-yl)vinyl)bicyclo[2.2.1]hept-2-ene-2-formic acid (180 mg) was dissolved in toluene (2 mL), and added with diphenylphosphoryl azide (154 mg) and triethylamine (78 mg) to react at room temperature for 3 hours. After the reaction was completed, the system was pre-purified by preparative thin layer chromatography and dissolved in toluene (2 mL) to continuously react at 90°C for 3 hours. After the reaction was ended, the reaction system was concentrated, and the crude product was separated and purified by preparative thin layer chromatography to obtain 80.00 mg of the title compound.

MS (ESI) m/z (M+H)⁺= 345.2.

### Step 6: preparation of 3-(piperidine-4-yl)-5,6,7,8-tetrahydro-5,8-methanoquinoline-2(1H)-one hydrochloride

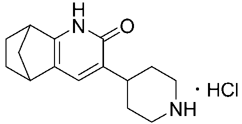

Tert-butyl 4-(2-oxo-1,2,5,6,7,8-hexahydro-5,8-methanoquinoline-3-yl)piperidine-1-formate (80 mg) was dissolved in hydrogen chloride/1,4-dioxane solution (4 M, 1 mL) to react at room temperature for 1 hour. After the reaction was ended, the reaction system was concentrated to obtain 78 mg of crude product.

MS (ESI) m/z (M+H)⁺ = 245.1

### Preparation Example 33: preparation of 6'-(piperidine-4-yl)-4',8'-dihydro-2'H,7'H-spiro[cyclopropane-1,3'-thiopyrano[2,3-b]pyridi ne]-7'-one hydrochloride (available for Example 46)

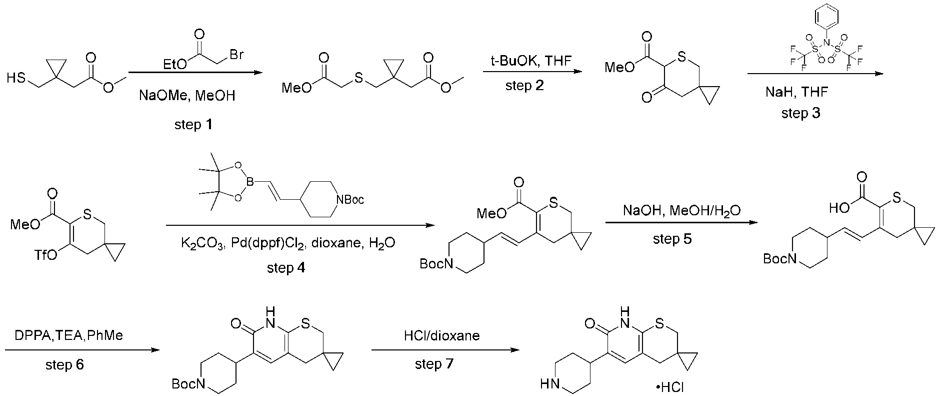

### Step 1: preparation of methyl 2-((1-(2-methoxy-2-oxoethyl)cyclopropyl)methyl)thio)acetate

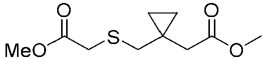

In ice water bath, methyl 2-(1-(mercaptomethyl)cyclopropyl)acetate (600 mg) was dissolved in methanol (12 mL), added with sodium methoxide (750 mg), stirred for 10 minutes, and added with ethyl bromoacetate (750 mg) to react for 2 hours. After LCMS showed that the reaction was completed, the reaction system was added with a hydrochloric acid solution (2N) to adjust to pH 7-8 and extracted with ethyl acetate, and the organic phases were combined, washed with a saturated sodium chloride solution, dried with anhydrous sodium sulfate, filtered and concentrated. The crude product was purified by column chromatography to obtain 650 mg of the title compound.

MS (ESI) m/z (M+H)+ = 233.1.

### Step 2: preparation of methyl 7-oxo-5-thiaspiro[2.5]octane-6-formate

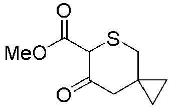

In ice water bath, methyl 2-((1-(2-methoxy-2-oxoethyl)cyclopropyl)methyl)thio)acetate (630 mg) was dissolved in tetrahydrofuran (10 mL), and added with potassium tert-butoxide (608 mg) in batches to react for 1 hour. After LCMS showed that the reaction was completed, the reaction system was added with a hydrochloric acid solution (2N) to adjust to pH 5-6 and extracted with ethyl acetate, and the organic phases were combined, washed with a saturated sodium chloride solution, dried with anhydrous sodium sulfate, filtered and concentrated. The crude product was purified by column chromatography to obtain 380 mg of the target compound.

MS (ESI) m/z (M+H)⁺ = 201.1.

Corresponding common commercially available reagents were used to subsequently prepare the title compound by a preparation method similar to that in Preparation Example 32 of intermediate compound above.

MS (ESI) m/z (M+H)⁺ = 277.1.

### Preparation Example 34: preparation of 6-(piperidine-4-yl)-1a,2,4,7b-tetrahydrocyclopropo[4,5]thiopyrano[2,3-b]pyridine-5(1H)-on e hydrochloride (available for Example 47)

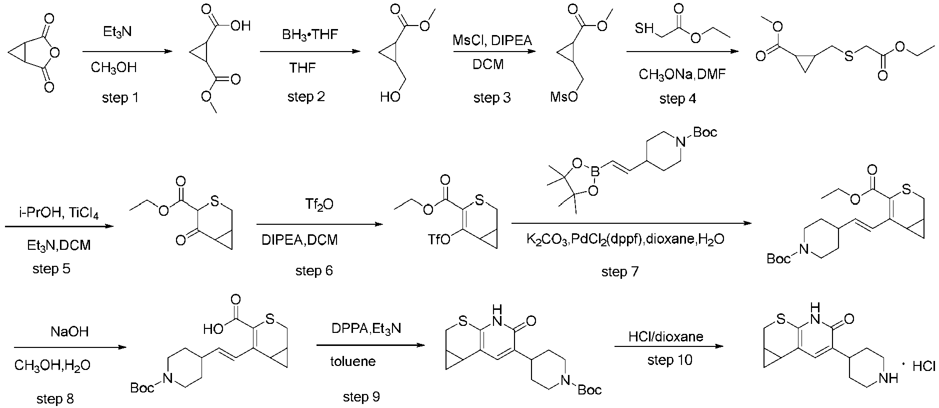

### Step 1: preparation of 2-(methoxycarbonyl)cyclopropane-1-formic acid

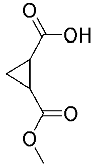

Triethylamine (901.8 mg) was dissolved in methanol (15 mL), and added with 3-oxabicyclo[3.1.0]hexane-2,4-dione (1 g) in batches to react at room temperature for 1 hour. After the reaction was ended, the reaction system was added with water for quenching to remove excess methanol, added with 1 N hydrochloric acid to adjust to pH 3-4, and extracted with ethyl acetate, and the organic phases were combined, dried with anhydrous sodium sulfate, filtered and concentrated to obtain 1.15 g of crude product.

### Step 2: preparation of methyl 2-(hydroxymethyl)cyclopropane-1-formate

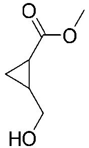

In ice water bath, 2-(methoxycarbonyl)cyclopropane-1-formic acid (1.15 g) was dissolved in anhydrous tetrahydrofuran (2 mL), dropwise added with a borane tetrahydrofuran solution (1 M, 16 mL), and transferred to room temperature to react for 3 hours. After the reaction was ended, the reaction system was added with an appropriate amount of methanol for quenching and concentrated to obtain a crude product, and the obtained crude product was separated and purified by column chromatography to obtain 750 mg of the title compound.

MS (ESI) m/z (M+H)⁺= 131.1 .

### Step 3: preparation of methyl 2-((methylsulfonyl)oxy)methyl)cyclopropane-1-formate

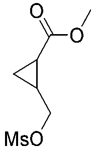

Methyl 2-(hydroxymethyl)cyclopropane-1-formate (750 mg) was dissolved in dichloromethane (10 mL) at -10°C, and added with N,N-diisopropylethylamine (1.49 g). Methanesulfonyl chloride (856 mg) was diluted with dichloromethane (3 mL), dropwise added into the above reaction system, and recovered to room temperature to react for 30 minutes. After the reaction was ended, the reaction system was added with water for quenching and extracted with ethyl acetate, and the organic phases were combined, dried with anhydrous sodium sulfate, filtered and concentrated. The crude product was separated and purified by column chromatography to obtain 1.1 g of the title compound.

MS (ESI) m/z (M+H)⁺= 209.0.

### Step 4: preparation of methyl 2-((2-ethoxy-2-oxoethyl)thio)methyl)cyclopropane-1-formate

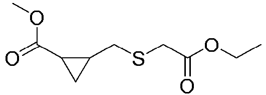

In ice water bath, in a nitrogen atmosphere, methyl 2-((methylsulfonyl)oxy)methyl)cyclopropane-1-formate (1.1 g) and ethyl 2-mercaptoacetate (936 mg) were dissolved in dry N,N-dimethylformamide (12 mL), added with sodium methoxide (421 mg), and recovered to room temperature to react for 2 hours. After the reaction was ended, the reaction system was added with water for quenching and extracted with ethyl acetate, and the organic phases were combined, dried with anhydrous sodium sulfate, filtered and concentrated. The crude product was separated and purified by column chromatography to obtain 1.10 g of the title compound.

MS (ESI) m/z (M+H)⁺= 233.0 .

### Step 5: preparation of ethyl 5-oxo-3-thiabicyclo[4.1.0]heptane-4-formate

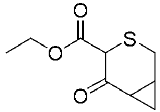

In a nitrogen atmosphere, titanium tetrachloride (1.51 g) was dissolved in dry dichloromethane (20 mL) at -10°C, added with isopropanol (518 mg), and stirred for 30 minutes. Methyl 2-((2-ethoxy-2-oxoethyl)thio)methyl)cyclopropane-1-formate (1.54 g) was dissolved in dry dichloromethane (5 mL), slowly dropwise added into the above reaction system, kept at an internal temperature of -10°C, and continuously stirred for 30 minutes. The reaction system was added with triethylamine (2.35 g) to continuously react for 1.5 hours. After the reaction was ended, the reaction system was added with an appropriate amount of 1 N hydrochloric acid for quenching and extracted with dichloromethane, and the organic phases were combined, dried with anhydrous sodium sulfate, filtered and concentrated. The crude product was separated and purified by column chromatography to obtain 740 mg of the title compound.

MS (ESI) m/z (M+H)⁺= 201.0 .

### Step 6: preparation of ethyl 5-(((trifluoromethyl)sulfonyl)oxy)-3-thiabicyclo[4.1.0]hept-4-ene-4-formate

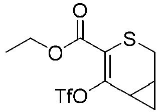

Ethyl 5-oxo-3-thiabicyclo[4.1.0]heptane-4-formate (570 mg) was dissolved in dry dichloromethane (15 mL) at -78°C, and added with N,N-diisopropylethylamine (1.1 g). Trifluoromethanesulfonic anhydride (1.36 g) was diluted with dry dichloromethane (5 mL) and dropwise added into the above system to continuously react for 30 minutes. After the reaction was ended, the reaction system was added with water for quenching and extracted with ethyl acetate, and the organic phases were combined, dried with anhydrous sodium sulfate, filtered and concentrated. The crude product was separated and purified by column chromatography to obtain 700 mg of the title compound.

MS (ESI) m/z (M+H)⁺ =333.0 .

Corresponding common commercially available reagents were used to subsequently prepare the title compound by a preparation method similar to that in Preparation Example 32 of intermediate compound above.

MS (ESI) m/z (M+H)⁺ =263.1.

### Preparation Example 35: preparation of 6-(piperidine-4-yl)-2,8-dihydro-7H-thiopyrano[2,3-b]pyridine-7-one hydrochloride (available for Example 48)

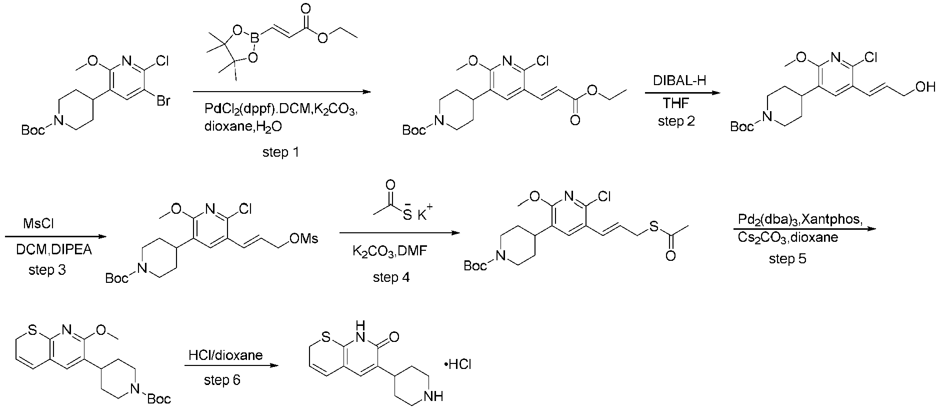

### Step 1: preparation of tert-butyl (E)-4-(6-chloro-5-(3-ethoxy-3-oxopropan-1-ene-1-yl)-2-methoxypyridine-3-yl)piperidine-1-form ate

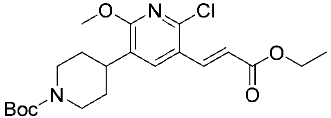

In a nitrogen atmosphere, tert-butyl 4-(5-bromo-6-chloro-2-methoxypyridine-3-yl)piperidine-1-formate (1.4 g), potassium carbonate (1.19 g) and a [1,1'-bis(diphenylphosphino)ferrocene]palladium dichloride dichloromethane complex (281 mg) were dissolved in 1,4-dioxane (30 mL) and water (5 mL). The system was heated at 90 °C, and slowly dropwise added with an ethyl (E)-3-(4,4,5,5-tetramethyl-1,3,2-dioxaborane-2-yl)acrylate (1.17 g dissolved in 8 mL of 1,4-dioxane) solution to react for 2 hours. The reaction solution was transferred to room temperature, added with a small amount of water for quenching, and filtered by suction, the filtrate was collected and extracted with ethyl acetate, and the organic phases were combined, dried with anhydrous sodium sulfate, filtered and concentrated. The crude product was separated and purified by silica gel column chromatography to obtain 1 g of the title compound.

MS (ESI) m/z (M+H)⁺ = 425.1.

### Step 2: preparation of tert-butyl (E)-4-(6-chloro-5-(3-hydroxypropan-1-alkenyl)-2-methoxypyridine-3-yl)piperidine-1-formate

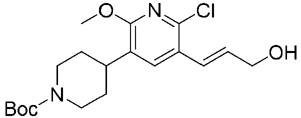

Tert-butyl (E)-4-(6-chloro-5-(3-ethoxy-3-oxopropan-1-ene-1-yl)-2-methoxypyridine-3-yl)piperidine-1-form ate (980 mg) was dissolved in dry tetrahydrofuran (20 mL) at -40°C, slowly dropwise added with diisobutyl aluminum hydride (1.5 M, 7.7 mL), and transferred to room temperature to react for 1.5 hours. The reaction solution was added with a small amount of water for quenching, then treated with a sodium hydroxide aqueous solution (2.5 N, 24 mL), and extracted with ethyl acetate, and the organic phases were combined, dried with anhydrous sodium sulfate, filtered and concentrated. The crude product was separated and purified by silica gel column chromatography to obtain 380 mg of the title compound.

MS (ESI) m/z (M+H)⁺ = 383.1.

### Step 3: preparation of tert-butyl (E)-4-(6-chloro-2-methoxy-5-(3-((methylsulfonyl)oxy)prop-1-ene-1-yl)pyridine-3-yl)piperidine-1-formate

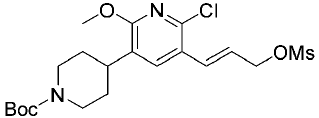

In ice water bath, tert-butyl (E)-4-(6-chloro-5-(3-hydroxypropan-1-alkenyl)-2-methoxypyridine-3-yl)piperidine-1-formate (370 mg) was dissolved in dichloromethane (10 mL), and added with N,N-diisopropylethylamine (499 mg). Methanesulfonyl chloride (172 mg) was dissolved in dichloromethane (2 mL) and slowly dropwise added into the above reaction system to react for 0.5 hour. The reaction solution was added with a small amount of water for quenching and extracted with ethyl acetate, and the organic phases were combined, dried with anhydrous sodium sulfate, filtered and concentrated to obtain 442 mg of crude product.

MS (ESI) m/z (M+H)⁺ = 461.1.

### Step 4: preparation of tert-butyl (E)-4-(5-(3-(acetylthio)prop-1-ene-1-yl)-6-chloro-2-methoxypyridine-3-yl)piperidine-1-formate

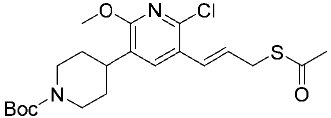

In a nitrogen atmosphere, tert-butyl (E)-4-(6-chloro-2-methoxy-5-(3-((methylsulfonyl)oxy)prop-1-ene-1-yl)pyridine-3-yl)piperidine-1-formate (445 mg) was dissolved in *N,N*-dimethylformamide (10 mL), and added with potassium carbonate (398 mg) and potassium thioacetate (329 mg) to react at room temperature for 2 hours. After the reaction was ended, the reaction system was added with a small amount of water for quenching and extracted with ethyl acetate, and the organic phases were combined, dried with anhydrous sodium sulfate, filtered and concentrated. The crude product was separated and purified by silica gel column chromatography to obtain 390 mg of the title compound.

MS (ESI) m/z (M+H)⁺ = 441.1.

### Step 5: preparation of tert-butyl 4-(7-methoxy-2H-thiopyrano[2,3-b]pyridine-6-yl)piperidine-1-formate

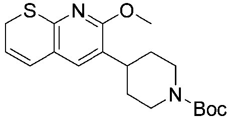

In a nitrogen atmosphere, tert-butyl (E)-4-(5-(3-(acetylthio)prop-1-ene-1-yl)-6-chloro-2-methoxypyridine-3-yl)piperidine-1-formate (150 mg), tris(dibenzylideneacetone)dipalladium (155 mg), 4,5-bis(diphenylphosphino)-9,9-dimethylxanthene (196 mg) and potassium carbonate (276 mg) were dissolved in 1,4-dioxane (10 mL) to react under induction of microwave at 115°C for 5 hours. After the reaction was ended, the reaction solution was filtered by suction through diatomite, and the filtrate was collected and concentrated. The crude product was separated and purified by a preparative high-performance liquid phase to obtain 11 mg of the title compound.

MS (ESI) m/z (M+H)⁺ = 363.1.

### Step 6: preparation of 6-(piperidine-4-yl)-2,8-dihydro-7H-thiopyrano[2,3-b]pyridine-7-one hydrochloride

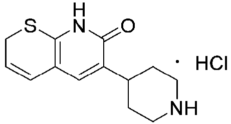

Tert-butyl 4-(7-methoxy-2H-thiopyrano[2,3-*b*]pyridine-6-yl)piperidine-1-formate (11 mg) was dissolved in hydrochloric acid/1,4-dioxane solution (4 N, 3 mL) to react at 90°C for 4 hours. After the reaction was ended, the reaction solution was concentrated to obtain 9 mg of crude product.

MS (ESI) m/z (M+H)⁺ = 249.1.

### Preparation Example 36: preparation of 3-(piperidine-4-yl)-1,5,6,7-tetrahydro-2H-cyclopentadieno[b]pyridine-2-one hydrochloride (available for Example 51)

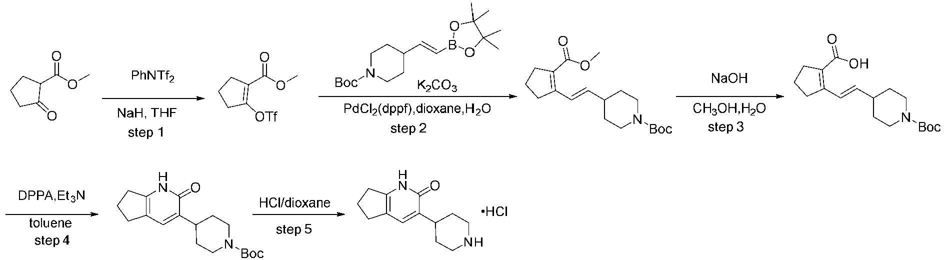

Corresponding common commercially available reagents were used to subsequently prepare the title compound by a preparation method similar to that in Preparation Example 32 of intermediate compound above.

MS (ESI) m/z (M+H)⁺= 219.1 .

### Preparation Example 37: preparation of 3-piperidine-4-yl)-5,6,7,8-tetrahydroquinoline-2(1H)-one hydrochloride (available for Example 52)

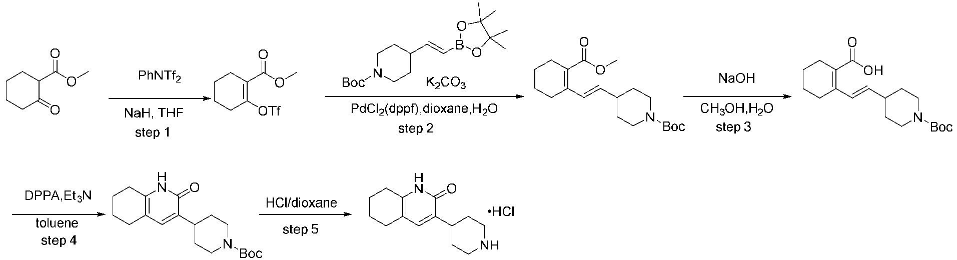

Corresponding common commercially available reagents were used to subsequently prepare the title compound by a preparation method similar to that in Preparation Example 32 of intermediate compound above.

MS (ESI) m/z (M+H)⁺ = 233.2.

A synthesis method for the compound of the present invention is illustratively provided as follows. The raw materials or reagents required for the synthesis of the compound of the present invention in the following embodiments may all be commercially available, or may be acquired according to the synthesis method for the intermediate compound provided by the present invention above, or may be acquired through the methods reported in the existing technical literatures combined with the conventional chemical reaction synthesis means.

### Example 1: preparation of (R)-4-(3,3-dimethyl-6-oxo-2,3,6,7-tetrahydrofuro[2,3-b]pyridine-5-yl)-N-(3-(7-methyl-1H-in dazole-5-yl)-1-(4-(1-methylpiperidine-4-yl)piperazine-1-yl)-1-oxopropan-2-yl)piperidine-1-f ormamide

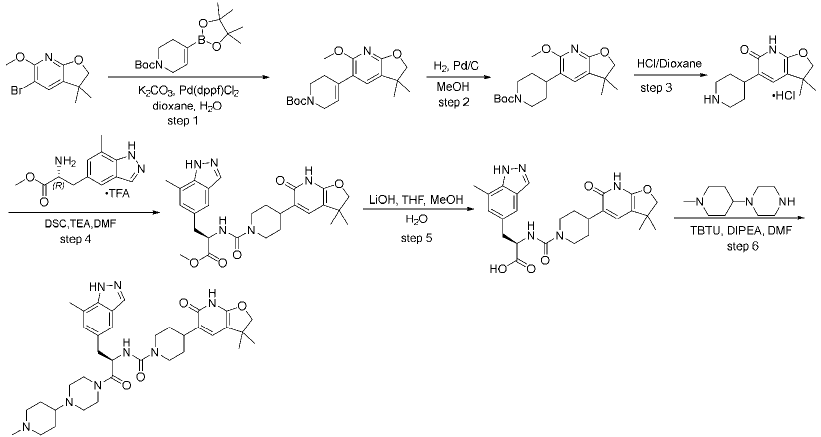

### Step 1: preparation of tert-butyl 4-(6-methoxy-3,3-dimethyl-2,3-dihydrofuro[2,3-b]pyridine-5-yl)-3,6-dihydropyridine-1(2H)-formate

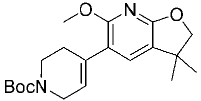

In a nitrogen atmosphere, 5-bromo-6-methoxy-3,3-dimethyl-2,3-dihydrofuro[2,3-*b*]pyridine (610.0 mg), *N*-tert-butoxycarbonyl-1,2,5,6-tetrahydropyridine-4-boronic acid pinacol ester (880.0 mg) and potassium carbonate (981.0 mg) were dissolved in 1,4-dioxane (7 mL) and water (0.7 mL), and added with 1,1'-bis(diphenylphosphino)ferrocene palladium dichloride (173.0 mg) to react at 90°C for 2 hours. After TLC showed that the reaction was completed, the reaction system was added with 30 mL of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 620.0 mg of the title compound.

MS (ESI) m/z (M+H)⁺ = 361.2.

### Step 2: preparation of tert-butyl 4-(6-methoxy-3,3-dimethyl-2,3-dihydrofuro[2,3-b]pyridine-5-yl)piperidine-1-formate

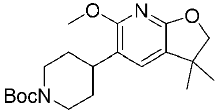

Tert-butyl 4-(6-methoxy-3,3-dimethyl-2,3-dihydrofuro[2,3-*b*]pyridine-5-yl)-3,6-dihydropyridine-1(2H)-for mate (610.0 mg) was dissolved in methanol (30 mL), added with palladium carbon (10%, 61.0 mg), subjected to air replacement, and continuously introduced with hydrogen to react at room temperature for 1 hour. After TLC showed that the reaction was completed, the system was filtered, and the filtrate was collected and concentrated. The obtained crude product was purified by column chromatography to obtain 550.0 mg of the title compound.

MS (ESI) m/z (M+H)⁺ = 363.2.

### Step 3: preparation of 3,3-dimethyl-5-(piperidine-4-yl)-3,7-dihydrofuro[2,3-b]pyridine-6(2H)-one hydrochloride

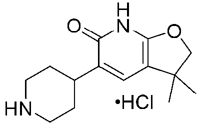

Tert-butyl 4-(6-methoxy-3,3-dimethyl-2,3-dihydrofuro[2,3-*b*]pyridine-5-yl)piperidine-1-formate (200.0 mg) was dissolved in a hydrochloric acid - 1,4-dioxane solution (4 M, 3 mL) to react at 90°C for 3 hours. After TLC showed that the reaction was completed, the system was concentrated to obtain 230.0 mg of crude product.

MS (ESI) m/z (M+H)⁺ = 249.2.

### Step 4: preparation of methyl (R)-2-(4-(3,3-dimethyl-6-oxo-2,3,6,7-tetrahydrofuro[2,3-b]pyridine-5-yl)piperidine-1-formylami no)-3-(7-methyl-1H-indazole-5-yl)propionate

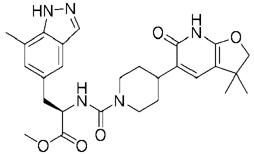

(R)-2-amino-3-(7-methyl-1H-indazole-5-yl)methyl propionate trifluoroacetate (187.9 mg) and *N,N'*-disuccinimyl carbonate (206.4 mg) were dissolved in *N,N*-dimethylformamide (3 mL), and added with triethylamine (244.3 mg) to react at room temperature for 30 minutes. The above system was added with 3,3-dimethyl-5-(piperidine-4-yl)-3,7-dihydrofuro[2,3-b]pyridine-6(2H)-one hydrochloride (200.0 mg) to react at room temperature for 1 hour. After TLC showed that the reaction was completed, the system was filtered, and the filtrate was collected. The crude product was subjected to reverse phase preparation to obtain 90.0 mg of the title compound.

MS (ESI) m/z (M+H)⁺ = 508.2.

### Step 5: preparation of (R)-2-(4-(3,3-dimethyl-6-oxo-2,3,6,7-tetrahydrofuro[2,3-b]pyridine-5-yl)piperidine-1-formylami no)-3-(7-methyl-1H-indazole-5-yl)propionic acid

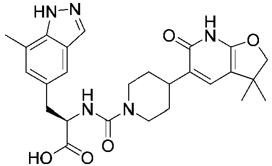

Methyl (*R*)-2-(4-(3,3-dimethyl-6-oxo-2,3,6,7-tetrahydrofuro[2,3-*b*]pyridine-5-yl)piperidine-1-formylami no)-3-(7-methyl-1*H*-indazole-5-yl)propionate (90.0 mg) was dissolved in tetrahydrofuran (5 mL) and methanol (1 mL), and added with a lithium hydroxide (8.2 mg) aqueous solution (1 mL) to react at room temperature for 1 hour. After TLC showed that the reaction was completed, the system was added with 1 M dilute hydrochloric acid to adjust to about pH 7, and the system was concentrated to obtain 100.0 mg of crude product.

MS (ESI) m/z (M+H)⁺ = 494.2.

### Step 6: preparation of (R)-4-(3,3-dimethyl-6-oxo-2,3,6,7-tetrahydrofuro[2,3-b]pyridine-5-yl)-N-(3-(7-methyl-1H-indaz ole-5-yl)-1-(4-(1-methylpiperidine-4-yl)piperazine-1-yl)-1-oxopropan-2-yl)piperidine-1-formami de

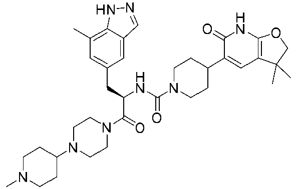

(*R*)-2-(4-(3,3-dimethyl-6-oxo-2,3,6,7-tetrahydrofuro[2,3-b]pyridine-5-yl)piperidine-1-formylami no)-3-(7-methyl-1*H*-indazole-5-yl)propionic acid (100.0 mg) and 2-(1H-benzotriazol-1-yl)-1,1,3,3-tetramethyluronium tetrafluoroborate (78 mg) was dissolved in *N,N*-dimethylformamide (2 mL), and added with *N,N*-diisopropylethylamine (77.4 mg) to react at room temperature for 10 minutes. The above system was added with 1-(1-methylpiperidine-4-yl)piperazine (43.9 mg) to react at room temperature for 20 minutes. After TLC showed that the reaction was completed, the system was filtered, and the filtrate was subjected to reverse phase preparation. The crude product was freeze-dried to obtain 36.0 mg of the title compound.

MS (ESI) m/z (M+H)⁺ = 659.4.

¹H NMR (400 MHz, DMSO-*d*₆) δ 13.02 (s, 1H), 10.62 (brs, 1H), 7.96 (s, 1H), 7.36 (s, 1H), 7.29 (s, 1H), 7.00 (s, 1H), 6.63 (d, *J* = 8.0 Hz, 1H), 4.78 (q, *J* = 7.7 Hz, 1H), 4.16 (s, 2H), 4.11 (d,*J =* 13.0 Hz, 2H), 3.62 - 3.55 (m, 1H), 3.22 (t, *J =* 9.9 Hz, 2H), 3.12-3.06 (m, 1H), 3.00-2.86 (m, 2H), 2.80-2.63 (m, 5H), 2.47 (s, 3H), 2.35 (d, *J =* 7.7 Hz, 1H), 2.27-2.20 (m, 1H), 2.09 (s, 3H), 1.98-1.85 (m, 2H), 1.75-1.62 (m, 4H), 1.50 (t, *J =* 9.1 Hz, 1H), 1.43-1.34 (m, 4H),, 1.25 (d, *J =* 2.2 Hz, 6H), 1.21-1.09 (m, 2H).

### Example 2: preparation of (R)-4-(3,3-dimethyl-6-oxo-2,3,6,7-tetrahydrothieno[2,3-b]pyridine-5-yl)-N-(3-(7-methyl-1H-indazole-5-yl)-1-(4-(1-methylpiperidine-4-yl)piperazine-1-yl)-1-oxopropan-2-yl)piperidine-1-formamide

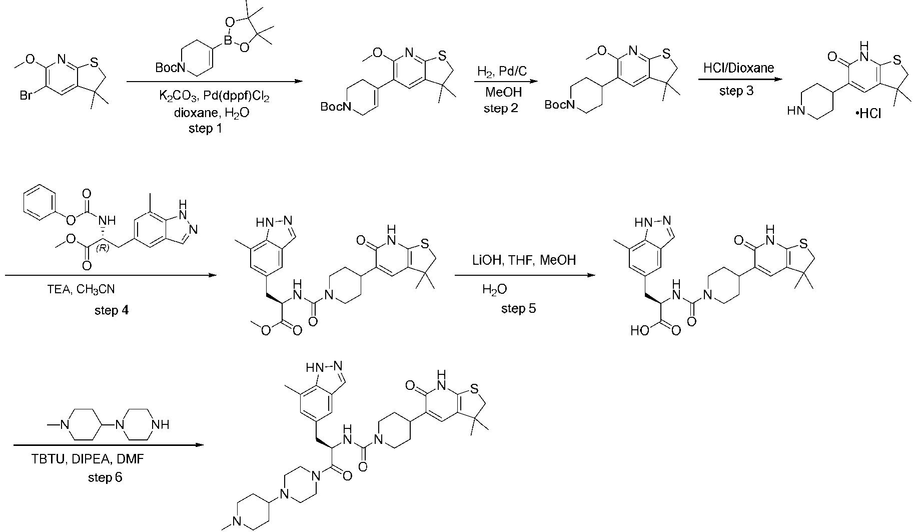

### Step 1: preparation of tert-butyl 4-(6-methoxy-3,3-dimethyl-2,3-dihydrothieno[2,3-b]pyridine-5-yl)-3,6-dihydropyridine-1(2H)-formate

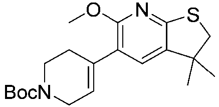

In a nitrogen atmosphere, 5-bromo-6-methoxy-3,3-dimethyl-2,3-dihydrothieno[2,3-*b*]pyridine (300.0 mg), *N*-tert-butoxycarbonyl-1,2,5,6-tetrahydropyridine-4-boronic acid pinacol ester (441.4 mg) and potassium carbonate (454.9 mg) were dissolved in 1,4-dioxane (4 mL) and water (0.4 mL), added with 1,1'-bis(diphenylphosphino)ferrocene palladium dichloride (80.4 mg), and heated to 90°C to react for 2 hours. After TLC showed that the reaction was completed, the reaction system was added with 30 mL of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 300.0 mg of the title compound.

MS (ESI) m/z (M+H)⁺ = 377.2.

### Step 2: preparation of tert-butyl 4-(6-methoxy-3,3-dimethyl-2,3-dihydrothieno[2,3-b]pyridine-5-yl)piperidine-1-formate

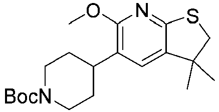

Tert-butyl 4-(6-methoxy-3,3-dimethyl-2,3-dihydrothieno[2,3-*b*]pyridine-5-yl)-3,6-dihydropyridine-1(2H)-f ormate (300.0 mg) was dissolved in methanol (20 mL), added with palladium carbon (10%, 30.0 mg), subjected to air replacement, and continuously introduced with hydrogen to react at room temperature for 1 hour. After TLC showed that the reaction was completed, the system was filtered, and the filtrate was collected and concentrated. The obtained crude product was purified by column chromatography to obtain 550.0 mg of the title compound.

MS (ESI) m/z (M+H)⁺ = 379.2.

### Step 3: preparation of 3,3-dimethyl-5-(piperidine-4-yl)-3,7-dihydrothieno[2,3-b]pyridine-6(2H)-one hydrochloride

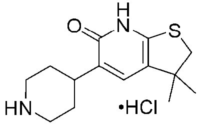

Tert-butyl 4-(6-methoxy-3,3-dimethyl-2,3-dihydrothieno[2,3-*b*]pyridine-5-yl)piperidine-1-formate (300.0 mg) was dissolved in a hydrochloric acid - 1,4-dioxane solution (10 mL) to react at 90°C for 3 hours. After TLC showed that the reaction was completed, the system was concentrated to obtain 340.0 mg of crude product.

MS (ESI) m/z (M+H)⁺ = 265.1.

### Step 4: preparation of methyl (R)-2-(4-(3,3-dimethyl-6-oxo-2,3,6,7-tetrahydrothieno[2,3-b]pyridine-5-yl)piperidine-1-formyla mino)-3-(7-methyl-1H-indazole-5-yl)propionate

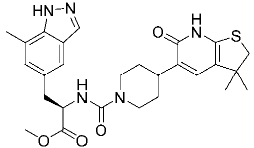

3,3-dimethyl-5-(piperidine-4-yl)-3,7-dihydrothieno[2,3-b]pyridine-6(2H)-one hydrochloride (62.9 mg) and methyl (*R*)-3-(7-methyl-1*H*-indazole-5-yl)-2-((phenoxycarbonyl)amino)propionate (67.2 mg) were dissolved in acetonitrile (4 mL), added with triethylamine (121.2 mg), and heated to 60°C to react for 7 hours. After TLC showed that the reaction was completed, the system was concentrated. The obtained crude product was purified by column chromatography to obtain 90.0 mg of the title compound.

MS (ESI) m/z (M+H)⁺ = 524.2.

### Step 5: preparation of (R)-2-(4-(3,3-dimethyl-6-oxo-2,3,6,7-tetrahydrothieno[2,3-b]pyridine-5-yl)piperidine-1-formyla mino)-3-(7-methyl-1H-indazole-5-yl)propionic acid

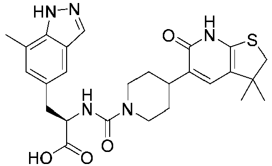

Methyl (*R*)-2-(4-(3,3-dimethyl-6-oxo-2,3,6,7-tetrahydrothieno[2,3-b]pyridine-5-yl)piperidine-1-formyla mino)-3-(7-methyl-1*H*-indazole-5-yl)propionate (90.0 mg) was dissolved in tetrahydrofuran (3 mL) and methanol (0.6 mL), and added with a lithium hydroxide (22.0 mg) aqueous solution (0.6 mL) to react at room temperature for 1 hour. After TLC showed that the reaction was completed, the system was added with 1 M dilute hydrochloric acid to adjust to about pH 7, and concentrated to obtain 110.0 mg of crude product.

MS (ESI) m/z (M+H)⁺ = 510.2.

### Step 6: preparation of (R)-4-(3,3-dimethyl-6-oxo-2,3,6,7-tetrahydrothieno[2,3-b]pyridine-5-yl)-N-(3-(7-methyl-1H-ind azole-5-yl)-1-(4-(1-methylpiperidine-4-yl)piperazine-1-yl)-1-oxopropan-2-yl)piperidine-1-forma mide

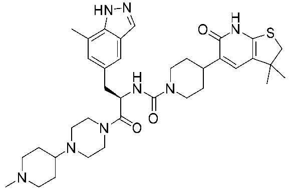

(*R*)-2-(4-(3,3-dimethyl-6-oxo-2,3,6,7-tetrahydrothieno[2,3-b]pyridine-5-yl)piperidine-1-formyla mino)-3-(7-methyl-1*H*-indazole-5-yl)propionic acid (80.0 mg) and 2-(1H-benzotriazol-1-yl)-1,1,3,3-tetramethyluronium tetrafluoroborate (61 mg) was dissolved in *N,N*-dimethylformamide (2 mL), and added with *N,N*-diisopropylethylamine (60.8 mg) to react at room temperature for 10 minutes. The above system was added with 1-(1-methylpiperidine-4-yl)piperazine (34.5 mg) to react at room temperature for 20 minutes. After TLC showed that the reaction was completed, the system was filtered, and the filtrate was subjected to reverse phase preparation. The crude product was freeze-dried to obtain 20.0 mg of the title compound.

MS (ESI) m/z (M+H)⁺ = 675.4.

¹H NMR (400 MHz, DMSO-*d*₆) δ 12.94 (s, 1H), 11.37 (s, 1H), 7.89 (s, 1H), 7.29 (s, 1H), 7.00 (s, 1H), 6.93 (s, 1H), 6.56 (d, *J =* 8.1 Hz, 1H), 4.70 (q, *J =* 7.7 Hz, 1H), 4.04 (d, *J =* 12.9 Hz, 2H), 3.51 (d, *J =* 13.1 Hz, 1H), 3.13 (s, 3H), 3.04 - 2.99 (m, 1H), 2.92 - 2.87 (m, 1H), 2.84 - 2.79 (m, 1H), 2.70 - 2.58 (m, 5H), 2.40 (s, 3H), 2.34 - 2.20 (m, 2H), 2.17 - 2.14 (m, 1H), 2.03 (s, 3H), 1.86 - 1.78 (m, 2H), 1.66 (t, *J =* 11.6 Hz, 2H), 1.57 (d, *J =* 12.3 Hz, 2H), 1.43 (t, *J =* 9.4 Hz, 1H), 1.34 - 1.28 (m, 4H), 1.18 (s, 6H), 1.14 - 1.07 (m, 2H).

### Example 3: preparation of (R)-N-(3-(7-methyl-1H-indazole-5-yl)-1-(4-(1-methylpiperidine-4-yl)piperazine-1-yl)-1-oxop ropan-2-yl)-4-(8-oxo-2,3,4,5,8,9-hexahydro-oxepino[2,3-b]pyridine-7-yl)piperidine-1-forma mide

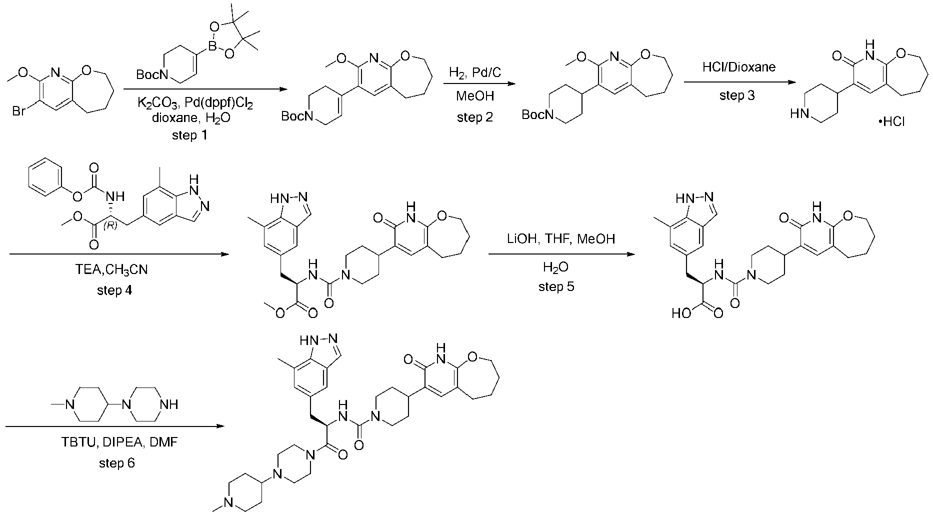

Corresponding common commercially available reagents and the products in the preparation examples of intermediate compound and the examples above were used as raw materials to prepare the title compound by a preparation method similar to that in Example 2 above.

MS (ESI) m/z (M+H)⁺ = 659.4.

¹H NMR (400 MHz, DMSO-*d*₆) δ 12.95 (brs, 1H), 11.27 (brs, 1H), 7.96 (s, 1H), 7.36 (s, 1H), 7.08 (s, 1H), 7.00 (d, *J =* 1.3 Hz, 1H), 6.64 (d, *J =* 8.0 Hz, 1H), 4.78 (q, *J =* 7.7 Hz, 1H), 4.13 - 4.06 (m, 2H), 4.02 (dd, *J =* 6.0, 4.0 Hz, 2H), 3.59 - 3.54 (m, 1H), 3.25 - 3.19 (m, 1H), 3.13 - 3.10 (m, 1H), 2.99 - 2.94 (m, 1H), 2.89 - 2.86 (m, 1H), 2.74 - 2.66 (m, 4H), 2.66 - 2.61 (m, 1H), 2.56 - 2.53 (m, 2H), 2.47 (s, 3H), 2.36 - 2.30 (m, 1H), 2.25 - 2.20 (m, 1H), 2.10 (s, 3H), 1.97 - 1.83 (m, 4H), 1.76 - 1.70 (m, 2H), 1.66 - 1.60 (m, 4H), 1.55 - 1.48 (m, 1H), 1.40 - 1.33 (m, 4H), 1.28 - 1.09 (m, 3H).

### Example 4: preparation of (R)-N-(3-(7-methyl-1H-indazole-5-yl)-1-(4-(1-methylpiperidine-4-yl)piperazine-1-yl)-1-oxop ropan-2-yl)-4-(8-oxo-2,3,4,5,8,9-hexahydrothiepino[2,3-b]pyridine-7-yl)piperidine-1-forma mide

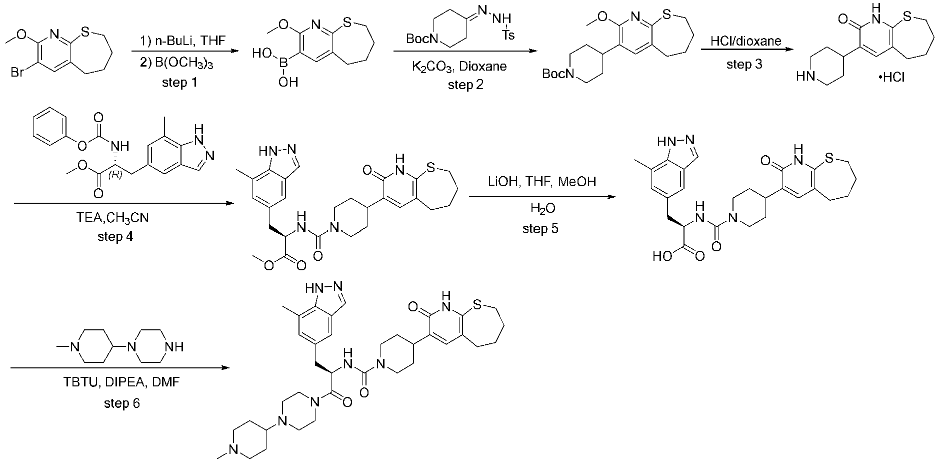

### Step 1: preparation of (8-methoxy-2,3,4,5-tetrahydro-thiepino[2,3-b]pyridine-7-yl)boric acid

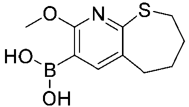

In a nitrogen atmosphere, 7-bromo-8-methoxy-2,3,4,5-tetrahydro-thiepino[2,3-*b*]pyridine (230.0 mg) was dissolved in tetrahydrofuran (3 mL), and dropwise added with n-butyl lithium (2.5 M, 0.4 mL) at -78°C to react for 30 minutes. The reaction system was added with trimethyl borate (113.9 mg) to continuously react for 40 minutes. After TLC showed that the reaction was completed, the reaction system was transferred to room temperature, added with 10 mL of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 90.0 mg of the title compound.

MS (ESI) m/z (M+H)⁺ = 240.1.

### Step 2: preparation of tert-butyl 4-(8-methoxy-2,3,4,5-tetrahydro-thiepino[2,3-b] pyridine-7-yl)piperidine-1-formate

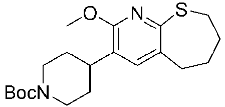

(8-methoxy-2,3,4,5-tetrahydro-thiepino[2,3-*b*]pyridine-7-yl)boric acid (90.0 mg), tert-butyl 4-(2-tosylhydrazone)piperidine-1-formate (165.0 mg) and potassium carbonate (156.0 mg) were dissolved in 1,4-dioxane (5 mL), and heated at 120°C to react for 8 hours. After TLC showed that the reaction was completed, the reaction system was transferred to room temperature, added with 10 mL of water, and extracted with ethyl acetate for three times, and the organic phases were combined, backwashed with a saturated sodium chloride solution once, dried with anhydrous sodium sulfate, filtered and concentrated. The obtained crude product was purified by column chromatography to obtain 78.0 mg of the title compound.

MS (ESI) m/z (M+H)⁺ = 379.2.

Corresponding common commercially available reagents and the products in the preparation examples of intermediate compound and the examples above were used as raw materials in subsequent steps to prepare the title compound by a preparation method similar to that in Example 2 above.

MS (ESI) m/z (M+H)⁺ = 675.4.

¹H NMR (400 MHz, DMSO-*d*₆) δ 12.94 (s, 1H), 11.45 (s, 1H), 7.89 (s, 1H), 7.29 (s, 1H), 7.00 (s, 1H), 6.93 (s, 1H), 6.58 (d, *J =* 8.1 Hz, 1H), 4.70 (q, *J =* 7.7 Hz, 1H), 4.03 (d, *J =* 12.4 Hz, 2H), 3.54 - 3.45 (m, 1H), 3.16 (dd, *J =* 11.8, 8.1 Hz, 1H), 3.05 - 3.00 (m, 1H), 2.92 - 2.79 (m, 2H), 2.75 - 2.71 (m, 2H), 2.67 - 2.55 (m, 7H), 2.40 (s, 3H), 2.28 - 2.24 (m, 1H), 2.19 - 2.12 (m, 1H), 2.03 (s, 3H), 1.91 - 1.80(m, 4H), 1.69 -1.56 (m, 4H), 1.53 - 1.42 (m, 3H), 1.32 - 1.29 (m, 2H), 1.26 - 1.04 (m, 5H).

### Example 5: preparation of (R)-N-(3-(7-methyl-1H-indazole-5-yl)-1-(4-(1-methylpiperidine-4-yl)piperazine-1-yl)-1-oxop ropan-2-yl)-4-(7-oxo-3,4,7,8-tetrahydro-2H-pyrano[2,3-b]pyridine-6-yl)piperidine-1-forma mide

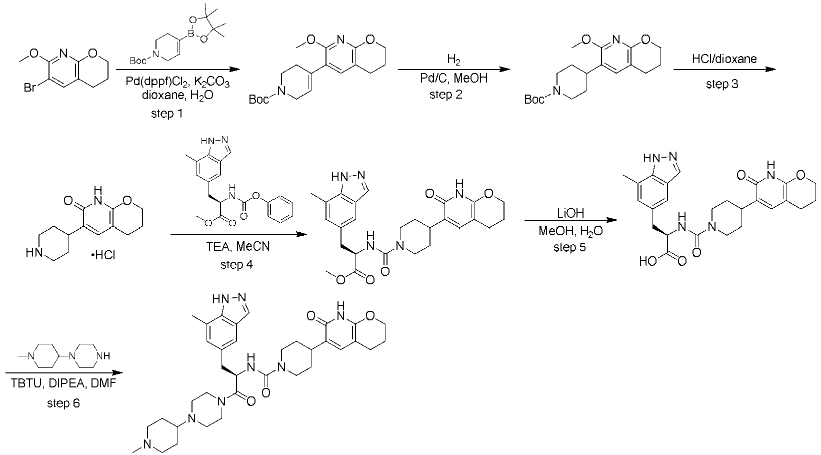

Corresponding common commercially available reagents and the products in the preparation examples of intermediate compound and the examples above were used as raw materials to prepare the title compound by a preparation method similar to that in Example 2 above.

MS (ESI) m/z (M+H)⁺ = 645.4.

¹H NMR (400 MHz, DMSO-*d*₆) δ 12.99 (s, 1H), 11.15 (s, 1H), 7.96 (s, 1H), 7.36 (s, 1H), 7.00 (s, 1H), 6.96 (s, 1H), 6.64 (d, *J =* 8.0 Hz, 1H), 4.87-4.69 (m, 1H), 4.24-4.13 (m, 2H), 4.09 (d, *J =* 12.0, 2H), 3.60-3.52 (m, 1H), 3.30-3.19 (m, 2H), 3.18-3.08 (m, 1H), 3.03-2.93 (m, 1H), 2.92-2.82 (m, 1H), 2.79-2.57 (m, 6H), 2.47 (s, 4H), 2.37-2.30 (m, 1H), 2.26-2.18 (m, 1H), 2.11 (s, 3H), 1.99-1.82 (m, 4H), 1.80-1.70 (m, 2H), 1.68-1.59 (m, 2H), 1.58-1.48 (m, 1H), 1.43-1.34 (m, 2H), 1.31-1.14 (m, 4H).

### Example 6: preparation of (R)-N-(3-(7-methyl-1H-indazole-5-yl)-1-(4-(1-methylpiperidine-4-yl)piperazine-1-yl)-1-oxop ropan-2-yl)-4-(7-oxo-3,4,7,8-tetrahydro-2H-thiopyrano[2,3-b]pyridine-6-yl)piperidine-1-for mamide

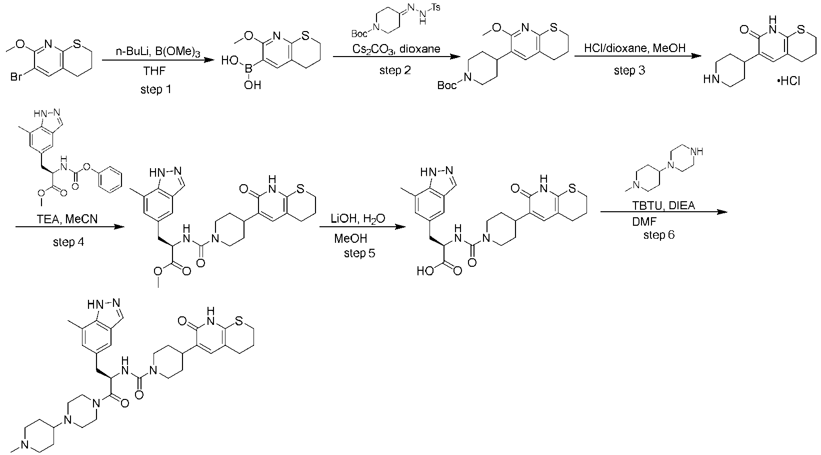

Corresponding common commercially available reagents and the products in the preparation examples of intermediate compound and the examples above were used as raw materials to prepare the title compound by a preparation method similar to that in Example 4 above.

MS (ESI) m/z (M+H)⁺ = 660.8.

¹H NMR (400 MHz, DMSO-*d*₆) δ 13.01 (s, 1H), 11.31 (s, 1H), 7.96 (s, 1H), 7.36 (s, 1H), 7.00 (s, 1H), 6.86 (s, 1H), 6.64 (d, *J =* 8.0, 1H), 4.80-4.72 (m, 1H), 4.14-4.02 (m, 2H), 3.57-3.53 (m, 1H), 3.27-3.18 (m, 2H), 3.14-3.08 (m, 1H), 3.04-3.00 (m, 2H), 2.98-2.93 (m, 1H), 2.92-2.86 (m, 1H), 2.74-2.60 (m, 6H), 2.56-2.54 (m, 1H), 2.47 (s, 3H), 2.37-2.31 (m, 1H), 2.25-2.19 (m, 1H), 2.09 (s, 3H), 2.01-1.89 (m, 4H), 1.74-1.61 (m, 4H), 1.55-1.49 (m, 1H), 1.40-1.34 (m, 2H), 1.30-1.13 (m, 4H).

### Example 7: preparation of (R)-4-(5,5-dimethyl-2-oxo-2,5,6,7-tetrahydro-1H-cyclopentadieno[b]pyridine-3-yl)-N-(3-7-methyl-1H-indazole-5-yl)-1-(4-(1-methylpiperidine-4-yl)piperazine-1-yl)-1-oxopropan -2-yl)piperidine-1-formamide

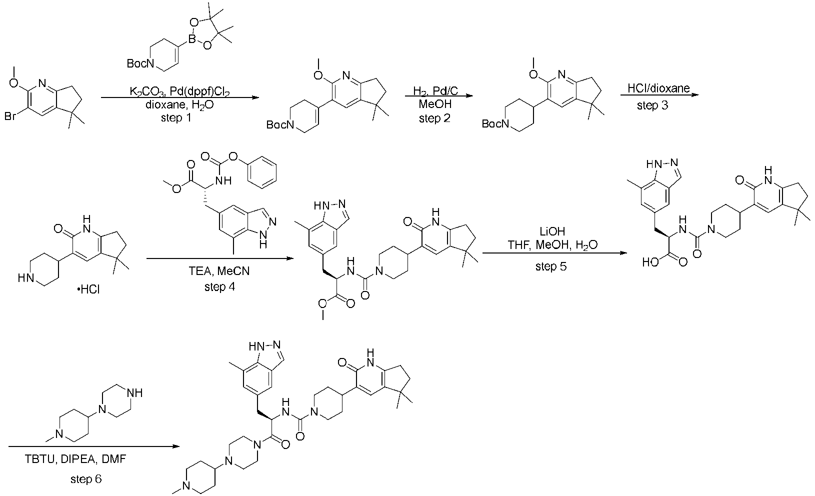

Corresponding common commercially available reagents and the products in the preparation examples of intermediate compound and the examples above were used as raw materials to prepare the title compound by a preparation method similar to that in Example 2 above.

MS (ESI) m/z (M+H)⁺= 657.1.

¹H NMR (400 MHz, DMSO-*d*₆) δ 13.02 (s, 1H), 11.59 (s, 1H), 7.96 (s, 1H), 7.36 (s, 1H), 7.07 (s, 1H), 7.00 (s, 1H), 6.61 (d, *J =* 8.0 Hz, 1H), 4.77 (q, *J =* 7.7 Hz, 1H), 4.10 (d, *J =* 12.8 Hz, 2H), 3.58 (d, *J =* 13.0 Hz, 1H), 3.21 (s, 1H), 3.12 - 3.05 (m, 1H), 2.97 - 2.86 (m, 2H), 2.79 - 2.63 (m, 8H), 2.47 (s, 3H), 2.33 (s, 2H), 2.22 (s, 1H), 2.11 (s, 3H), 1.97 - 1.87 (m, 2H), 1.82 (t, *J =* 7.2 Hz, 2H), 1.76 (s, 1H), 1.66 (d, *J =* 12.4 Hz, 2H), 1.49 (t, *J =* 9.1 Hz, 1H), 1.38 (d, *J =* 12.7 Hz, 2H), 1.32 - 1.20 (m, 4H), 1.14 (d, *J =* 1.9 Hz, 6H).

### Example 8: preparation of (R)-N-(3-(7-methyl-1H-indazole-5-yl)-1-(4-(1-methylpiperidine-4-yl)piperazine-1-yl)-1-oxop ropan-2-yl)-4-(6-oxo-2,3,6,7-tetrahydrothieno[2,3-b]pyridine-5-yl)piperidine-1-formamide

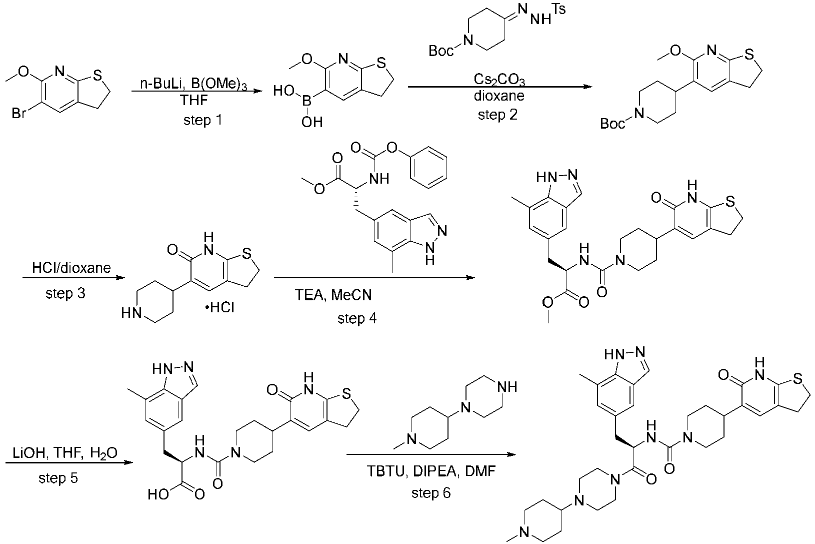

Corresponding common commercially available reagents and the products in the preparation examples of intermediate compound and the examples above were used as raw materials to prepare the title compound by a preparation method similar to that in Example 4 above.

MS (ESI) m/z (M+H)⁺= 647.1.

¹H NMR (400 MHz, DMSO-*d*₆) δ 13.01 (s, 1H), 11.38 (s, 1H), 7.96 (s, 1H), 7.36 (s, 1H), 7.09 (s, 1H), 7.00 (s, 1H), 6.65 (d, *J =* 8.0 Hz, 1H), 4.77 (q, *J =* 7.7 Hz, 1H), 4.09 (d, *J =* 12.5 Hz, 2H), 3.55 (d, *J =* 13.6 Hz, 1H), 3.40 (s, 2H), 3.24 (s, 1H), 3.11 (s, 1H), 3.06 (t, *J =* 8.1 Hz, 2H), 2.96 (dd, *J =* 13.1, 7.8 Hz, 1H), 2.88 (dd, *J =* 13.1, 7.1 Hz, 1H), 2.74 - 2.67 (m, 4H), 2.63 (d, *J =* 11.1 Hz, 1H), 2.47 (s, 3H), 2.37 - 2.31 (m, 1H), 2.23 (d, *J=* 10.1 Hz, 1H), 2.11 (s, 3H), 1.99 - 1.90 (m, 2H), 1.74 (t, *J =* 11.5 Hz, 2H), 1.67 - 1.59 (m, 2H), 1.57 - 1.51 (m, 1H), 1.38 (d, *J =* 11.9 Hz, 2H), 1.27 - 1.18 (m, 5H).

### Example 9: preparation of 4-(3,3-dimethyl-1-oxidation-6-oxo-2,3,6,7-tetrahydrothieno[2,3-b]pyridine-5-yl)-N-((R)-3-(7 -methyl-1H-indazole-5-yl)-1-(4-(1-methylpiperidine-4-yl)piperazine-1-yl)-1-oxopropan-2-yl )piperidine-1-formamide

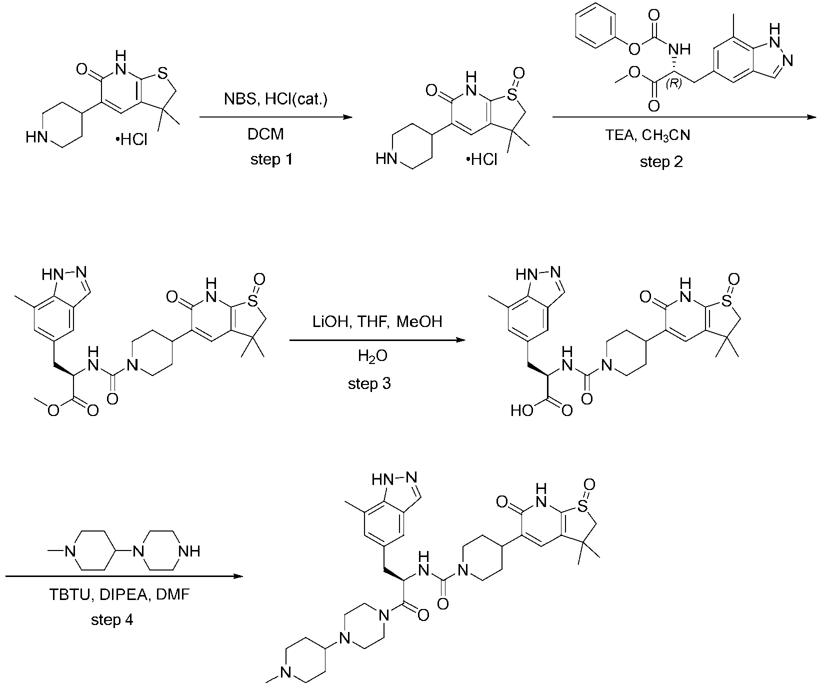

### Step 1: preparation of 3,3-dimethyl-5-(piperidine-4-yl)-3,7-dihydrothieno[2,3-b]pyridine-6(2H)-one 1-oxide hydrochloride

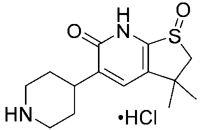

3,3-dimethyl-5-(piperidine-4-yl)-3,7-dihydrothieno[2,3-b]pyridine-6(2H)-one hydrochloride (140.0 mg) was dissolved in dichloromethane (10 mL), and added with dilute hydrochloric acid (2 M, 0.01 mL) and N-bromosuccinimide (94.0 mg) to react at room temperature for 30 minutes. After TLC showed that the reaction was completed, the system was concentrated to obtain 210.0 mg of crude product.

MS (ESI) m/z (M+H)⁺ = 281.1.

Corresponding common commercially available reagents and the products in the preparation examples of intermediate compound and the examples above were used as raw materials in subsequent steps to prepare the title compound by a preparation method similar to that in Example 2 above.

MS (ESI) m/z (M+H)⁺ = 691.4.

¹H NMR (400 MHz, DMSO-*d*₆) δ 12.95 (s, 1H), 7.90 (s, 1H), 7.47 (s, 1H), 7.30 (s, 1H), 6.93 (s, 1H), 6.60 (d, *J =* 8.0 Hz, 1H), 4.71 (q, *J =* 7.8 Hz, 1H), 4.09 (d, *J =* 12.9 Hz, 2H), 3.54 - 3.51 (m, 2H), 3.23 - 3.16 (m, 3H), 3.01 (d, *J =* 9.3 Hz, 1H), 2.97 - 2.90 (m, 2H), 2.82 - 2.80 (m, 2H), 2.68 - 2.63 (m, 4H), 2.40 (s, 3H), 2.30 - 2.24 (m, 1H), 2.18 - 2.11 (m, 1H), 2.03 (s, 3H), 1.88 - 1.78 (m, 2H), 1.69 -1.61 (m, 4H), 1.42 - 1.40 (m, 2H), 1.38 (s, 3H), 1.29 (d, *J =* 12.2 Hz, 2H), 1.24 (s, 3H), 1.16 - 1.06 (m, 2H).

### Example 10: preparation of (R)-4-(3,3-dimethyl-6-oxo-2,3,6,7-tetrahydrofuro[2,3-b]pyridine-5-yl)-N-(3-(7-methyl-1H-in dazole-5-yl)-1-(4-(1-methylpiperidine-4-yl)piperazine-1-yl)-1-oxopropan-2-yl)piperidine-1-t hioformamide

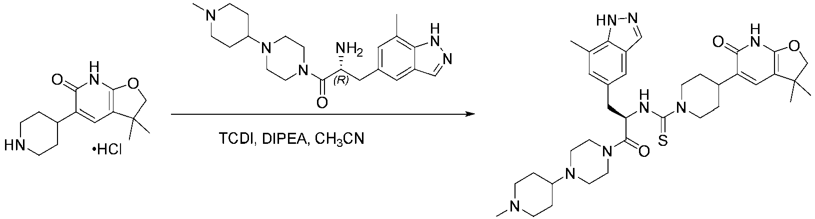

(*R*)-2-amino-3-(7-methyl-1*H*-indazole-5-yl)-1-(4-(1-methylpiperidine-4-yl)piperazine-1-yl)propa n-1-one (23.0 mg) was dissolved in acetonitrile (1 mL), and added with *N,N*-diisopropylethylamine (54.0 mg) and *N,N'*-thiocarbonyl diimidazole (11.0 mg) to react at room temperature for 1 hour. The system was added with 3,3-dimethyl-5-(piperidine-4-yl)-3,7-dihydrofuro[2,3-b]pyridine-6(2H)-one hydrochloride (15.0 mg) and heated to 40°C to react for 1 hour. After TLC showed that the reaction was completed, the reaction system was filtered, and the filtrate was collected and purified by reverse phase preparation. The crude product was freeze-dried to obtain 2.0 mg of the title compound.

MS (ESI) m/z (M+H)⁺ = 675.4.

¹H NMR (400 MHz, DMSO-*d*₆) δ 12.97 (s, 1H), 10.65 (brs, 1H), 7.91 (s, 1H), 7.58 (d, *J =* 7.5 Hz, 1H), 7.32 (s, 1H), 7.23 (s, 1H), 6.95 (s, 1H), 5.47 (q, *J =* 7.5 Hz, 1H), 4.76 (t, *J =* 13.3 Hz, 2H), 4.10 (s, 2H), 3.46-3.41 (m, 1H), 3.22-3.20 (m, 2H), 3.14-3.10 (m, 1H), 3.05 - 2.92 (m, 3H), 2.93 - 2.78 (m, 3H), 2.66-2.63 (m, 2H), 2.41 (s, 3H), 2.28 (d, *J =* 6.7 Hz, 1H), 2.23 - 2.17 (m, 1H), 2.03 (s, 3H), 2.00 - 1.93 (m, 1H), 1.89-1.81 (m, 1H), 1.69-1.63 (m, 4H), 1.44-1.32 (m, 5H), 1.18 (d, *J =* 2.9 Hz, 6H), 1.14-1.09 (m, 1H).

### Example 11: preparation of (R)-N-(3-(7-methyl-1H-indazole-5-yl)-1-(4-(1-methylpiperidine-4-yl)piperazine-1-yl)-1-oxop ropan-2-yl)-4-(2-oxo-1,2,5,7-tetrahydrothieno[3,4-b] pyridine-3-yl)piperidine-1-formamide

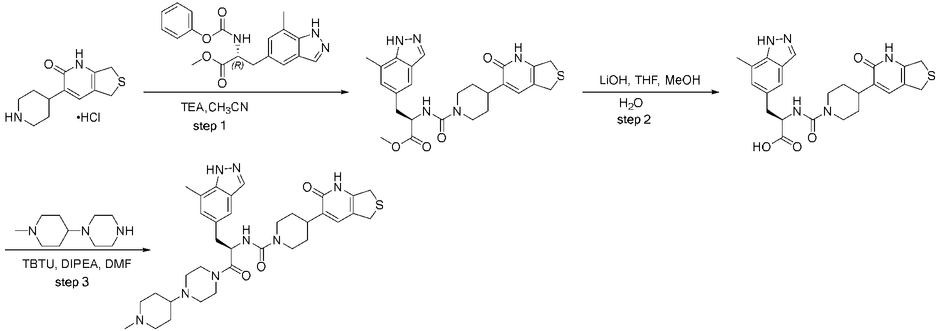

### Step 1: preparation of methyl (R)-3-(7-methyl-1H-indazole-5-yl)-2-(4-(2-oxo-1,2,5,7-tetrahydrothieno[3,4-b]pyridine-3-yl)pip eridine-1-formylamino)propionate

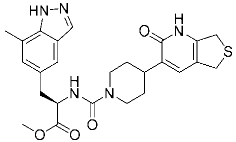

3-(piperidine-4-yl)-5,7-dihydrothieno[3,4-b]pyridine-2(1H)-one hydrochloride (51.0 mg) and methyl (*R*)-3-(7-methyl-1*H*-indazole-5-yl)-2-((phenoxycarbonyl)amino)propionate (68.0 mg) were dissolved in acetonitrile (3 mL), added with triethylamine (64.0 mg), and heated to 60°C to react for 8 hours. After TLC showed that the reaction was completed, the system was concentrated, and the obtained crude product was purified by column chromatography to obtain 65.0 mg of the title compound.

MS (ESI) m/z (M+H)⁺ =496.2.

### Step 2: preparation of (R)-3-(7-methyl-1H-indazole-5-yl)-2-(4-(2-oxo-1,2,5,7-tetrahydrothieno[3,4-b]pyridine-3-yl)pip eridine-1-formylamino)propionic acid

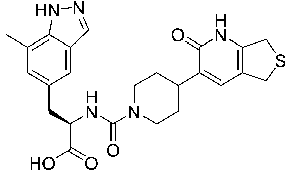

Methyl (*R*)-3-(7-methyl-1*H*-indazole-5-yl)-2-(4-(2-oxo-1,2,5,7-tetrahydrothieno[3,4-b]pyridine-3-yl)pip eridine-1-formylamino)propionate (30.0 mg) was dissolved in tetrahydrofuran (2 mL) and methanol (0.5 mL), and added with a lithium hydroxide (7.6 mg) aqueous solution (0.5 mL) to react at room temperature for 1 hour. After TLC showed that the reaction was completed, the system was added with 1 M dilute hydrochloric acid to adjust pH to be neutral, and the mother solution was concentrated to obtain 40.0 mg of crude product.

MS (ESI) m/z (M+H)⁺ = 482.2.

### Step 3: preparation of (R)-N-(3-(7-methyl-1H-indazole-5-yl)-1-(4-(1-methylpiperidine-4-yl)piperazine-1-yl)-1-oxoprop an-2-yl)-4-(2-oxo-1,2,5,7-tetrahydrothieno[3,4-b]pyridine-3-yl)piperidine-1-formamide

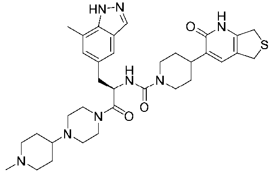

(*R*)-3-(7-methyl-1*H*-indazole-5-yl)-2-(4-(2-oxo-1,2,5,7-tetrahydrothieno[3,4-b]pyridine-3-yl)pip eridine-1-formylamino)propionic acid (30.0 mg) and 2-(1*H*-benzotriazo-1-yl)-1,1,3,3-tetramethyluronium tetrafluoroborate (22.0 mg) were dissolved in N,N-dimethylformamide (2 mL), and added with N,N-diisopropylethylamine (24.0 mg) to react at room temperature for 10 minutes. The system was continuously added with 1-(1-methylpiperidine-4-yl)piperazine (17.0 mg) to react at room temperature for 20 minutes. After TLC showed that the reaction was completed, the system was filtered, and the mother solution was purified by reverse phase preparation. The crude product was freeze-dried to obtain 11.3 mg of the title compound.

MS (ESI) m/z (M+H)⁺ = 647.3.

¹H NMR (400 MHz, DMSO-*d*6) *δ* 13.01 (s, 1H), 11.76 (brs, 1H), 7.96 (s, 1H),7.36 (s, 1H), 7.06 (s, 1H), 7.00 (s, 1H), 6.66 (d, J = 8.1 Hz, 1H), 4.77 (q, J = 7.7 Hz, 1H), 4.09 (d, J = 12.9 Hz, 2H), 4.01 - 3.93 (m, 4H), 3.58 - 3.52 (m, 1H),, 3.27 - 3.22 (m, 1H), 3.15 - 3.09 (m, 1H), 2.98 - 2.85 (m, 2H), 2.78 - 2.62 (m, 5H), 2.47 (s, 3H), 2.36 - 2.29 (m, 1H), 2.27 - 2.20 (m, 1H), 2.10 (s, 3H), 1.99 - 1.86 (m, 2H), 1.76 - 1.63 (m, 4H), 1.57 - 1.53 (m, 1H), 1.40 - 1.37 (m, 2H),, 1.26 - 1.15 (m, 5H).

### Example 12: preparation of (R)-N-(3-(7-methyl-1H-indazole-5-yl)-1-(4-(1-methylpiperidine-4-yl)piperazine-1-yl)-1-oxop ropan-2-yl)-4-(6-oxo-3,4,5,6-tetrahydro-2H-thiopyrano[3,2-b]pyridine-7-yl)piperidine-1-for mamide

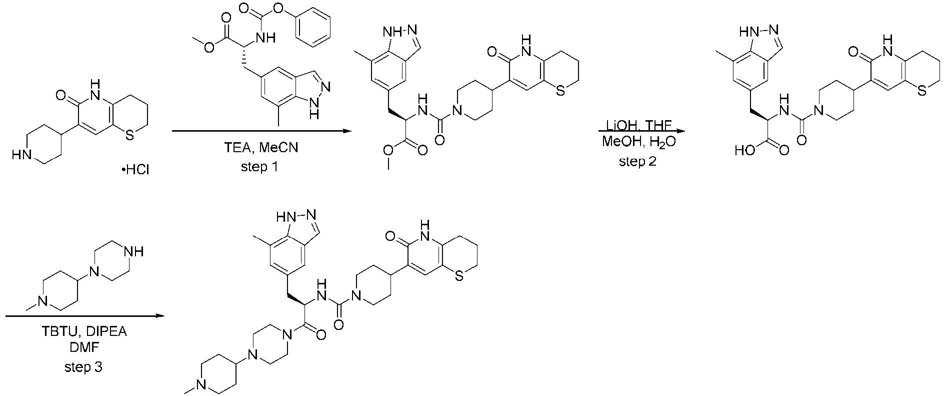

Corresponding common commercially available reagents and the products in the preparation examples of intermediate compound and the examples above were used as raw materials to prepare the title compound by a preparation method similar to that in Example 11 above.

MS (ESI) m/z (M+H)⁺= 661.1.

¹H NMR (400 MHz, DMSO-*d*₆) δ 12.94 (s, 1H), 11.40 (s, 1H), 7.89 (s, 1H), 7.28 (s, 1H), 6.93 (s, 1H), 6.71 (s, 1H), 6.57 (d, *J =* 8.0 Hz, 1H), 4.70 (q, *J =* 7.7 Hz, 1H), 4.02 (s, 2H), 3.49 (d, *J =* 13.5 Hz, 1H), 3.15 (t, *J =* 9.6 Hz, 1H), 3.03 (t, *J =* 9.4 Hz, 1H), 2.92 - 2.79 (m, 4H), 2.69 - 2.55 (m, 5H), 2.48 (t, *J =* 6.3 Hz, 2H), 2.41 (s, 3H), 2.26 (s, 1H), 2.13 (d, *J =* 8.8 Hz, 1H), 2.02 (s, 3H), 1.96 - 1.80 (m, 4H), 1.69 - 1.57 (m, 4H), 1.42 (s, 1H), 1.30 (d, *J =* 12.0 Hz, 2H), 1.21 - 1.07 (m, 5H).

### Example 13: preparation of (R)-N-(3-(7-methyl-1H-indazole-5-yl)-1-(4-(1-methylpiperidine-4-yl)piperazine-1-yl)-1-oxop ropan-2-yl)-4-(2-oxo-2,5,6,8-tetrahydro-1H-thiopyrano[3,4-b]pyridine-3-yl)piperidine-1-for mamide

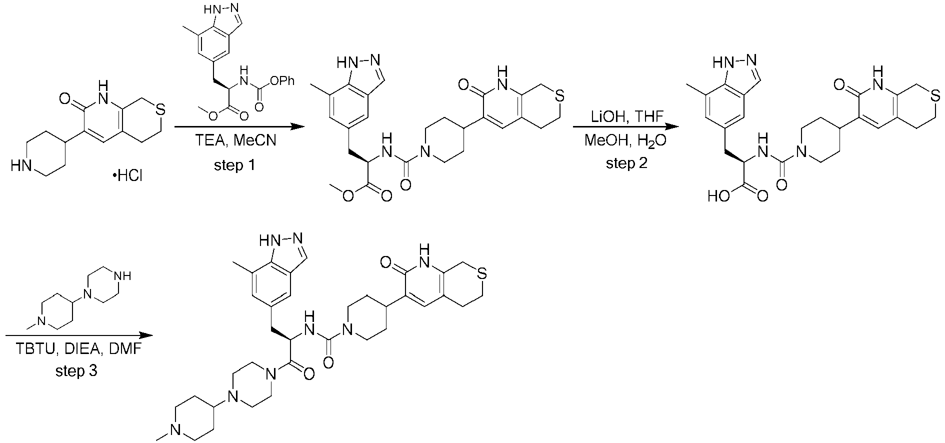

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 660.8.

¹H NMR (400 MHz, DMSO-*d*₆) *δ* 13.01 (s, 1H), 11.36 (s, 1H), 7.96 (s, 1H), 7.36 (s, 1H), 7.00 (s, 1H), 6.89 (s, 1H), 6.64 (d, *J* = 8.0 Hz, 1H), 4.82-4.72 (m, 1H), 4.10 (d, *J* = 12.0 Hz, 2H), 3.60-3.54 (m, 1H), 3.50 (s, 2H), 3.25-3.18 (m, 1H), 3.15-3.08 (m, 1H), 2.98-2.92 (m, 1H), 2.90-2.85 (m, 1H), 2.81-2.78 (m, 3H), 2.75-2.62 (m, 6H), 2.47 (s, 3H), 2.37-2.31 (m, 1H), 2.25-2.19 (m, 1H), 2.09 (s, 3H), 2.01-1.85 (m, 2H), 1.75-1.64 (m, 4H), 1.58-1.46 (m, 1H), 1.43-1.32 (m, 2H), 1.29-1.11 (m, 5H).

### Example 14: preparation of (R)-N-(3-(7-methyl-1H-indazole-5-yl)-1-(4-(1-methylpiperidine-4-yl)piperazine-1-yl)-1-oxop ropan-2-yl)-4-(2-oxo-1,2,5,7-tetrahydrothieno[3,4-b]pyridine-3-yl-7,7-d2)piperidine-1-form amide

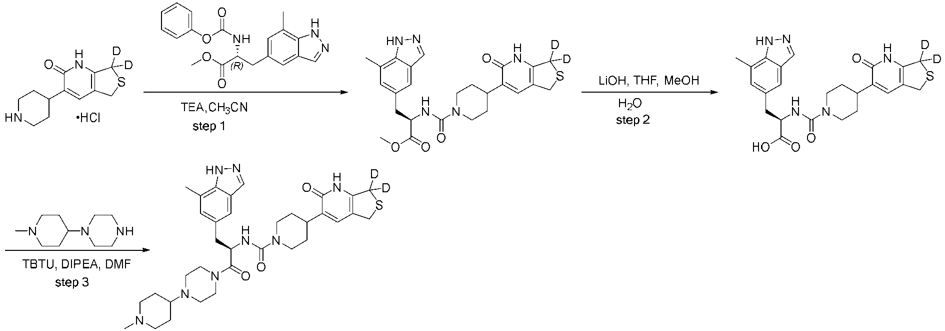

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 649.4.

¹H NMR (400 MHz, DMSO-d6) δ 13.01 (s, 1H), 11.75 (brs, 1H), 7.96 (s, 1H), 7.36 (s, 1H), 7.06 (s, 1H), 7.00 (s, 1H), 6.65 (d, J = 8.0 Hz, 1H), 4.77 (q, J = 7.7 Hz, 1H), 4.09 (d, J = 12.9 Hz, 2H), 3.95 (d, J = 2.2 Hz, 2H), 3.58 - 3.51 (m, 1H), 3.26 - 3.21 (m, 1H), 3.17 - 3.09 (m, 1H), 2.99 - 2.93 (m, 1H), 2.90 - 2.85 (m, 1H), 2.79 - 2.62 (m, 5H), 2.47 (s, 3H), 2.36 - 2.31 (m, 1H), 2.24 - 2.20 (m, 1H), 2.10 (s, 3H), 1.99 - 1.87 (m, 2H), 1.75 - 1.62 (m, 4H), 1.58 - 1.53 (m, 1H), 1.46 - 1.37 (m, 2H), 1.27 - 1.12 (m, 5H).

### Example 15: preparation of (R)-N-(3-(7-methyl-1H-indazole-5-yl)-1-(4-(1-methylpiperidine-4-yl)piperazine-1-yl)-1-oxop ropan-2-yl)-4-(2-oxo-1,5,7,8-tetrahydro-2H-thiopyrano[4,3-b]pyridine-3-yl)piperidine-1-for mamide

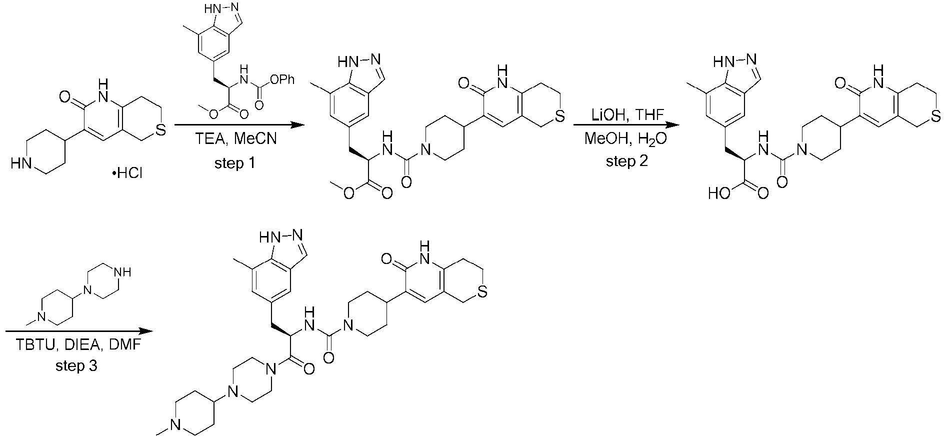

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 661.3.

¹H NMR (400 MHz, DMSO-*d*₆) *δ* 13.01 (s, 1H), 11.35 (s, 1H), 7.96 (s, 1H), 7.36 (s, 1H), 7.00 (s, 1H), 6.90 (s, 1H), 6.65 (d, *J =* 8.0 Hz, 1H), 4.77 (q, *J =* 7.7 Hz, 1H), 4.09 (d, *J =* 12.8 Hz, 2H), 3.52 (d, *J =* 17.4 Hz, 3H), 3.23 (s, 1H), 3.15 - 3.06 (m, 1H), 3.01 - 2.83 (m, 2H), 2.82 (t, *J =* 5.9 Hz, 2H), 2.69 (dt, *J =* 20.1, 8.4 Hz, 6H), 2.47 (s, 3H), 2.33 (s, 1H), 2.23 (d, *J =* 10.4 Hz, 1H), 2.10 (s, 3H), 1.95 (dd, *J=* 25.7, 15.2, 9.4 Hz, 3H), 1.75 (d, *J =* 11.4 Hz, 1H), 1.71 - 1.61 (m, 2H), 1.54 (t*, J =* 8.9 Hz, 1H), 1.38 (d, *J =* 12.4 Hz, 2H), 1.30 - 1.10 (m, 6H).

### Example 16: preparation of (R)-N-(3-(7-methyl-1H-indazole-5-yl)-1-(4-(1-methylpiperidine-4-yl)piperazine-1-yl)-1-oxop ropan-2-yl)-4-(6-oxo-2,3,6,7-tetrahydrothieno[2,3-b]pyridine-5-yl)piperidine-1-thioformami de

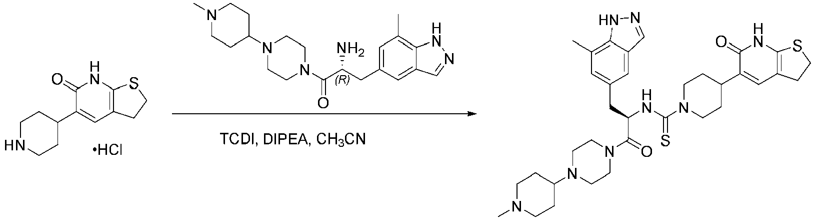

Corresponding common commercially available reagents and the products in the preparation examples of intermediate compound and the examples above were used as raw materials to prepare the title compound by a preparation method similar to that in Example 10 above.

MS (ESI) m/z (M+H)⁺ = 663.3.

¹H NMR (400 MHz, DMSO-*d*₆) δ 13.04 (s, 1H), 11.49 (brs, 1H), 7.98 (s, 1H), 7.67 (d, *J=* 7.5 Hz, 1H), 7.40 (s, 1H), 7.10 (s, 1H), 7.02 (s, 1H), 5.54 (q, *J =* 7.5 Hz, 1H), 4.80 (t, *J =* 12.5 Hz, 2H), 3.50 - 3.45 (m, 1H), 3.42 - 3.38 (m, 2H), 3.25 - 3.18 (m, 2H), 3.09 - 2.96 (m, 4H), 2.99 - 2.83 (m, 3H), 2.72 (d, *J=* 10.9 Hz, 2H), 2.48 (s, 3H), 2.35 - 2.23 (m, 2H), 2.10 (s, 3H), 2.08 - 1.99 (m, 1H), 1.97 - 1.89 (m, 1H), 1.77 - 1.67 (m, 4H), 1.65 - 1.59 (m, 1H), 1.44 - 1.31 (m, 4H), 1.29 - 1.10 (m, 3H).

### Example 17: preparation of (R)-N-(3-(7-methyl-1H-indazole-5-yl)-1-(4-(1-methylpiperidine-4-yl)piperazine-1-yl)-1-oxop ropan-2-yl)-4-(5-oxo-2,3,4,5-tetrahydrothieno[3,2-b]pyridine-6-yl)piperidine-1-formamide

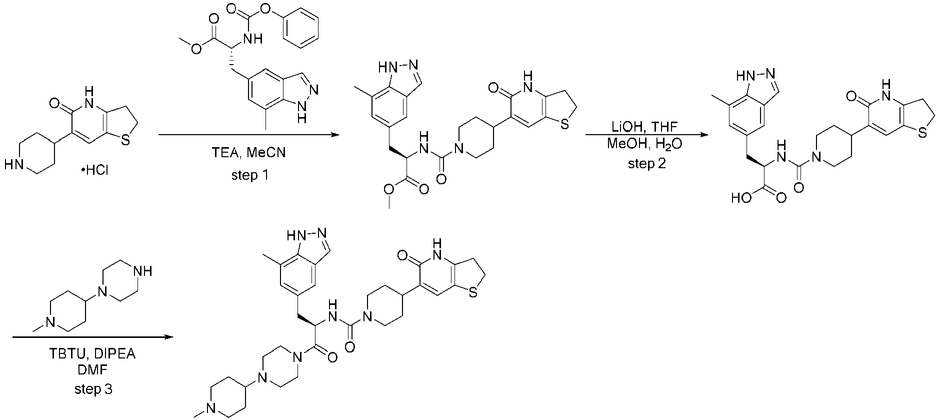

Corresponding common commercially available reagents and the products in the preparation examples of intermediate compound and the examples above were used as raw materials to prepare the title compound by a preparation method similar to that in Example 11 above.

MS (ESI) m/z (M+H)⁺= 647.1.

¹H NMR (400 MHz, DMSO-*d*₆) δ 12.94 (s, 1H), 11.67 (s, 1H), 7.89 (s, 1H), 7.28 (s, 1H), 7.07 (s, 1H), 6.92 (s, 1H), 6.58 (d, *J =* 8.0 Hz, 1H), 4.70 (q, *J =* 7.7 Hz, 1H), 4.03 (t, *J =* 11.1 Hz, 2H), 3.54 - 3.44 (m, 1H), 3.24 (s, 2H), 3.18 - 3.11 (m, 1H), 3.00 (t, *J =* 8.1 Hz, 3H), 2.90 (dd, *J =* 13.1, 8.1 Hz, 1H), 2.81 (dd, *J =* 13.1, 6.8 Hz, 1H), 2.72 - 2.54 (m, 5H), 2.40 (s, 3H), 2.29 - 2.22 (m, 1H), 2.17 - 2.10 (m, 1H), 2.02 (s, 3H), 1.91 - 1.77 (m, 2H), 1.69 - 1.55 (m, 4H), 1.41 (t, *J =* 9.1 Hz, 1H), 1.32 - 1.05 (m, 7H).

### Example 18: preparation of (R)-N-(3-(7-methyl-1H-indazole-5-yl)-1-(4-(1-methylpiperidine-4-yl)piperazine-1-yl)-1-oxop ropan-2-yl)-4-(7-oxo-3,4,7,8-tetrahydro-2H-thiopyrano[2,3-b]pyridine-6-yl)piperazine-1-for mamide

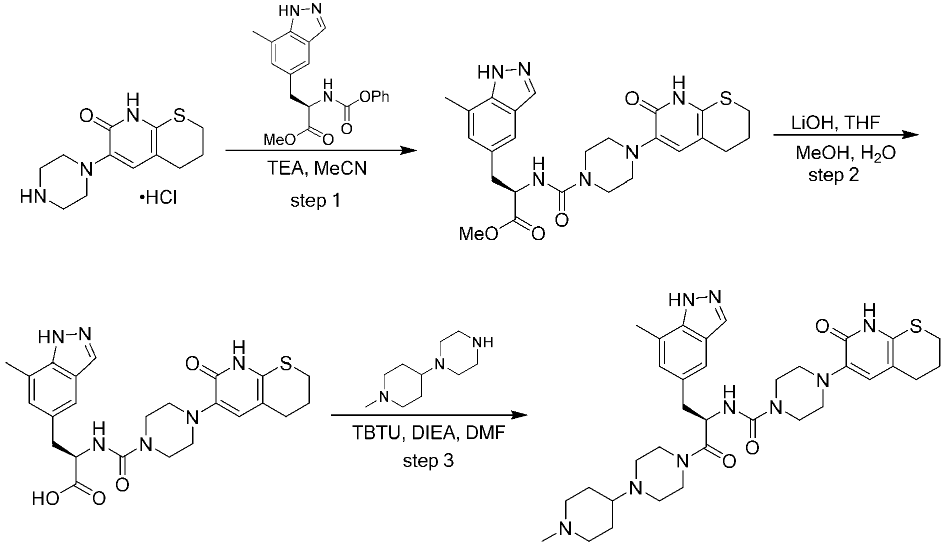

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 661.8.

¹H NMR (400 MHz, DMSO-*d*₆) δ 13.01 (s, 1H), 11.41(s, 1H), 7.96 (s, 1H), 7.36 (s, 1H), 6.99 (s, 1H), 6.78 (d, *J =* 8.0 Hz, 1H), 6.45 (s, 1H), 4.82-4.72 (m, 1H), 3.60-3.53 (m, 1H), 3.40-3.35 (m, 3H), 3.27-3.18 (m, 1H), 3.15-3.05 (m, 1H), 3.05-2.92 (m, 3H), 2.90-2.80 (m, 5H), 2.75-2.62 (m, 2H), 2.55-2.51 (m, 2H), 2.47 (s, 3H), 2.35-2.25 (m, 1H), 2.23-2.18(m, 1H), 2.09 (s, 3H), 2.01-1.85 (m, 4H), 1.76-1.64 (m, 2H), 1.55-1.46 (m, 1H), 1.42-1.32 (m, 2H), 1.29-1.09 (m, 4H).

### Example 19: preparation of (R)-N-(3-(7-methyl-1H-indazole-5-yl)-1-(4-(4-methylpiperazine-1-yl)piperidine-1-yl)-1-oxop ropan-2-yl)-4-(6-oxo-2,3,6,7-tetrahydrothieno[2,3-b]pyridine-5-yl)piperidine-1-formamide

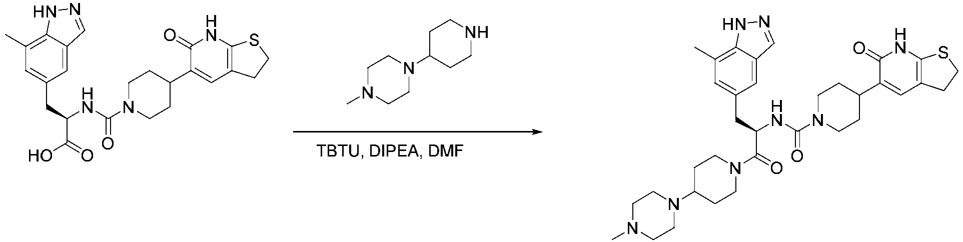

Corresponding common commercially available reagents and the products in the preparation examples of intermediate compound and the examples above were used as raw materials to prepare the title compound by a preparation method similar to that in Example 11 above.

MS (ESI) m/z (M+H)⁺= 647.1.

¹H NMR (400 MHz, DMSO-*d*₆) δ 12.93 (s, 1H), 11.40 (s, 1H), 7.89 (d, *J =* 3.2 Hz, 1H), 7.30 (d, *J= 14.3* Hz, 1H), 6.94 (d, *J=* 5.6 Hz, 2H), 6.59 (dd, *J=* 12.8, 8.1 Hz, 1H), 4.74 (dq, *J =* 15.2, 7.6 Hz, 1H), 4.26 (d, *J =* 12.7 Hz, 1H), 4.03 (s, 2H), 3.85 (t, *J =* 16.0 Hz, 1H), 3.33 (t, *J =* 7.9 Hz, 2H), 2.99 (t, *J =* 8.0 Hz, 2H), 2.94 - 2.86 (m, 1H), 2.81 (dd, *J =* 13.1, 6.4 Hz, 1H), 2.73 - 2.55 (m, 4H), 2.40 (d, *J =* 4.1 Hz, 3H), 2.34 (s, 1H), 2.30 - 2.07 (m, 5H), 2.04 (d, *J =* 5.1 Hz, 3H), 1.95 - 1.81 (m, 3H), 1.57 (d, *J =* 14.5 Hz, 3H), 1.37 (d, *J =* 12.2 Hz, 1H), 1.27 - 1.10 (m, 4H), 0.82 - 0.42 (m, 1H).

### Example 20: preparation of (R)-N-(1-([1,4'-bipiperidine]-1'-yl)-3-(7-methyl-1H-indazole-5-yl)-1-oxopropan-2-yl)-4-(6-o xo-2,3,6,7-tetrahydrothieno[2,3-b]pyridine-5-yl)piperidine-1-formamide

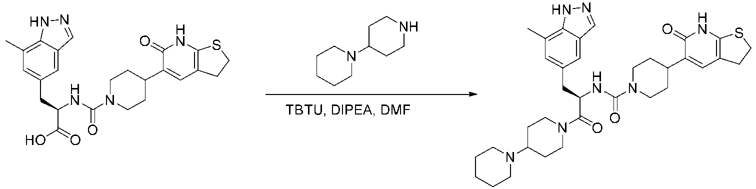

Corresponding common commercially available reagents and the products in the preparation examples of intermediate compound and the examples above were used as raw materials to prepare the title compound by a preparation method similar to that in Example 11 above.

MS (ESI) m/z (M+H)⁺= 632.1.

¹H NMR (400 MHz, DMSO-*d*₆) δ 12.99 (s, 1H), 11.45 (s, 1H), 7.96 (d, *J =* 3.1 Hz, 1H), 7.37 (d, *J= 14.4* Hz, 1H), 7.01 (d, *J=* 5.0 Hz, 2H), 6.65 (dd, *J=* 14.3, 8.1 Hz, 1H), 4.80 (dq, *J=* 24.1, 7.9 Hz, 1H), 4.34 (d, *J =* 12.1 Hz, 1H), 4.10 (s, 2H), 3.94 (t, *J =* 16.8 Hz, 1H), 3.40 (t, *J =* 7.9 Hz, 2H), 3.06 (t, *J =* 7.9 Hz, 2H), 3.00 - 2.92 (m, 1H), 2.88 (dd, *J =* 13.3, 6.3 Hz, 1H), 2.78 - 2.61 (m, 4H), 2.47 (d, *J =* 4.7 Hz, 3H), 2.38 (s, 2H), 2.30 - 2.19 (m, 1H), 2.02 - 1.79 (m, 3H), 1.62 (s, 3H), 1.45 - 1.20 (m, 10H), 0.74 - 0.55 (m, 1H).

### Example 21: preparation of (R)-N-(3-(7-methyl-1H-indazole-5-yl)-1-(4-(1-methylpiperidine-4-yl)piperazine-1-yl)-1-oxop ropan-2-yl)-1-(7-oxo-3,4,7,8-tetrahydro-2H-thiopyrano[2,3-b]pyridine-6-yl)piperidine-4-for mamide

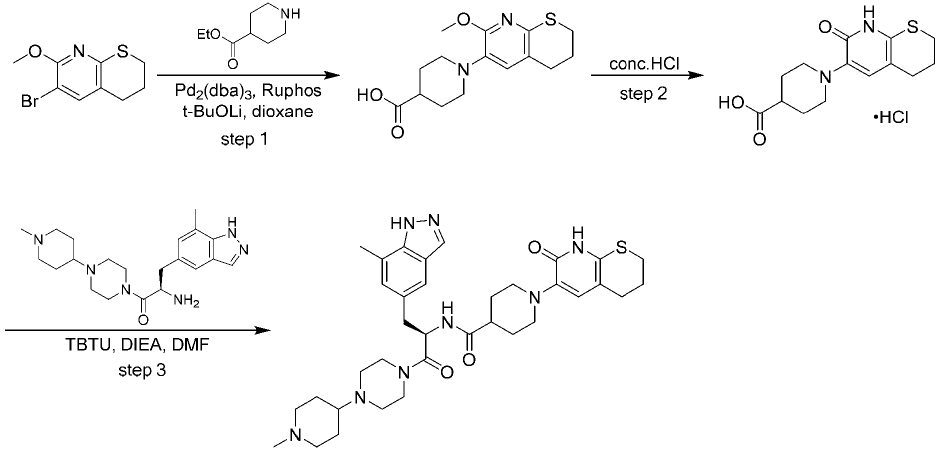

### Step 1: preparation of 1-(7-methoxy-3,4-dihydro-2H-thiopyrano[2,3-b]pyridine-6-yl)piperidine-4-formic acid

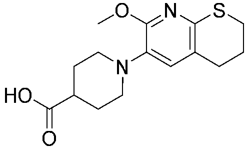

In a nitrogen atmosphere, 6-bromo-7-methoxy-3,4-dihydro-2*H*-thiopyrano[2,3-*b*]pyridine (50.0 mg), ethyl piperidine-4-formate (36.3 mg), tris(dibenzylideneacetone)dipalladium (17.4 mg), 2-dicyclohexylphosphine-2',6'-diisopropylbiphenyl (17.7 mg) and lithium tert-butoxide (30.4 mg) were dissolved in 1,4-dioxane (1 mL) to react at 100°C for 2 hours. After LCMS showed that some raw materials were left, and the reaction was ended, the reaction solution was filtered, and the filtrate was collected and concentrated. The obtained crude product was purified by column chromatography to obtain 40.0 mg of the title compound.

MS (ESI) m/z (M+H)⁺ =308.9.

### Step 2: preparation of 1-(7-oxo-3,4,7,8-tetrahydro-2H-thiopyrano[2,3-b]pyridine-6-yl)piperidine-4-formic acid hydrochloride

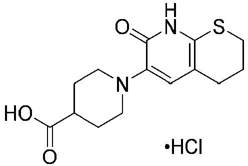

1-(7-methoxy-3,4-dihydro-2*H*-thiopyrano[2,3-*b*]pyridine-6-yl)piperidine-4-formic acid (40 mg) was dissolved in concentrated hydrochloric acid (2 mL) to react at 95°C for 2 hours. After LCMS showed that the reaction was completed, the reaction solution was concentrated to obtain 38 mg of crude product, which was directly used in the next step of reaction.

MS (ESI) m/z (M+H)⁺ = 294.9.

### Step 3: preparation of (R)-N-(3-(7-methyl-1H-indazole-5-yl)-1-(4-(1-methylpiperidine-4-yl)piperazine-1-yl)-1-oxoprop an-2-yl)-1-(7-oxo-3,4,7,8-tetrahydro-2H-thiopyrano[2,3-b]pyridine-6-yl)piperidine-4-formamide

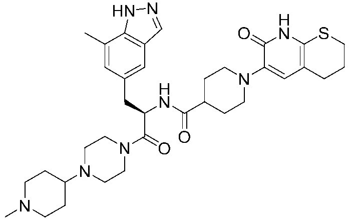

1-(7-oxo-3,4,7,8-tetrahydro-2*H*-thiopyrano[2,3-*b*]pyridine-6-yl)piperidine-4-formic acid hydrochloride (38 mg), N,N-diisopropylethylamine (50.0 mg) and (*R*)-2-amino-3-(7-methyl-1H-indazole-5-yl)-1-(4-(1-methylpiperidine-4-yl)piperazine-1-yl)prop an-1-one (54.6 mg) were dissolved in N,N-dimethylformamide (1.0 mL), and added with 2-(1H-benzotriazol-1-yl)-1,1,3,3-tetramethyluronium tetrafluoroborate (62.2 mg) to react for 1 hour. After LCMS showed that the reaction was completed, the reaction solution was concentrated, and the crude product was purified by a preparative liquid phase, and freeze-dried to obtain 17.3 mg of the title compound.

MS (ESI) m/z (M+H)⁺ = 660.9.

¹H NMR (400 MHz, DMSO-*d*₆) *δ* 13.01 (s, 1H), 11.31 (s, 1H), 8.21 (d, *J =* 8.0 Hz, 1H), 7.96 (s, 1H), 7.34 (s, 1H), 6.99 (s, 1H), 6.45(s, 1H), 4.94-4.89 (m, 1H), 3.60-3.45 (m, 3H), 3.28-3.23 (m, 2H), 3.20-3.08 (m, 1H), 3.08-2.92 (m, 3H), 2.88-2.80 (m, 1H), 2.75-2.65 (m, 2H), 2.47 (s, 3H), 2.37-2.30 (m, 3H), 2.28-2.19 (m, 2H), 2.09 (s, 3H), 2.05-1.96 (m, 1H), 1.95-1.86 (m, 3H), 1.79-1.68 (m, 2H), 1.65-1.48 (m, 5H), 1.45-1.35 (m, 2H), 1.33-1.08 (m, 4 H).

### Example 22: preparation of (R)-N-(3-(7-methyl-1H-indazole-5-yl)-1-(4'-methyl-[1,4'-bipiperidine]-1'-yl)-1-oxopropan-2-yl)-4-(6-oxo-2,3,6,7-tetrahydrothieno[2,3-b]pyridine-6-yl)piperidine-1-formamide

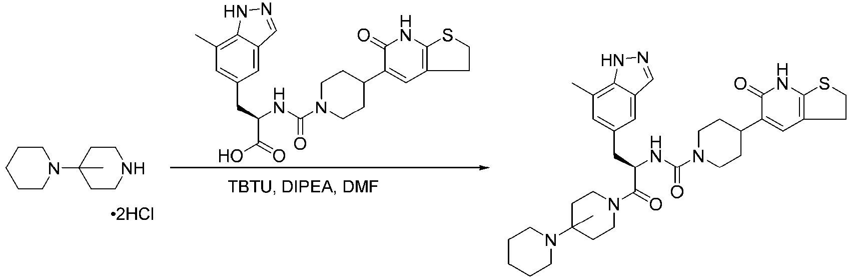

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 646.3.

¹H NMR (400 MHz, DMSO-*d*₆) δ 13.02 (s, 1H), 7.96 (d, *J =* 5.7 Hz, 1H), 7.37 (d, *J =* 6.5 Hz, 1H), 7.09 (d, *J =* 13.4 Hz, 1H), 6.99 (d, *J =* 15.0 Hz, 1H), 6.61 (t, *J =* 6.9 Hz, 1H), 4.78 (s, 1H), 4.09 (d, *J =* 13.0 Hz, 2H), 3.40 (s, 1H), 3.06 (t, *J =* 8.0 Hz, 2H), 2.93 (dd, *J =* 19.0, 9.0 Hz, 2H), 2.74 (d, *J =* 14.2 Hz, 1H), 2.66 (d, *J =* 12.7 Hz, 2H), 2.47 (d, *J =* 2.5 Hz, 3H), 2.36 - 2.18 (m, 5H), 2.00 (q, *J =* 7.1 Hz, 1H), 1.62 (s, 3H), 1.52 - 1.39 (m, 4H), 1.38 - 1.30 (m, 4H), 1.30 -1.17 (m, 5H), 0.85 (t, *J =* 6.5 Hz, 1H), 0.82 - 0.68 (m, 2H), 0.59 - 0.38 (m, 2H).

### Example 23: preparation of (R)-N-(3-(7-methyl-1H-indazole-5-yl)-1-(4-(1-methylpiperidine-4-yl)piperazine-1-yl)-1-oxop ropan-2-yl)-4-(7-oxo-3,4,7,8-tetrahydro-2H-thiopyrano[2,3-b]pyridine-6-yl)piperidine-1-thi oformamide

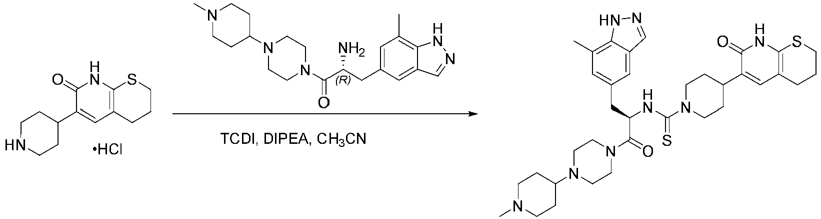

Corresponding common commercially available reagents and the products in the preparation examples of intermediate compound and the examples above were used as raw materials to prepare the title compound by a preparation method similar to that in Example 10 above.

MS (ESI) m/z (M+H)⁺ = 677.3

1H NMR (400 MHz, DMSO-d6) δ 13.03 (s, 1H), 11.36 (s, 1H), 7.97 (s, 1H), 7.66 (d, J = 7.6 Hz, 1H), 7.39 (s, 1H), 7.02 (s, 1H), 6.87 (s, 1H), 5.54 (q, J = 7.5 Hz, 1H), 4.80 (t, J = 11.3 Hz, 2H), 3.53 - 3.43 (m, 1H), 3.32 - 3.25 (m, 2H), 3.24 - 3.16 (m, 1H), 3.07 - 2.90 (m, 6H), 2.88 -2.76 (m, 1H), 2.71 (d, J = 11.0 Hz, 2H), 2.53 (t, J = 6.1 Hz, 2H), 2.48 (s, 3H), 2.36 -2.30 (m, 1H), 2.29 - 2.20 (m, 1H), 2.09 (s, 3H), 2.08 - 2.00 (m, 1H), 1.98 - 1.88(m, 3H), 1.76 - 1.66 (m, 4H), 1.64 - 1.58 (m, 1H), 1.45 - 1.31 (m, 4H), 1.29 - 1.10 (m, 2H).

### Example 24: preparation of (R)-N-(3-(7-methyl-1H-indazole-5-yl)-1-(4'-methyl-[1,4'-bipiperidine]-1'-yl)-1-oxopropan-2-yl)-4-(7-oxo-3,4,7,8-tetrahydro-2H-thiopyrano[2,3-b]pyridine-6-yl)piperidine-1-formamide

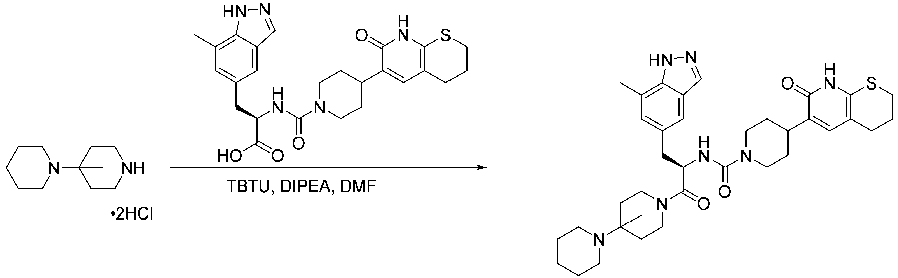

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 660.3 .

1H NMR (400 MHz, DMSO-d6) δ 13.01 (s, 1H), 11.33 (s, 1H), 7.96 (s, 0.5H), 7.94 (s, 0.5H), 7.36 (s, 0.5H), 7.35 (s, 0.5H), 7.01 (s, 0.5H), 6.97 (s, 0.5H), 6.87 (s, 0.5H), 6.84 (s, 0.5H), 6.62 - 6.58 (m, 1H), 4.82-4.73 (m, 1H), 4.09 (d, J = 13.0 Hz, 2H), 3.80-3.75 (m, 1H), 3.63 - 3.58(m, 0.5H), 3.39-3.35 (m, 0.5H), 3.20 (t, J = 11.6 Hz, 0.5H), 3.13 - 3.05 (m,0.5H), 3.05 - 2.83 (m, 5H), 2.74 - 2.59 (m, 3H), 2.55-2.52 (m, 1.5H), 2.47 (s, 1.5H), 2.46 (s, 1.5H),2.34-2.18 (m, 4H), 1.98-1.91 (m, 2H), 1.64-1.56 (m, 3H), 1.50 - 1.10 (m, 10.5H), 0.77 (s, 1.5H), 0.76-0.70(m,0.5H),0.47 (s, 1.5H), 0.17-0.11 (m, 0.5H).

### Example 25: preparation of (R)-N-(3-(7-methyl-1H-indazole-5-yl)-1-(4-(4-methylpiperazine-1-yl)piperidine-1-yl)-1-oxop ropan-2-yl)-4-(7-oxo-3,4,7,8-tetrahydro-2H-thiopyrano[2,3-b]pyridine-6-yl)piperidine-1-for mamide

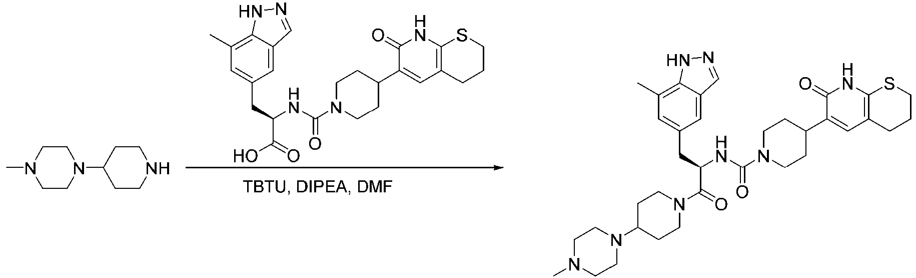

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 661.4.

¹H NMR (400 MHz, DMSO-*d*₆) δ 12.99 (s, 1H), 11.32 (s, 1H), 7.96 (d, *J* = 3.1 Hz, 1H), 7.36 (d, *J* = 13.7 Hz, 1H), 7.00 (d, *J* = 3.8 Hz, 1H), 6.90 (s, 1H), 6.65 (dd, *J =* 13.9, 8.1 Hz, 1H), 4.80 (dq, *J* = 25.6, 7.7 Hz, 1H), 4.34 (d, *J =* 12.5 Hz, 1H), 4.10 (s, 2H), 3.93 (t, *J =* 15.5 Hz, 1H), 3.05 - 2.99 (m, 2H), 2.99 - 2.77 (m, 2H), 2.76 - 2.55 (m, 4H), 2.53 (d, *J =* 6.0 Hz, 4H), 2.47 (d, *J =* 4.4 Hz, 3H), 2.41 - 2.30 (m, 2H), 2.23 (dd, *J =* 23.8, 11.9 Hz, 1H), 1.91 (ddt, *J =* 33.0, 10.8, 5.6 Hz, 4H), 1.65 (d, *J =* 12.6 Hz, 3H), 1.43 (s, 2H), 1.37 - 0.81 (m, 8H), 0.86 - 0.56 (m, 1H).

### Example 26: preparation of (R)-N-(1-([1,4'-bipiperidine]-1'-yl)-3-(7-methyl-1H-indazole-5-yl)-1-oxopropan-2-yl)-4-(7-o xo-3,4,7,8-tetrahydro-2H-thiopyrano[2,3-b]pyridine-6-yl)piperidine-1-formamide

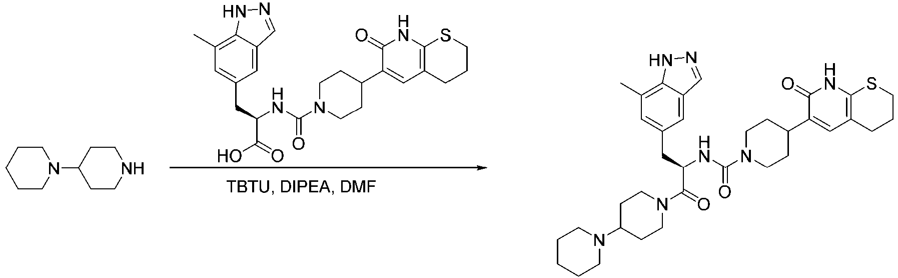

Corresponding common commercially available reagents and the products in the preparation examples of intermediate compound and the examples above were used as raw materials to prepare the title compound by a preparation method similar to that in Example 11 above.

MS (ESI) m/z (M+H)+ = 646.3.

¹H NMR (400 MHz, DMSO-*d*₆) δ 13.00 (s, 1H), 11.33 (s, 1H), 7.96 (d, *J =* 3.3 Hz, 1H), 7.36 (d, *J=* 13.7 Hz, 1H), 7.01 (d, *J* = 4.2 Hz, 1H), 6.85 (d, *J* = 38.7 Hz, 1H), 6.70 - 6.60 (m, 1H), 4.80 (dq, *J=* 24.2, 7.8 Hz, 1H), 4.32 (d, *J* = 12.6 Hz, 1H), 4.10 (s, 2H), 3.91 (t, *J* = 14.3 Hz, 1H), 3.09 - 2.92 (m, 3H), 2.87 (dt, *J =* 13.1, 6.2 Hz, 1H), 2.69 (td, *J =* 25.3, 22.8, 11.9 Hz, 4H), 2.54 (t, *J =* 6.2 Hz, 2H), 2.47 (d, *J* = 3.7 Hz, 3H), 2.43 - 2.31 (m, 2H), 2.23 (t, *J* = 28.8 Hz, 4H), 2.10 (d,*J* = 4.6 Hz, 3H), 1.94 (td, *J* = 10.8, 5.7 Hz, 5H), 1.65 (d, *J* = 12.9 Hz, 2H), 1.44 (d, *J* = 12.3 Hz, 1H), 1.36 - 1.15 (m, 4H), 0.87 - 0.49 (m, 1H).

### Example 27: preparation of 2-((7-methyl-1H-indazole-5-yl)methyl)-1-(4-(1-methylpiperidine-4-yl)piperazine-1-yl)-4-(4-( 7-oxo-3,4,7,8-tetrahydro-2H-thiopyrano[2,3-b]pyridine-6-yl)piperidine-1-yl)butane-1,4-dio ne

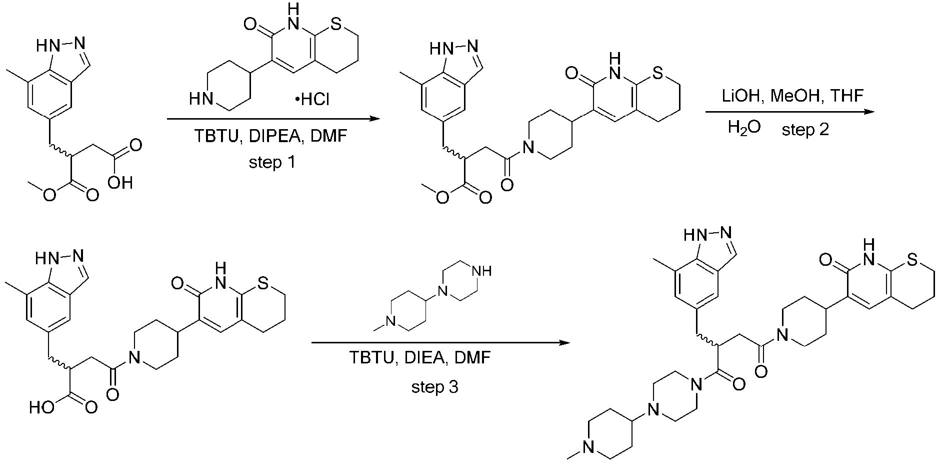

### Step 1: preparation of methyl 2-((7-methyl-1H-indazole-5-yl)methyl)-4-oxo-4-(4-(7-oxo-3,4,7,8-tetrahydro-2H-thiopyrano[2,3 -b]pyridine-6-yl)piperidine-1-yl)butyrate

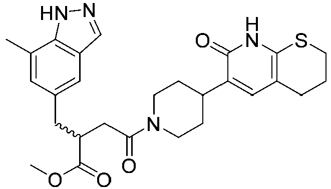

4-methoxy-3-((7-methyl-1H-indazole-5-yl)methyl)-4-oxobutyric acid (80 mg), 6-(piperidine-4-yl)-2,3,4,8-tetrahydro-7H-thiopyrano[2,3-b]pyridine-7-one hydrochloride (72 mg), O-benzotriazole-N,N,N',N'-tetramethylurea tetrafluoroborate (111 mg) and N,N-diisopropylethylamine (112 mg) were dissolved in N,N-dimethylformamide (2.0 mL) to react at room temperature for 2 hours. After LCMS showed that the reaction was completed, the reaction solution was quenched with a saturated ammonium chloride solution (5mL) and extracted with ethyl acetate, and the organic phases were combined, washed with a saturated sodium chloride solution, dried with anhydrous sodium sulfate, filtered and concentrated. The crude product was purified by a preparative liquid phase to obtain 45 mg of the title compound.

MS (ESI) m/z (M+H)+ = 509.2.

Corresponding common commercially available reagents and the products in the preparation examples of intermediate compound and the examples above were used as raw materials to prepare the title compound by a preparation method similar to that in Example 11 above.

MS (ESI) m/z (M+H)+ = 660.4.

¹H NMR (400 MHz, DMSO-*d*₆) δ 13.01 (s, 1H), 11.32 (s, 1H), 7.96 (s, 1H), 7.29 (s, 1H), 6.94 (d, *J=* 7.8 Hz, 2H), 4.44 (d, *J* = 12.6 Hz, 1H), 3.99 - 3.89 (m, 1H), 3.67 (d, *J =* 12.9 Hz, 1H), 3.43 (s, 1H), 3.32(s, 1H), 3.11 - 2.97 (m, 4H), 2.93 - 2.77 (m, 3H), 2.71 (dd, *J =* 19.5, 9.4 Hz, 4H), 2.54 (d, *J =* 6.1 Hz, 2H), 2.48 (s, 3H), 2.42 - 2.27 (m, 2H), 2.16 (d, *J =* 11.1 Hz, 1H), 2.08 (s, 3H), 1.94 (p, *J =* 6.1 Hz, 2H), 1.82 - 1.62 (m, 6H), 1.43 - 1.34 (m, 1H), 1.31 - 1.19 (m, 4H), 1.11 (ddt, *J* = 20.4, 11.9, 5.8 Hz, 3H).

### Example 28: preparation of (S)-N-(3-(7-methyl-1H-indazole-5-yl)-1-(4-(1-methylpiperidine-4-yl)piperazine-1-yl)-1-oxop ropan-2-yl)-4-(7-oxo-3,4,7,8-tetrahydro-2H-thiopyrano[2,3-b]pyridine-6-yl)piperidine-1-for mamide

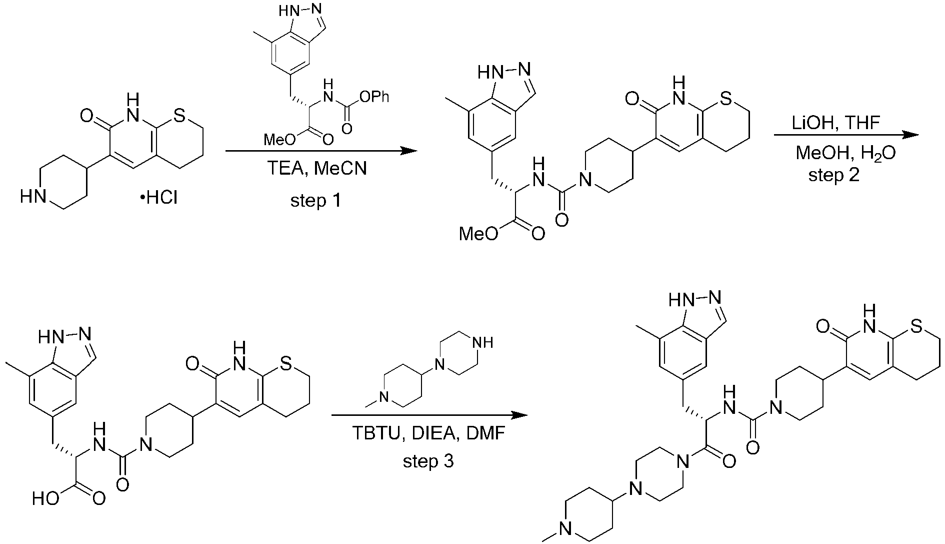

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 661.3 .

¹H NMR (400 MHz, DMSO-*d*₆) δ 13.01 (s, 1H), 11.27 (s, 1H), 7.96 (s, 1H), 7.36 (s, 1H), 7.00 (s, 1H), 6.86 (s, 1H), 6.64 (d, *J* = 8.1 Hz, 1H), 4.77 (q, *J* = 7.7 Hz, 1H), 4.09 (d, *J* = 12.9 Hz, 2H), 3.62 - 3.51 (m, 1H), 3.26 - 3.17 (m, 1H), 3.15 - 3.06 (m, 1H), 3.04 - 2.85 (m, 4H), 2.76 - 2.59 (m, 5H), 2.54 (t, *J =* 6.1 Hz, 2H), 2.47 (s, 3H), 2.37 - 2.29 (m, 1H), 2.26 - 2.18 (m, 1H), 2.09 (s, 3H), 2.00 - 1.85 (m, 4H), 1.76 - 1.62 (m, 4H), 1.52 (t, *J* = 9.3 Hz, 1H), 1.37 (d, *J =* 12.2 Hz, 2H), 1.32-1.14 (m, 5H).

### Example 29: preparation of (R)-N-(3-(7-methyl-1H-indazole-5-yl)-1-(4-(1-(methyl-d3)piperidine-4-yl)piperazine-1-yl)-1-oxopropan-2-yl)-4-(6-oxo-2,3,6,7-tetrahydrothieno[2,3-b]pyridine-5-yl)piperidine-1-forma mide

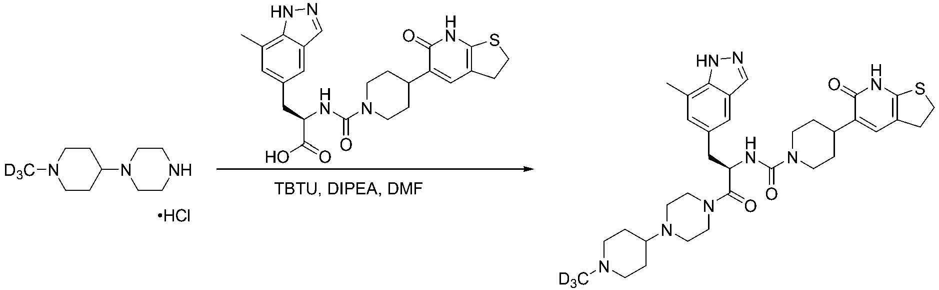

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 650.4.

¹H NMR (400 MHz, DMSO-*d*₆) *δ* 13.01 (s, 1H), 11.45 (brs, 1H), 7.96 (s, 1H), 7.36 (s, 1H), 7.09 (s, 1H), 7.00 (s, 1H), 6.65 (d, *J =* 8.0 Hz, 1H), 4.77 (q, *J* = 7.7 Hz, 1H), 4.14 - 4.05 (m, 2H), 3.58 - 3.52 (m, 1H), 3.42 - 3.38 (m, 2H), 3.27 - 3.21 (m, 1H), 3.16 - 3.10 (m, 1H), 3.10 - 3.02 (m, 3H), 2.98 - 2.86 (m, 2H), 2.75 - 2.70 (m, 3H), 2.69 - 2.60 (m, 2H), 2.47 (s, 3H), 2.36 - 2.31 (m, 1H), 2.26 - 2.19 (m, 1H),1.98 - 1.89 (m, 2H), 1.80 - 1.75 (m, 2H), 1.68 - 1.60 (m, 2H), 1.57 - 1.52 (m, 1H), 1.41 - 1.38 (m, 1H), 1.31 - 1.16 (m, 5H).

### Example 30: preparation of (R)-N-(3-(7-methyl-1H-indazole-5-yl)-1-(4-(1-(methyl-d3)piperidine-4-yl)piperazine-1-yl)-1-oxopropan-2-yl)-4-(7-oxo-3,4,7,8-tetrahydro-2H-thiopyrano[2,3-b]pyridine-6-yl)piperidine-1-formamide

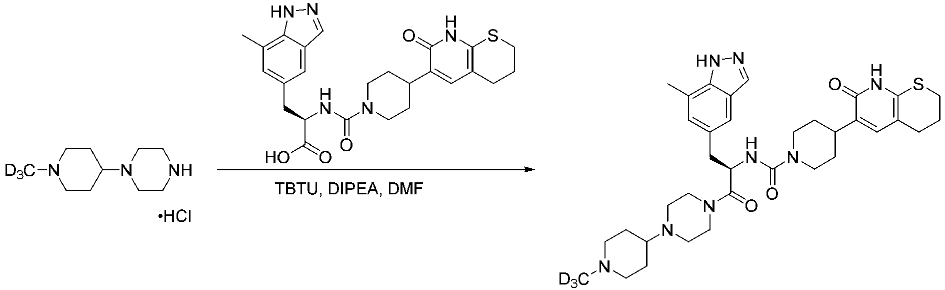

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 664.4.

¹H NMR (400 MHz, DMSO-*d*₆) δ 13.01 (s, 1H),11.32 (s, 1H), 7.96 (s, 1H), 7.36 (s, 1H), 7.00 (s, 1H), 6.86 (s, 1H), 6.64 (d, *J* = 8.1 Hz, 1H), 4.77 (q, *J* = 7.7 Hz, 1H), 4.09 (d, *J* = 12.6 Hz, 2H), 3.57 - 3.54 (m, 1H), 3.25 - 3.20 (m, 1H), 3.13 - 3.07 (m, 1H), 3.04 - 3.01 (m, 2H), 2.98 - 2.86 (m, 2H), 2.72 - 2.67 (m, 4H), 2.64 - 2.61 (m, 1H), 2.58 - 2.54 (m, 2H), 2.47 (s, 3H), 2.35 - 2.32 (m, 1H), 2.25 - 2.19 (m, 1H), 1.98 - 1.93 (m, 3H), 1.92 - 1.84 (m, 1H), 1.75 - 1.70 (m, 2H), 1.68 - 1.63 (m, 2H), 1.55 - 1.51 (m, 1H), 1.39 - 1.36 (m, 2H), 1.29 - 1.14 (m, 5H).

### Example 31: preparation of 2-((7-methyl-1H-indazole-5-yl)methyl)-1-(4-(1-methylpiperidine-4-yl)piperazine-1-yl)-4-(4-( 6-oxo-2,3,6,7-tetrahydrothieno[2,3-b]pyridine-5-yl)piperidine-1-yl)butane-1,4-dione

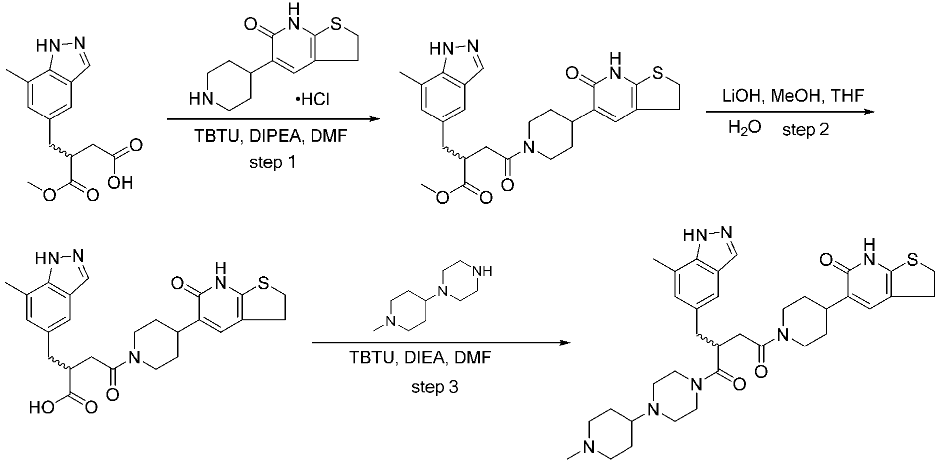

Corresponding common commercially available reagents and the products in the preparation examples of intermediate compound and the examples above were used as raw materials to prepare the title compound by a preparation method similar to that in Example 27 above.

MS (ESI) m/z (M+H)⁺ = 646.5.

1H NMR (400 MHz, DMSO-*d*₆) *δ* 13.01 (s, 1H), 11.46 (s, 1H), 7.96 (s, 1H), 7.29 (s, 1H), 7.18 (d, *J* = 3.3 Hz, 1H), 6.95 (s, 1H), 4.44 (d, *J =* 12.8 Hz, 1H), 3.95 (d, *J =* 13.3 Hz, 1H), 3.67 (d, *J =* 12.4 Hz, 1H), 3.39 (t, *J =* 7.9 Hz, 3H), 3.29 (s, 1H), 3.04 (q, *J =* 6.7, 5.7 Hz, 3H), 2.90 - 2.78 (m, 3H), 2.71 (dd, *J =* 19.5, 9.2 Hz, 4H), 2.48 (s, 3H), 2.40 (dd, *J =* 15.8, 3.6 Hz, 1H), 2.31 (d, *J =* 12.4 Hz, 2H), 2.08 (s, 4H), 1.86 - 1.64 (m, 7H), 1.38 (d, *J =* 12.3 Hz, 1H), 1.32 - 1.21 (m, 5H), 1.20-1.05 (m, 1H).

### Example 32: preparation of N-((R)-3-(7-methyl-1H-indazole-5-yl)-1-(4-(1-methylpiperidine-4-yl)piperazine-1-yl)-1-oxop ropan-2-yl)-4-(1-oxidation-7-oxo-3,4,7,8-tetrahydro-2H-thiopyrano[2,3-b]pyridine-6-yl)pipe ridine-1-formamide

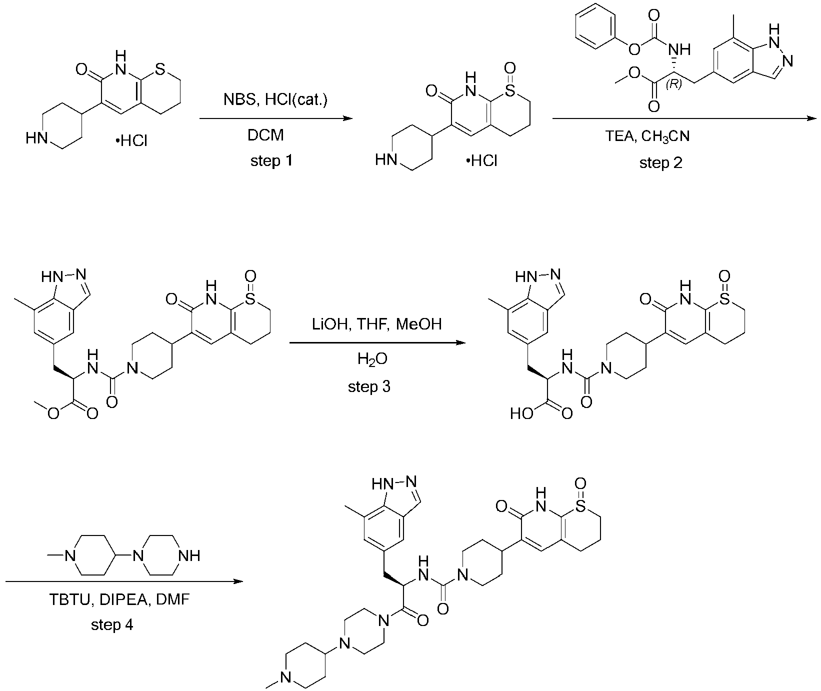

Corresponding common commercially available reagents and the products in the preparation examples of intermediate compound and the examples above were used as raw materials to prepare the title compound by a preparation method similar to that in Example 9 above.

MS (ESI) m/z (M+H)+ = 677.4.

¹H NMR (400 MHz, DMSO-*d*₆) δ 13.01 (s, 1H), 12.03 (s, 1H), 7.96 (d, *J =* 1.6 Hz, 1H), 7.36 (s, 1H), 7.23 (s, 1H), 7.00 (s, 1H), 6.68 (d, *J =* 8.0 Hz, 1H), 4.77 (q, *J =* 7.6 Hz, 1H), 4.12 (d, *J* = 13.2 Hz, 2H), 3.56 (d, *J =* 14.0 Hz, 1H), 3.22 (d, *J* = 10.4 Hz, 2H), 3.12 (dt, *J =* 19.2, 9.7 Hz, 2H), 3.04 - 2.93 (m, 2H), 2.91 - 2.81 (m, 2H), 2.77 - 2.63 (m, 5H), 2.47 (s, 3H), 2.33 (dd, *J* = 11.2, 6.4 Hz, 1H), 2.21 (d, *J* = 10.4 Hz, 2H), 2.10 (s, 3H), 2.02 - 1.85 (m, 3H), 1.79 - 1.60 (m, 4H), 1.52 (t, *J* = 9.4 Hz, 1H), 1.43 - 1.28 (m, 4H), 1.19 (ddd, *J* = 23.6, 12.4, 3.2 Hz, 3H).

### Example 33: preparation of (R)-N-(3-(7-methyl-1H-indazole-5-yl)-1-(4-(1-methylpiperidine-4-yl)piperazine-1-yl)-1-oxop ropan-2-yl)-4-(6'-oxo-6',7'-dihydro-2'H-spiro[cyclopropane-1,3'-thieno][2,3-b]pyridine-5'-y l)piperidine-1-formamide

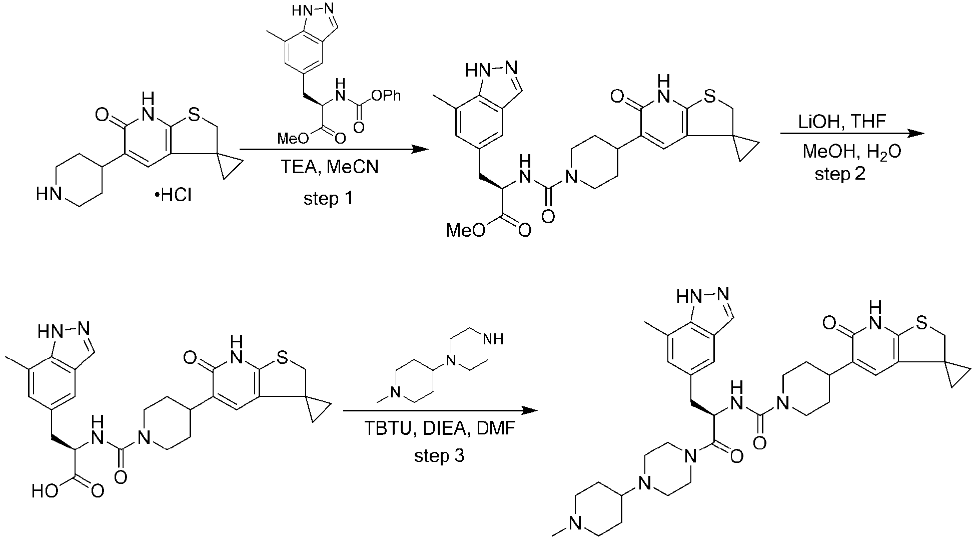

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 673.4.

¹H NMR (400 MHz, DMSO-*d*₆) *δ* 13.01 (s, 1H), 11.31 (s, 1H), 7.96 (s, 1H), 7.35 (s, 1H), 6.99 (s, 1H), 6.68 (s, 1H), 6.61 (d, *J* = 8.1 Hz, 1H), 4.76 (q, *J* = 7.8 Hz, 1H), 4.10 (d, *J* = 12.9 Hz, 2H), 3.57 (d, *J =* 12.8 Hz, 1H), 3.38 (s, 2H), 3.20 (t, *J =* 9.8 Hz, 1H), 3.07 (t, *J =* 10.0 Hz, 1H), 2.96 (dd, *J =* 13.1, 8.1 Hz, 1H), 2.87 (dd, *J =* 13.1, 6.8 Hz, 1H), 2.74 - 2.59 (m, 5H), 2.47 (s, 3H), 2.38 - 2.29 (m, 1H), 2.21 (d, *J =* 11.4 Hz, 1H), 2.09 (s, 3H), 2.03 - 1.83 (m, 2H), 1.72 (tt, *J* = 11.8, 2.9 Hz, 2H), 1.60 (d, *J =* 12.6 Hz, 2H), 1.47 (t, *J =* 9.1 Hz, 1H), 1.34 (tt, *J =* 12.0, 5.3 Hz, 4H), 1.24 (d, *J* = 3.6 Hz, 1H), 1.21 - 1.08 (m, 2H), 0.97 - 0.89 (m, 4H).

### Example 34: preparation of N-((2R)-3-(7-methyl-1H-indazole-5-yl)-1-oxo-1-(3-(piperidine-1-yl)-8-azabicyclo[3.2.1]octan e-8-yl)propan-2-yl)-4-(6-oxo-2,3,6,7-tetrahydrothieno[2,3-b]pyridine-5-yl)piperidine-1-form amide

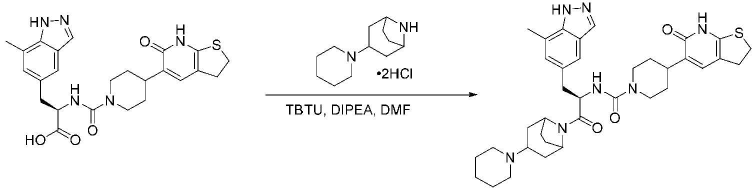

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 658.1.

¹H NMR (400 MHz, DMSO-*d*₆) δ 12.99 (s, 1H), 11.38 (s, 1H), 7.95 (t, *J* = 2.3 Hz, 1H), 7.37 (d, *J* = 7.2 Hz, 1H), 7.14 - 6.98 (m, 2H), 6.69 (q, *J =* 8.4 Hz, 1H), 4.66 - 4.57 (m, 1H), 4.53 - 4.38 (m, 1H), 4.33 - 3.95 (m, 3H), 3.40 (t, *J =* 7.9 Hz, 2H), 3.05 (t, *J =* 7.9 Hz, 2H), 2.90 (d, *J =* 7.1 Hz, 1H), 2.87 - 2.62 (m, 4H), 2.46 (d, *J* = 2.8 Hz, 3H), 2.34 (d, *J* = 20.2 Hz, 3H), 2.11 - 1.96 (m, 1H), 1.86 - 1.75 (m, 2H), 1.61 (s, 4H), 1.54 - 1.19 (m, 13H).

### Example 35: preparation of N-((R)-3-(7-methyl-1H-indazole-5-yl)-1-(4-(1-methylpiperidine-4-yl)piperazine-1-yl)-1-oxop ropan-2-yl)-4-(1-oxidation-6-oxo-2,3,6,7-tetrahydrothieno[2,3-b]pyridine-5-yl)piperidine-1-formamide

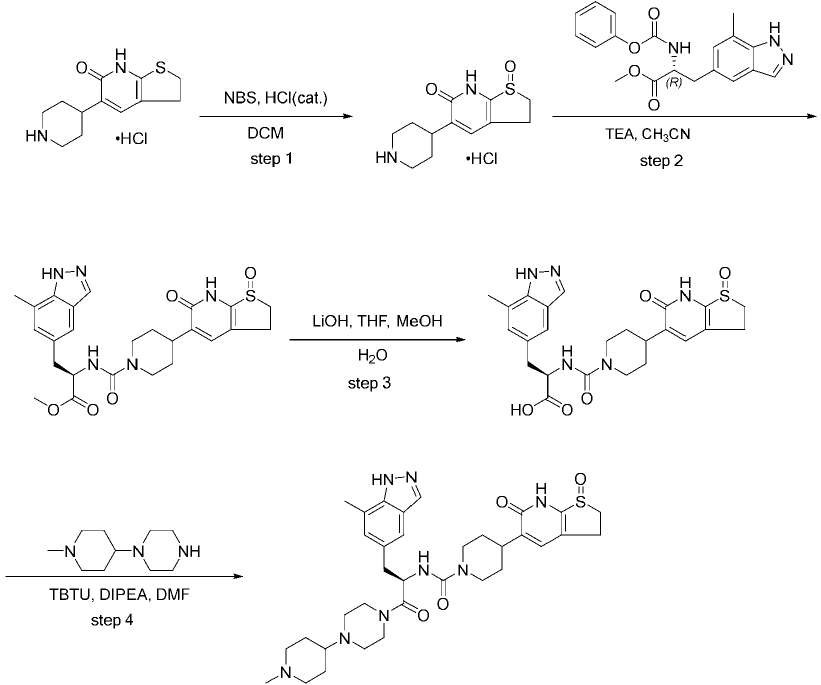

Corresponding common commercially available reagents and the products in the preparation examples of intermediate compound and the examples above were used as raw materials to prepare the title compound by a preparation method similar to that in Example 9 above.

MS (ESI) m/z (M+H)⁺ = 663.3.

¹H NMR (400 MHz, DMSO-*d*₆) *δ* 13.03 (s, 1H), 12.01 (s, 1H), 7.97 (d, *J =* 2.7 Hz, 1H), 7.45 (d, *J = 2.8* Hz, 1H), 7.38 (s, 1H), 7.02 (s, 1H), 6.71 (d, *J =* 8.0 Hz, 1H), 4.78 (q, *J =* 7.8 Hz, 1H), 4.12 (d, *J =* 12.9 Hz, 2H), 3.64 - 3.37 (m, 5H), 3.26 - 3.02 (m, 6H), 3.01 - 2.81 (m, 3H), 2.78 - 2.53 (m, 5H), 2.48 (s, 3H), 2.44 - 2.09 (m, 3H), 1.99 (q, *J =* 13.2, 7.3 Hz, 2H), 1.79 - 1.53 (m, 3H), 1.46 (s, 1H), 1.40 - 1.14 (m, 5H).

### Example 36: preparation of N-((2R)-3-(7-methyl-1H-indazole-5-yl)-1-oxo-1-(3-(piperidine-1-yl)-8-azabicyclo[3.2.1]octan e-8-yl)propan-2-yl)-4-(6-oxo-2,3,6,7-tetrahydrothieno[2,3-b]pyridine-5-yl)piperidine-1-form amide

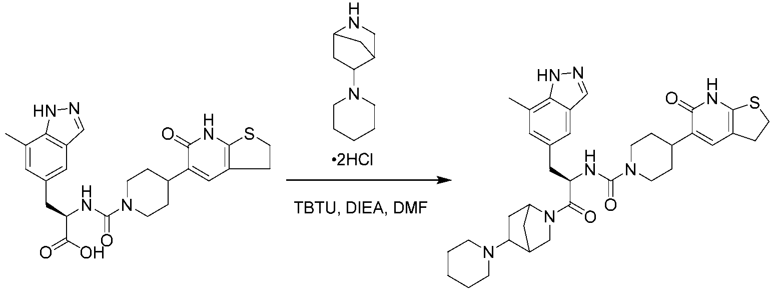

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 644.3.

¹H NMR (400 MHz, DMSO-*d*₆) δ 12.99 (s, 1H), 11.46 (s, 1H), 7.95 (d, *J =* 2.2 Hz, 1H), 7.52 - 7.31 (m, 1H), 7.12 - 6.97 (m, 2H), 6.70 - 6.39 (m, 1H), 4.74 - 4.43 (m, 1H), 4.29 (d, *J* = 23.3 Hz, 1H), 4.08 (t, *J =* 12.2 Hz, 3H), 3.47 - 3.37 (m, 5H), 3.19 - 2.99 (m, 3H), 2.96 - 2.81 (m, 2H), 2.78 - 2.58 (m, 2H), 2.47 (d, *J =* 4.4 Hz, 3H), 2.31 (d, *J =* 64.9 Hz, 3H), 2.04 - 1.88 (m, 1H), 1.74 (t, *J* = 11.0 Hz, 1H), 1.60 (d, *J =* 12.5 Hz, 2H), 1.45 (d, *J =* 10.8 Hz, 2H), 1.40 - 1.28 (m, 6H), 1.24 (d, 3H).

### Example 37: preparation of (R)-N-(3-(7-methyl-1H-indazole-5-yl-3-d)-1-(4-(1-methylpiperidine-4-yl)piperazine-1-yl)-1-oxopropan-2-yl)-4-(7-oxo-3,4,7,8-tetrahydro-2H-thiopyrano[2,3-b]pyridine-6-yl)piperidine-1-formamide

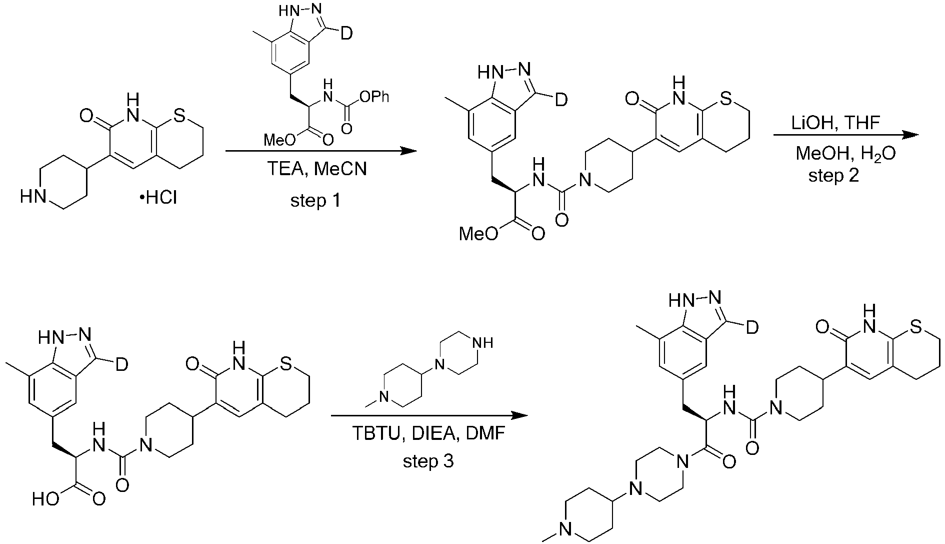

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 661.8.

¹H NMR (400 MHz, DMSO-*d*₆) *δ* 13.01 (s, 1H), 11.31 (s, 1H), 7.36 (s, 1H), 7.00 (s, 1H), 6.86 (s, 1H), 6.64 (d, *J* = 8.0 Hz, 1H), 4.80-4.72 (m, 1H), 4.14-4.02 (m, 2H), 3.57-3.53 (m, 1H), 3.27-3.18 (m, 2H), 3.14-3.08 (m, 1H), 3.04-3.00 (m, 2H), 2.98-2.93 (m, 1H), 2.92-2.86 (m, 1H), 2.74-2.60 (m, 6H), 2.56-2.54 (m, 1H), 2.47 (s, 3H), 2.37-2.31 (m, 1H), 2.25-2.19 (m, 1H), 2.09 (s, 3H), 2.01-1.89 (m, 4H), 1.74-1.61 (m, 4H), 1.55-1.49 (m, 1H), 1.40-1.34 (m, 2H), 1.30-1.13 (m, 4H).

### Example 38: preparation of (R)-N-(3-(7-methyl-1H-indazole-5-yl-3-d)-1-(4-(1-(methyl-d3)piperidine-4-yl)piperazine-1-y l)-1-oxopropan-2-yl)-4-(7-oxo-3,4,7,8-tetrahydro-2H-thiopyrano[2,3-b]pyridine-6-yl)piperid ine-1-formamide

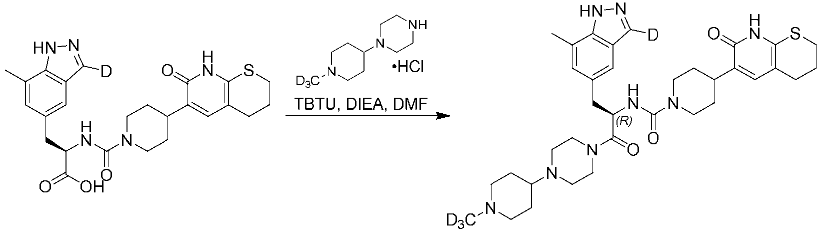

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 665.8.

¹H NMR (400 MHz, DMSO-*d*₆) *δ* 13.01 (s, 1H), 11.31 (s, 1H), 7.36 (s, 1H), 7.00 (s, 1H), 6.86 (s, 1H), 6.64 (d, *J* = 8.0 Hz, 1H), 4.80-4.72 (m, 1H), 4.14-4.02 (m, 2H), 3.57-3.53 (m, 1H), 3.27-3.18 (m, 2H), 3.14-3.08 (m, 1H), 3.04-3.00 (m, 2H), 2.98-2.93 (m, 1H), 2.92-2.86 (m, 1H), 2.74-2.60 (m, 6H), 2.56-2.54 (m, 1H), 2.47 (s, 3H), 2.37-2.31 (m, 1H), 2.25-2.19 (m, 1H), 2.01-1.89 (m, 4H), 1.74-1.61 (m, 4H), 1.55-1.49 (m, 1H), 1.40-1.34 (m, 2H), 1.30-1.13 (m, 4H).

### Example 39: preparation of N-((2R)-3-(7-methyl-1H-indazole-5-yl)-1-oxo-1-(3-(piperidine-1-yl)-8-azabicyclo[3.2.1]octan e-8-yl)propan-2-yl)-4-(7-oxo-3,4,7,8-tetrahydro-2H-thiopyrano[2,3-b]pyridine-6-yl)piperidi ne-1-formamide

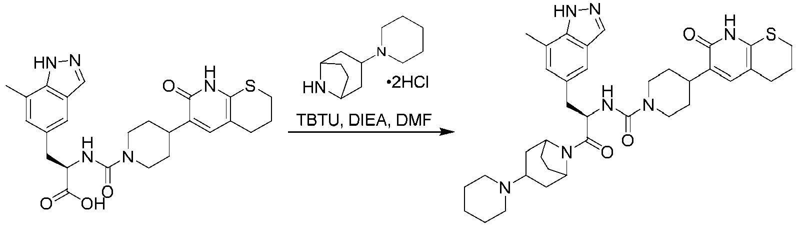

Corresponding common commercially available reagents and the products in the preparation examples of intermediate compound and the examples above were used as raw materials to prepare the title compound by a preparation method similar to that in Example 11 above.

MS (ESI) m/z (M+H)+ = 672.4.

¹H NMR (400 MHz, DMSO-*d*₆) δ 12.99 (s, 1H), 10.97 (s, 1H), 8.05 - 7.84 (m, 1H), 7.37 (d, *J* = 8.7 Hz, 1H), 7.07 - 6.97 (m, 1H), 6.91 - 6.80 (m, 1H), 6.68 (dt, *J=* 14.7, 7.6 Hz, 1H), 4.59 (dd, *J* = 17.1, 8.3 Hz, 1H), 4.40 (dd, *J* = 40.4, 32.5 Hz, 2H), 4.05 (d, *J =* 44.4 Hz, 2H), 3.08 - 2.99 (m, 2H), 2.91 (tt, *J* = 23.6, 6.7 Hz, 2H), 2.75 - 2.61 (m, 3H), 2.57 - 2.51 (m, 2H), 2.46 (d, *J* = 2.6 Hz, 3H), 2.43 - 2.21 (m, 3H), 2.07 (s, 1H), 1.99 - 1.93 (m, 2H), 1.87 - 1.73 (m, 2H), 1.63 (t, *J =* 10.4 Hz, 4H), 1.48 - 1.36 (m, 4H), 1.25 (d, *J* = 11.2 Hz, 6H), 0.94 (d, *J =* 6.4 Hz, 3H).

### Example 40: preparation of N-((2R)-3-(7-methyl-1H-indazole-5-yl)-1-oxo-1-(5-(piperidine-1-yl)-2-azabicyclo[2.2.1]hept-2-yl)propan-2-yl)-4-(7-oxo-3,4,7,8-tetrahydro-2H-thiopyrano[2,3-b]pyridine-6-yl)piperidine -1-formamide

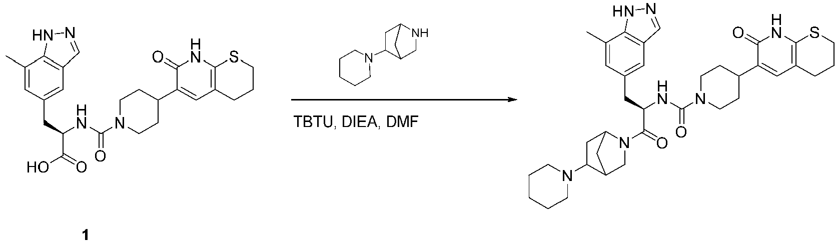

Corresponding common commercially available reagents and the products in the preparation examples of intermediate compound and the examples above were used as raw materials to prepare the title compound by a preparation method similar to that in Example 11 above.

MS (ESI) m/z (M+H)+ = 658.3.

¹H NMR (400 MHz, DMSO-*d*₆) δ 13.01 (d, *J =* 15.6 Hz, 1H), 11.38 (s, 1H), 7.95 (d, *J =* 2.4 Hz, 1H), 7.46 - 7.35 (m, 1H), 7.02 (dd, *J =* 13.2, 10.4 Hz, 1H), 6.83 (d, *J =* 14.0 Hz, 1H), 6.67 - 6.52 (m, 1H), 4.58 (ddt, *J =* 30.2, 22.6, 7.6 Hz, 1H), 4.43 - 4.24 (m, 1H), 4.08 (t, *J =* 13.6 Hz, 2H), 3.67 - 3.37 (m, 1H), 3.04 - 3.00 (m, 2H), 2.94 - 2.84 (m, 2H), 2.67 (t, *J =* 13.2 Hz, 3H), 2.47 (d, *J= 4.4* Hz, 3H), 2.35 - 2.11 (m, 4H), 1.95 (s, 3H), 1.74 (t, *J* = 10.0 Hz, 1H), 1.62 (d, *J =* 12.6 Hz, 2H), 1.45 (d, *J =* 11.3 Hz, 4H), 1.32 (d, *J =* 17.6 Hz, 4H), 1.23 (s, 5H), 0.90 (dd, *J =* 34.6, 6.4Hz, 2H).

### Example 41: preparation of 3-(7-methyl-1H-indazole-5-yl)-1-(4-(1-methylpiperidine-4-yl)piperazine-1-yl)-1-oxopropan-2-yl)-4-(7-oxo-3,4,7,8-tetrahydro-2H-thiopyrano[2,3-b]pyridine-6-yl)piperidine-1-formate

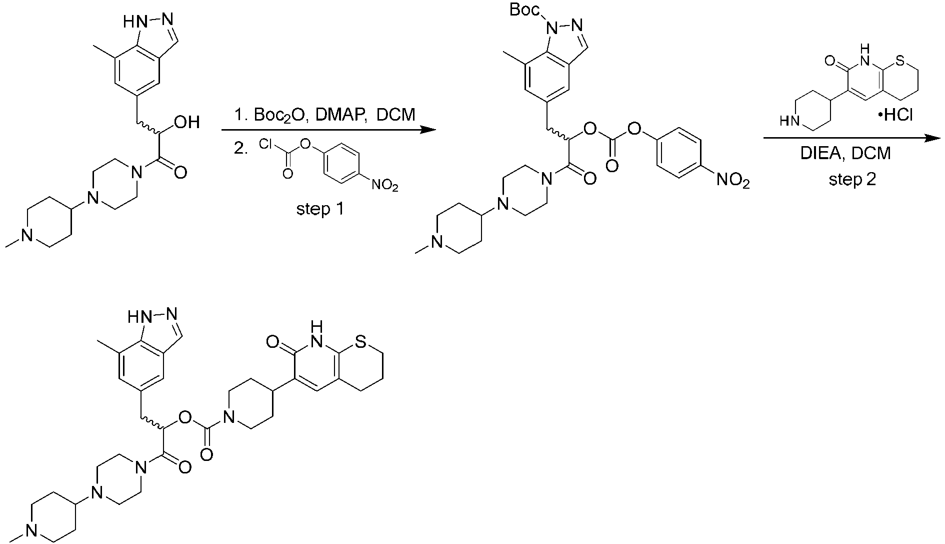

### Step 1: preparation of tert-butyl 7-methyl-5-(3-(4-(1-methylpiperidine-4-yl)piperazine-1-yl)-2-(((4-nitrophenoxy)carbonyl)oxy)-3-oxopropyl)-1H-indazole-1-formate

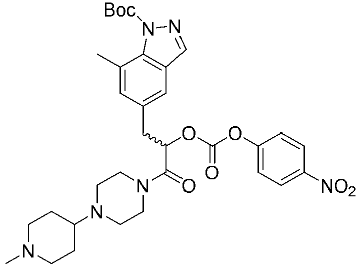

In **ice** water bath, 2-hydroxy-3-(7-methyl-1H-indazole-5-yl)-1-(4-(1-methylpiperidine-4-yl)piperazine-1-yl)propan -1-one (40.0 mg) and 4-dimethylaminopyridine (1.27 mg) were dissolved in anhydrous dichloromethane (2.0 mL), and slowly dropwise added with di-tert-butyl dicarbonate (24.9 mg) to react for 1 hour. The system was added with p-nitrophenyl chloroformate (31.4 mg) in batches and transferred to room temperature to react for 15 hours. After LCMS showed that the reaction was completed, a half of the reaction solution was extracted and directly used in the next step of reaction.

MS (ESI) m/z (M+H)⁺ = 651.9.

### Step 2: preparation of 3-(7-methyl-1H-indazole-5-yl)-1-(4-(1-methylpiperidine-4-yl)piperazine-1-yl)-1-oxopropan-2-yl )-4-(7-oxo-3,4,7,8-tetrahydro-2H-thiopyrano[2,3-b]pyridine-6-yl)piperidine-1-formate

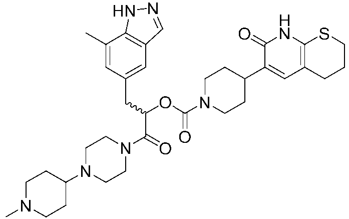

N,N-diisopropylethylamine (7.94 mg) and anhydrous dichloromethane (1.0 mL) were added into the above reaction solution, and added with 6-(piperidine-4-yl)-2,3,4,8-tetrahydro-7H-thiopyrano[2,3-b]pyridine-7- one hydrochloride (9.23 mg) to react for 1 hour. After LCMS showed that the reaction was completed, the reaction solution was filtered, and the filtrate was collected and concentrated. The crude product was purified by a preparative liquid phase to obtain 4.0 mg of the title compound.

MS (ESI) m/z (M+H)⁺ = 661.8.

1H NMR (400 MHz, DMSO-*d*₆) δ 12.99 (s, 1H), 7.98 (s, 1H), 7.41 (s, 1H), 7.04 (s, 1H), 7.05-6.75 (m, 1H), 5.42-5.35 (m, 1H), 4.25-3.89 (m, 3H), 3.65-3.50 (m, 2H), 3.23-2.95 (m, 6H), 2.94-2.66 (m, 5H), 2.48 (s, 3H), 2.43-2.24 (m, 2H), 2.10 (s, 3H), 2.06-1.88 (m, 4H), 1.81-1.60 (m, 5H), 1.50-1.35 (m, 2H), 1.32-1.10 (m, 5H).

### Example 42: preparation of 3-(7-methyl-1H-indazole-5-yl)-1-(4-(1-methylpiperidine-4-yl)piperazine-1-yl)-1-oxopropan-2-yl-4-(6-oxo-2,3,6,7-tetrahydrothieno[2,3-b]pyridine-5-yl)piperidine-1-formate

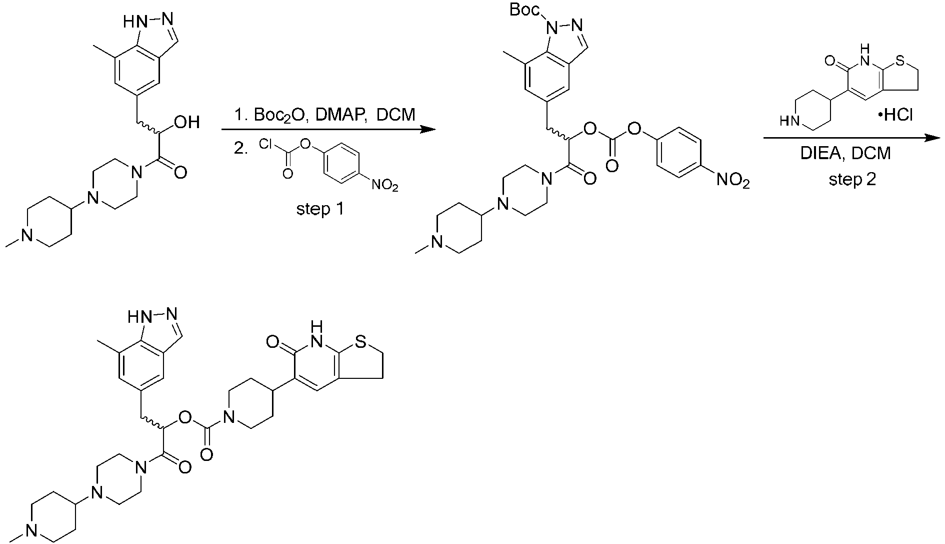

Corresponding common commercially available reagents and the products in the preparation examples of intermediate compound and the examples above were used as raw materials to prepare the title compound by a preparation method similar to that in Example 41 above.

MS (ESI) m/z (M+H)⁺ = 647.8.

¹H NMR (400 MHz, DMSO-*d*₆) *δ* 13.06 (s, 1H), 11.44 (s, 1H), 7.98 (s, 1H), 7.42 (s, 1H), 7.04 (s, 1H), 7.05-6.75 (m, 1H), 5.42-5.31 (m, 1H), 4.25-3.89 (m, 2H), 3.75-3.50 (m, 1H), 3.45-23.35 (m, 3H), 3.20-2.95 (m, 5H), 2.92-2.66 (m, 4H), 2.48 (s, 3H), 2.43-2.24 (m, 2H), 2.10 (s, 3H), 2.06-1.88 (m, 2H), 1.81-1.60 (m, 4H), 1.50-1.35 (m, 2H), 1.35-1.10 (m, 7H).

### Example 43: preparation of (R)-N-(3-(7-methyl-1H-indazole-5-yl-3-d)-1-(4-(1-(methyl-d3)piperidine-4-yl)piperazine-1-y l)-1-oxopropan-2-yl)-4-(6-oxo-2,3,6,7-tetrahydrothieno[2,3-b]pyridine-5-yl)piperidine-1-for mamide

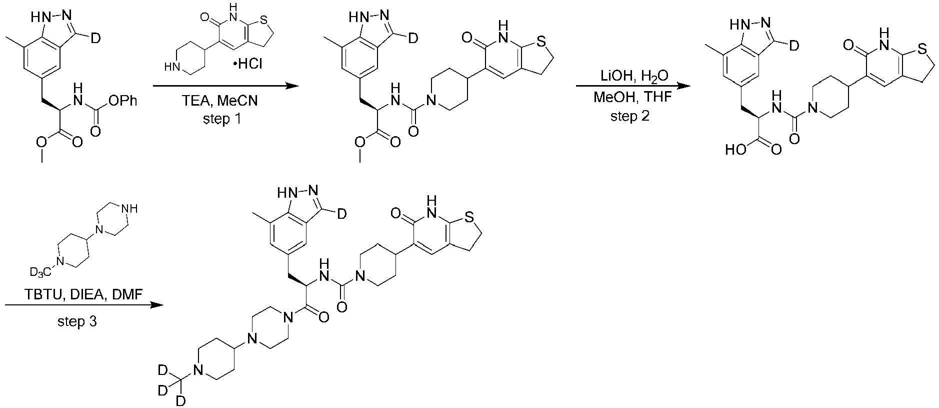

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 650.8.

¹H NMR (400 MHz, DMSO-*d*₆) *δ* 13.00 (s, 1H), 11.43 (s, 1H), 7.36 (s, 1H), 7.09 (s, 1H), 7.00 (s, 1H), 6.65 (d, *J* = 8.0 Hz, 1H), 4.82-4.72 (m, 1H), 4.09 (d, *J* = 16.0, 2H), 3.65-3.50 (m, 1H), 3.45-3.35 (m, 2H), 3.29-3.21 (m, 1H), 3.17-3.03 (m, 2H), 3.00-2.83 (m, 2H), 2.80-2.60 (m, 4H), 2.47 (s, 3H), 2.37-2.31 (m, 1H), 2.25-2.19 (m, 1H), 2.05-1.85 (m, 3H), 1.80-1.69 (m, 2H), 1.67-1.51 (m, 3H), 1.44-1.34 (m, 2H), 1.32-1.13 (m, 6H).

### Example 44: preparation of (R)-N-(3-(7-methyl-1H-indazole-5-yl-3-d)-1-(4-(1-methylpiperidine-4-yl)piperazine-1-yl)-1-oxopropan-2-yl)-4-(6-oxo-2,3,6,7-tetrahydrothieno[2,3-b]pyridine-5-yl)piperidine-1-forma mide

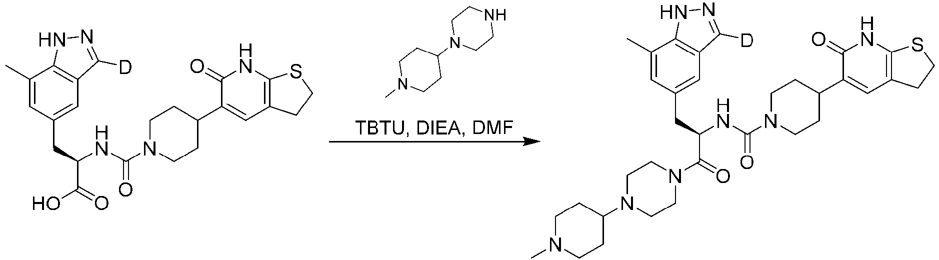

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 647.8.

¹H NMR (400 MHz, DMSO-*d*₆) *δ* 13.00 (s, 1H), 11.44 (s, 1H), 7.36 (s, 1H), 7.09 (s, 1H), 7.00 (s, 1H), 6.65 (d, J = 8.0 Hz, 1H), 4.82-4.72 (m, 1H), 4.09 (d, J = 12.0 Hz, 2H), 3.65-3.50 (m, 1H), 3.45-3.35 (m, 2H), 3.29-3.21 (m, 1H), 3.13-3.02 (m, 2H), 3.00-2.83 (m, 2H), 2.79-2.61 (m, 4H), 2.47 (s, 3H), 2.37-2.31 (m, 1H), 2.25-2.19 (m, 1H), 2.10 (s, 3H), 2.05-1.85 (m, 2H), 1.80-1.69 (m, 2H), 1.67-1.51 (m, 3H), 1.44-1.34 (m, 2H), 1.32-1.13 (m, 7H).

### Example 45: preparation of N-((R)-3-(7-methyl-1H-indazole-5-yl)-1-(4-(1-methylpiperidine-4-yl)piperazine-1-yl)-1-oxop ropan-2-yl)-4-(2-oxo-1,2,5,6,7,8-hexahydro-5,8-methanoquinoline-3-yl)piperidine-1-forma mide

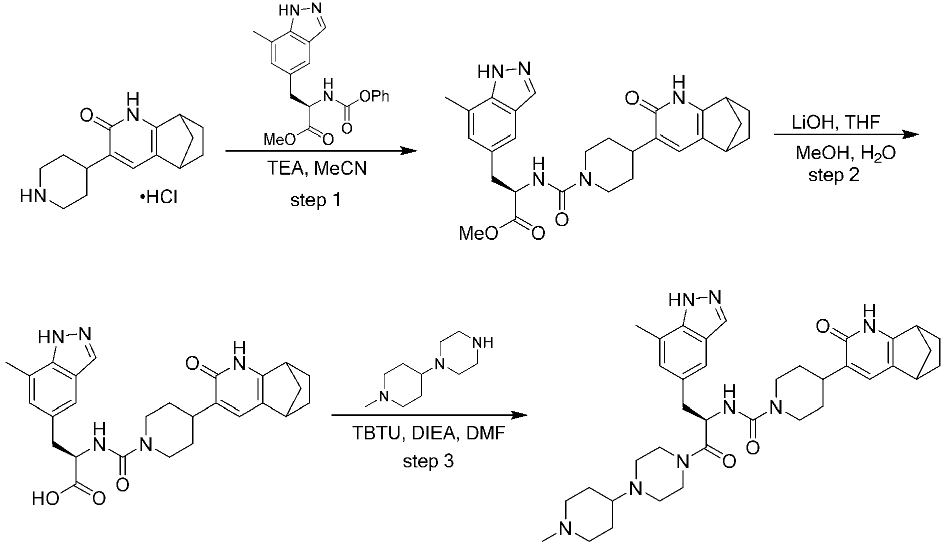

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 655.4

¹H NMR (400 MHz, DMSO-*d*₆) δ 13.01 (s, 1H), 11.72 (s, 1H), 7.96 (s, 1H), 7.36 (s, 1H), 7.06 (s, 1H), 7.00 (s, 1H), 6.63 (d, *J =* 8.0 Hz, 1H), 4.77 (q, *J =* 7.7 Hz, 1H), 4.08 (d, *J =* 12.7 Hz, 2H), 3.55 (d, *J =* 13.6 Hz, 1H), 3.38-3.33(m, 1H), 3.26 - 3.20 (m, 2H), 3.18 - 3.05 (m, 2H), 3.00 - 2.84 (m, 2H), 2.76-2.60 (m, 5H), 2.47 (s, 3H), 2.37 - 2.29 (m, 1H), 2.27 - 2.18 (m, 1H), 2.09 (s, 3H), 1.99 - 1.52 (m, 10H), 1.43 - 1.33 (m, 3H), 1.29-1.21 (m, 4H), 1.06 - 0.96 (m, 2H).

### Example 46: preparation of (R)-N-(3-(7-methyl-1H-indazole-5-yl)-1-(4-(1-methylpiperidine-4-yl)piperazine-1-yl)-1-oxop ropan-2-yl)-4-(7'-oxo-7',8'-dihydro-2'H,4'H-spiro[cyclopropane-1,3'-thiopyrano[2,3-b]pyri dine]-6'-yl)piperidine-1-formamide

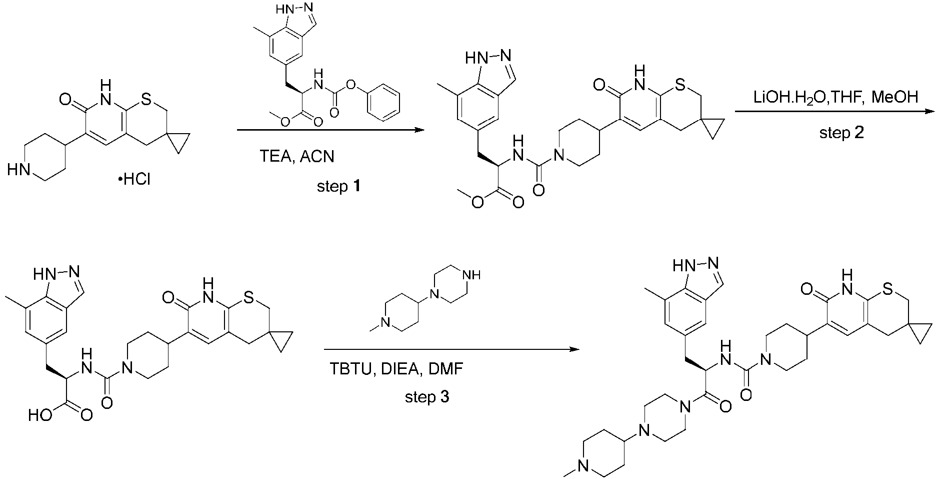

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 687.4.

¹H NMR (400 MHz, DMSO-*d*₆) *δ* 13.01 (s, 1H), 11.36 (s, 1H), 7.95 (s, 1H), 7.35 (s, 1H), 7.00 (s, 1H), 6.78 (s, 1H), 6.64 (d, *J* = 8.0 Hz, 1H), 4.76 (q, *J* = 7.6 Hz, 1H), 4.08 (d, *J* = 12.8 Hz, 2H), 3.55 (d, *J* = 13.2 Hz, 1H), 3.23 (s, 1H), 3.10 (t, *J =* 9.6 Hz, 1H), 2.95 (dd, *J =* 13.2, 7.6 Hz, 1H), 2.88 (d, *J =* 16.0 Hz, 3H), 2.67 (dt, *J* = 25.6, 11.2 Hz, 5H), 2.47 (s, 3H), 2.41 (s, 2H), 2.33 (s, 1H), 2.22 (s, 1H), 2.09 (s, 3H), 1.92 (dt, *J* = 23.6, 11.2 Hz, 2H), 1.72 (t, *J* = 11.6 Hz, 2H), 1.64 (d, *J=* 12.6 Hz, 2H), 1.54 (t, *J =* 9.2 Hz, 1H), 1.38 (d, *J =* 12.0 Hz, 2H), 1.32 - 1.09 (m, 5H), 0.68 - 0.50 (m, 4H).

### Example 47: preparation of N-((R)-3-(7-methyl-1H-indazole-5-yl)-1-(4-(1-methylpiperidine-4-yl)piperazine-1-yl)-1-oxop ropan-2-yl)-4-(5-oxo-1,1a,2,4,5,7b-hexahydrocyclopropo[4,5]thiopyrano[2,3-b]pyridine-6-yl )piperidine-1-formamide

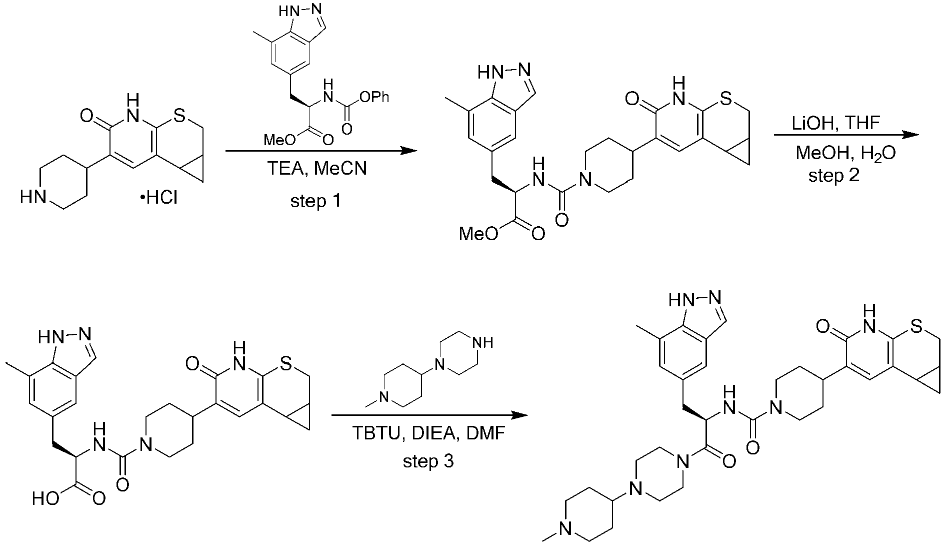

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 673.3

¹H NMR (400 MHz, DMSO-*d*₆) δ 13.01 (s, 1H), 11.31 (s, 1H), 7.96 (s, 1H), 7.36 (s, 1H), 7.16 (d, *J* = 7.5 Hz, 1H), 7.00 (s, 1H), 6.65 (d, *J* = 7.9 Hz, 1H), 4.77 (q, *J =* 7.7 Hz, 1H), 4.11 (d, *J =* 12.9 Hz, 2H), 3.56 (d, *J* = 13.3 Hz, 1H), 3.40-3.36 (m, 1H),3.24-3.19 (m, 1H), 3.12-3.06 (m, 3H), 2.99-2.93 (m, 1H), 2.90-2.84 (m, 1H), 2.77 - 2.60 (m, 5H), 2.47 (s, 3H), 2.36-2.29 (m, 1H), 2.26 - 2.17 (m, 1H), 2.09 (s, 3H), 1.97 - 1.78 (m, 4H), 1.76 - 1.61 (m, 4H), 1.52 (t, *J =* 9.8 Hz, 1H), 1.42 - 1.27 (m, 5H), 1.24 - 1.10 (m, 2H), 0.85-0.77 (m, 1H).

### Example 48: preparation of (R)-N-(3-(7-methyl-1H-indazole-5-yl)-1-(4-(1-methylpiperidine-4-yl)piperazine-1-yl)-1-oxop ropan-2-yl)-4-(7-oxo-7,8-dihydro-2H-thiopyrano[2,3-b]pyridine-6-yl)piperidine-1-formami de

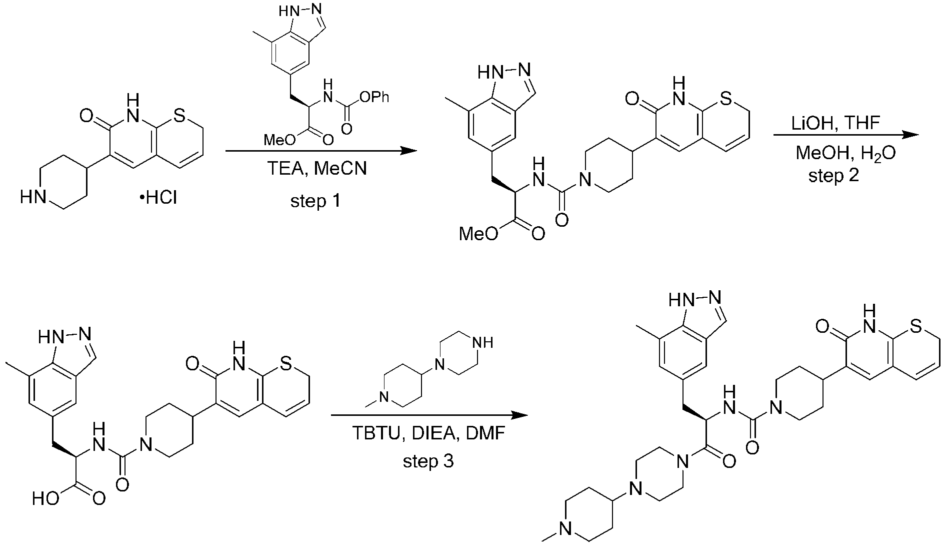

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 659.3 .

¹H NMR (400 MHz, DMSO-*d*₆) δ 13.01 (s, 1H), 11.57 (s, 1H), 7.96 (s, 1H), 7.36 (s, 1H), 7.02 (s, 1H), 6.99 (s, 1H),6.65 (d, *J* = 7.9 Hz, 1H), 6.33 (d, *J=* 9.9 Hz, 1H), 5.62-5.57 (m, 1H), 4.79-4.73 (m, 1H), 4.10 (d, *J =* 12.5 Hz, 2H), 3.63 - 3.50 (m, 3H), 3.30-3.18 (m, 1H), 3.14 - 3.05 (m, 1H), 3.05 - 2.84 (m, 3H), 2.75 - 2.62 (m, 5H), 2.47 (s, 3H), 2.35-2.28 (m, 1H), 2.24-2.16 (m, 1H), 2.10 (s, 3H), 2.00 - 1.86 (m, 2H), 1.77 - 1.61 (m, 4H), 1.51 (t, *J =* 8.7 Hz, 1H), 1.39 - 1.11 (m, 6H).

### Example 49: preparation of (R)-N-(3-(7-methyl-1H-indazole-5-yl)-1-oxo-1-(4-(piperidine-4-yl)piperazine-1-yl)propan-2-yl)-4-(7-oxo-3,4,7,8-tetrahydro-2H-thiopyrano[2,3-b]pyridine-6-yl)piperidine-1-formamide

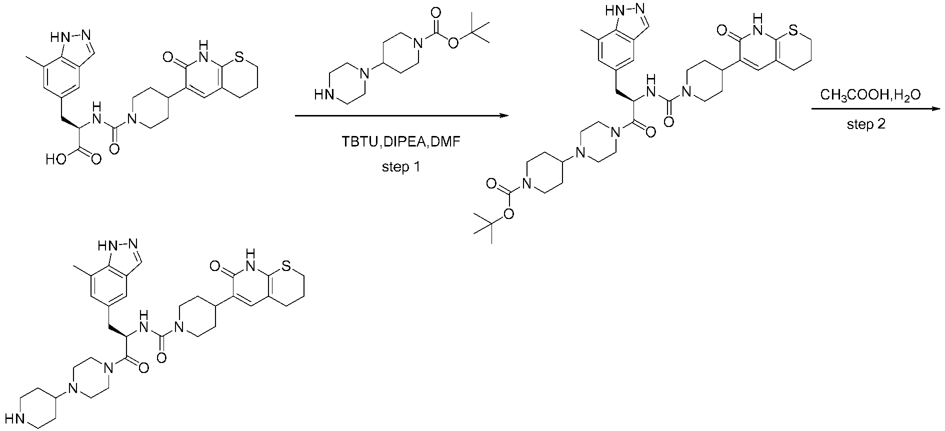

### Step 1: preparation of tert-butyl (R)-4-(4-(3-(7-methyl-1H-indazole-5-yl)-2-(4-(7-oxo-3,4,7,8-tetrahydro-2H-thiopyrano[2,3-b]py ridine -6- yl)piperidine-1-formylamino)propionyl)piperazine-1-yl)piperidine-1-formate

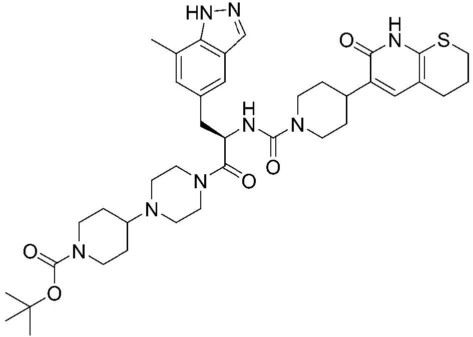

(R)-3-(7-methyl-1H-indazole-5-yl)-2-(4-(7-oxo-3,4,7,8-tetrahydro-2H-thiopyrano[2,3-b]pyridine -6-yl)piperidine-1-formylamino)propionic acid (66 mg), tert-butyl 4-(piperazine-1-yl)piperidine-1-formate (43 mg) and N,N-diisopropylethylamine (43 mg) were dissolved in N,N-dimethylformamide (2 mL), and added with 2-(1H-benzotriazol-1-yl)-1,1,3,3-tetramethylurea tetrafluoroborate (43 mg) to react at room temperature for 4 hours. After the reaction was ended, the reaction solution was separated and purified by preparative HPLC to obtain 30 mg of the title compound.

MS (ESI) m/z (M+H)⁺ = 747.3 .

### Step 2: preparation of (R)-N-(3-(7-methyl-1H-indazole-5-yl)-1-oxo-1-(4-(piperidine-4-yl)piperazine-1-yl)propan-2-yl)-4-(7-oxo-3,4,7,8-tetrahydro-2H-thiopyrano[2,3-b]pyridine-6-yl)piperidine-1-formamide

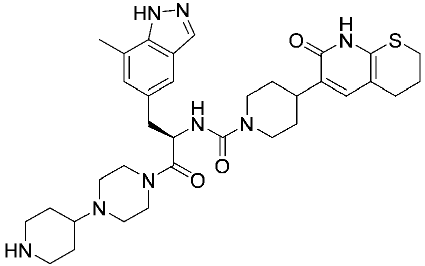

Tert-butyl (R)-4-(4-(3-(7-methyl-1H-indazole-5-yl)-2-(4-(7-oxo-3,4,7,8-tetrahydro-2H-thiopyrano[2,3-b]py ridine -6- yl)piperidine-1-formylamino)propionyl)piperazine-1-yl)piperidine-1-formate (30 mg) was dissolved in acetic acid and water (2 mL, v/v = 1/5) to react at 100°C for 2 hours. After the reaction was ended, the reaction system was concentrated, the crude product was dissolved in an appropriate amount of methanol, then added with a saturated potassium carbonate solution to adjust a pH value of the system to be 8-9 and filtered, and the filtrate was separated and purified by preparative HPLC, and freeze-dried to obtain 10.00 mg of the title compound.

MS (ESI) m/z (M+H)⁺ = 647.3 .

1H NMR (400 MHz, DMSO-d6) δ 13.02 (s, 1H), 7.96 (s, 1H), 7.36 (s, 1H), 7.00 (s, 1H), 6.86 (s, 1H), 6.63 (d, J = 8.0 Hz, 1H), 4.77 (q, J = 7.7 Hz, 1H), 4.08 (d, J = 12.6 Hz, 2H), 3.60-3.47 (m, 1H), 3.30-3.20 (m, 1H), 3.15-3.06 (m, 2H), 3.05 - 2.84 (m, 7H), 2.75 - 2.59 (m, 3H), 2.56-2.51 (m, 2H), 2.47 (s, 3H), 2.41 - 2.18 (m, 4H), 2.06-1.91 (m, 4H), 1.70 - 1.52 (m, 3H), 1.41 - 1.17 (m, 4H), 1.09 - 0.97 (m, 2H).

### Example 50: preparation of (R)-N-(3-(7-methyl-1H-indazole-5-yl)-1-oxo-1-(4-(piperidine-4-yl)piperazine-1-yl)propan-2-yl)-4-(6-oxo-2,3,6,7-tetrahydrothieno[2,3-b]pyridine-5-yl)piperidine-1-formamide

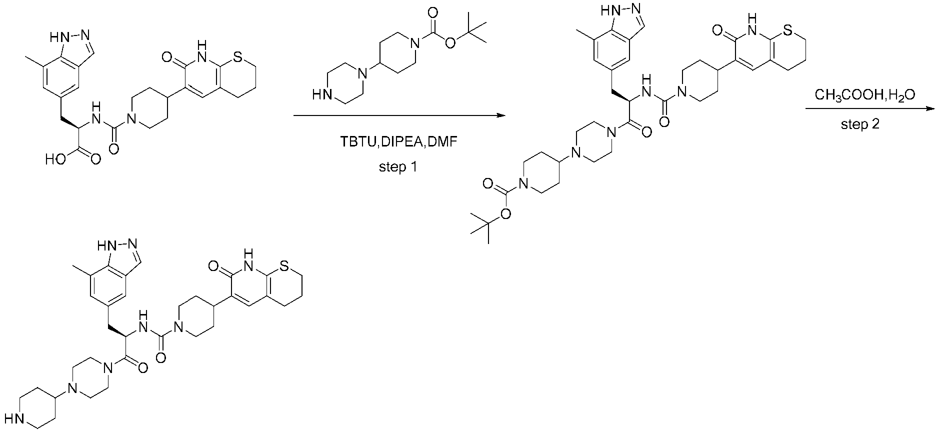

Corresponding common commercially available reagents and the products in the preparation examples of intermediate compound and the examples above were used as raw materials to prepare the title compound by a preparation method similar to that in Example 49 above.

MS (ESI) m/z (M+H)⁺ = 633.3 .

¹H NMR (400 MHz, DMSO-*d*₆) δ 13.02 (s, 1H), 7.96 (s, 1H), 7.36 (s, 1H), 7.08 (s, 1H), 7.00 (s, 1H), 6.64 (d, *J =* 8.1 Hz, 1H), 4.77 (q, *J =* 7.7 Hz, 1H), 4.15 - 4.06 (m, 2H), 3.63-3.48(m, 1H), 3.16 - 3.10 (m, 1H), 3.06 (t, *J =* 8.1 Hz, 2H), 2.99 - 2.84 (m, 5H), 2.75 - 2.60 (m, 4H), 2.47 (s, 3H), 2.38 - 2.20 (m, 4H), 2.05 - 1.95 (m, 2H), 1.69 - 1.54 (m, 3H), 1.39 - 1.20 (m, 7H), 1.11-0.97 (m, 2H).

### Example 51: preparation of (R)-N-(3-(7-methyl-1H-indazole-5-yl)-1-(4-(1-methylpiperidine-4-yl)piperazine-1-yl)-1-oxop ropan-2-yl)-4-(2-oxo-2,5,6,7-tetrahydro-1H-cyclopentadieno[b]pyridine-3-yl)piperidine-1-f ormamide

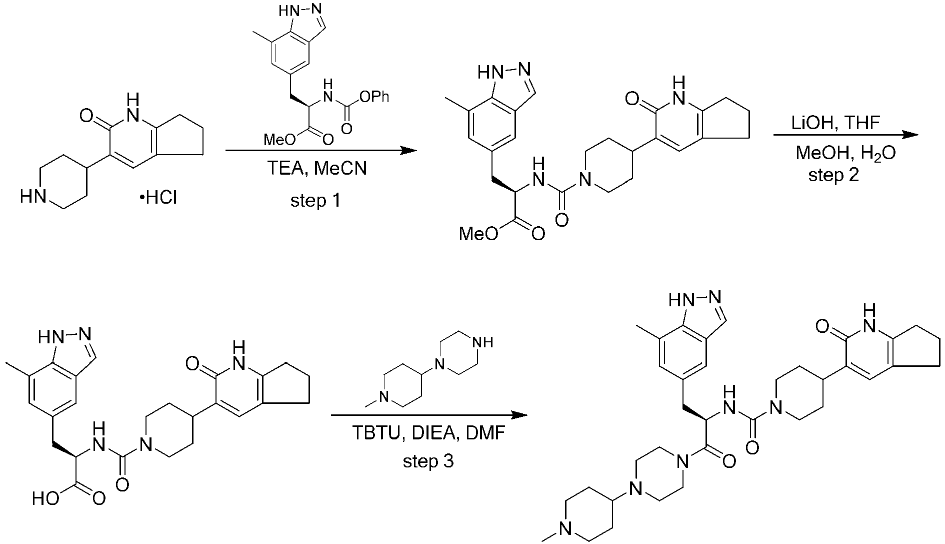

Corresponding common commercially available reagents and the products in the preparation examples of intermediate compound and the examples above were used as raw materials to prepare the title compound by a preparation method similar to that in Example 11 above.

MS (ESI) m/z (M+H)⁺ = 629.3 .

¹H NMR (400 MHz, DMSO-*d*₆) δ 13.01 (s, 1H), 11.66 (s, 1H), 7.96 (s, 1H), 7.36 (s, 1H), 7.03 (s, 1H), 7.00 (d, *J* = 1.2 Hz, 1H), 6.64 (d, *J* = 8.1 Hz, 1H), 4.77 (q, *J* = 7.7 Hz, 1H), 4.16 - 4.03 (m, 2H), 3.59 - 3.49 (m, 1H), 3.38-3.36 (m, 1H), 3.28 - 3.19 (m, 1H), 3.15-3.07 (m, 1H), 2.99-2.92 (m, 1H), 2.91-2.84 (m, 1H), 2.79 - 2.56 (m, 9H), 2.47 (s, 3H), 2.37 - 2.28 (m, 1H), 2.26 - 2.17 (m, 1H), 2.09 (s, 3H), 2.04 - 1.84 (m, 4H), 1.78 - 1.60 (m, 4H), 1.54 (t, *J* = 8.8 Hz, 1H), 1.41-1.33 (m, 2H), 1.29 - 1.09 (m, 4H).

### Example 52: preparation of (R)-N-(3-(7-methyl-1H-indazole-5-yl)-1-(4-(1-methylpiperidine-4-yl)piperazine-1-yl)-1-oxop ropan-2-yl)-4-(2-oxo-1,2,5,6,7,8-hexahydroquinoline-3-yl)piperidine-1-formamide

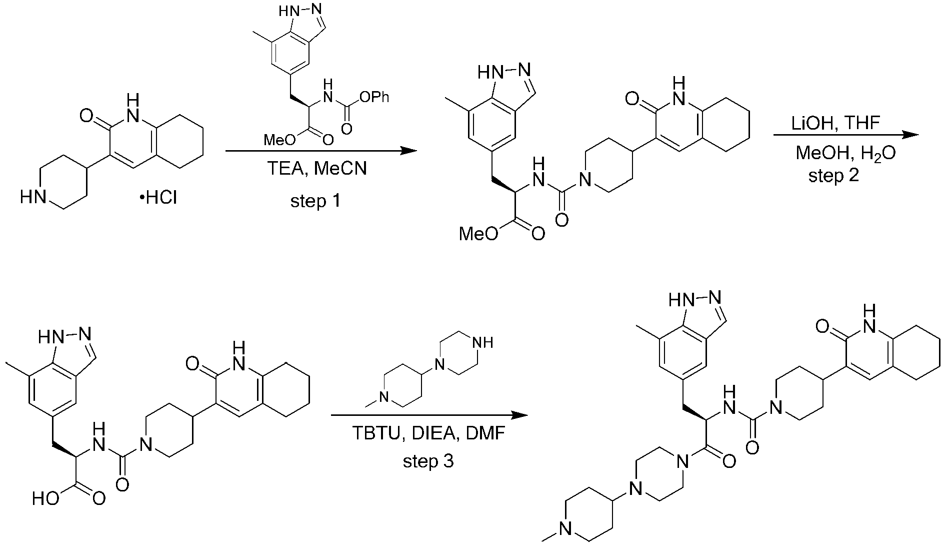

Corresponding common commercially available reagents and the products in the preparation examples of intermediate compound and the examples above were used as raw materials to prepare the title compound by a preparation method similar to that in Example 11 above.

MS (ESI) m/z (M+H)+ = 643.4.

¹H NMR (400 MHz, DMSO-*d*₆) δ 13.02 (s, 1H), 11.23 (s, 1H), 7.96 (s, 1H), 7.37 (s, 1H), 7.00 (t, *J* = 1.2 Hz, 1H), 6.85 (s, 1H), 6.64 (d, *J* = 8.1 Hz, 1H), 4.77 (q, *J =* 7.7 Hz, 1H), 4.10 (d, *J =* 12.9 Hz, 2H), 3.56 (d, *J =* 13.4 Hz, 1H), 3.24 (t, *J =* 10.2 Hz, 1H), 3.12 (t, *J =* 9.9 Hz, 1H), 2.97 (dd, *J* = 13.1, 7.9 Hz, 1H), 2.88 (dd, *J =* 13.1, 7.0 Hz, 1H), 2.82 - 2.54 (m, 5H), 2.48 (s, 3H), 2.39 (d, *J* = 19.6 Hz, 5H), 2.23 (d, *J =* 11.0 Hz, 1H), 2.10 (s, 3H), 2.03 - 1.80 (m, 2H), 1.78 - 1.60 (m, 7H), 1.55 (t, *J* = 9.2 Hz, 1H), 1.38 (d, *J =* 12.3 Hz, 2H), 1.32 - 1.08 (m, 6H).

### Biological test data

Unless otherwise specified, the experimental materials, reagents, operations and methods used in the following activity test examples were all commercially available, or easily known or prepared based on the prior art.

### Test Example 1: determination of cell functional antagonism - cAMP determination

1.Experimental principle
A CGRP receptor complex was coupled with Gs in G protein, the combination of CGRP and CGRP receptor complex led to Gs activation, and cAMP (3',5'-cyclic adenosine monophosphate) was produced.
2.Experimental purpose
The ability of the compound of the present invention to inhibit CGRP-stimulated cAMP formation in SK-N-MC cells was determined.
3.Experimental materials
3.1Experimental cell line:
SK-N-MC (neuroepithelioma cells) from National Science and Technology Infrastructure - Biomedical Materials and Cell Resources.
3.2Reagents and consumables

| Name | Brand | Article number |
|---|---|---|
| LANCE Ultra cAMP Kit | PerkinElmer | TRF0262 |
| α-CGRP (human) | MCE | HY-P1071 |
| IBMX | sigma | I7018-100MG |
| HBSS (1×) | Gibco | 14025076 |
| HEPES 1M | Gibco | 15630080 |

4.Experimental steps
4.1Cell preparation:
   The SK-N-MC cells were resuscitated and cultured in advance, and a cell confluence degree should be 70%-80% before use.
4.2A sample dilution, a tested compound and α-CGRP (human) were prepared.
4.3Cell seeding and drug effect:
   The SK-N-MC cells were digested, centrifuged, then resuspended with the sample dilution, and inoculated into a 384-well plate. Each well was added with the α-CGRP (human) and the tested compound respectively, and blown and mixed evenly. The well plate was adhered with a sealing film, and placed on an enzyme-labeled incubator to incubate at 25°C.
4.4cAMP determination: carried out according to LANCE Ultra cAMP Kit specification Eu-cAMP tracer and ULight-anti-cAMP working solutions were prepared in the dark, respectively added into the well plate, and blown and mixed evenly. The well plate was adhered with a sealing film, and placed on an enzyme-labeled incubator to incubate at 25°C.
4.5A luminescence value was read by a chemiluminescent module of an automatic enzyme-labeled instrument.
4.6Data analysis:
   A compound concentration LOG value was taken as an abscissa and Inhibition was taken as an ordinate to carry out fitting calculation on an IC₅₀ value by a nonlinear formula, and results were as shown in the table below.

IC₅₀ values of compounds of the present invention to inhibit CGRP-stimulated cAMP formation in SK-N-MC cells

| Serial number of example | IC₅₀ (nM) | Serial number of example | IC₅₀ (nM) |
|---|---|---|---|
| 1 | 1.35 | 2 | 0.65 |
| 3 | 0.62 | 4 | 1.00 |
| 5 | 1.94 | 6 | 0.21 |
| 7 | 6.02 | 8 | 0.41 |
| 9 | 0.86 | 10 | 9.40 |
| 11 | 7.83 | 12 | 16.88 |
| 13 | 1.44 | 14 | 6.85 |
| 15 | 10.08 | 16 | 0.71 |
| 17 | 15.45 | 18 | 0.95 |
| 19 | 0.39 | 20 | 0.37 |
| 21 | 589.60 | 22 | 0.81 |
| 23 | 1.12 | 24 | 1.02 |
| 25 | 0.79 | 26 | 0.10 |
| 27 | 1.24 | 28 | 10.10 |
| 29 | 0.23 | 30 | 0.19 |
| 31 | 2.05 | 32 | 0.29 |
| 33 | 0.37 | 34 | 0.20 |
| 35 | 0.69 | 36 | 4.61 |
| 37 | 0.15 | 38 | 0.07 |
| 39 | 0.13 | 40 | 3.47 |
| 41 | 0.38 | 42 | 0.27 |
| 43 | 0.10 | 44 | 0.06 |
| 45 | 26.04 | 46 | 0.15 |
| 47 | 0.24 | 48 | 0.15 |
| 49 | 0.14 | 50 | 0.07 |
| 51 | 1.94 | 52 | 1.04 |

### 5.Conclusion

The novel compounds provided by the present invention could significantly inhibit CGRP-stimulated cAMP formation, and had lower IC₅₀ values. Especially, the compounds represented by Examples 1, 2, 3, 4, 5, 6, 8, and 9 had remarkable inhibitory activities, and the IC₅₀ values of the compounds could reach a pM level.

The above experimental results showed that the compounds in the examples with different structures showed considerable differences in activity. Taking the compounds in Example 21 (IC₅₀: 589.60 nM) and Example 38 (IC₅₀: 0.07 nM) as examples, there was an 8000 times difference in inhibitory activity between the two compounds. Therefore, a general formula structure based on formula I of the present invention was disclosed, wherein different selections of variable groups had an important influence on the inhibitory activities of the compounds.

### Test Example 2: Competitive binding determination of in-vitro [¹²⁵I] CGRP

### 1. Experimental purpose:

The ability of the compound to compete for a radiolabelled endogenous peptide human αCGRP ([¹²⁵I] CGRP) was determined.

### 2. Experimental principle:

The SK-N-MC cells endogenously expressed a CGRP receptor with the same sequence as the human CGRP receptor. A tissue homogenate of SK-N-MC cell membrane was used as a receptor source, and the radiolabelled endogenous peptide human αCGRP ([¹²⁵I] CGRP) and the compound competitively bound with the receptor source. After washing the unbound part of [¹²⁵I] CGRP, the binding of the reaction compound on the receptor could be detected by a radioactive signal.

### 3. Experimental materials:

3.1 Experimental cell line: SK-N-MC (human neuroepithelioma cells) from Cell Resource Center, Peking Union Medical College
3.2 Detection equipment: MicroBeta2 (PerkinElmer), and UNIFILTER-96 well cell collector (PerkinElmer, C961961)
3.3 Experimental reagents and consumables

| Name | Brand | Article number |
|---|---|---|
| Tris base | Sigma | T1503-1KG |
| MgCl₂ | Sigma | M1028 |
| PEI (Poly ethyleneimine) | sigma | P3143 |
| Bovine Serum Albumin | Sigma | B2064-50G |
| HEPES 1M | Gibco | 15630080 |
| Microscint 20 cocktail | PerkinElmer | 6013329 |
| Unifilter-96 GF/B filter plates | Perkin Elmer | 6055690 |
| 96 well conical polypropylene plates | Agilent | 5042-1385 |
| TopSeal-A sealing film | Perkin Elmer | 6050185 |
| ¹²⁵I-CGRP (Human) | | NET354050UC |
| α CGRP (Human) | MCE | HY-P1071 |

3.4 Experimental steps
3.4.1 Extraction and quantification of membrane protein: fresh SK-N-MC cells were collected. The cell pellet was resuspended with an experimental buffer (Tris-HCl 50 mM, pH 7.4). The cells were homogenized 10 times by a homogenizer, and centrifuged at 1000 g and 4°C for 10 minutes, then nuclear/mitochondrial fragments were removed, and a supernatant tissue homogenate was collected. The supernatant was centrifuged at 50000 g and 4°C for 1 hour, and then a supernatant was removed. Particles were suspended with an appropriate buffer, and after determing the protein concentration, the suspension was packaged and then stored at -80°C.
3.4.2 Determination of binding Ki (inhibition constant) of compound: 1 µL of continuously diluted tested compound was transferred to an assay plate. 50 µL of membrane stock solution (15 µg) and 50 µL of ¹²⁵I-CGRP (Human) with a final concentration of 200 pM were added into the plate, and shaken and mixed evenly. Each well was added with 50 µL of 0.3% PEI to soak the mixture for 1 hour. The reaction mixture was filtered by a UNIFILTER-96 well cell collector, and the plate was washed with a pre-cooled buffer. After the filter plate was dried, the bottom of the well plate was sealed with a Unifilter-96 backing seal tape, each well was added with 50 µL of Microscint 20 cocktail, and the top of the well plate was sealed with a TopSeal-A sealing film. A ³H signal was captured by a MicroBeta2 Reader counting filter.
3.4.3 Data analysis

Data were analyzed by Prism 5. A "log(inhibitor) vs. response - Variable slope" model was used for data fitting, and a Kd value of 240.6 pM determined for the ¹²⁵I-CGRP (Human) in a saturation binding assay was used to calculate IC₅₀ and Ki (Ki = IC₅₀/(1+[L]/Kd), [L]: Radioligand concentration (200pM)) values of the compound and the ¹²⁵I-CGRP (Human) competitively bound with the SK-N-MC cell membrane.

### 4. Experimental results

The Ki values of the compounds in the representative examples of the present invention which bound with the CGRP receptor were as shown in the following table:

| Compound | Ki (pM) | Ki ratio |
|---|---|---|
| Zavegepant | 49.76 | / |
| Example 6 | 19.48 | 0.39 |
| Example 8 | 11.58 | 0.23 |
| Example 30 | 16.62 | 0.33 |

| | | |
|---|---|---|
| Note: Ki ratio= Ki (compound)/Ki (Zavegepant). | | |

According to in-vitro data in the above table, affinities of the compounds of the present invention to the CGRP receptor reached a pM level, and the compounds showed better in-vitro target affinity compared with the Zavegepant.

### Test Example 3: stability test in liver microsomes

### 1. Experimental purpose

The stability of the compound of the present invention in liver microsomes of rat, monkey and human was determined. Tested compounds of the present invention were the compounds in Examples 6, 8 and 29, and a control drug was Zavegepant.

### 2. Reagents and consumables

| Name of reagent | Supplier | Batch number | Concentration (mg protein/mL) |
|---|---|---|---|
| Liver microsome of rat | RILD | WJYW | 20 |
| Liver microsome of monkey | RILD | MZYM | 20 |
| Liver microsome of human | BioreclamationIVT | YZB | 20 |

### 3 . Experimental steps

### 3.1 Test incubation system

| Concentration of liver microsome | Tested substance: 0.5 mg/mL; and Verapamil: 0.2 mg/mL |
|---|---|
| Incubation buffer | Phosphate buffer (100 mM, pH 7.4) |
| Concentration of tested substance | 1.0 µM |
| Incubation time | 0, 5, 15, 30, 45 and 60 minutes |
| MgCl₂ | 3.0 mM |
| NADPH | 1.0 mM |
| Parallel test | Two parts |

3.2 An appropriate amount of liver microsome solution was transferred into a 1 mL 96-deep-well plate, added with a tested drug liquid (or a probe substrate solution), and pre-incubated in a thermal mixer at 37°C for 5 minutes. Two parts of mixed solutions were taken out from the system and added with 1×PBS to replace NADPH, and the mixed solutions were taken out at 0 and 60 minutes respectively, and added with methanol (containing an internal standard) to terminate the reaction. In the remaining mixed system, each well was added with NADPH to start the reaction. In the tested substance group, mixed solutions were taken out from the system at 0, 5, 15, 30, 45 and 60 minutes, and in the control group, mixed solutions were taken out from the system at 0, 30 and 60 minutes, and the mixed solutions were added methanol (containing an internal standard) to terminate the reaction. All samples of the terminated reaction were mixed evenly and centrifuged in a centrifuge at 3800 rpm for 15 minutes, and the supernatant was taken for LC-MS/MS analysis.

### 3.3 Data analysis

A peak area was determined by an extracted ion chromatogram. A slope value k was determined by linear regression of a remaining percentage of a parent drug relative to a natural logarithm of an incubation time curve. According to the slope, in-vitro half-life periods (t_{1/2}) were respectively calculated, and in-vitro intrinsic clearance rates (CLᵢₙₜ, represented by µL/min/mg protein) were calculated. Calculation formulas were as follows: ${t}_{1 / 2} = ln 2 / k = 0.693 / k ; and {CL}_{int} = 0.693 / {t}_{1 / 2} / liver microsome protein concentration$

Experimental results were as shown in the following table:

### Stability data of compounds of the present invention in liver microsomes of rat, monkey and human

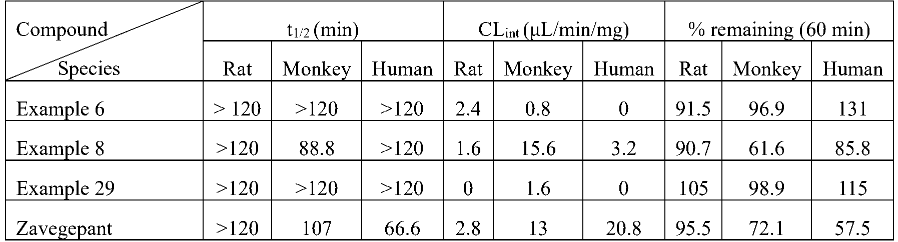

### Test Example 4: plasma protein binding rate test

### 1. Experimental purpose

Protein binding rates of the compound of the present invention in plasma of rat, monkey and human were determined by an equilibrium dialysis method. Tested compounds of the present invention were the compounds in Examples 6, 8 and 29, and a control drug was Zavegepant.

### 2. Test matrix

| **Matrix/species** | **Anticoagulant** |
|---|---|
| Plasma of SD rat | EDTA-K₂ |
| Plasma of cynomolgus monkey | EDTA-K₂ |
| Plasma of human | EDTA-K₂ |

### 3. Experimental steps

3.1 A dry dialysis membrane was rinsed with ultrapure water for 2-3 times, and then soaked in a phosphate buffer for 1 hour for later use. Polytetrafluoroethylene module was soaked in 20% ethanol for 30 minutes, and then surface moisture was dried with dust-free paper for later use. The pretreated dialysis membrane was assembled into a dialysis plate according to the product specification, and one side (receptor compartment) of an osmotic membrane in each dialysis well was added with 100 µL of receptor fluid (100 mM phosphate buffer solution plus 0.002% Tween 80).

3.2 The plasma was put into a centrifuge tube, a working solution of a tested substance was added into the plasma, wherein a final concentration was 1 µM, and the mixture was turned upside down and mixed evenly (this step was carried out in ice bath). 20 µL of drug-containing plasma each was taken, and was respectively transferred to a 96-well sample plate according to two parts in parallel, and stored in a refrigerator at -20°C as a T₀ sample.

3.3 Another 100 µL of the above drug-containing plasma was transferred to the other side (sample compartment) of the membrane in the dialysis device according to two parts in parallel, and incubated under vibration at a constant temperature of 37°C for 6 hours. After 6 hours of incubation, 20 µL of the plasma was respectively taken out from the receptor compartment and the sample compartment after equilibration to obtain samples B and A. The samples B and A were respectively added with corresponding volumes of corresponding blank plasma or phosphate buffer solution (containing 0.002% Tween 80), so that a volume ratio of the plasma to the buffer solution in each sample well was 1: 1.

3.4 All sample wells were added with 250 µL of methanol solution containing an internal standard, mixed evenly and then centrifuged in a centrifuge at 3800 rpm for 10 minutes. 20 µL of supernatant was added with 180 µL of methanol, and mixed evenly in a vortexed mode for LC-MS/MS sample injection analysis.

### 3.5 Data analysis

A plasma protein binding rate and a recovery rate of the compound in plasma were calculated by the following formulas: $dissociation percentage \left(%\right) = {C}_{B} / {C}_{A} ;$ $plasma protein binding rate \left({f}_{b} %\right) = 1 - dissociation percentage \left(%\right) ;$ $recovery rate \left(%\right) = \left({C}_{B}+{C}_{A}\right) / {C}_{T 0}$ wherein C_{B} was a concentration of the compound in the receptor fluid after equilibrium dialysis; C_{A} was a concentration of the compound in the drug-containing plasma after equilibrium dialysis; and C_{T0} was an initial concentration of the compound in plasma.

Experimental results were as shown in the following Table:

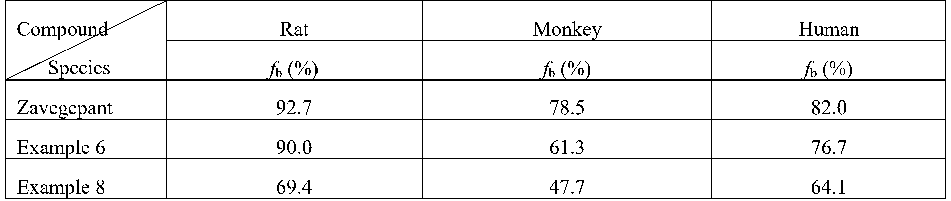

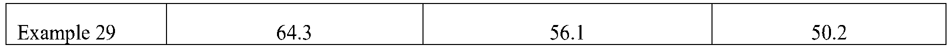

### Test Example 5: in-vivo pharmacokinetic study on SD rats intravenously and intranasally administrated with tested compound

### 1. Experimental animals

Species: SD rats, male, SPF level. Source: SD rats purchased from Chengdu Dossy Experimental Animals Co., Ltd., with the production license number of experimental animals: SCXK(Chuan)2020-030. Quantity: 2 SD rats were intravenously administered and 4 SD rats were intranasally administered.

### 2. Preparation of tested sample

2.1 An appropriate amount of drug was accurately weighed and added with 50 mM succinate buffer (diluted with D5W, pH=5-6), and mixed evenly and dissolved fully in an ultrasonic and vortexed mode to obtain a 0.2 mg/mL drug solution for intravenous (IV) administration.

2.2 An appropriate amount of drug was accurately weighed and added with 50 mM succinate buffer (diluted with pure water, pH=5-6), and mixed evenly and dissolved fully in an ultrasonic and vortexed mode to obtain a 10 mg/mL drug solution for intranasal (IN) administration.

### 3. Experiment design

| Group | Quantity | Dosage (mg/kg) | Concentration (mg/mL) | Administration volume | Administration mode | Sample collection |
|---|---|---|---|---|---|---|
| 1 | 2 | 1 | 0.2 | 5 mL/kg | IV | Plasma |
| 2 | 4 | 2 | 10 | 50 µL/animal | IN | Plasma |

Tested drugs were the compounds in Examples 6 and 8, and a control drug was Zavegepant.

### 4. Time point of blood sampling

5 minutes, 10 minutes, 15 minutes, 0.5 hour, 1 hour, 2 hours, 4 hours, 8 hours and 24 hours after administration.

### 5. Sample collection and disposition

Blood was collected through a jugular vein, about 0.2 mL of each sample was collected, EDTA-K2 was used for anticoagulation, the collected blood sample was placed on ice, and plasma was centrifugally separated within 2 hours (at 6000 g and 2-8°C for 5 minutes). The collected plasma samples were stored in a refrigerator at -80°C before analysis, and the remaining plasma samples after analysis were continuously stored in the refrigerator at -80°C for temporary storage.

### 6. Biological analysis and data processing

A concentration of a specified compound in plasma was determined by an LC-MS/MS method, and main pharmacokinetic parameters were calculated by a Winnolin 8.3 non-compartmental model. When the pharmacokinetic parameters were calculated, BLQ before Cₘₐₓ (comprising "No peak") was calculated according to 0; and BLQ (comprising "No peak") after Cₘₐₓ did not participate in the calculation.

### Data of in-vivo pharmacokinetic study on SD rats intravenously and intranasally administrated with tested compound

| Compound | | Example 6 | Example 8 | Zavegepant |
|---|---|---|---|---|
| IV@1 mg/kg | Half-life period t_{1/2} (h) | 2.52 | 7.58 | 2.58 |
| | Area under curve AUC (ng.h/mL) | 5992 | 2852 | 3622 |
| | Apparent clearance CL (mL/min/kg) | 2.77 | 5.94 | 4.59 |
| | Volume of distribution steady-state Vₛₛ (L/kg) | 0.29 | 0.50 | 0.35 |
| IN@2 mg/kg | Half-life period t_{1/2} (h) | 3.80 | 3.41 | 1.08 |
| | Concentration to peak Cₘₐₓ (ng/mL) | 540 | 204 | 645 |
| | Area under curve AUC (ng.h/mL) | 2401 | 626 | 385 |
| | Bioavailability F% | 20.1 | 11.0 | 5.31 |

### Test Example 6: in-vivo pharmacodynamic study on tested compound to capsaicin-induced change of blood flow in forearm skin of cynomolgus monkeys

### 1.Experimental principle

Capsaicin induced the release of CGRP after acting on skin, a CGRP binding receptor played a role of vasodilatation and promoted the increase of blood flow, the tested compound inhibited the binding of the CGRP with the receptor and inhibited the increase of blood flow caused by the release of CGRP, and the blood flow was detected by a blood flow meter, so as to reflect a drug effect.

### 2.Experimental steps

2.1Animal information:
   Species and strain: cynomolgus monkeys, male (5-8 kg), animal license: SYXK(Su)2019-0004, depilated 3 days before the experiment.
2.2Preparation of test sample:
   An appropriate amount of drug was accurately weighed and dissolved in sterile saline solution, and mixed evenly and dissolved fully in an ultrasonic and vortexed mode to obtain a 0.05 mg/ml drug solution for intravenous (IV) administration.
2.3In a pre-test stage, a blood flow in a forearm of an anesthetized animal was scanned by a blood flow meter, and an O-shaped rubber ring was placed on an inner side of the arm, avoiding obvious blood vessels. After a position of the rubber ring was confirmed, the blood flow in the rubber ring was scanned by the blood flow meter as a baseline blood flow value, then 2 mg of capsaicin was applied to a part inside the rubber ring, and blood flows were recorded by the blood flow meter 10 minutes, 20 minutes and 30 minutes after the capsaicin treatment.
2.4In an administration stage, a rubber ring was placed on the same part of the arm of the anesthetized monkey, baseline blood flow data were collected, then the tested compound (dosage: 0.05 mg/kg) was injected intravenously at a dosage of 1 mL/kg, 2 mg of capsaicin was applied to a part inside the rubber ring after the administration, and blood flows were recorded by the blood flow meter 10 minutes, 20 minutes and 30 minutes after the capsaicin treatment.
2.5Data processing: in the pre-test stage, the blood flow was recorded as P, the blood flow in the rubber ring was recorded as Pₜ when time =t, and the baseline blood flow was recorded as P₀; and in the administration stage, the blood flow was recorded as D, the blood flow in the rubber ring was recorded as Dₜ when time =t, and the baseline blood flow was recorded as D₀, so that a formula for calculating a blood flow inhibition rate when time =t was as follows: ${I}_{t} = \left(\left({P}_{t}-{P}_{0}\right)/{P}_{0}-\left({D}_{t}-{D}_{0}\right)/{D}_{0}\right) * 100 %$

### 3.Experimental results:

Blood flow inhibition rates at different time points were as shown in FIG. 1. All the tested compounds could inhibit the capsaicin-induced increase of blood flow. At the time point of 10 minutes, the blood flow inhibition rate of Example 6 (Mean = 355.3%) was significantly higher than that of the Zavegepant (Mean = 89.7%), and blood flow inhibition effects of all the tested compounds lasted to the time point of 30 minutes.

The present invention is not limited to the above optional embodiments, and anyone can get other products in various forms under the inspiration of the present invention. The above specific embodiments should not be construed as limiting the scope of protection of the present invention, the scope of protection of the present invention should be defined in the claims, and the specification can be used to explain the claims.

### Cited documents:

[1] Ashina M, Terwindt GM, Al-Karagholi MA, et al. Migraine: disease characterisation, biomarkers, and precision medicine. Lancet. 2021;397(10283):1496-1504. doi:10.1016/S0140-6736(20)32162-0
[2] Neurology Doctor Branch of Chinese Medical Doctor Association, Professional Committee of Headache and Sensory Disorder of Chinese Research Hospital Association. Chinese Guidelines for Diagnosis and Treatment of Migraine (2022 Edition) [J]. Chinese Journal of Pain Medicine, 2022,28(12):881-898.
[3] Ashina M, Katsarava Z, Do TP, et al. Migraine: epidemiology and systems of care. Lancet. 2021;397(10283):1485-1495. doi:10.1016/S0140-6736(20)32160-7
[4] Russo AF. Calcitonin gene-related peptide (CGRP): a new target for migraine. Annu Rev Pharmacol Toxicol. 2015;55:533-552. doi:10.1146/annurev-pharmtox-010814-124701
[5] Iyengar S, Ossipov MH, Johnson KW. The role of calcitonin gene-related peptide in peripheral and central pain mechanisms including migraine. Pain. 2017;158(4):543-559. doi:10.1097/j.pain.0000000000000831
[6] Hargreaves R, Olesen J. Calcitonin Gene-Related Peptide Modulators - The History and Renaissance of a New Migraine Drug Class. Headache. 2019;59(6):951-970. doi:10.1111/head.13510
[7] Russell FA, King R, Smillie SJ, Kodji X, Brain SD. Calcitonin gene-related peptide: physiology and pathophysiology. Physiol Rev. 2014;94(4):1099-1142. doi: 10. 1 1 52/physrev.00034.20 13
[8] Clemow DB, Johnson KW, Hochstetler HM, Ossipov MH, Hake AM, Blumenfeld AM. Lasmiditan mechanism of action - review of a selective 5-HT1F agonist. J Headache Pain. 2020;21(1):71. Published 2020 Jun 10. doi:10.1186/s10194-020-01132-3.

## Claims

1. A compound as represented by the following formula I, or an isomer, pharmaceutically acceptable salt or solvate thereof: 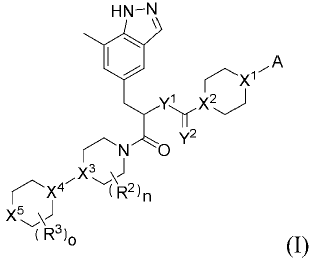 wherein,
X¹ and X² are respectively independently CH or N, and X² is preferably N;
X³ and X⁴ are respectively independently C, CH or N;
X⁵ is C, CH, CH₂, NH or N;
Y¹ is CH₂, O or NH, and Y¹ is preferably O or NH;
Y² is O or S, and Y² is preferably O;
R² is independently -H, -(C₁-C₆)alkyl or -(C₃-C₈)cycloalkyl when occurring each time, and n is 0, 1, 2, 3, 4, 5 or 6,
or, two R² form a 3-6 membered aliphatic ring or aliphatic heterocyclic ring together with atoms to which R² attach, the aliphatic ring or aliphatic heterocyclic ring optionally forms a spiro structure, a fused ring structure or a bridged ring structure with the ring to which R² attach, and heteroatoms in the aliphatic heterocyclic ring are O, N or S,
or, -(R²)ₙ forms a 3-6 membered aliphatic ring or aliphatic heterocyclic ring together with atoms to which -(R²)ₙ attach, the aliphatic ring or aliphatic heterocyclic ring optionally forms a spiro structure, a fused ring structure or a bridged ring structure with the ring to which -(R²)ₙ attach, and heteroatoms in the aliphatic heterocyclic ring are O, N or S;
R³ is independently -H, -(C₁-C₆)alkyl or -(C₃-C₈)cycloalkyl when occurring each time, and o is 0, 1, 2, 3, 4, 5 or 6,
or, two R³ form a 3-6 membered aliphatic ring or aliphatic heterocyclic ring together with atoms to which R³ attach, the aliphatic ring or aliphatic heterocyclic ring optionally forms a spiro structure, a fused ring structure or a bridged ring structure with the ring to which R³ attach, and heteroatoms in the aliphatic heterocyclic ring are O, N or S,
or, -(R³)ₒ forms a 3-6 membered aliphatic ring or aliphatic heterocyclic ring together with atoms to which -(R³)ₒ attach, the aliphatic ring or aliphatic heterocyclic ring optionally forms a spiro structure, a fused ring structure or a bridged ring structure with the ring to which -(R³)ₒ attach, and heteroatoms in the aliphatic heterocyclic ring are O, N or S;
A has a structure as represented by the following formula II: 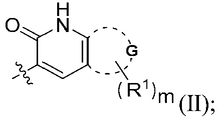
a G-chain group forms a 5-7 membered aliphatic ring or aliphatic heterocyclic ring together with two carbon atoms to which the G-chain group attaches, and ring-forming heteroatoms in the aliphatic heterocyclic ring are -O-, -S- or 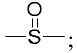
R¹ is independently -H, -(C₁-C₆)alkyl or -(C₃-C₈)cycloalkyl when occurring each time, and m is 0, 1, 2, 3, 4, 5 or 6,
or, two R¹ form a 3-8 membered aliphatic ring or aliphatic heterocyclic ring together with atoms to which R¹ attach, the aliphatic ring or aliphatic heterocyclic ring optionally forms a spiro structure, a fused ring structure or a bridged ring structure with the ring to which R¹ attach, and heteroatoms in the aliphatic heterocyclic ring are O, N or S,
or, -(R¹)ₘ forms a 3-6 membered aliphatic ring or aliphatic heterocyclic ring together with atoms to which -(R¹)ₘ attach, the aliphatic ring or aliphatic heterocyclic ring optionally forms a spiro structure, a fused ring structure or a bridged ring structure with the ring to which -(R¹)ₘ attach, and heteroatoms in the aliphatic heterocyclic ring are O, N or S.

2. The compound, or the isomer, pharmaceutically acceptable salt or solvate thereof according to claim 1:
wherein, the compound is as represented by formula I-2 or I-3:

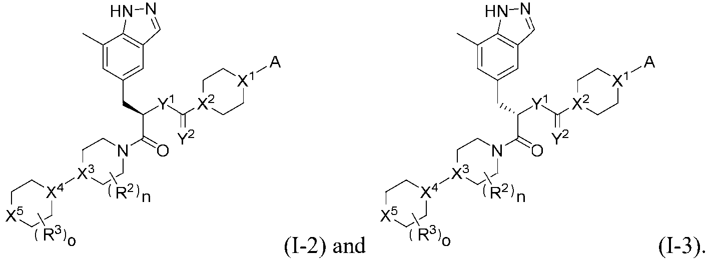

3. The compound, or the isomer, pharmaceutically acceptable salt or solvate thereof according to claim 1 or 2:
wherein, in A:
in a case that the G-chain group forms a 5 or 6 membered aliphatic ring together with two carbon atoms to which the G-chain group attaches, the G-chain group optionally has 0 or 1 double bond, and the remaining bonds are all single bonds;
in a case that the G-chain group forms a 7 membered aliphatic ring together with two carbon atoms to which the G-chain group attaches, the G-chain group optionally has 0, 1 or 2 double bonds, and the remaining bonds are all single bonds;
in a case that the G-chain group forms a 5 membered aliphatic heterocyclic ring together with two carbon atoms to which the G-chain group attaches, bonds on the G-chain group are all single bonds; and
in a case that the G-chain group forms a 6 or 7 membered aliphatic heterocyclic ring together with two carbon atoms to which the G-chain group attaches, the G-chain group optionally has 0 or 1 double bond, and the remaining bonds are all single bonds.

4. The compound, or the isomer, pharmaceutically acceptable salt or solvate thereof according to claim 1 or 2:
wherein, in A, the G-chain group is selected from:

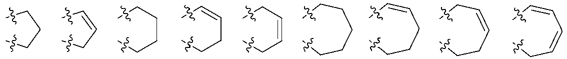

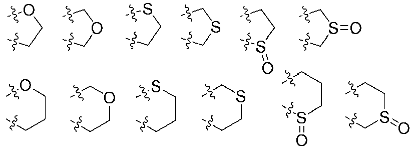

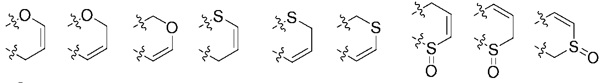

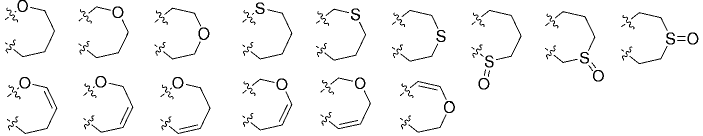

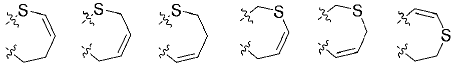

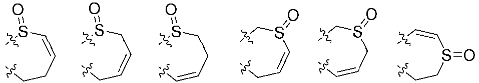

5. The compound, or the isomer, pharmaceutically acceptable salt or solvate thereof according to claim 1 or 2:
wherein, in A,
R¹ is independently -H, -(C₁-C₃)alkyl or -(C₃-C₆)cycloalkyl when occurring each time, and m is 0, 1, 2 or 3,
or, two R¹ form a 3-6 membered aliphatic ring or aliphatic heterocyclic ring together with atoms to which R¹ attach, the aliphatic ring or aliphatic heterocyclic ring optionally forms a spiro structure, a fused ring structure or a bridged ring structure with the ring to which R¹ attaches, and heteroatoms in the aliphatic heterocyclic ring are O, N or S,
or, -(R¹)ₘ forms a 3-6 membered aliphatic ring or aliphatic heterocyclic ring together with atoms to which -(R¹)ₘ attach, the aliphatic ring or aliphatic heterocyclic ring optionally forms a spiro structure, a fused ring structure or a bridged ring structure with the ring to which -(R¹)ₘ attach, and heteroatoms in the aliphatic heterocyclic ring are O, N or S.

6. The compound, or the isomer, pharmaceutically acceptable salt or solvate thereof according to claim 5:
wherein, in A,
R¹ is independently -H, methyl, ethyl or cyclopropyl when occurring each time, and m is 0, 1 or 2,
or, two R¹ form cyclopropane, cyclobutane or cyclopentane together with atoms linked to the R¹, and the cyclopropane, the cyclobutane or the cyclopentane forms a spiro structure, a fused ring structure or a bridged ring structure with the ring to which R¹ attach,
or, -(R¹)ₘ forms cyclopropane, cyclobutane or cyclopentane together with atoms to which -(R¹)ₘ attach, and the cyclopropane, the cyclobutane or the cyclopentane forms a spiro structure, a fused ring structure or a bridged ring structure with the ring to which -(R¹)ₘ attach.

7. The compound, or the isomer, pharmaceutically acceptable salt or solvate thereof according to claim 1, 2, 5 or 6:
wherein, A has a structure as represented by the following formula II-1, II-2 or II-3: 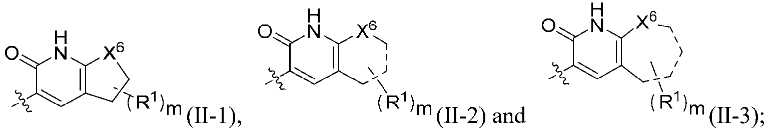
in the formulas II-1, II-2 and II-3, X⁶ is -O-, -S- or 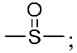
in the formula II-2, two "- - -" both refer to single bonds, or any one of the two "- - -" is a double bond and the other one is a single bond; and
in the formula II-3, three "- - -" all refer to single bonds, or any one of the three "- - -" is a double bond and the other two are both single bonds.

8. The compound, or the isomer, pharmaceutically acceptable salt or solvate thereof according to claim 7:
wherein, Y² is S, and X⁶ is -S-.

9. The compound, or the isomer, pharmaceutically acceptable salt or solvate thereof according to claim 1, 2, 5 or 6:
wherein, A has a structure as represented by the following formula II-4 or II-5: 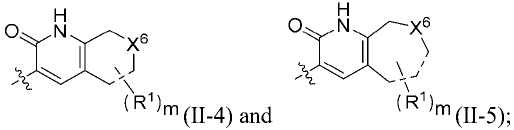
in the formula II-4 or II-5, X⁶ is -O-, -S- or 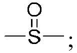
in the formula II-4, "- - -" refers to a single bond or a double bond; and
in the formula II-5, two "- - -" both refer to single bonds, or any one of the two "- - -" is a double bond and the other one is a single bond.

10. The compound, or the isomer, pharmaceutically acceptable salt or solvate thereof according to claim 1 or 2:
wherein, the G-chain group in A forms a 5-7 membered aliphatic ring together with two carbon atoms to which the G-chain group attaches, and m is 0.

11. The compound, or the isomer, pharmaceutically acceptable salt or solvate thereof according to claim 1 or 2:
wherein, A is selected from: 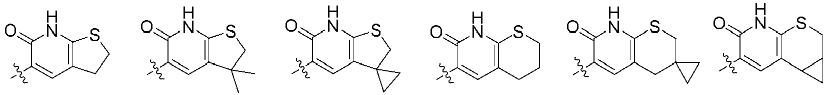 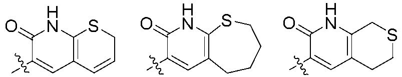 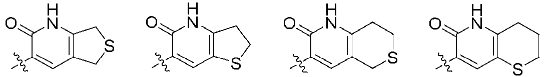 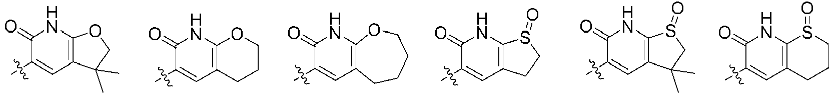 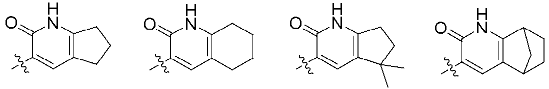 , and
A is further preferably selected from:

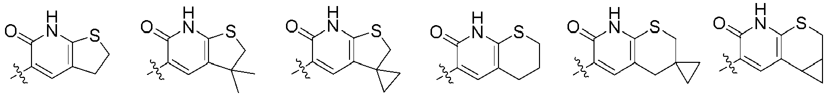

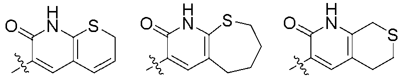

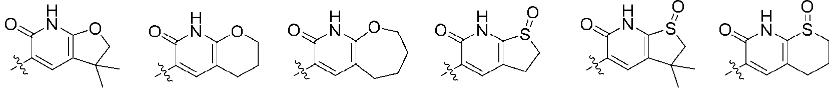

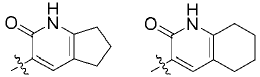

12. The compound, or the isomer, pharmaceutically acceptable salt or solvate thereof according to claim 1 or 2:
wherein, A is selected from: 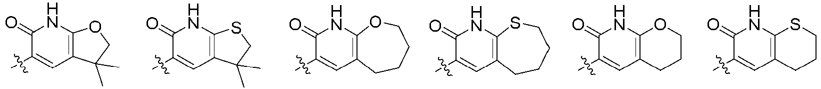 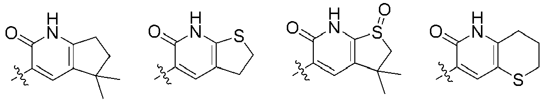 , and
A is further preferably selected from:

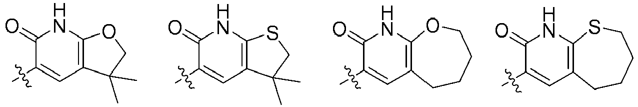

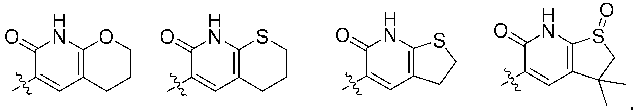

13. The compound, or the isomer, pharmaceutically acceptable salt or solvate thereof according to claim 1 or 2:
wherein, R² is independently -H, -(C₁-C₃)alkyl or -(C₃-C₆)cycloalkyl when occurring each time, and n is 0, 1, 2 or 3,
or, two R² form a 3-6 membered aliphatic ring or aliphatic heterocyclic ring together with atoms to which R² attach, the aliphatic ring or aliphatic heterocyclic ring optionally forms a spiro structure, a fused ring structure or a bridged ring structure with the ring to which R² attach, and ring-carried atoms provided by the two R² in the aliphatic ring or aliphatic heterocyclic ring are both C atoms,
or, -(R²)ₙ forms a 3-6 membered aliphatic ring or aliphatic heterocyclic ring together with atoms to which -(R²)ₙ attach, the aliphatic ring or aliphatic heterocyclic ring optionally forms a spiro structure, a fused ring structure or a bridged ring structure with the ring to which -(R²)ₙ attach, and ring-carried atoms provided by -(R²)ₙ in the aliphatic ring or aliphatic heterocyclic ring are all C atoms;
R³ is independently -H, -(C₁-C₃)alkyl or -(C₃-C₆)cycloalkyl when occurring each time, and o is 0, 1, 2 or 3,
or, two R³ form a 3-6 membered aliphatic ring or aliphatic heterocyclic ring together with atoms to which R³ attach, the aliphatic ring or aliphatic heterocyclic ring optionally forms a spiro structure, a fused ring structure or a bridged ring structure with the ring to which R³ attach, and ring-carried atoms provided by the two R³ in the aliphatic ring or aliphatic heterocyclic ring are both C atoms,
or, -(R³)ₒ forms a 3-6 membered aliphatic ring or aliphatic heterocyclic ring together with atoms to which -(R³)ₒ attach, the aliphatic ring or aliphatic heterocyclic ring optionally forms a spiro structure, a fused ring structure or a bridged ring structure with the ring to wihch -(R³)ₒ attach, and ring-carried atoms provided by the -(R³)ₒ in the aliphatic ring or aliphatic heterocyclic ring are all C atoms.

14. The compound, or the isomer, pharmaceutically acceptable salt or solvate thereof according to claim 13:
wherein, R² is independently -H, methyl, ethyl, cyclopropyl or cyclobutyl when occurring each time, and n is 0, 1 or 2,
or, two R² form a 3-5 membered aliphatic ring or aliphatic heterocyclic ring together with atoms to which R² attach, the aliphatic ring or aliphatic heterocyclic ring optionally forms a spiro structure with the ring to which R² attach, and ring-carried atoms provided by the two R² in the aliphatic ring or aliphatic heterocyclic ring are both C atoms,
or, -(R²)ₙ forms a 3-5 membered aliphatic ring or aliphatic heterocyclic ring together with atoms to which -(R²)ₙ attach, the aliphatic ring or aliphatic heterocyclic ring optionally forms a spiro structure with the ring to which -(R²)ₙ attach, and ring-carried atoms provided by -(R²)ₙ in the aliphatic ring or aliphatic heterocyclic ring are all C atoms;
R³ is independently -H, -(C₁-C₃)alkyl or -(C₃-C₆)cycloalkyl when occurring each time, and o is 0, 1, 2 or 3,
or, two R³ form a 3-5 membered aliphatic ring or aliphatic heterocyclic ring together with atoms to which R³ attach, the aliphatic ring or aliphatic heterocyclic ring optionally forms a spiro structure with the ring to which R³ attach, and ring-carried atoms provided by the two R³ in the aliphatic ring or aliphatic heterocyclic ring are both C atoms,
or, -(R³)ₒ forms a 3-5 membered aliphatic ring or aliphatic heterocyclic ring together with atoms to which -(R³)ₒ attach, the aliphatic ring or aliphatic heterocyclic ring optionally forms a spiro structure with the ring to which -(R³)ₒ attach, and ring-carried atoms provided by the -(R³)ₒ in the aliphatic ring or aliphatic heterocyclic ring are all C atoms.

15. The compound, or the isomer, pharmaceutically acceptable salt or solvate thereof according to claim 1 or 2:
wherein, X³ and X⁴ are not N at the same time.

16. The compound, or the isomer, pharmaceutically acceptable salt or solvate thereof according to claim 1:
the compound is as represented by the following formula I-1: 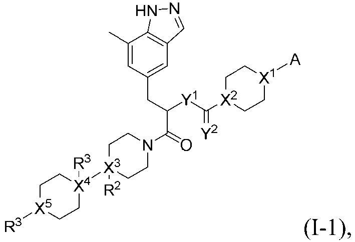
wherein, X¹, X², X³, X⁴, X⁵, Y¹ and Y² are as defined in claim 1, and A is as defined in claim 6 or 11; and
R² and R³ are independently -H, -CH₃ or absent when occurring each time.

17. The compound, or the isomer, pharmaceutically acceptable salt or solvate thereof according to claim 1, 2 or 16:
wherein, more than one H in the compound are substituted by D.

18. The compound, or the isomer, pharmaceutically acceptable salt or solvate thereof according to claim 17:
wherein, more than one H in R³ are substituted by D.

19. The compound, or the isomer, pharmaceutically acceptable salt or solvate thereof according to claim 17:
wherein, R³ attached to X⁵ is -CD₃.

20. The compound, or the isomer, pharmaceutically acceptable salt or solvate thereof according to claim 1, 2 or 16:
wherein, H on a ring of a pyrazole group in indazolyl in the compound are substituted by D.

21. The compound, or the isomer, pharmaceutically acceptable salt or solvate thereof according to claim 3:
wherein, more than one H in the G-chain group or R¹ are substituted by D.

22. The compound, or the isomer, pharmaceutically acceptable salt or solvate thereof according to claim 1:
the compound is selected from:

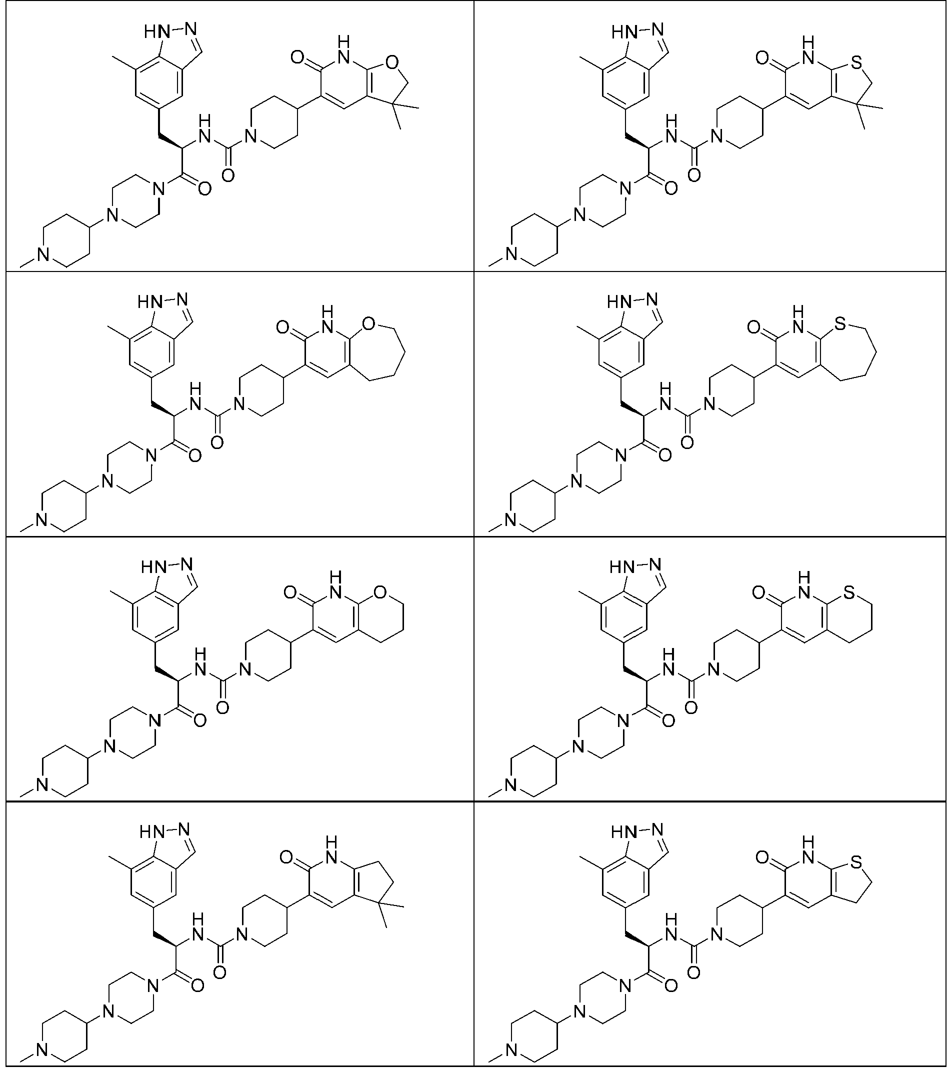

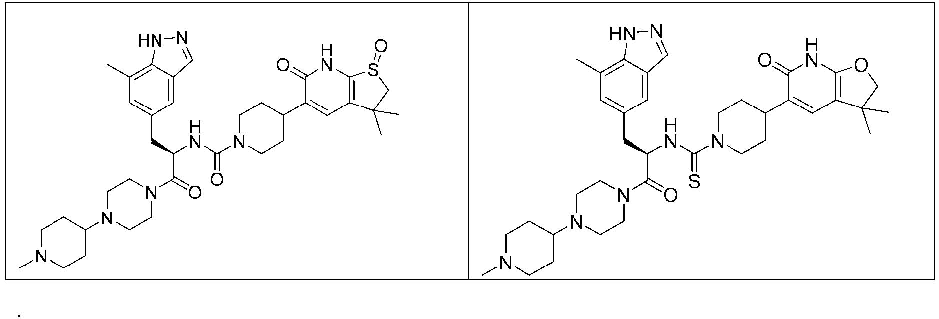

23. The compound, or the isomer, pharmaceutically acceptable salt or solvate thereof according to claim 1:
the compound is selected from:

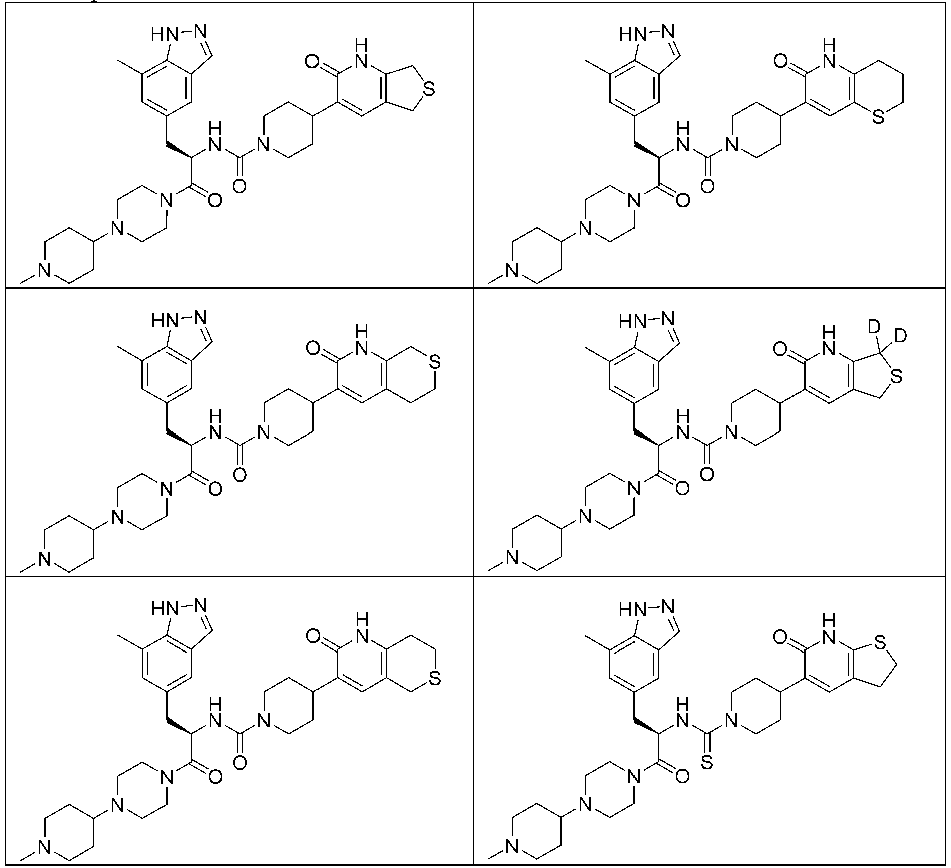

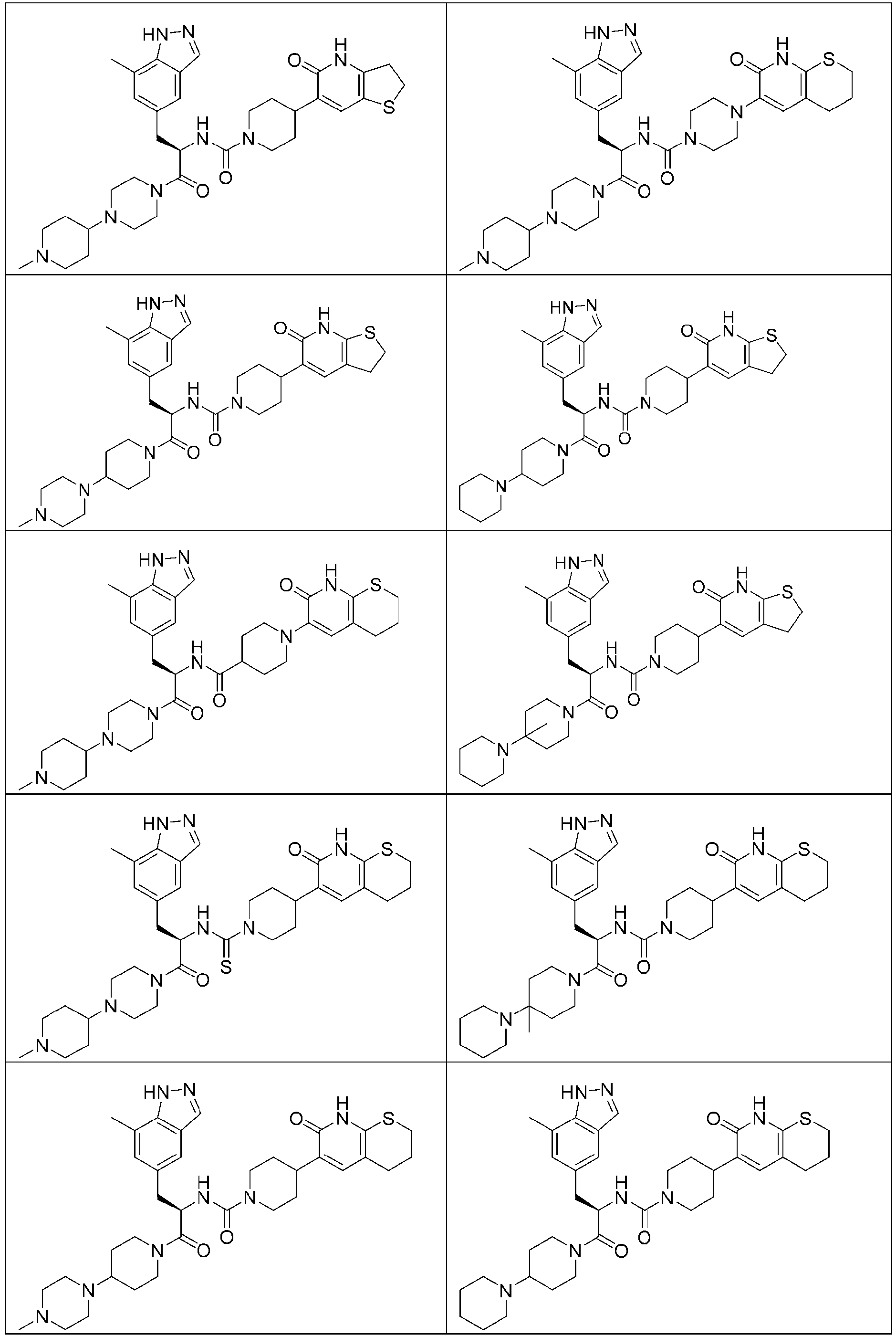

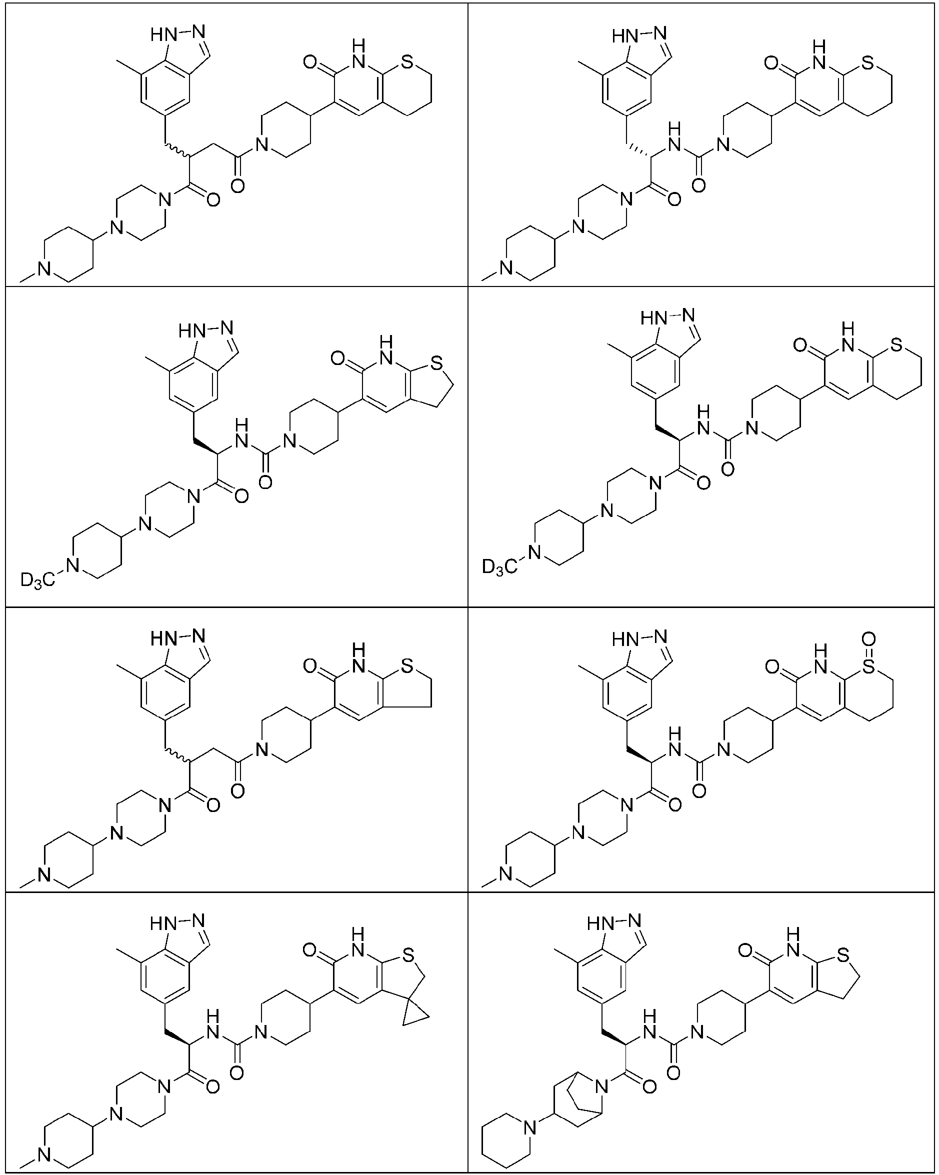

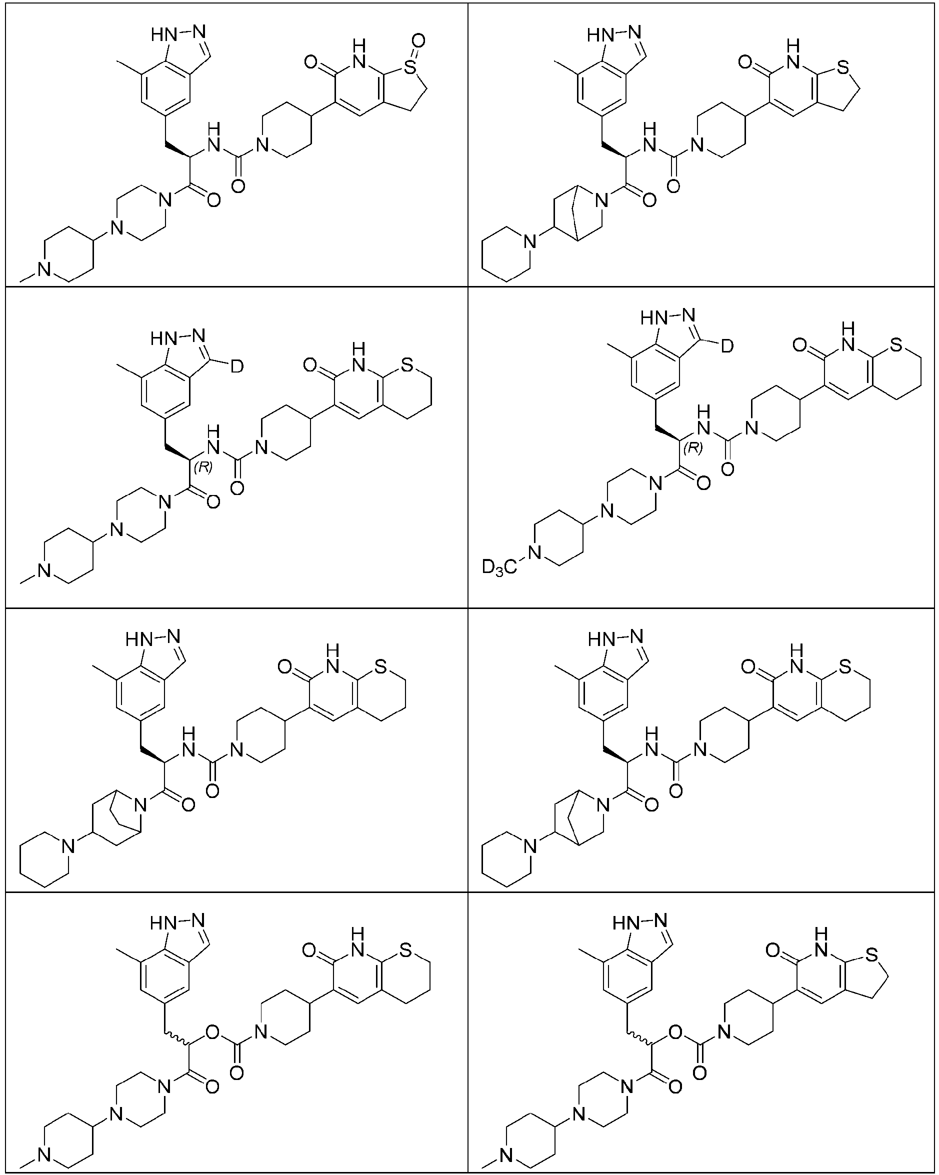

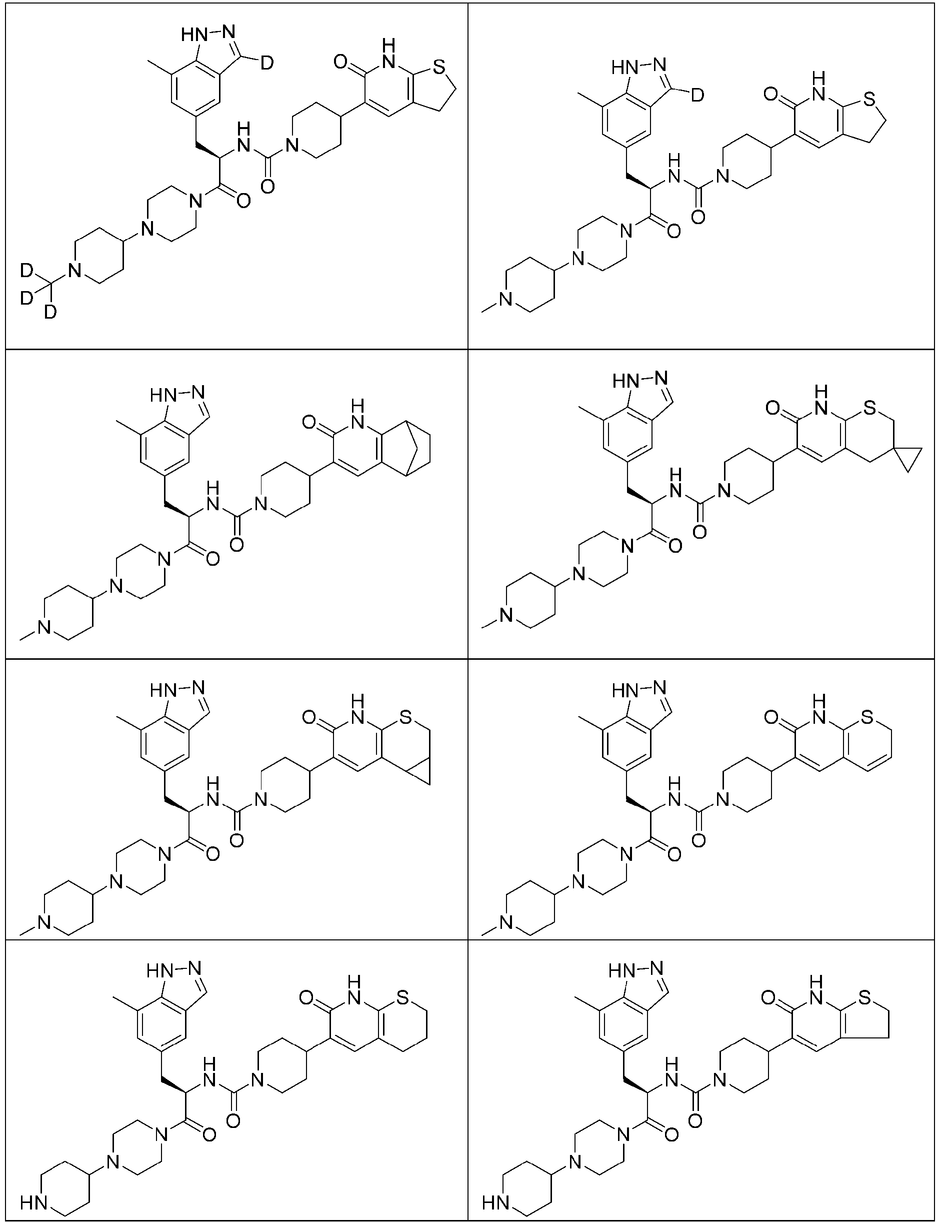

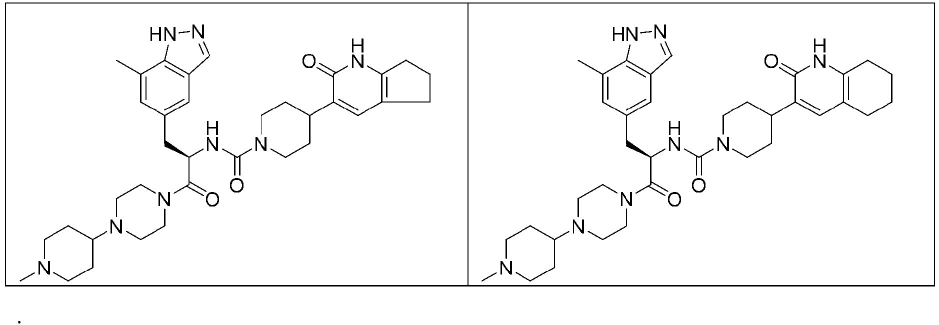

24. A use of the compound, or the isomer, pharmaceutically acceptable salt or solvate thereof according to any one of claims 1 to 23 as a CGRP antagonist.

25. The use according to claim 24, wherein the compound, or the isomer, pharmaceutically acceptable salt or solvate thereof is used for preparing a drug for preventing, treating or relieving CGRP-mediated diseases.

26. The use according to claim 25, wherein the diseases comprise migraine or neuropathic pain.

27. A method for preventing, treating or relieving CGRP-mediated diseases, such as migraine or neuropathic pain, comprising: administering the compound, or the isomer, pharmaceutically acceptable salt or solvate thereof according to any one of claims 1 to 23.

28. An intermediate compound as represented by the following formula III, IV or V, or an isomer, pharmaceutically acceptable salt or solvate thereof, wherein: 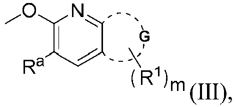 in the formula III, R^{a} is Cl, Br, I, -OTf or -B(OH)₂, the G-chain group is as defined in any part of the compound in claim 1, 3, 4 or 10, and R₁ and m are as defined in any part of the compound in claim 1, 5 or 6, under conditions that:
when R^{a} is Br, the ring formed by the G-chain group together with the two carbon atoms attached to the G-chain group is not cyclopentane, cyclohexane and 1,4-dioxane,
when R^{a} is Cl, the ring formed by the G-chain group together with the two carbon atoms attached to the G-chain group is not cyclohexane,
when the ring formed by the G-chain group together with the two carbon atoms attached to the G-chain group is tetrahydrofuran, and m is 1, R¹ is not isopropyl; 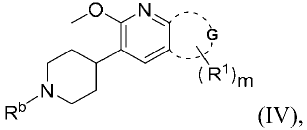
in the formula IV, R^{b} is -H or an amino protecting group, and the amino protecting group is optionally -Cbz, -Boc, -Fmoc, -PMB, -Bn, -Trt, -Tos or -Alloc, and the amino protecting group is preferably -Boc,
the G-chain group is as defined in any part of the compound in claim 1, 3, 4 or 10, and R¹ and m are as defined in any part of the compound in claim 1, 5 or 6; 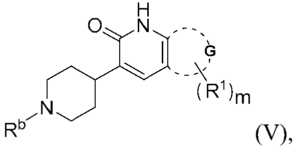
in the formula V, R^{b} is -H or an amino protecting group, and the amino protecting group is optionally -Cbz, -Boc, -Fmoc, -PMB, -Bn, -Trt, -Tos or -Alloc, and the amino protecting group is preferably -Boc,
when R^{b} is -Boc, the formula V is optionally not 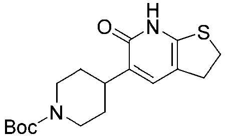 or 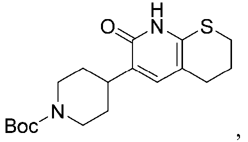 and
the G-chain group is as defined in any part of the compound in Claim 1, 2, 3 or 9, and R¹ and m are as defined in any part of the compound in claim 1, 4 or 5.

29. The intermediate compound, or the isomer, pharmaceutically acceptable salt or solvate thereof according to claim 28, wherein:
the intermediate compound as represented by the formula III is selected from formula III-1, III-2, III-3, III-4 or III-5, 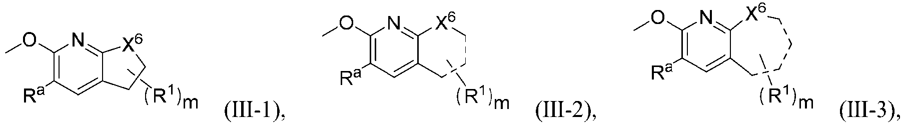 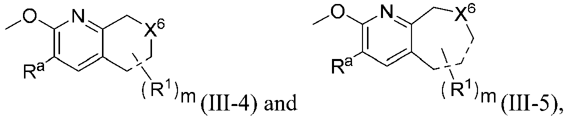
in the formulas III-1, III-2, III-3, III-4 and III-5, X⁶ is -O-, -S- or 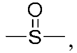
in the formula III-2, two "- - -" both refer to single bonds, or any one of the two "- - -" is a double bond and the other one is a single bond,
in the formula III-3, three "---" all refer to single bonds, or any one of the three " --- " is a double bond and the other two are both single bonds,
in the formula III-4, "- - -" refers to a single bond or a double bond, and
in the formula III-5, two "- - -" both refer to single bonds, or any one of the two "- - -" is a double bond and the other one is a single bond;
the intermediate compound as represented by the formula IV is selected from formula IV-1, IV-2, IV-3, IV-4 or IV-5, 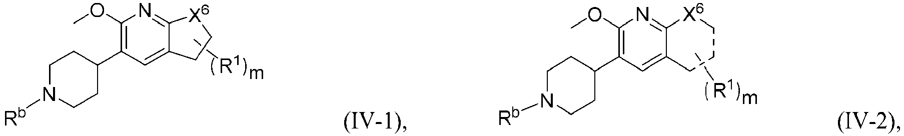 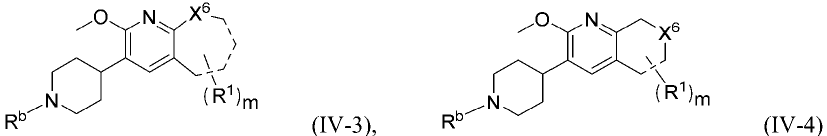 and 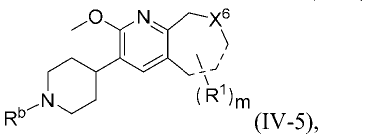
in the formulas IV-1, IV-2, IV-3, IV-4 and IV-5, X⁶ is -O-, -S- or 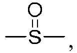
in the formula IV-2, two "- - -" both refer to single bonds, or any one of the two "- - -" is a double bond and the other one is a single bond,
in the formula IV-3, three "- - -" all refer to single bonds, or any one of the three "- - -" is a double bond and the other two are both single bonds,
in the formula IV-4, "- - -" refers to a single bond or a double bond, and
in the formula IV-5, two "- - -" both refer to single bonds, or any one of the two "- - -" is a double bond and the other one is a single bond;
the intermediate compound as represented by the formula V is selected from formula V-1, V-2, V-3, V-4 or V-5, 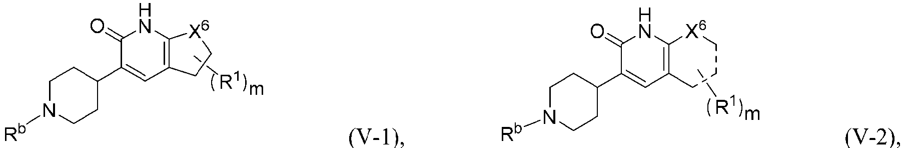 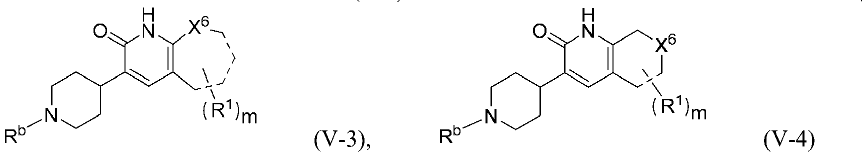 and 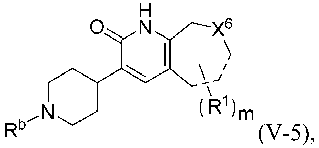
in the formulas V-1, V-2, V-3, V-4 and V-5, X⁶ is -O-, -S- or 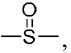
in the formula V-2, two "- - -" both refer to single bonds, or any one of the two "- - -" is a double bond and the other one is a single bond,
in the formula V-3, three "- - -" all refer to single bonds, or any one of the three "- - -" is a double bond and the other two are both single bonds,
in the formula V-4, "- - -" refers to a single bond or a double bond, and
in the formula V-5, two "- - -" both refer to single bonds, or any one of the two "- - -" is a double bond and the other one is a single bond.

30. The intermediate compound, or the isomer, pharmaceutically acceptable salt or solvate thereof according to claim 28 or 29:
wherein, the salt is hydrochloride or trifluoroacetate.

31. The intermediate compound, or the isomer, pharmaceutically acceptable salt or solvate thereof according to claim 28 or 29:
wherein, more than one H in the intermediate compound are substituted by D, and optionally, more than one H in the G-chain group or R¹ are substituted by D.

32. The intermediate compound, or the isomer, pharmaceutically acceptable salt or solvate thereof according to any one of claims 28 to 31:
wherein, the intermediate compound is selected from:

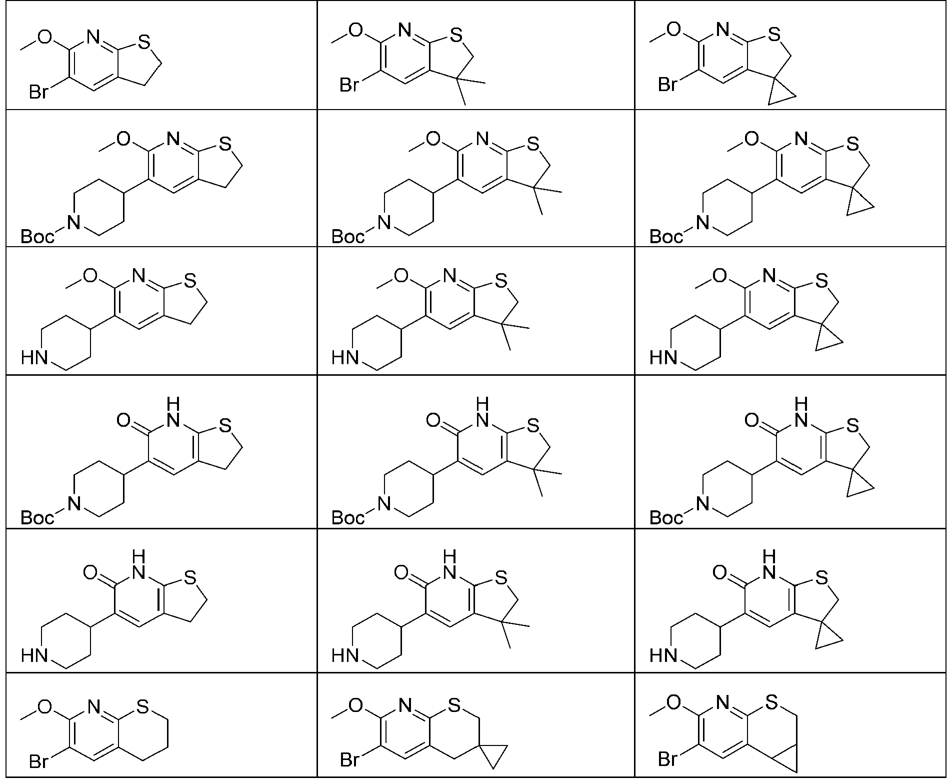

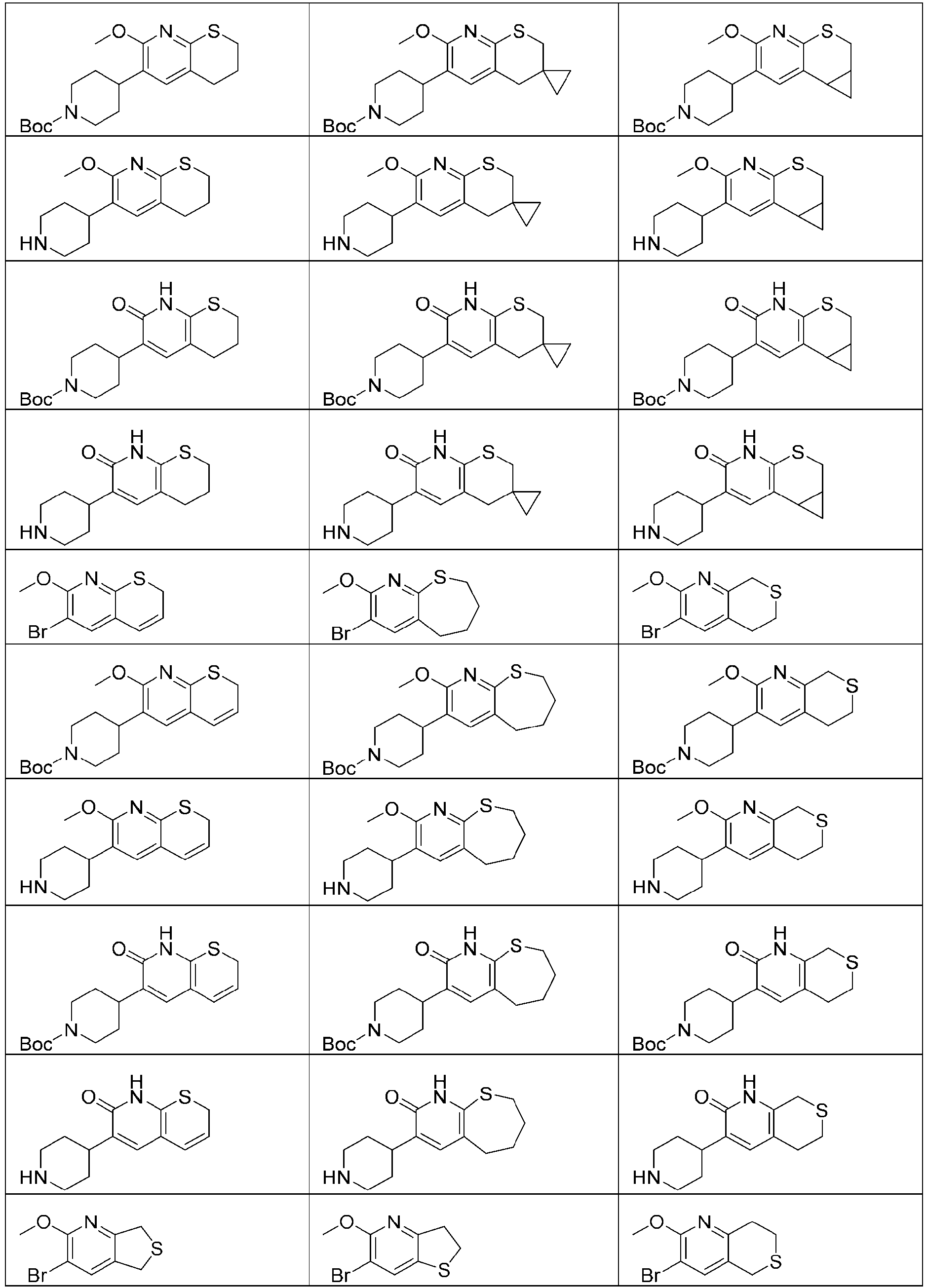

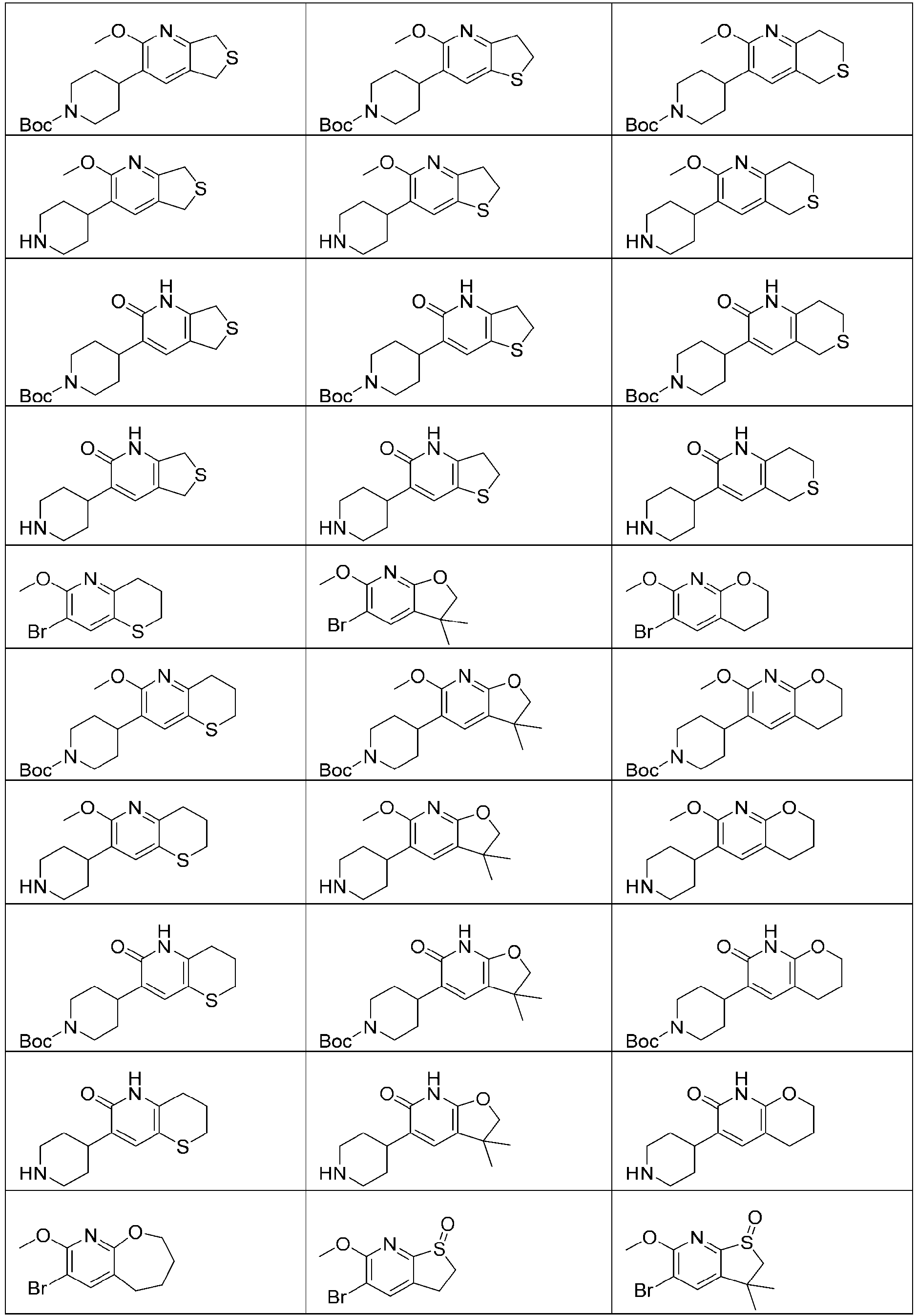

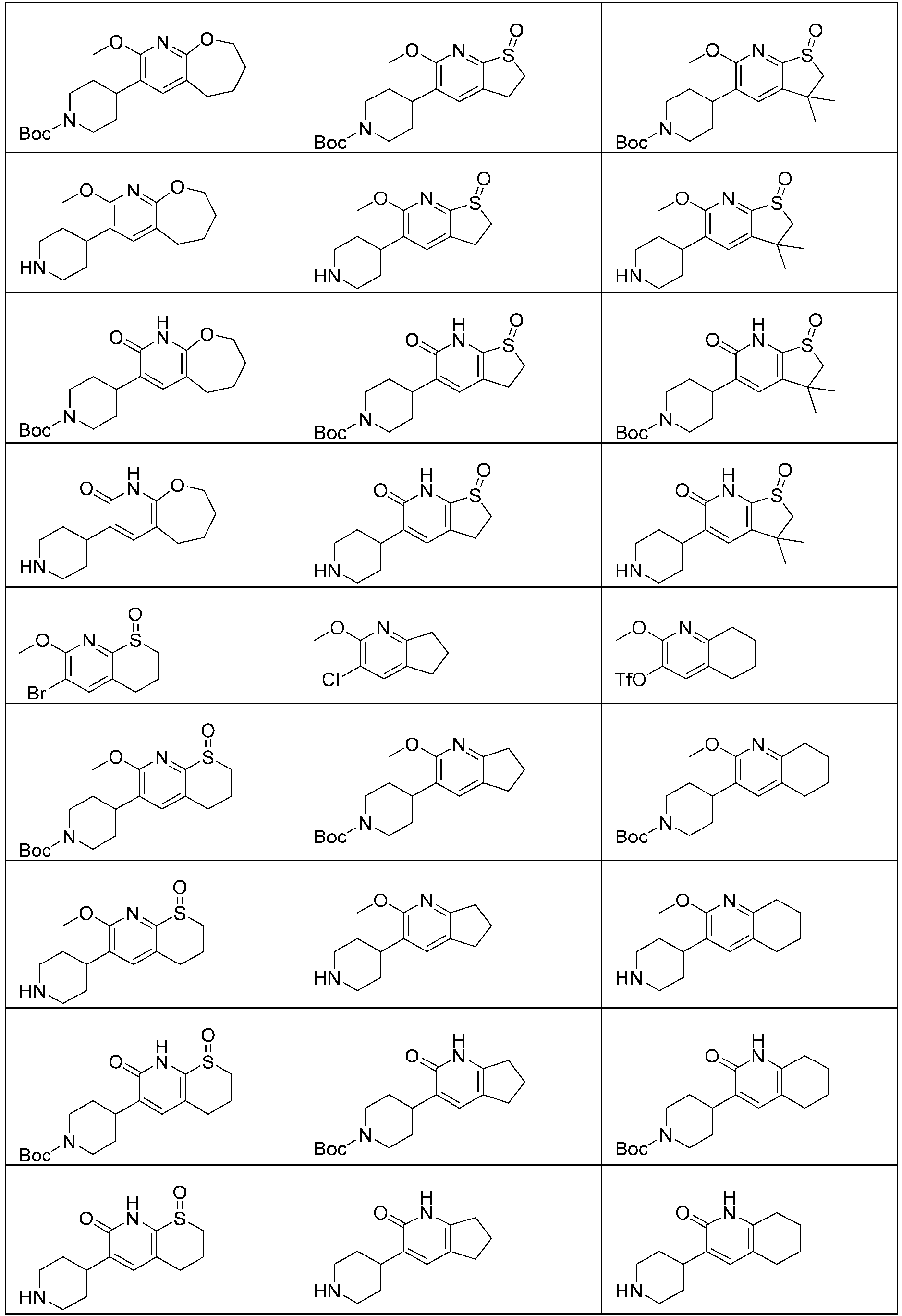

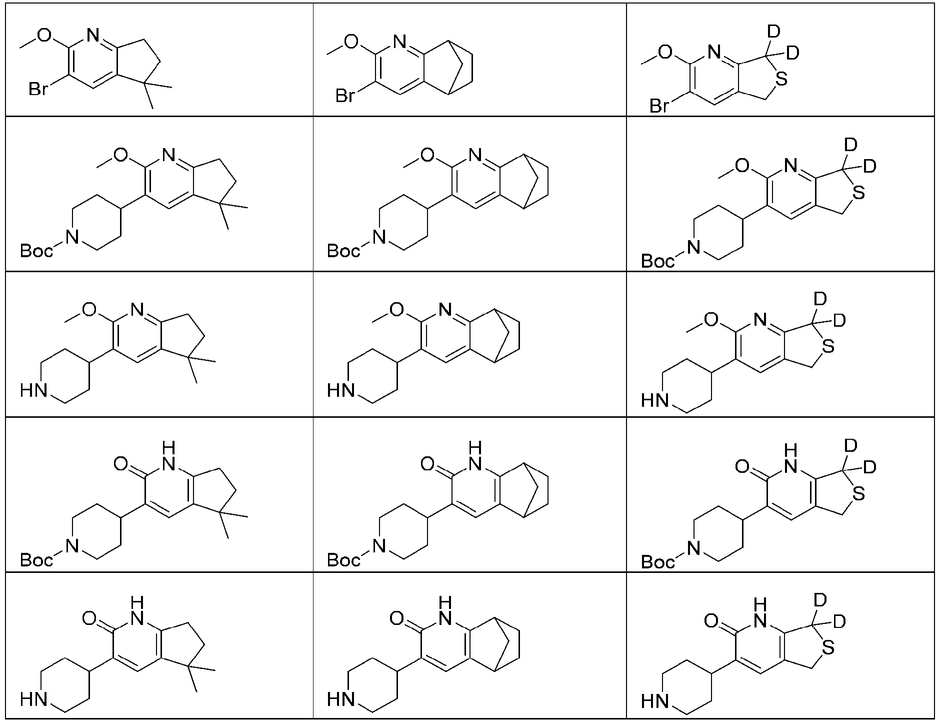

33. An intermediate compound as represented by the following formula VI, or an isomer, pharmaceutically acceptable salt or solvate thereof:

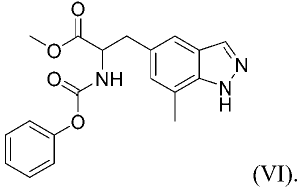

34. The intermediate compound, or the isomer, pharmaceutically acceptable salt or solvate thereof according to claim 33, wherein:
the intermediate compound as represented by the formula VI is selected from any one of the following structures: 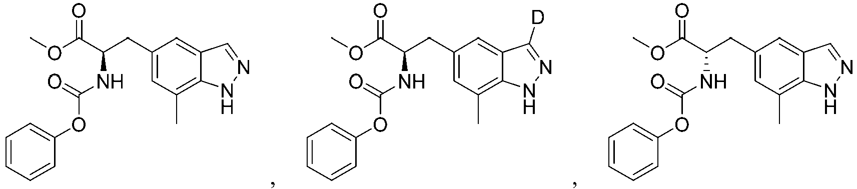 and

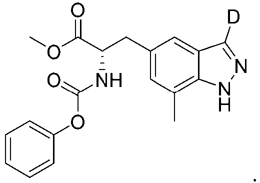

35. A preparation method for an intermediate compound as represented by formula I, comprising any one or a combination of the following synthetic routes (1)-(3): 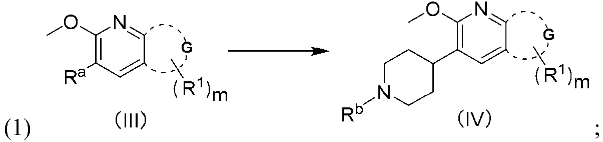 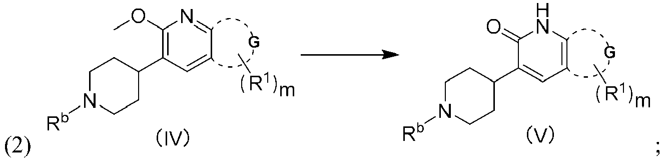 and 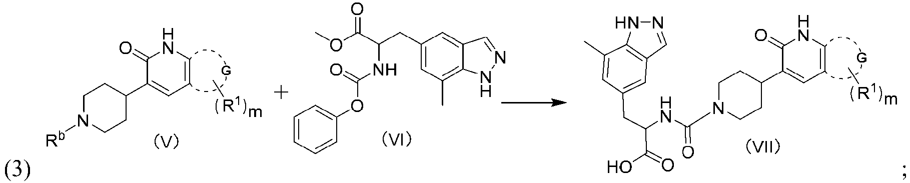 wherein, formulas III, IV and V are respectively as defined in the formulas III, IV and V of the intermediate compound in claim 28 sequentially, formula VI is as defined in the formula VI of the intermediate compound in claim 33, and the G-chain group, R¹ and m in formula VII are as defined in the G-chain group, R¹ and m in any structure of the formula III, IV or V in the intermediate compound in claim 28.

36. A synthetic method for a compound as a CGRP antagonist, comprising the following synthetic route: 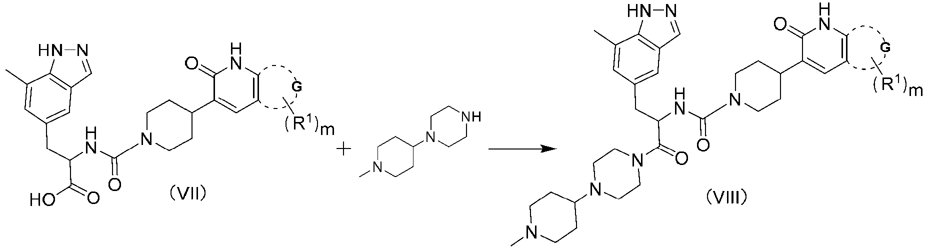 wherein, the G-chain group, R¹ and m in formulas VII and VIII are as defined in claim 1.

37. A synthetic method for a compound as a CGRP antagonist, comprising the following synthetic route: 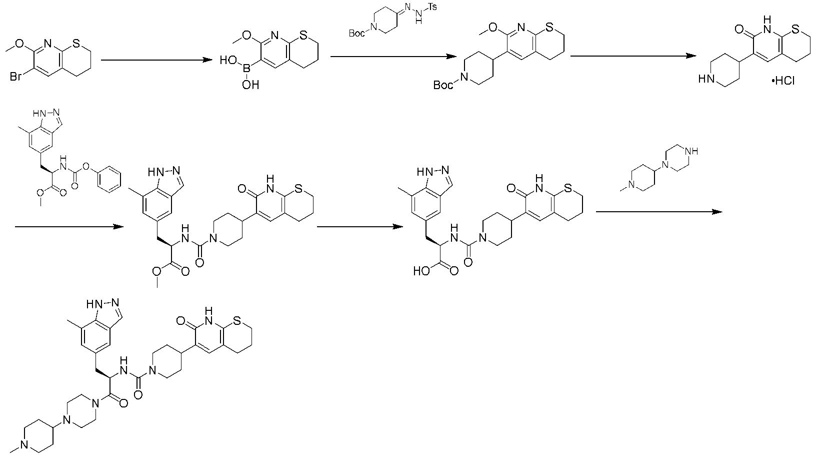 or,

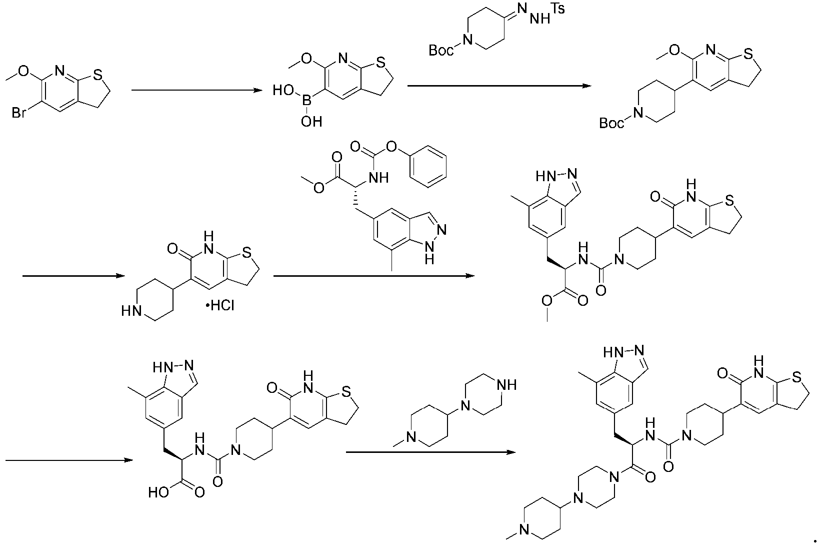

38. A pharmaceutical composition, comprising the compound, or the isomer, pharmaceutically acceptable salt or solvate thereof according to any one of claims 1 to 23, and a pharmaceutically acceptable excipient.
